# EUROPEAN PATENT APPLICATION

(11) **EP 3 260 450 A1**
(43) Date of publication of application: **27.12.2017**
(21) Application number: 16752421.4
(22) Date of filing: 15.02.2016
(51) Int. Cl.: C07D 319/06, C09K 19/12, C09K 19/14, C09K 19/18, C09K 19/20, C09K 19/30, C09K 19/32, C09K 19/34, C09K 19/38, C09K 19/42, C09K 19/54, G02F 1/13

(54) **COMPOUND HAVING SATURATED SIX-MEMBERED RING AND ALKOXY GROUP OR ALKOXYALKYL GROUP, LIQUID CRYSTAL COMPOSITION AND LIQUID CRYSTAL DISPLAY ELEMENT**

(30) Priority: 17.02.2015 JP 2015028973
(71) Applicant: JNC Corporation, Chiyoda-ku Tokyo 100-8105 (JP); JNC Petrochemical Corporation, Tokyo 100-0004 (JP)
(72) Inventor: OOKAWA, Hiroki, Ichihara-shi Chiba 290-8551 (JP)
(74) Representative: Müller-Boré & Partner Patentanwälte PartG mbB
(86) International application number: PCT/JP2016/054230
(87) International publication number: WO 2016/133035

(57) **Abstract**

Provided is a liquid crystal compound satisfying at least one of physical properties such as high stability to light or the like, a high clearing point, low minimum temperature of a liquid crystal phase, small viscosity, suitable optical anisotropy and large dielectric anisotropy, a liquid crystal composition containing the compound, and a liquid crystal display device including the composition.

A compound is represented by formula (1).

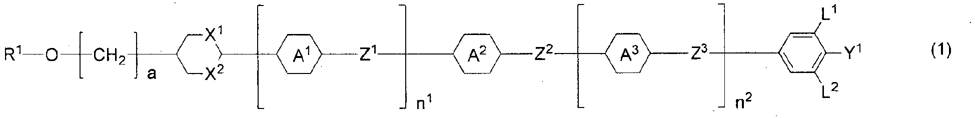

In formula (1), for example,
R¹ is alkyl having 1 to 12 carbons;
ring A¹, ring A² and ring A³ are independently 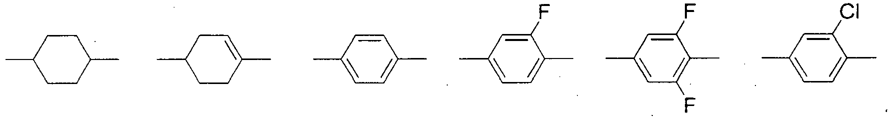 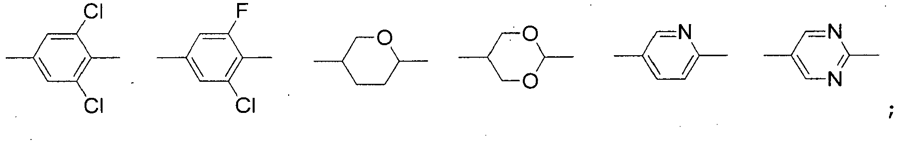 in which, X¹ and X² are independently -O- or -CH₂-;
Y¹ is fluorine, -CF3 or -OCF₃;
Z¹ and Z³ are independently a single bond, -CF₂O- or -COO-;
Z² is a single bond, -CF₂O- or -COO-;
L¹ and L² are independently hydrogen or halogen;
a is 0, 1, 2 or 3; and
n¹ and n² are independently 0, 1 or 2.

## Description

### Technical Field

The invention relates to a liquid crystal compound, a liquid crystal composition and a liquid crystal display device. More specifically, the invention relates to a compound having an alkoxy group or an alkoxyalkyl group, and a saturated six-membered ring, a liquid crystal composition that contains the compound and has a nematic phase, and a liquid crystal display device including the composition.

A liquid crystal display device has been widely used in a display of a personal computer, a television and so forth. The device utilizes optical anisotropy and dielectric anisotropy of a liquid crystal compound, and so forth. As an operating mode of the liquid crystal display device, a phase change (PC) mode, a twisted nematic (TN) mode, a super twisted nematic (STN) mode, a bistable twisted nematic (BTN) mode, an electrically controlled birefringence (ECB) mode, an optically compensated bend (OCB) mode, an in-plane switching (IPS) mode, a vertical alignment (VA) mode, a fringe field switching (FFS) mode, a polymer sustained alignment (PSA) mode and so forth are known. In recent years, a mode in which an electric field is applied in an optically isotropic liquid crystal phase to develop an electric refractive index has been also actively studied.

A proposal has been made on a wavelength variable filter utilizing electric birefringence in a blue phase as one of optically isotropic liquid crystal phases, a wavefront control device, a liquid crystal lens, an aberration correction device, an aperture control device, an optical head device and so forth.

A classification based on a driving mode in the device includes a passive matrix (PM) and an active matrix (AM). The passive matrix (PM) is classified into static, multiplex and so forth, and the active matrix (AM) is classified into a thin film transistor (TFT), a metal insulator metal (MIM) and so forth according to a type of the switching device.

In such a liquid crystal display device, a liquid crystal composition having suitable physical properties is used. In order to further improve characteristics of the liquid crystal display device, the liquid crystal compound contained in the composition preferably has physical properties described in (1) to (8) below.
(1) High stability to heat, light or the like,
(2) a high clearing point,
(3) a low minimum temperature of a liquid crystal phase,
(4) small viscosity (η),
(5) suitable optical anisotropy (Δn),
(6) large dielectric anisotropy (Δε),
(7) a suitable elastic constant (K), and
(8) excellent compatibility with other liquid crystal compounds.

In particular, in the optically isotropic liquid crystal phase, a liquid crystal compound having both large dielectric anisotropy and large refractive index anisotropy is preferred from a viewpoint of reducing driving voltage.

An effect of physical properties of the liquid crystal compound on the characteristics of the device is as described below. A compound having the high stability to heat, light and so forth as described in (1) increases a voltage holding ratio of the device. Thus, a service life of the device becomes longer. A compound having the high clearing point as described in (2) extends a temperature range in which the device can be used. A compound having low minimum temperature of the liquid crystal phase such as the nematic phase and a smectic phase as described in (3), particular a compound having low minimum temperature of the nematic phase, also extends the temperature range in which the device can be used. A compound having small viscosity as described in (4) shortens a response time of the device.

A compound having the suitable optical anisotropy as described in (5) improves contrast of the device. According to a design of the device, a compound having large optical anisotropy or small optical anisotropy, more specifically, a compound having suitable optical anisotropy is required. When the response time is shortened by decreasing a cell gap of the device, a compound having large optical anisotropy is suitable. A compound having the large dielectric anisotropy as described in (6) decreases a threshold voltage of the device. Thus, electric power consumption of the device is reduced. On the other hand, a compound having small dielectric anisotropy shortens the response time of the device by decreasing viscosity of the composition.

With regard to (7), a compound having a large elastic constant shortens the response time of the device driven in the nematic phase. A compound having a small elastic constant decreases the threshold voltage of the device driven in the nematic phase. Therefore, the suitable elastic constant is required according to the characteristics that are desirably improved. A compound having excellent compatibility with other liquid crystal compounds as described in (8) is preferred. The reason is that the physical properties of the composition are adjusted by mixing liquid crystal compounds having different physical properties. In recent years, a liquid crystal display device in which display performance such as contrast, display capacity and response time characteristics is higher has been particularly required. A liquid crystal composition having low driving voltage is further requested as liquid crystal material used. Moreover, use of a liquid crystal compound having large dielectric anisotropy and large refractive index anisotropy is preferred in order to drive at low voltage an optical device to be driven in the optically isotropic liquid crystal phase.

A variety of liquid crystal compounds having large dielectric anisotropy have so far been prepared. The reason is that excellent physical properties that are not found in conventional compounds are expected from a new compound. The reason is that a suitable balance is expected to be obtained regarding at least two of physical properties by adding the new compound to a liquid crystal composition. In view of such a situation, with regard to physical properties (1) to (8) described above, desire has been expressed for development of a compound having excellent physical properties and a suitable balance, above all, a compound having large dielectric anisotropy (Δε).

### Citation List

### Patent Literature

Patent literature No. 1: EP 452274 A.
Patent literature No. 2: JP 2007-091796 A.
Patent literature No. 3: JP 2006-070080 A.
Patent literature No. 4: JP 2006-008928 A.
Patent literature No. 5: US 20090302273 B.
Patent literature No. 6: JP H05-065280 A.

### Summary of Invention

### Technical Problem

A first object of the invention is to provide a liquid crystal compound satisfying at least one of physical properties such as high stability to light, a high clearing point, a low minimum temperature of a liquid crystal phase, small viscosity, suitable optical anisotropy, large dielectric anisotropy, a suitable elastic constant and excellent compatibility with other liquid crystal compounds. In particular, the object is to provide a compound having large dielectric anisotropy. A second object is to provide a liquid crystal composition that contains the compound and satisfies at least one of physical properties such as a high maximum temperature of a nematic phase, a low minimum temperature of the nematic phase, the small viscosity, the suitable optical anisotropy, the large dielectric anisotropy and the suitable elastic constant. The object is to provide a liquid crystal composition having a suitable balance regarding at least two of the physical properties. A third object is to provide a liquid crystal display device that includes the composition and has a wide temperature range in which the device can be used, a short response time, a large voltage holding ratio, low threshold voltage, a large contrast ratio and a long service life.

### Solution to Problem

The invention concerns a compound represented by formula (1), a liquid crystal composition containing the compound, and a liquid crystal display device including the composition: 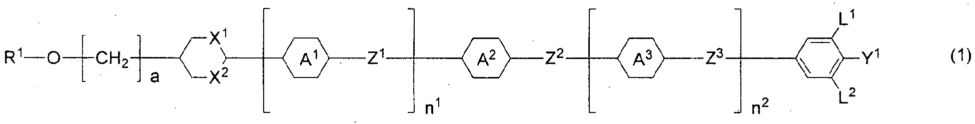 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine;
ring A¹, ring A² and ring A³ are independently represented by a formula described below; 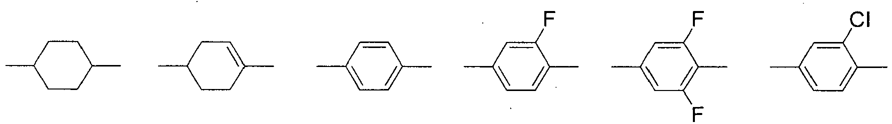 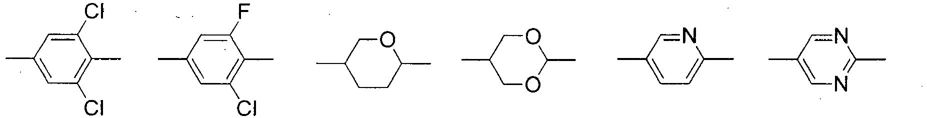 wherein, X¹ and X² are independently -O-, -S- or -CH₂-, and a case where both of X¹ and X² are -CH₂- is excluded;
Y¹ is hydrogen, fluorine, chlorine, -C≡N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-;
Z¹ and Z³ are independently a single bond, -CH₂CH₂-, -CH₂O-, -CF₂O-, -OCF₂-, -COO-, -OCO-, -CH=CH- or -C=C-;
Z² is a single bond, -CF₂O- or -COO-;
L¹ and L² are independently hydrogen or halogen;
a is 0, 1, 2 or 3; and
n¹ and n² are independently 0, 1 or 2, and an expression: n¹ + n² ≤ 2 holds; and
when n¹ + n² = 0 and Z² is -CF₂O, Y¹ is hydrogen, fluorine, chlorine, -C=N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

### Advantageous Effects of Invention

A first advantage of the invention is to provide a liquid crystal compound satisfying at least one of physical properties such as high stability to light, a high clearing point, a low minimum temperature of a liquid crystal phase, small viscosity, suitable optical anisotropy, large dielectric anisotropy, a suitable elastic constant and excellent compatibility with other liquid crystal compounds. The advantage is to provide a compound having particularly large dielectric anisotropy.

A second advantage is to provide a liquid crystal composition that contains the compound and satisfies at least one of physical properties such as a high maximum temperature of a nematic phase, a low minimum temperature of the nematic phase, the small viscosity, the suitable optical anisotropy, the large dielectric anisotropy and the suitable elastic constant.

A third advantage is to provide a liquid crystal display device that includes the composition and has a wide temperature range in which the device can be used, a short response time, a large voltage holding ratio, low threshold voltage, a large contrast ratio and a long service life. An optical device driven in an optically isotropic liquid crystal phase has a wide temperature range in which the device can be used and low driving voltage.

### Brief Description of Drawings

Figure 1 shows a comb-shaped electrode substrate used in Examples.
Figure 2 shows an optical system used in Examples.

### Description of Embodiments

In the invention, a liquid crystal compound represents a compound having a mesogen, and is not limited to a compound having a liquid crystal phase. A liquid crystal medium is a generic term for a liquid crystal composition and a polymer-liquid crystal composite. Moreover, an optical device means various kinds of devices performing a function such as light modulation and optical switching by utilizing an electro-optical effect, and specific examples include a light modulation device used in a display device (liquid crystal display device), an optical communication system, an optical information processing or various sensor systems. A Kerr effect is known as light modulation utilizing change of a refractive index by voltage application to an optically isotropic liquid crystal medium. The Kerr effect is a phenomenon in which an electric birefringence value Δn (E) is proportional to a square of electric field E, and a material showing the Kerr effect satisfies an equation: Δn (E) = KλE² (K: Kerr coefficient (Kerr constant), λ: wavelength). Here, the electric birefringence value is expressed in terms of a value of refractive index anisotropy induced when an electric field is applied to an isotropic medium.

Usage of terms herein is as described below. A liquid crystal compound is a generic term for a compound having a liquid crystal phase such as a nematic phase and a smectic phase, and a compound having no liquid crystal phase but to be added for the purpose of adjusting physical properties of a composition such as maximum temperature, minimum temperature, viscosity and dielectric anisotropy. The compounds have a six-membered ring such as 1,4-cyclohexylene and 1,4-phenylene, and have rod-like molecular structure. A liquid crystal composition is prepared by mixing such liquid crystal compounds . A proportion (content) of the liquid crystal compounds is expressed in terms of weight percent (% by weight) based on the weight of the liquid crystal composition. An additive such as a polymerizable compound, a polymerization initiator, an optically active compound, an antioxidant, an ultraviolet light absorber, a light stabilizer, a heat stabilizer, an antifoaming agent and a dye is added to the composition when necessary. A proportion (amount of addition) of the additive is expressed in terms of weight percent (% by weight) based on the weight of the liquid crystal composition in a manner similar to the proportion of the liquid crystal compounds. Weight parts per million (ppm) may be occasionally used. A chiral agent is the optically active compound, and added in order to give a desired twisted molecular arrangement to the liquid crystal composition. The liquid crystal display device is a generic term for a liquid crystal display panel and a liquid crystal display module. The liquid crystal compound, the liquid crystal composition and the liquid crystal display device may be occasionally abbreviated as "compound, " "composition" and "device, " respectively. A clearing point is a transition temperature between the liquid crystal phase and an isotropic phase in the liquid crystal compound. A minimum temperature of the liquid crystal phase is a transition temperature between a solid and the liquid crystal phase (the smectic phase, the nematic phase or the like) in the liquid crystal compound. A maximum temperature of the nematic phase is a transition temperature between the nematic phase and the isotropic phase in the liquid crystal composition, and may be occasionally abbreviated as "maximum temperature. " A minimum temperature of the nematic phase may be occasionally abbreviated as "minimum temperature."

A compound represented by formula (1) may be occasionally abbreviated as compound (1). The abbreviation may occasionally apply also to a compound represented by formula (2) or the like. In formula (1) to formula (16), a symbol such as A¹, B¹ and C¹ surrounded by a hexagonal shape corresponds to ring A¹, ring B¹ and ring C¹, respectively. A symbol of terminal group R¹¹ is used in a plurality of compounds. In the compounds, two groups represented by two pieces of arbitrary R¹¹ may be identical or different. For example, in one case, R¹¹ of compound (2) is ethyl and R¹¹ of compound (3) is ethyl. In another case, R¹¹ of compound (2) is ethyl and R¹¹ of compound (3) is propyl. A same rule applies also to a symbol of any other terminal group, a ring or the like. In formula (6), when i is 2, two of ring C¹ exists. In the compound, two groups represented by two of ring C¹ may be identical or different. A same rule applies also to arbitrary two when i is larger than 2. A same rule applies also to a symbol of any other ring, a bonding group or the like.

An expression "at least one piece of "A" may be replaced by "B"" means that, when the number of "A" is 1, a position of "A" is arbitrary, and also when the number of "A" is 2 or more, positions thereof can be selected without restriction. An expression "at least one piece of A may be replaced by B, C or D" includes a case where arbitrary A is replaced by B, a case where arbitrary A is replaced by C, and a case where arbitrary A is replaced by D, and also a case where a plurality of pieces of A are replaced by at least two of B, C and D. For example, alkyl in which at least one piece of -CH₂- may be replaced by -O- or -CH=CH- includes alkyl, alkenyl, alkoxy, alkoxyalkyl, alkoxyalkenyl and alkenyloxyalkyl. In addition, a case where two pieces of consecutive -CH₂- are replaced by -O- to form -O-O- is not preferred. In alkyl or the like, a case where -CH₂- of a methyl part (-CH₂-H) is replaced by -O- to form -O-H is not preferred, either.

Then, 2-fluoro-1,4-phenylene means two divalent groups described below. In a chemical formula, fluorine may be leftward (L) or rightward (R). A same rule applies also to an asymmetrical divalent ring such as tetrahydropyran-2, 5-diyl.

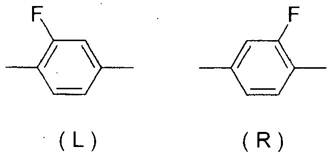

The invention includes the content described in items 1 to item 37 below.

Item 1. A liquid crystal composition, containing at least one compound represented by formula (1): 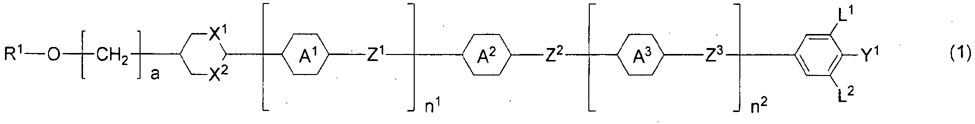 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine;
ring A¹, ring A² and ring A³ are independently represented by a formula described below; 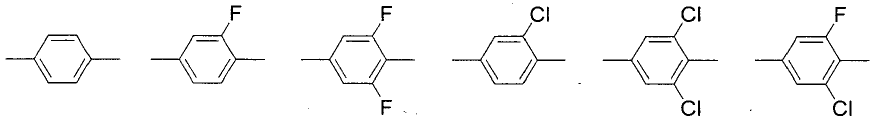 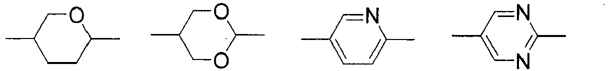 wherein, X¹ and X² are independently -O-, -S- or -CH₂-, and a case where both X¹ and X² are -CH₂- is excluded;
Y¹ is hydrogen, fluorine, chlorine, -C=N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-;
Z¹ and Z³ are independently a single bond, -CH₂CH₂-, -CH₂O-, -CF₂O-, -OCF₂-, -COO-, -OCO-, -CH=CH- or -C=C-;
Z² is a single bond, -CF₂O- or -COO-;
L¹ and L² are independently hydrogen or halogen;
a is 0, 1, 2 or 3; and
n¹ and n² are independently 0, 1 or 2, and an expression: n¹ + n² ≤ 2 holds; and
when n¹ + n² = 0 and Z² is -CF₂O, Y¹ is hydrogen, fluorine, chlorine, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

Item 2. The liquid crystal composition according to item 1, containing at least one compound represented by formula (1-1): 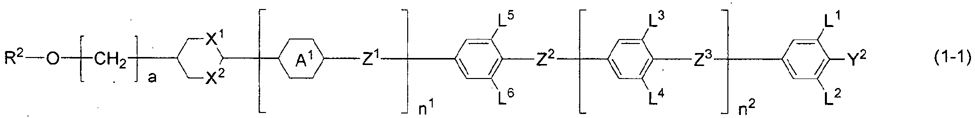 wherein, in formula (1-1),
R² is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
ring A¹ is represented by a formula described below; 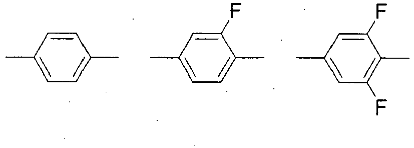 wherein, X¹ and X² are independently -O- or -CH₂-, and a case where both X¹ and X² are -CH₂- is excluded;
Y² is hydrogen, fluorine, chlorine, -C=N, -CH₂F, -CHF₂, -CF₃, -OCH₂F, -OCHF₂, -OCF₃, -CH=CHF, -CH=CF₂, -CF=CHF, -CH=CHCF₃, -OCH=CF₂,-OCF=CF₂ or -OCH=CHCF₃;
Z¹ and Z³ are independently a single bond, -CH₂CH₂-, -CH₂O-, -CF₂O-, -OCF₂-, -COO-, -OCO-, -CH=CH- or -C≡C-;
Z² is a single bond, -CF₂O- or -COO-;
L¹, L², L³, L⁴, L⁵ and L⁶ are independently hydrogen, fluorine or chlorine;
a is 0, 1, 2 or 3; and
n¹ and n² are independently 0, 1 or 2, and an expression: n¹ + n² ≤ 2 holds.

Item 3. The liquid crystal composition according to item 1 or 2, containing at least one compound represented by formula (1-1-1): 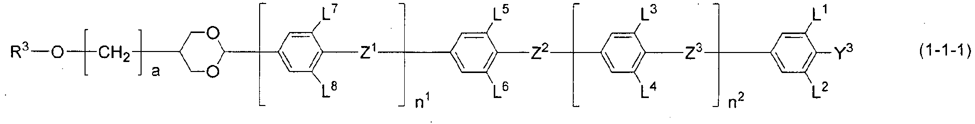 wherein, in formula (1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C=N, -CF₃, -OCF₃, -OCH=CF₂ or -OCH=CHCF₃ ;
Z¹ and Z³ are independently a single bond, -CH₂O-, -CF₂O-, -COO-, -CH=CH- or -C≡C-;
Z² is a single bond, -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

Item 4. The liquid crystal composition according to any one of items 1 to 3, wherein a is 1.

Item 5. The liquid crystal composition according to any one of items 1 to 3, wherein a is 2.

Item 6. The liquid crystal composition according to any one of items 1 to 5, containing at least one compound represented by formula (1-1-1-1) : 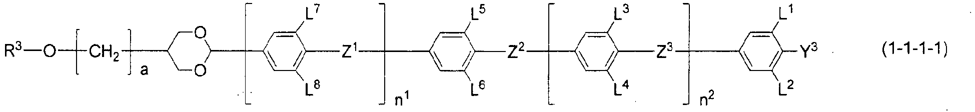 wherein, in formula (1-1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C=N, -CF₃, -OCF₃, -OCH=CF₂ or -OCH=CHCF₃ ;
Z¹ and Z³ are independently a single bond, -CF₂O-, -COO- or -C≡C-;
Z² is -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

Item 7. The liquid crystal composition according to any one of items 1 to 6, containing at least one compound represented by formulas (1-1-1-1-1) to (1-1-1-1-5): 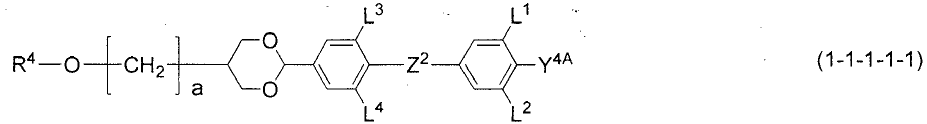 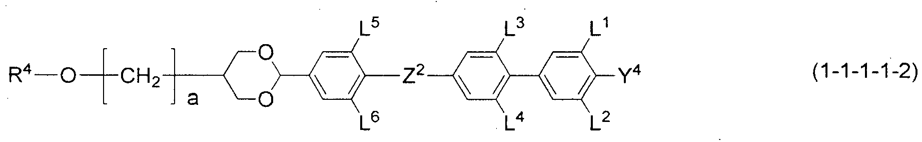 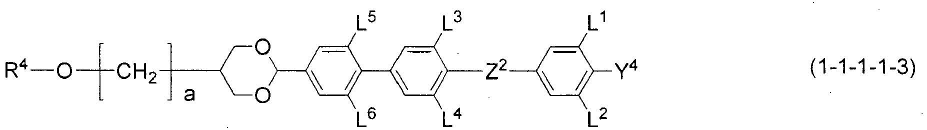 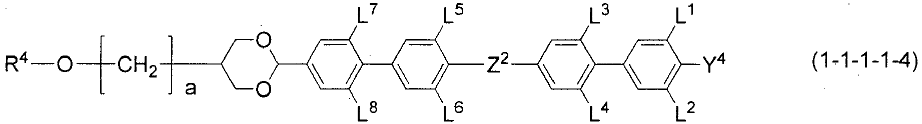 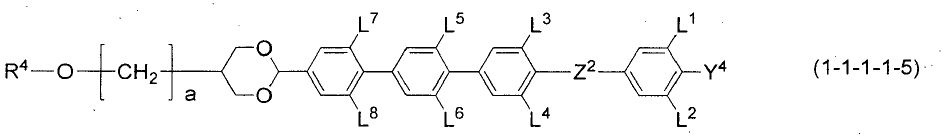 wherein, in formulas (1-1-1-1-1) to (1-1-1-1-5),
R⁴ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁴ is hydrogen, fluorine, chlorine, -C=N, -CF₃, -OCF₃, -OCH=CF₂ or -OCH=CHCF₃ ;
Y^{4A} is hydrogen, fluorine, chlorine, -CF₃, -OCF₃, -OCH=CF₂ or -OCH=CHCF₃ ;
Z² is -CF₂O- or -COO-;
L¹, L², L³, L⁴, L⁵, L⁶, L⁷ and L⁸ are independently hydrogen or fluorine; and
a is 0, 1, 2 or 3.

Item 8. The compound according to any one of items 1 to 6, containing at least one compound represented by formula (1-1-1-1-11) or (1-1-1-1-12): 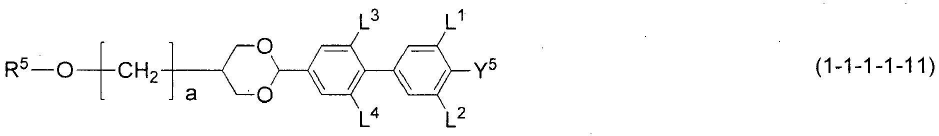 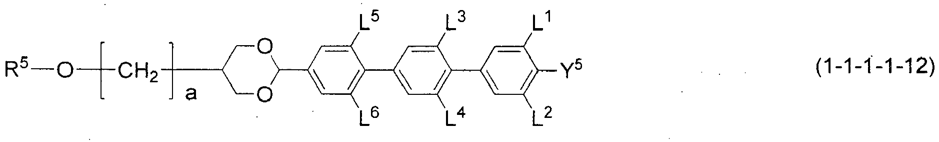 wherein, in formulas (1-1-1-1-11) and (1-1-1-1-12),
R⁵ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁵ is hydrogen, fluorine, chlorine, -C=N, -CF₃, -OCF₃, -OCH=CF₂ or -OCH=CHCF₃ ;
L¹, L², L³, L⁴, L⁵ and L⁶ are independently hydrogen or fluorine; and
a is 0, 1, 2 or 3.

Item 9. The liquid crystal composition according to any one of items 1 to 8, further containing at least one compound selected from the group of compounds represented by formulas (2) to (5): 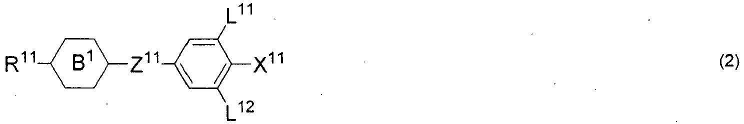 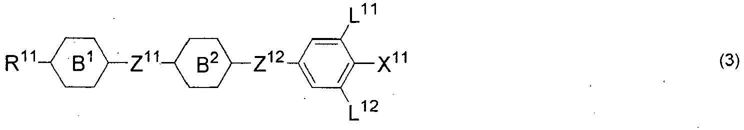 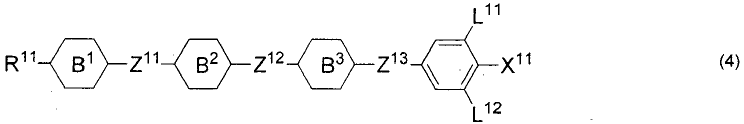 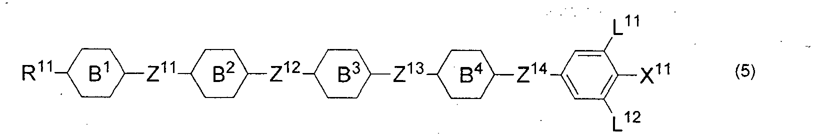 wherein, in formulas (2) to (5),
R¹¹ is alkyl having 1 to 10 carbons or alkenyl having 2 to 10 carbons, and in the alkyl and the alkenyl, at least one piece of hydrogen may be replaced by fluorine;
X¹¹ is hydrogen, fluorine, chlorine, -OCF₃, -OCHF₂, -CF₃, -CHF₂, -CH₂F, -OCF₂CHF₂ or -OCF₂CHFCF₃;
ring B¹, ring B², ring B³ and ring B⁴ are independently 1,4-cyclohexylene, 1,4-phenylene in which at least one piece of hydrogen may be replaced by fluorine, tetrahydropyran-2,5-diyl, 1,3-dioxane-2,5-diyl or pyrimidine-2,5-diyl;
Z¹¹, Z¹², Z¹³ and Z¹⁴ are independently a single bond, -CH₂CH₂-, -CH=CH-, -C≡C-, -COO-, -CF₂O-, -OCF₂-, -CH₂O- or -(CH₂)₄-; and
L¹¹ and L¹² are independently hydrogen or fluorine.

Item 10. The liquid crystal composition according to any one of items 1 to 9, further containing at least one compound selected from the group of compounds represented by formula (6): 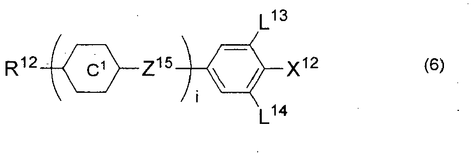 wherein, in formula (6),
R¹² is alkyl having 1 to 10 carbons or alkenyl having 2 to 10 carbons, and in the alkyl and the alkenyl, at least one piece of hydrogen may be replaced by fluorine;
X¹² is -C=N or -C=C-CN;
ring C¹ is 1,4-cyclohexylene, 1,4-phenylene in which at least one piece of hydrogen may be replaced by fluorine, tetrahydropyran-2,5-diyl, 1,3-dioxane-2,5-diyl or pyrimidine-2,5-diyl;
Z¹⁵ is a single bond, -CH₂CH₂-, -C≡C-, -COO-, -CF₂O-, -OCF₂- or -CH₂O-;
L¹³ and L¹⁴ are independently hydrogen or fluorine; and
i is 1, 2, 3 or 4.

Item 11. The liquid crystal composition according to any one of items 1 to 10, further containing at least one compound selected from the group of compounds represented by formulas (7) to (13): 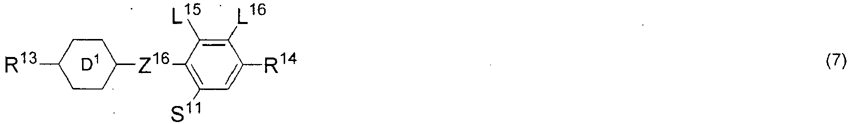 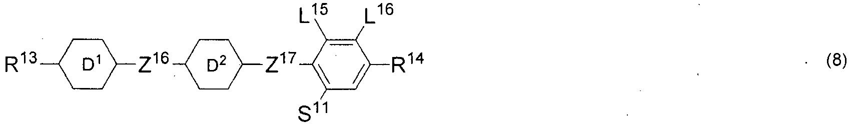 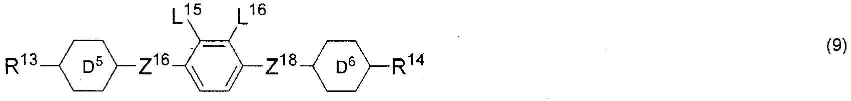 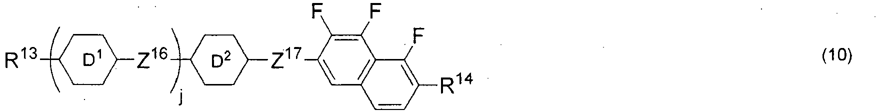 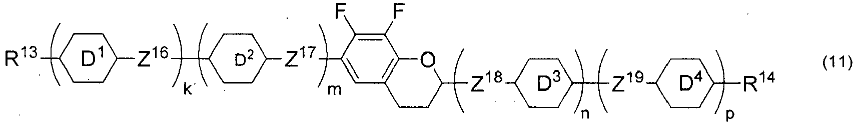 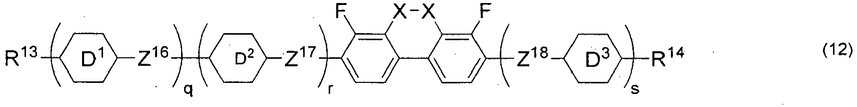 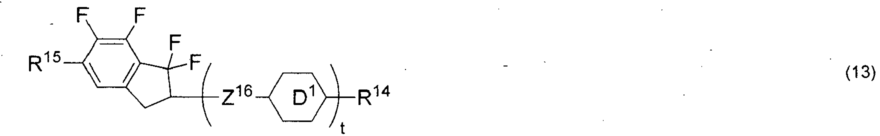 wherein, in formulas (7) to (13),
R¹³ and R¹⁴ are independently alkyl having 1 to 10 carbons or alkenyl having 2 to 10 carbons, and in the alkyl and the alkenyl, at least one piece of -CH₂- may be replaced by -O-, and at least one piece of hydrogen may be replaced by fluorine;
R¹⁵ is hydrogen, fluorine, alkyl having 1 to 10 carbons or alkenyl having 2 to 10 carbons, and in the alkyl and the alkenyl, at least one piece of -CH₂- may be replaced by -O-, and at least one piece of hydrogen may be replaced by fluorine;
S¹¹ is hydrogen or methyl;
X is -CF₂-, -O- or -CHF-;
ring D¹, ring D², ring D³ and ring D⁴ are independently 1,4-cyclohexylene, 1,4-cyclohexenylene, 1,4-phenylene in which at least one piece of hydrogen may be replaced by fluorine, tetrahydropyran-2,5-diyl or decahydronaphthalene-2,6-diyl;
ring D⁵ and ring D⁶ are independently 1,4-cyclohexylene, 1,4-cyclohexenylene, 1,4-phenylene, tetrahydropyran-2,5-diyl or decahydronaphthalene-2,6-diyl;
Z¹⁶, Z¹⁷, Z¹⁸ and Z¹⁹ are independently a single bond, -CH₂CH₂-, -COO-, -CH₂O-, -OCF₂- or -OCF₂CH₂CH₂-;
L¹⁵ and L¹⁶ are independently fluorine or chlorine; and
j, k, m, n, p, q, r and s are independently 0 or 1, a sum of k, m, n and p is 1 or 2, a sum of q, r and s is 0, 1, 2 or 3, and t is 1, 2 or 3.

Item 12. The liquid crystal composition according to any one of items 1 to 11, further containing at least one compound selected from the group of compounds represented by formulas (14) to (16): 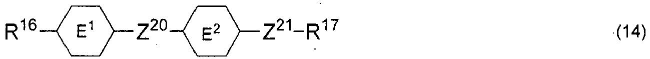 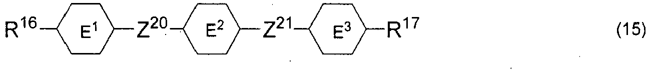 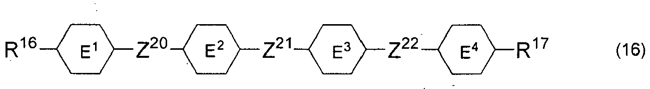 wherein, in formulas (14) to (16),
R¹⁶ and R¹⁷ are independently alkyl having 1 to 10 carbons or alkenyl having 2 to 10 carbons;
ring E¹, ring E², ring E³ and ring E⁴ are independently 1,4-cyclohexylene, 1,4-phenylene, 2-fluoro-1,4-phenylene, 2,5-difluoro-1,4-phenylene or pyrimidine-2,5-diyl; and
Z²⁰, Z²¹ and Z²² are independently a single bond, -CH₂CH₂-, -CH=CH-, -C≡C- or -COO-.

Item 13. The liquid crystal composition according to any one of items 1 to 12, further containing at least one of a polymerizable compound, an optically active compound, an antioxidant, an ultraviolet light absorber, a light stabilizer, a heat stabilizer and an antifoaming agent.

Item 14. A liquid crystal composition that contains a compound represented by formula (1) and a chiral agent, and develops an optically isotropic liquid crystal phase: 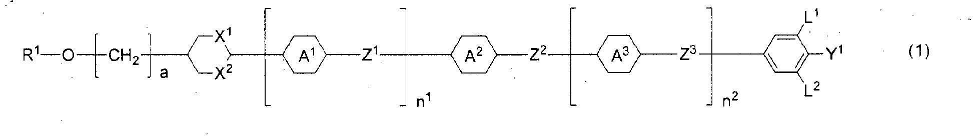 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine;
ring A¹, ring A² and ring A³ are independently represented by a formula described below; 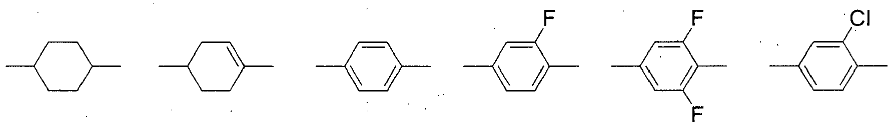 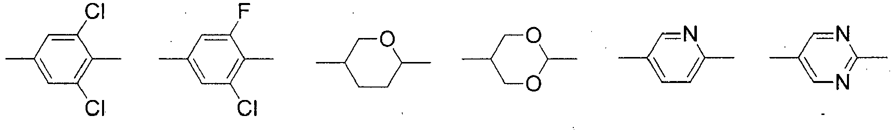 wherein, X¹ and X² are independently -O-, -S- or -CH₂-, and a case where both X¹ and X² are -CH₂- is excluded;
Y¹ is hydrogen, fluorine, chlorine, -C=N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-;
Z¹ and Z³ are independently a single bond, -CH₂CH₂-, -CH₂O-, -CF₂O-, -OCF₂-, -COO-, -OCO-, -CH=CH- or -C≡C-;
Z² is a single bond, -CF₂O- or -COO-;
L¹ and L² are independently hydrogen or halogen;
a is 0, 1, 2 or 3; and
n¹ and n² are independently 0, 1 or 2, and an expression: n¹ + n² ≤ 2 holds; and
when n¹ + n² = 0 and Z² is -CF₂O, Y¹ is hydrogen, fluorine, chlorine, -C≡N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

Item 15. The liquid crystal composition according to item 14, containing at least one compound represented by formula (1-1): 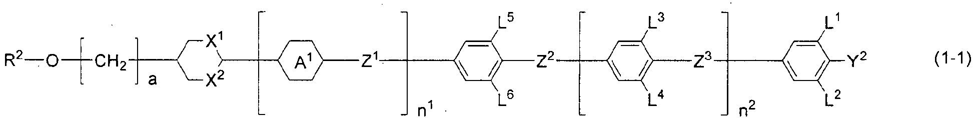 wherein, in formula (1-1),
R² is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
ring A¹ is represented by a formula described below; 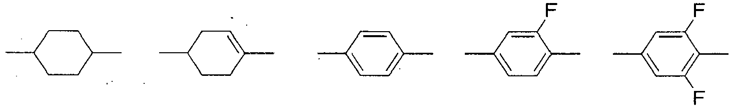 wherein, X¹ and X² are independently -O- or -CH₂-, and a case where both X¹ and X² are -CH₂- is excluded;
Y² is hydrogen, fluorine, chlorine, -C=N, -CH₂F, -CHF₂, -CF₃, -OCH₂F, -OCHF₂, -OCF₃, -CH=CHF, -CH=CF₂, -CF=CHF, -CH=CHCF₃, -OCH=CF₂, -OCF=CF₂ or -OCH=CHCF₃ ;
Z¹ and Z³ are independently a single bond, -CH₂CH₂-, -CH₂O-, -CF₂O-, -OCF₂-, -OCO-, -CH=CH- or -C≡C-;
Z² is a single bond, -CF₂O- or -COO-;
L¹, L², L³, L⁴, L⁵ and L⁶ are independently hydrogen, fluorine or chlorine;
a is 0, 1, 2 or 3; and
n¹ and n² are independently 0, 1 or 2, and an expression: n¹ + n² ≤ 2 holds.

Item 16. The liquid crystal composition according to item 14 or 15, containing at least one compound represented by formula (1-1-1) : 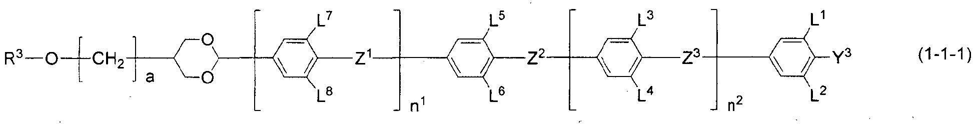 wherein, in formula (1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C=N, -CF₃, -OCF₃, -OCH=CF₂ or -OCH=CHCF₃ ;
Z¹ and Z³ are independently a single bond, -CH₂O-, -CF₂O-, -CH=CH- or -C≡C-;
Z² is a single bond, -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

Item 17. The liquid crystal composition according to any one of items 14 to 16, wherein a is 1.

Item 18. The liquid crystal composition according to any one of items 14 to 16, wherein a is 2.

Item 19. The liquid crystal composition according to any one of items 14 to 18, containing at least one compound represented by formula (1-1-1-1) : 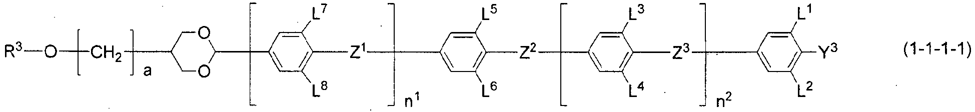 wherein, in formula (1-1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C=N, -CF₃, -OCF₃, -OCH=CF₂ or -OCH=CHCF₃ ;
Z¹ and Z³ are independently a single bond, -CF₂O-, -COO- or -C≡C-;
Z² is -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

Item 20. The liquid crystal composition according to any one of items 14 to 19, containing at least one compound represented by formulas (1-1-1-1-1) to (1-1-1-1-5): 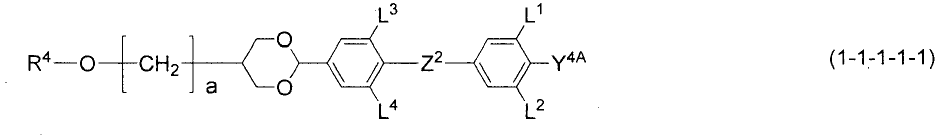 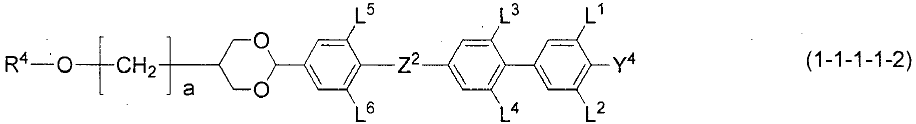 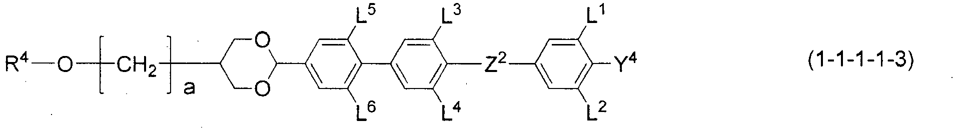 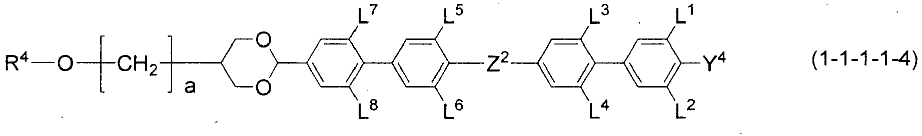 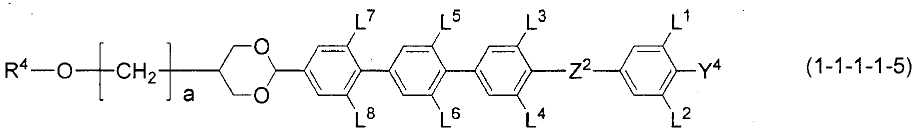 wherein, in formulas (1-1-1-1-1) to (1-1-1-1-5),
R⁴ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁴ is hydrogen, fluorine, chlorine, -C≡N, -CF₃, -OCF₃, -OCH=CF₂ or -OCH=CHCF₃ ;
Y^{4A} is hydrogen, fluorine, chlorine, -CF₃, -OCF₃, -OCH=CF₂ or -OCH=CHCF₃ ;
Z² is -CF₂O- or -COO-;
L¹, L², L³, L⁴, L⁵, L⁶, L⁷ and L⁸ are independently hydrogen or fluorine; and
a is 0, 1, 2 or 3.

Item 21. The liquid crystal composition according to any one of items 14 to 19, containing at least one compound represented by formula (1-1-1-1-11) or (1-1-1-1-12): 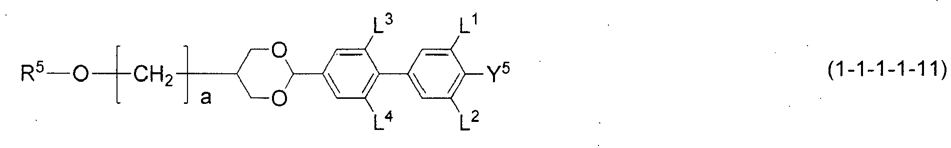 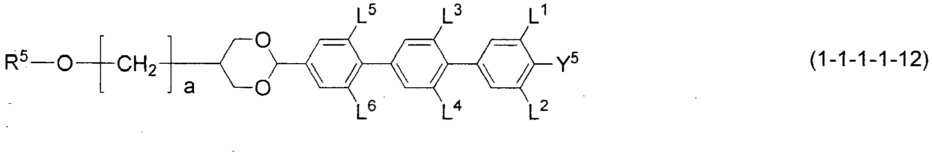 wherein, in formulas (1-1-1-1-11) and (1-1-1-1-12),
R⁵ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁵ is hydrogen, fluorine, chlorine, -C=N, -CF₃, -OCF₃, -OCH=CF₂ or -OCH=CHCF₃;
L¹, L², L³, L⁴, L⁵ and L⁶ are independently hydrogen or fluorine; and
a is 0, 1, 2 or 3.

Item 22. The liquid crystal composition according to any one of items 14 to 21, further containing at least one compound selected from the group of compounds represented by formulas (4A) to (4D) : 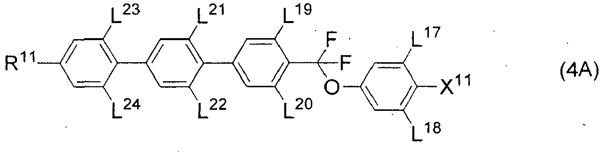 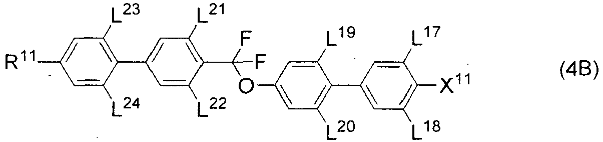 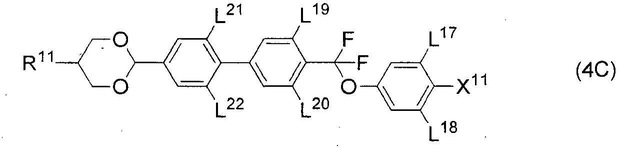 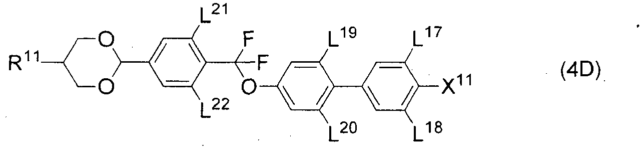 wherein, in formulas (4A) to (4D),
R¹¹ is alkyl having 1 to 10 carbons or alkenyl having 2 to 10 carbons, and in the alkyl and the alkenyl, at least one piece of hydrogen may be replaced by fluorine;
L¹⁷, L¹⁸, L¹⁹, L²⁰, L²¹, L²², L²³ and L²⁴ are independently hydrogen, fluorine or chlorine; and
X¹¹ is hydrogen, fluorine, chlorine, -OCF₃, -OCHF₂, -CF₃, -CHF₂, -CH₂F, -OCF₂CHF₂ or -OCF₂CHFCF₃.

Item 23. The liquid crystal composition according to any one of items 14 to 22, containing at least one compound selected from the group of compounds represented by formulas) (K21) to (K27) as a chiral agent (optically active compound): 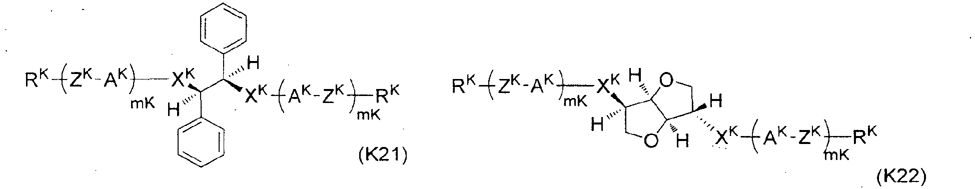 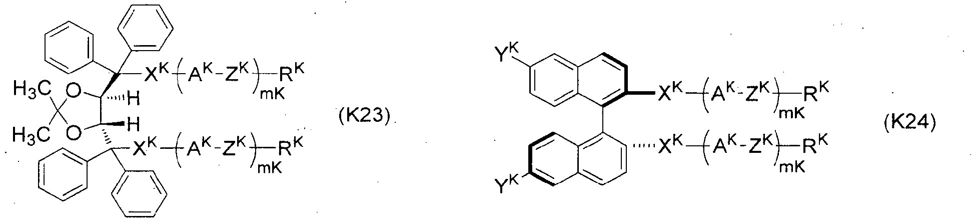 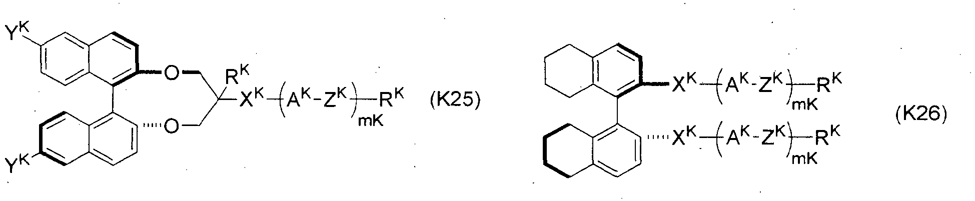 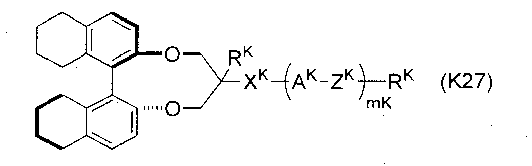 wherein, in formulas (K21) to (K27),
R^{K} is each independently hydrogen, halogen, -C=N, -N=C=O, -N=C=S or alkyl having 1 to 12 carbons, at least one piece of -CH₂- in R^{K} is may be replaced by -O-, -S-, -COO- or -OCO-, at least one piece of -CH₂-CH₂- in R^{K} may be replaced by -CH=CH-, -CF=CF- or -C=C-, and at least one piece of hydrogen in R^{K} may be replaced by fluorine or chlorine;
A^{K} is each independently an aromatic 6-membered ring to an aromatic 8-membered ring, a non-aromatic 3-membered ring to a non-aromatic 8-membered ring, or a condensed ring having 9 or more carbons, at least one piece of hydrogen in the rings may be replaced by halogen, alkyl having 1 to 3 carbons or haloalkyl, -CH₂- in the ring may be replaced by -O-, -S- or -NH-, and -CH= may be replaced by -N=;
Y^{K} is each independently hydrogen, halogen, alkyl having 1 to 3 carbons, haloalkyl having 1 to 3 carbons, an aromatic 6-membered ring to an aromatic 8-membered ring, a non-aromatic 3-membered ring to a non-aromatic 8-membered ring, or a condensed ring having 9 or more carbons, at least one piece of hydrogen in the rings may be replaced by halogen, alkyl having 1 to 3 carbons or haloalkyl, -CH₂- in the alkyl may be replaced by -O-, -S- or -NH-, and -CH= may be replaced by -N=;
Z^{K} is each independently a single bond and alkylene having 1 to 8 carbons, at least one piece of -CH₂- in Z^{K} may be replaced by -O-, -S-, -OCO-, -CSO-, -OCS-, -N=N-, -CH=N- or -N=CH-, at least one piece of -CH₂-CH₂- in Z^{K} may be replaced by -CH=CH-, -CF=CF- or -C≡C-, and at least one piece of hydrogen in Z^{K} may be replaced by halogen;
X^{K} is each independently a single bond, -OCO-, -CH₂O-, -OCH₂-, -CF₂O-, -OCF₂- or -CH₂CH₂-; and
mK is each independently an integer from 1 to 3).

Item 24. The liquid crystal composition according to any one of items 14 to 23, further containing at least one polymerizable compound selected from the group of compounds represented by formulas (M2-15), (M4-5) and (M21):

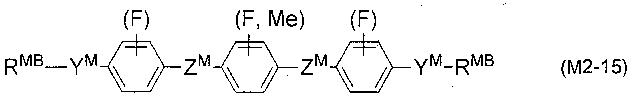

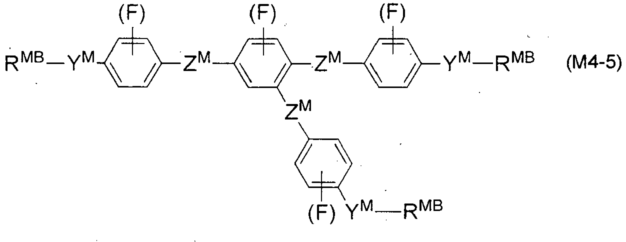

R^{MB}-R^{MC} (M21)

wherein, in formulas (M2-15), (M4-5) and (M21),
R^{MB} is each independently a polymerizable group in formulas (M3-1) to (M3-7), and R^{d} in formulas (M3-1) to (M3-7) is each independently hydrogen, halogen or alkyl having 1 to 5 carbons, and in the alkyl, at least one piece of hydrogen may be replaced by halogen; 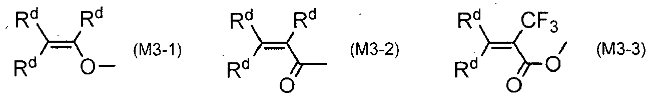 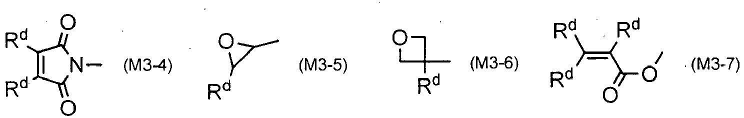 wherein, R^{MC} is each independently alkyl having 1 to 20 carbons or alkenyl having 2 to 20 carbons, and in the alkyl and the alkenyl, at least one piece of -CH₂- may be replaced by -O-, and at least one piece of hydrogen may be replaced by fluorine;
Y^{M} is each independently a single bond or alkylene having 1 to 20 carbons, and in the alkylene, at least one piece of -CH₂- may be replaced by -O- or -S-, and at least one piece of -CH₂-CH₂- in the alkyl may be replaced by -CH=CH-, -C≡C-, -COO- or -OCO-; and
Z^{M} is each independently a single bond, -(CH₂)ₘ₃-, -O(CH₂)ₘ₃-, -(CH₂)ₘ₃O-, -O(CH₂)ₘ₃O-, -CH=CH-, -C≡C-, -OCO-, -(CF₂)₂-, -(CH₂)₂-COO-, -OCO-(CH₂)₂-, -CH=CH-COO-, -OCO-CH=CH-, -C≡C-COO-, -OCO-C≡C-, -CH=CH-(CH₂)₂-, -(CH₂)₂-CH=CH-, -CF=CF-, -C≡C-CH=CH-, -CH=CH-C=C-, -OCF₂-(CH₂)₂-, - (CH₂)₂-CF₂O-, -OCF₂- or -CF₂O- (in the formulas described above, m3 is an integer from 1 to 20); and
in partial structure of a ring, partial structure (a1) represents 1, 4-phenylene in which at least one of hydrogen is replaced by fluorine, partial structure (a2) represents 1,4-phenylene in which at least one piece of hydrogen may be replaced by fluorine, partial structure (a3) represents 1,4-phenylene in which at least one piece of hydrogen may be replaced by any one of fluorine and methyl, and partial structure (a4) represents fluorene in which hydrogen in a 9 position may be replaced by methyl.

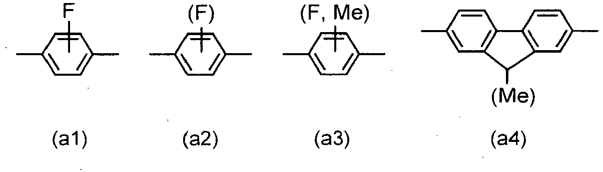

Item 25. The liquid crystal composition according to any one of items 14 to 24, having a chiral nematic phase in a temperature of any of -20°C to 70°C, wherein a helical pitch is 700 nanometers or less in at least part of the range of the temperature.

Item 26. The liquid crystal composition according to any one of items 14 to 24, used in a device driven in an optically isotropic liquid crystal phase.

Item 27. A polymer-liquid crystal composite material, obtained by polymerizing the liquid crystal composition according to item 24 and used in a device driven in an optically isotropic liquid crystal phase.

Item 28. An optical device, having an electrode arranged on one or both substrates, a liquid crystal medium arranged between the substrates, and an electric field applying means for applying an electric field to the liquid crystal medium through the electrode, wherein the optical device is prepared by using the liquid crystal composition according to any one of items 14 to 24 as the liquid crystal medium, or comprises the polymer-liquid crystal composite material according to claim 27.

Item 29. Use of the liquid crystal composition according to any one of items 14 to 24 in an optical device, or use of the polymer-liquid crystal composite material according to item 27 in the optical device.

Item 30. A compound, represented by formula (1) : 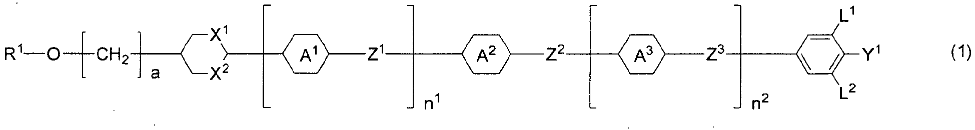 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine;
ring A¹, ring A² and ring A³ are independently represented by a formula described below; 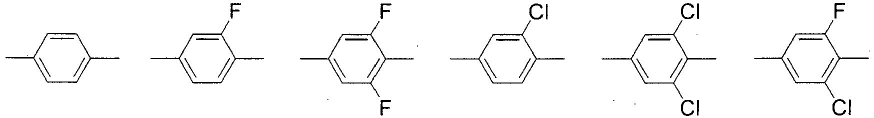 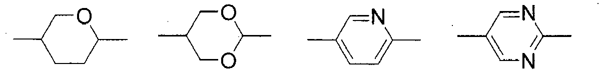 wherein, X¹ and X² are independently -O-, -S- or -CH₂-, and a case where both X¹ and X² are -CH₂- is excluded;
Y¹ is hydrogen, fluorine, chlorine, -C=N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-;
Z¹ and Z³ are independently a single bond, -CH₂CH₂-, -CH₂O-, -CF₂O-, -OCF₂-, -OCO-, -CH=CH- or -C≡C-;
Z² is a single bond, -CF₂O- or -COO-;
L¹ and L² are independently hydrogen or halogen;
a is 0, 1, 2 or 3; and
n¹ and n² are independently 0, 1 or 2, and an expression: n¹ + n² ≤ 2 holds; and
when n¹ + n² = 0 and Z² is -CF₂O, Y¹ is hydrogen, fluorine, chlorine, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

Item 31. The compound according to item 30, represented by formula (1-1) : 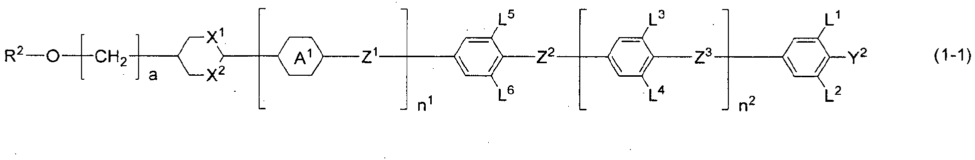 wherein, in formula (1-1),
R² is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
ring A¹ is represented by a formula described below; 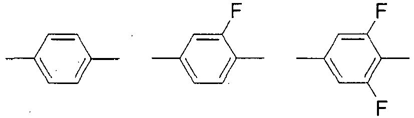 wherein, X¹ and X² are independently -O- or -CH₂-, and a case where both X¹ and X² are -CH₂- is excluded;
Y² is hydrogen, fluorine, chlorine, -C≡N, -CH₂F, -CHF₂, -CF₃, -OCH₂F, -OCHF₂, -OCF₃, -CH=CHF, -CH=CF₂, -CF=CHF, -CH=CHCF₃, -OCH=CF₂, -OCF=CF₂ or -OCH=CHCF₃;
Z¹ and Z³ are independently a single bond, -CH₂CH₂-, -CH₂O-, -CF₂O-, -OCF₂-,-OCO-, -CH=CH- or -C≡C-;
Z² is a single bond, -CF₂O- or -COO-;
L¹, L², L³, L⁴, L⁵ and L⁶ are independently hydrogen, fluorine or chlorine;
a is 0, 1, 2 or 3; and
n¹ and n² are independently 0, 1 or 2, and an expression: n¹ + n² ≤ 2 holds.

Item 32. The compound according to item 30 or 31, represented by formula (1-1-1): 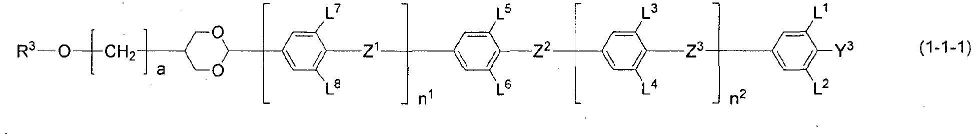 wherein, in formula (1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C=N, -CF₃, -OCF₃, -OCH=CF₂ or -OCH=CHCF₃ ;
Z¹ and Z³ are independently a single bond, -CH₂O-, -CF₂O-, -CH=CH- or -C≡C-;
Z² is a single bond, -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

Item 33. The compound according to any one of items 30 to 32, wherein a is 1.

Item 34. The compound according to any one of items 30 to 32, wherein a is 2.

Item 35. The compound according to any one of items 30 to 34, represented by formula (1-1-1-1): 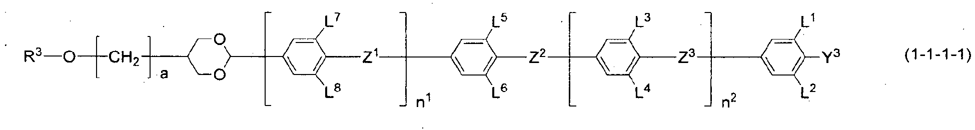 wherein, in formula (1-1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C=N, -CF₃, -OCF₃, -OCH=CF₂ or -OCH=CHCF₃ ;
Z¹ and Z³ are independently a single bond, -CF₂O-, -COO- or -C≡C-;
Z² is -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

Item 36. The compound according to any one of items 30 to 35, represented by formulas (1-1-1-1-1) to (1-1-1-1-5): 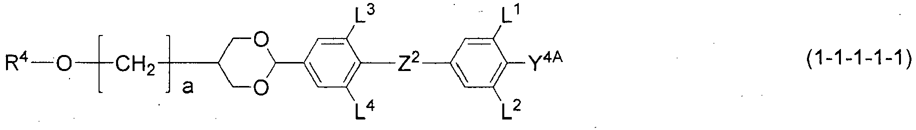 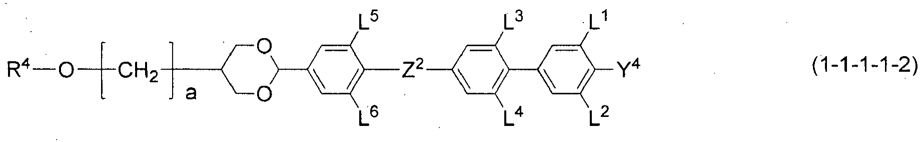 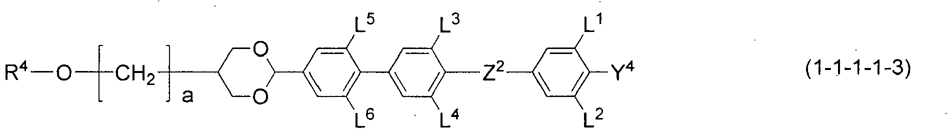 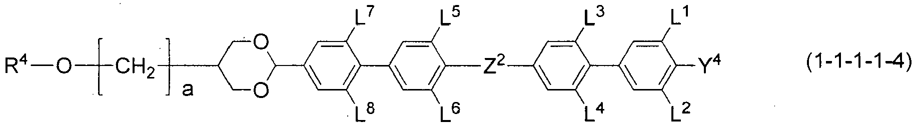 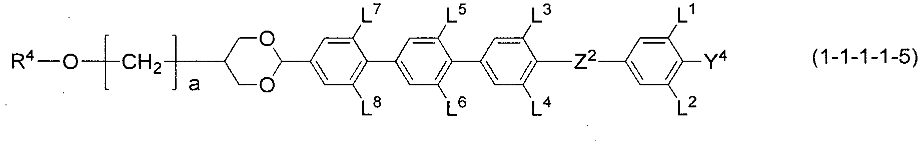 wherein, in formulas (1-1-1-1-1) to (1-1-1-1-5),
R⁴ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁴ is hydrogen, fluorine, chlorine, -C=N, -CF₃, -OCF₃, -OCH=CF₂ or -OCH=CHCF₃;
Y^{4A} is hydrogen, fluorine, chlorine, -CF₃, -OCF₃, -OCH=CF₂ or -OCH=CHCF₃;
Z² is -CF₂O- or -COO-;
L¹, L², L³, L⁴, L⁵, L⁶, L⁷ and L⁸ are independently hydrogen or fluorine; and
a is 0, 1, 2 or 3.

Item 37. The compound according to any one of items 30 to 35, represented by formula (1-1-1-1-11) or (1-1-1-1-12): 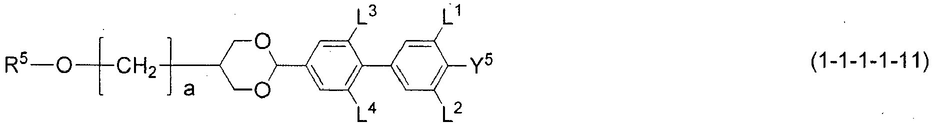 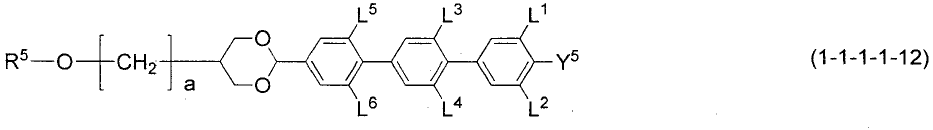 wherein, in formulas (1-1-1-1-11) and (1-1-1-1-12),
R⁵ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁵ is hydrogen, fluorine, chlorine, -C≡N, -CF₃, -OCF₃, -OCH=CF₂ or -OCH=CHCF₃;
L¹, L², L³, L⁴, L⁵ and L⁶ are independently hydrogen or fluorine; and
a is 0, 1, 2 or 3.

The compound, the liquid crystal composition and the liquid crystal display device according to the invention will be described in the order.

### 1-1. Compound (1)

Compound (1) of the invention is a compound having an alkoxy group or an alkoxyalkyl group, and a saturated six-membered ring to particularly have large dielectric anisotropy (Δε). Preferred examples of compound (1) of the invention will be described. Preferred examples of a terminal group, ring structure, a bonding group and a substituent in compound (1) applies also to a subordinate formula of formula (1) for compound (1): 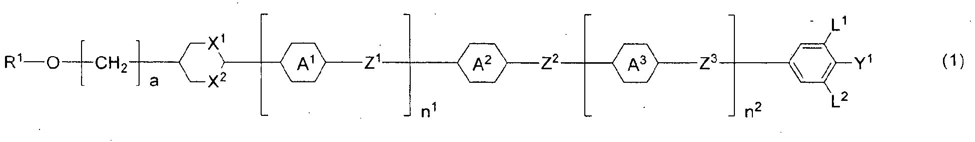 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine.

Specific examples of such group R¹ include alkyl, alkoxyalkyl, alkenyl, alkenyloxyalkyl and alkoxyalkenyl. In the groups, at least one piece of hydrogen may be replaced by halogen. Preferred halogen is fluorine and chlorine. Further preferred halogen is fluorine. The groups have a straight chain or a branched chain, but include no cyclic group such as cyclohexyl. In the groups, the straight chain is preferred to the branched chain.

A preferred configuration of -CH=CH- in the alkenyl depends on a position of a double bond. A trans configuration is preferred in alkenyl having the double bond in an odd-numbered position, such as -CH=CHCH₃, -CH=CHC₂H₅, -CH=CHC₃H₇, -CH=CHC₄H₉, -C₂H₄CH=CHCH₃ and -C₂H₄CH=CHC₂H₅. A cis configuration is preferred in alkenyl having the double bond in an even-numbered position, such as -CH₂CH=CHCH₃, -CH₂CH=CHC₂H₅ and -CH₂CH=CHC₃H₇. An alkenyl compound having the preferred configuration has a high clearing point or a wide temperature range of the liquid crystal phase. A detailed description is found in Mol. Cryst. Liq. Cryst., 1985, 131, 109 and Mol. Cryst. Liq. Cryst., 1985, 131, 327.

Specific examples of the alkyl include -CH₃, -C₂H₅, -C₃H₇, -C₄H₉, -C₅H₁₁, -C₆H₁₃ and -C₇H₁₅.

Specific examples of the alkoxyalkyl include -CH₂OCH₃, -CH₂OC₂H₅, -CH₂OC₃H₇, - (CH₂)₂-OCH₃, - (CH₂)₂-OC₂H₅, - (CH₂)₂-OC₃H₇, - (CH₂)₃-OCH₃, -(CH₂)₄-OCH₃ and - (CH₂)₅-OCH₃.

Specific examples of the alkenyl include -CH=CH₂, -CH=CHCH₃, -CH₂CH=CH₂, -CH=CHC₂H₅, -CH₂CH=CHCH₃, - (CH₂)₂-CH=CH₂, -CH=CHC₃H₇, -CH₂CH=CHC₂H₅, -(CH₂)₂-CH=CHCH₃ and -(CH₂)₃-CH=CH₂.

Specific examples of alkyl in which at least one piece of hydrogen is replaced by halogen include -CH₂F, -CHF₂, -CF₃, -(CH₂)₂-F, -CF₂CH₂F, -CF₂CHF₂, -CH₂CF₃, -CF₂CF₃, -(CH₂)₃-F, -(CF₂)₃-F, -CF₂CHFCF₃, -CHFCF₂CF₃, -(CH₂)₄-F, -(CF₂)₄-F, -(CH₂)₅-F, -(CF₂)₅-F, -CH₂Cl, -CHCl₂, -CCl₃, -(CH₂)₂-Cl, -CCl₂CH₂Cl, -CCl₂CHCl₂, -CH₂CCl₃, -CCl₂CCl₃, -(CH₂)₃-Cl, -(CCl₂)₃-Cl, -CCl₂CHClCCl₃, -CHClCCl₂CCl₃, -(CH₂)₄-Cl, -(CCl₂)₄-Cl, -(CH₂)₅-Cl and -(CCl₂)₅-Cl.

Specific examples of alkenyl in which at least one piece of hydrogen is replaced by halogen include -CH=CHF, -CH=CF₂, -CF=CHF, -CH=CHCH₂F, -CH=CHCF₃, -(CH₂)₂-CH=CF₂, -CH₂CH=CHCF₃, -CH=CHCF₂CF₃, -CH=CHCl, -CH=CCl₂, -CCl=CHCl, -CH=CHCH₂Cl, -CH=CHCCl₃, -(CH₂)₂-CH=CCl₂, -CH₂CH=CHCCl₃ and -CH=CHCCl₂CCl₃.

Preferred examples of R¹ include alkyl having 1 to 10 carbons, alkenyl having 2 to 10 carbons, alkyl having 1 to 10 carbons in which one or two pieces of hydrogen are replaced by fluorine, or alkenyl having 2 to 10 carbons in which one or two pieces of hydrogen are replaced by fluorine. Further preferred examples of R¹ include alkyl having 1 to 7 carbons and alkenyl having 2 to 8 carbons. Most preferred examples of R¹ include -CH₃, -C₂H₅, -C₃H₇, -C₄H₉, -C₅H₁₁, -CH=CH₂, -CH=CHCH₃, -(CH₂)₂-CH=CH₂, -CH₂CH=CHC₂H₅ and -(CH₂)₂-CH=CHCH₃.

In formula (1), ring A¹, ring A² and ring A³ are independently

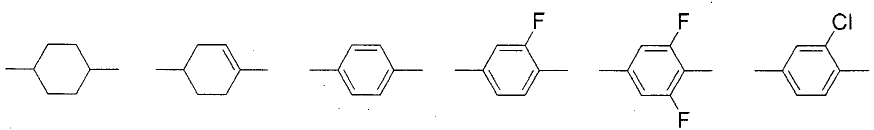

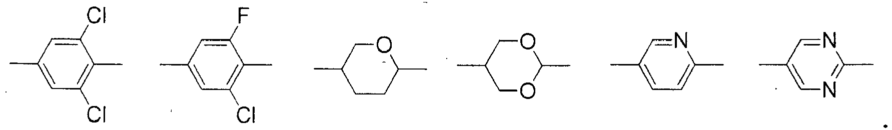

Preferred examples of ring A¹ include

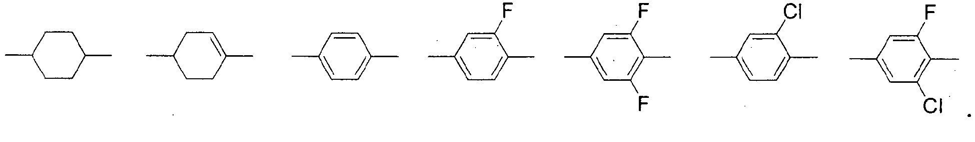

Further preferred examples of ring A¹ include

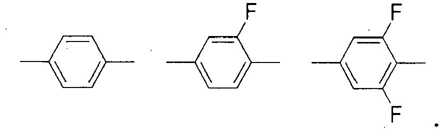

In 1,4-cyclohexylene, the cis configuration and the trans configuration exist. From a viewpoint of high maximum temperature, the trans configuration is preferred.

Preferred examples of ring A² and ring A³ include

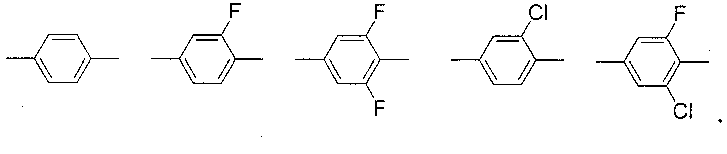

Further preferred examples of ring A² and ring A³ include

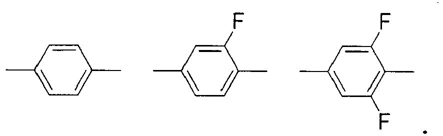

In formula (1), X¹ and X² are independently -O-, -S- or -CH₂-, and a case where both X¹ and X² are -CH₂- is excluded. In a preferred combination of X¹ and X², one of X¹ and X² is -O-, and the other is -CH₂-, or both X¹ and X² are -O-. In a further preferred combination, both X¹ and X² are -O-.

In formula (1), a substituent is hydrogen, fluorine, chlorine, -C=N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

Specific examples of such terminal group Y¹ include alkyl, alkoxy, alkoxyalkyl, alkoxyalkoxy, alkenyl, alkenyloxy, alkenyloxyalkyl and alkoxyalkenyl. In the groups, at least one piece of hydrogen may be replaced by halogen. Preferred halogen is fluorine and chlorine. Further preferred halogen is fluorine. The groups have a straight chain or a branched chain, but include no cyclic group such as cyclohexyl. In the groups, the straight chain is preferred to the branched chain.

A preferred configuration of -CH=CH- in the alkenyl depends on a position of a double bond. A trans configuration is preferred in alkenyl having the double bond in an odd-numbered position, such as -CH=CHCH₃, -CH=CHC₂H₅, -CH=CHC₃H₇, -CH=CHC₄H₉, -C₂H₄CH=CHCH₃ and -C₂H₄CH=CHC₂H₅. A cis configuration is preferred in alkenyl having the double bond in an even-numbered position, such as -CH₂CH=CHCH₃, -CH₂CH=CHC₂H₅ and -CH₂CH=CHC₃H₇. An alkenyl compound having the preferred configuration has the high clearing point or the wide temperature range of the liquid crystal phase. A detailed description is found in Mol. Cryst. Liq. Cryst., 1985, 131, 109 and Mol. Cryst. Liq. Cryst., 1985, 131, 327.

Specific examples of the alkyl include -CH₃, -C₂H₅, -C₃H₇, -C₄H₉, -C₅H₁₁, -C₆H₁₃ and -C₇H₁₅.

Specific examples of the alkoxy include -OCH₃, -OC₂H₅, -OC₃H₇, -OC₄H₉, -OC₅H₁₁ and -OC₆H₁₂.

Specific examples of the alkoxyalkyl include -CH₂OCH₃, -CH₂OC₂H₅, -CH₂OC₃H₇, -(CH₂)₂-OCH₃, -(CH₂)₂-OC₂H₅, -(CH₂)₂-OC₃H₇, -(CH₂)₃-OCH₃, -(CH₂)₄-OCH₃ and -(CH₂)₅-OCH₃.

Specific examples of the alkenyl include -CH=CH₂, -CH=CHCH₃, -CH₂CH=CH₂, -CH=CHC₂H₅, -CH₂CH=CHCH₃, -(CH₂)₂-CH=CH₂, -CH=CHC₃H₇, -CH₂CH=CHC₂H₅, -(CH₂)₂-CH=CHCH₃ and -(CH₂)₃-CH=CH₂.

Specific examples of the alkenyloxy include -OCH₂CH=CH₂, -OCH₂CH=CHCH₃ and -OCH₂CH=CHC₂H₅.

Specific examples of alkyl in which at least one piece of hydrogen is replaced by halogen include -CH₂F, -CHF₂, -CF₃, -(CH₂)₂-F, -CF₂CH₂F, -CF₂CHF₂, -CH₂CF₃, -CF₂CF₃, -(CH₂)₃-F, -(CF₂)₃-F, -CF₂CHFCF₃, -CHFCF₂CF₃, -(CH₂)₄-F, -(CF₂)₄-F, -(CH₂)₅-F, -(CF₂)₅-F, -CH₂Cl, -CHCl₂, -CCl₃, -(CH₂)₂-Cl, -CCl₂CH₂Cl, -CCl₂CHCl₂, -CH₂CCl₃, -CCl₂CCl₃, -(CH₂)₃-Cl, -(CCl₂)₃-Cl, -CCl₂CHClCCl₃, -CHClCCl₂CCl₃, -(CH₂)₄-Cl, -(CCl₂)₄-Cl, -(CH₂)₅-Cl and -(CCl₂)₅-Cl.

Specific examples of alkoxy in which at least one piece of hydrogen is replaced by halogen include -OCH₂F, -OCHF₂, -OCF₃, -O-(CH₂)₂-F, -OCF₂CH₂F, -OCF₂CHF₂, -OCH₂CF₃, -O-(CH₂)₃-F, -O-(CF₂)₃-F, -OCF₂CHFCF₃, -OCHFCF₂CF₃, -O(CH₂)₄-F, -O-(CF₂)₄-F, -O-(CH₂)₅-F, -O-(CF₂)₅-F, -OCH₂Cl, -OCHCl₂, -OCCl₃, -O-(CH₂)₂-Cl, -OCCl₂CH₂Cl, -OCCl₂CHCl₂, -OCH₂CCl₃, -O-(CH₂)₃-Cl, -O-(CCl₂)₃-Cl, -OCCl₂CHClCCl₃, -OCHClCCl₂CCl₃, -O(CH₂)₄-Cl, -O-(CCl₂)₄-Cl, -O-(CH₂)₅-Cl and -O-(CCl₂)₅-Cl.

Specific examples of alkenyl in which at least one piece of hydrogen is replaced by halogen include -CH=CHF, -CH=CF₂, -CF=CHF, -CH=CHCH₂F, -CH=CHCF₃, -(CH₂)₂-CH=CF₂, -CH₂CH=CHCF₃, -CH=CHCF₂CF₃, -CH=CHCl, -CH=CCl₂, -CCl=CHCl, -CH=CHCH₂Cl, -CH=CHCCl₃, -(CH₂)₂-CH=CCl₂, -CH₂CH=CHCCl₃ and -CH=CHCCl₂CCl₃.

Specific examples of alkenyloxy in which at least one piece of hydrogen is replaced by halogen include -OCH=CHF, -OCH=CF₂, -OCF=CHF, -OCF=CF₂, -OCH=CHCH₂F, -OCH=CHCF₃, -O(CH₂)₂-CH=CF₂, -OCH₂CH=CHCF₃, -OCH=CHCF₂CF₃, -OCH=CHCl, -OCH=CCl₂, -OCCl=CHCl, -OCH=CHCH₂Cl, -OCH=CHCCl₃, -O(CH₂)₂CH=CCl₂, O-CH₂CH=CHCCl₃ and -OCH=CHCCl₂CCl₃.

Preferred examples of Y¹ include hydrogen, fluorine, chlorine, -C=N, -N=C=S, alkyl having 1 to 5 carbons in which at least one piece of hydrogen is replaced by halogen, alkoxy having 1 to 5 carbons in which at least one piece of hydrogen is replaced by halogen, alkenyl having 2 to 5 carbons in which at least one piece of hydrogen is replaced by halogen, and alkenyloxy having 2 to 5 carbons in which at least one piece of hydrogen is replaced by halogen. Further preferred examples of Y¹ include hydrogen, fluorine, chlorine, -C=N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂ or -OCH=CHCF₃. Most preferred examples of Y¹ include hydrogen, fluorine, chlorine, -C=N, -CF₃, -OCF₃, OCH=CF₂ and -OCH=CHCF₃.

In formula (1), Z¹ and Z³ are independently a single bond, -CH₂CH₂-, -CH₂O-, -CF₂O-, -OCF₂-, -COO-, -OCO-, -CH=CH- and -C≡C-, and Z² is a single bond, -CF₂O- or -COO-.

Preferred examples of Z¹ and Z³ include a single bond, -CF₂O-, -COO-, -CH=CH- and -C=C-. Further preferred examples of Z¹ and Z³ include a single bond, -CF₂O- and -COO-. In a preferred combination of Z¹, Z² and Z³, Z¹ and Z³ are a single bond, Z² is -CF₂O-, and all of Z¹, Z² and Z³ are a single bond.

In formula (1), L¹ and L² are independently hydrogen or halogen. Preferred halogen is fluorine and chlorine. Further preferred halogen is fluorine. In a preferred combination of L¹ and L², one of L¹ and L² is hydrogen, and the other is fluorine. In a further preferred combination of L¹ and L², both L¹ and L² are fluorine.

In formula (1), a is 0, 1, 2 or 3. Preferred a is 0, 1 or 2. Further preferred a is 1 or 2. From a viewpoint of small viscosity, preferred a is 0. From a viewpoint of the large dielectric anisotropy, preferred a is 1 or 2.

In formula (1), n¹ and n² are independently 0, 1 or 2, wherein n¹ + n² ≤ 2. From a viewpoint of the small viscosity, in preferred combination of n¹ and n², both n¹ and n² are 0. From a viewpoint of compatibility with other liquid crystal compounds and the large dielectric anisotropy, in a preferred combination of n¹ and n², one of n¹ and n² is 0, and the other is 1. From a viewpoint of the high maximum temperature and the large dielectric anisotropy, in a preferred combination of preferred n¹ and n², n¹ is 2 and n² is 0, or n¹ is 1 and n² is 1.

### 1-2. Physical properties of compound (1)

In compound (1), Physical properties such as the clearing point, optical anisotropy and the dielectric anisotropy can be arbitrarily adjusted by suitably combining types of R¹, ring A¹, ring A², ring A³, X¹, X², Y¹, Z¹, Z², Z³, L¹ and L². Compound (1) may contain a larger amount of isotope such as ²H (deuterium) and ¹³C than the amount of natural abundance because no significant difference exists in the physical properties of the compound. A main effect of a type of R¹ or the like on the physical properties of compound (1) will be described below.

When left-terminal group R¹ has the straight chain, a temperature range of the liquid crystal phase is wide and the viscosity is small. When R¹ has the branched chain, the compatibility with other liquid crystal compounds is good.

A compound in which R¹ is optically active is useful as a chiral dopant. A reverse twisted domain to be generated in the liquid crystal display device can be prevented by adding the compound to the composition. A compound in which R¹ is not optically active is useful as a component of the composition. When R¹ is alkenyl, a preferred configuration depends on a position of a double bond. An alkenyl compound having the preferred configuration has the small viscosity, the high maximum temperature or the wide temperature range of the liquid crystal phase.

When at least one of ring A¹, ring A² and ring A³ is 1, 4-cyclohexylene, the clearing point is high and the viscosity is small. When at least one of ring A¹, ring A² and ring A³ is 1,4-phenylene, or 1,4-phenylene in which at least one piece of hydrogen is replaced by halogen, the optical anisotropy is comparatively large and an orientational order parameter is comparatively large. When all of ring A¹, ring A² and ring A³ are 1,4-phenylene, 1,4-phenylene in which at least one piece of hydrogen is replaced by halogen, or a combination thereof, the optical anisotropy is particularly large. When at least one of ring A¹, ring A² and ring A³ is 1,4-phenylene in which at least one piece of hydrogen is replaced by halogen, tetrahydropyran-2,5-diyl, pyrimidine-2, 5-diyl, pyridine-2,5-diyl and 1, 3-dioxane-2, 5-diyl, the dielectric anisotropy is large.

When right-terminal group Y¹ is fluorine or -OCH=CF₂, the dielectric anisotropy is large, and the viscosity is small. When right-terminal group Y¹ is -CF₃ or -OCH=CHCF₃, the dielectric anisotropy is particularly large. When right-terminal group Y¹ is -OCF₃, the dielectric anisotropy is large, and the compatibility with other compounds is high. When right-terminal group Y¹ is chlorine, the refractive index anisotropy is large. When right-terminal group Y¹ is -C=N, the dielectric anisotropy is large, and the refractive index anisotropy is large.

When bonding group Z¹ or Z³ is a single bond, -CH₂CH₂-, -CH=CH- or -CF₂O-, the viscosity is small. When Z¹ or Z³ is -CH=CH- or -CH₂O-, the temperature range of the liquid crystal phase is wide, and an elastic constant (K) is large. When Z¹ or Z³ is -CH=CH- or -C=C-, the optical anisotropy is large. When Z¹, Z² or Z³ is -CF₂O- or -COO-, the dielectric anisotropy is large. When Z¹ or Z³ is a single bond, -CH₂CH₂- or -CH₂O-, chemical stability is high.

When one of L¹ and L² is fluorine, the dielectric anisotropy is large. When both L¹ and L² are fluorine, the dielectric anisotropy is particularly large.

As described above, a compound having objective physical properties can be obtained by suitably selecting a type of the ring structure, the terminal group, the bonding group or the like. Accordingly, compound (1) is useful as a component of a liquid crystal composition used in a liquid crystal display device having a mode such as the PC mode, the TN mode, the STN mode, the ECB mode, the OCB mode, the IPS mode and the VA mode.

### 1-3. Preferred compound

Preferred examples of compound (1) include a compound represented by formula (1-1): 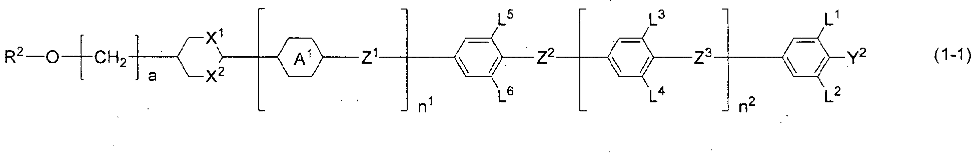 wherein, in formula (1-1),
R² is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
ring A¹ is represented by a formula described below; 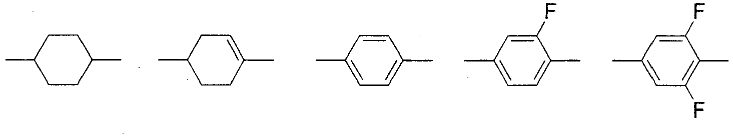 wherein, X¹ and X² are independently -O- or -CH₂-, and a case where both X¹ and X² are -CH₂- is excluded;
Y² is hydrogen, fluorine, chlorine, -C≡N, -N=C=S, -CH₂F, -CHF₂, -CF₃, -OCH₂F, -OCHF₂, -OCF₃, -CH=CHF, -CH=CF₂, -CF=CHF, -OCH=CF₂, -OCF=CF₂ or -OCH=CHCF₃;
Z¹ and Z³ are independently a single bond, -CH₂CH₂-, -CH₂O-, -CF₂O-, -OCF₂-, -COO-, -OCO-, -CH=CH- or -C≡C-;
Z² is a single bond, -CF₂O- or -COO-;
L¹ L², L³, L⁴, L⁵ and L⁶ are independently hydrogen, fluorine or chlorine;
a is 0, 1, 2 or 3; and
n¹ and n² are independently 0, 1 or 2, and an expression: n¹ + n² ≤ 2 holds; and
when n¹ + n² = 0 and Z² is -CF₂O, Y² is hydrogen, fluorine, chlorine, -SF₅, -CH₂F, -CHF₂, -CF₃, -OCH₂F, -OCHF₂, -OCF₃, -CH=CHF, -CH=CF₂, -CF=CHF, -OCH=CF₂, -OCF=CF₂ or -OCH=CHCF₃.

Further preferred examples of compound (1-1) include a compound represented by formula (1-1-1): 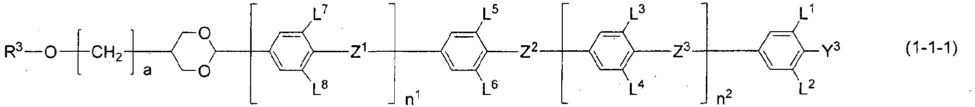 wherein, in formula (1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C=N, -N=C=S, -CF₃, -OCF₃, -OCH=CF₂ or -OCH=CHCF₃;
Z¹ and Z³ are independently a single bond, -CH₂O-, -CF₂O-, -COO-, -CH=CH- or -C≡C-;
Z² is a single bond, -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds; and
when n¹ + n² = 0 and Z² is -CF₂O, Y³ is hydrogen, fluorine, chlorine, -SF₅, -CF₃, -OCF₃, -OCH=CF₂ or -OCH=CHCF₃.

One of most preferred examples of compound (1-1-1) is compounds (1-1-1-1-1) to (1-1-1-1-5): 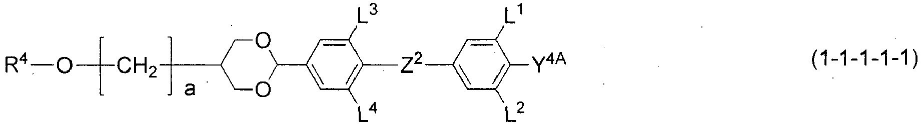 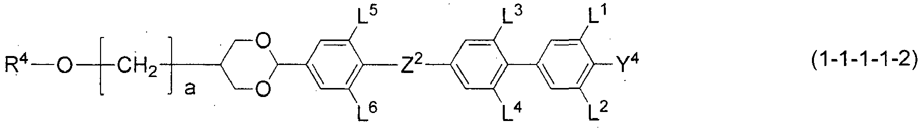 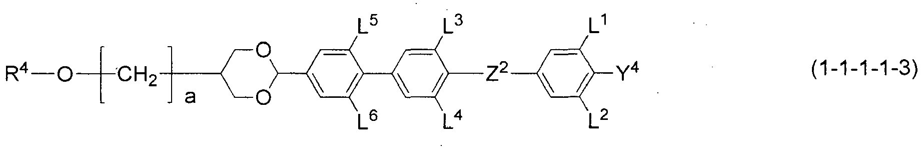 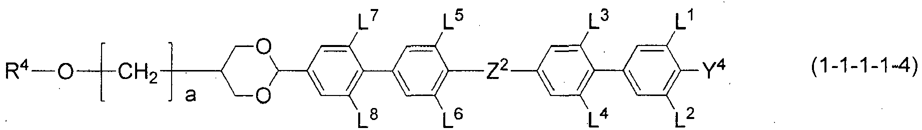 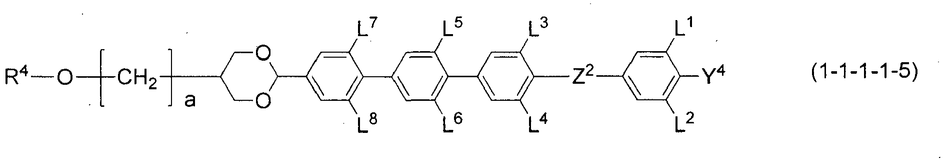 wherein, in formulas (1-1-1-1-1) to (1-1-1-1-5),
R⁴ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁴ is hydrogen, fluorine, chlorine, -C=N, -CF₃, -OCF₃, -OCH=CF₂ or -OCH=CHCF₃;
Y^{4A} is hydrogen, fluorine, chlorine, -CF₃, -OCF₃, -OCH=CF₂ or -OCH=CHCF₃;
Z² is -CF₂O- or -COO-;
L¹, L², L³, L⁴, L⁵, L⁶, L⁷ and L⁸ are independently hydrogen or fluorine; and
a is 0, 1, 2 or 3.

Another of most preferred examples of compound (1-1-1) is compound (1-1-1-1-11) or (1-1-1-1-12): 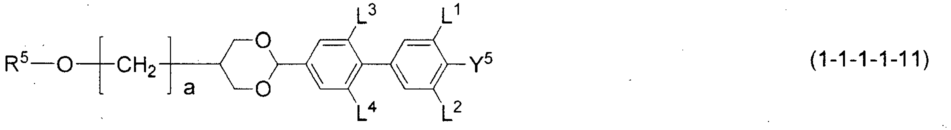 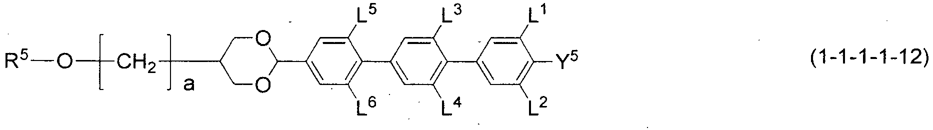 wherein, in formulas (1-1-1-1-11) and (1-1-1-1-12),
R⁵ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁵ is hydrogen, fluorine, chlorine, -C=N, -CF₃, -OCF₃, -OCH=CF₂ or -OCH=CHCF₃;
L¹, L², L³, L⁴, L⁵ and L⁶ are independently hydrogen or fluorine; and
a is 0, 1, 2 or 3.

### 1-4. Synthesis of compound (1)

A synthesis method of compound (1) will be described. Compound (1) can be prepared by suitably combining methods in synthetic organic chemistry. Methods for introducing an objective terminal group, ring and bonding group into a starting material are described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course (Shin Jikken Kagaku Koza in Japanese)" (Maruzen Co., Ltd.).

### 1-4-1. Formation of a bonding group

An example of a method for forming a bonding group in compound (1) is as described in a scheme below. In the scheme, MSG¹ (or MSG²) is a monovalent organic group having at least one ring. Monovalent organic groups represented by a plurality of MSG¹ (or MSG²) may be identical or different. Compounds (1A) to (1G) correspond to compound (1) or an intermediate of compound (1).

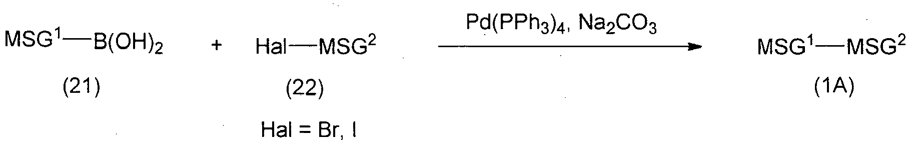

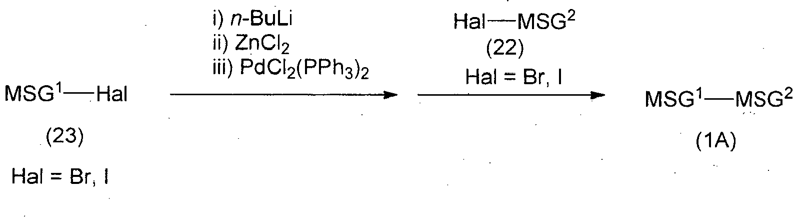

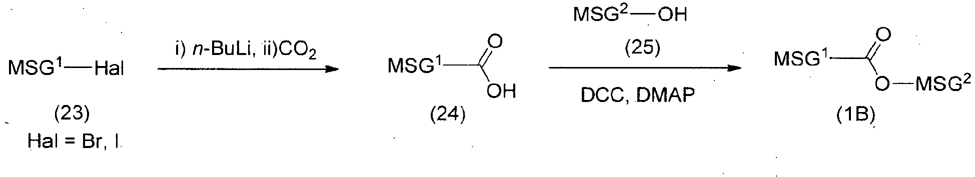

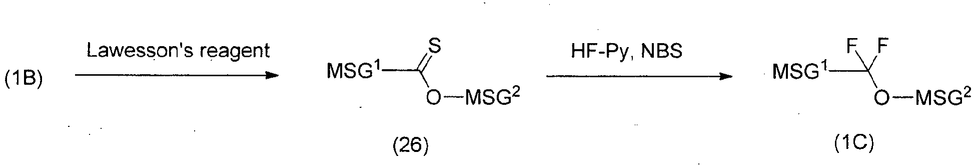

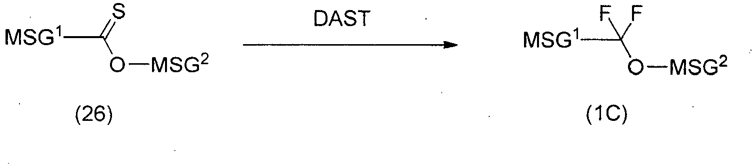

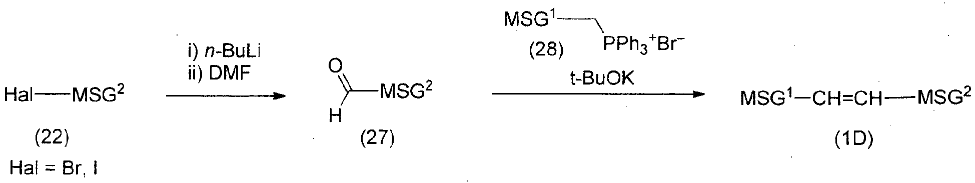

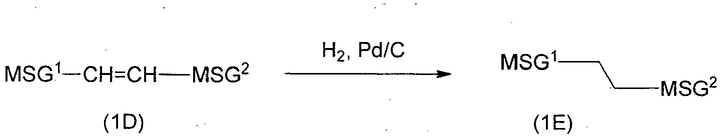

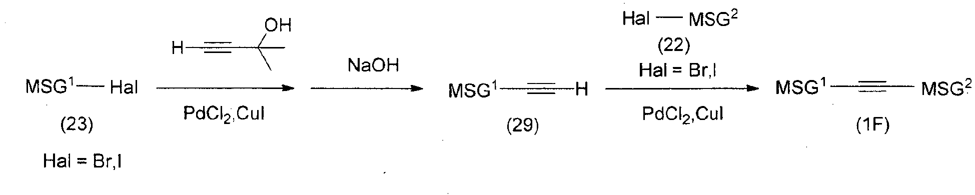

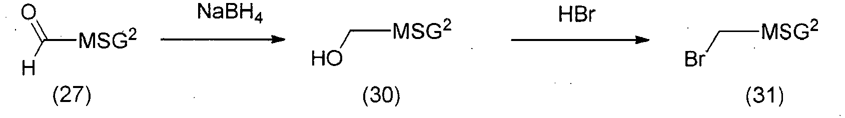

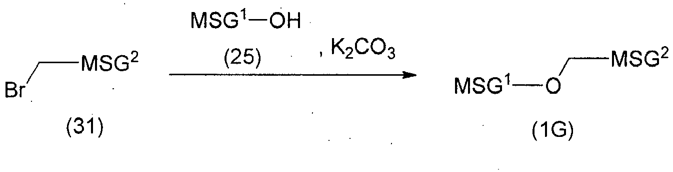

### (I) Formation of a single bond

Compound (1A) is prepared by allowing aryl boronic acid (21) to react with compound (22) in the presence of a carbonate and a tetrakis(triphenylphosphine)palladium catalyst. Compound (1A) is also prepared by allowing compound (23) to react with n-butyllithium and subsequently with zinc chloride, and further with compound (22) in the presence of a dichlorobis(triphenylphosphine)palladium catalyst.

### (II) Formation of -COO- and -OCO-

Carboxylic acid (24) is obtained by allowing compound (23) to react with n-butyllithium and subsequently with carbon dioxide. Compound (1B) having -COO- is prepared by dehydration of carboxylic acid (24) and phenol (25) derived from compound (21) in the presence of 1,3-dicyclohexylcarbodiimide (DCC) and 4-dimethylaminopyridine (DMAP). A compound having -OCO- is also prepared according to the method.

### (III) Formation of -CF₂O- and -OCF₂-

Compound (26) is obtained by sulfurating compound (1B) with a Lawesson's reagent. Compound (1C) having -CF₂O- is prepared by fluorinating compound (26) with a hydrogen fluoride-pyridine complex and N-bromosuccinimide (NBS). Refer to M. Kuroboshi et al., Chem. Lett., 1992, 827. Compound (1C) is also prepared by fluorinating compound (26) with (diethylamino) sulfur trifluoride (DAST). Refer to W. H. Bunnelle et al., J. Org. Chem. 1990, 55, 768. A compound having -OCF₂- is also prepared according to the method.

### (IV) Formation of -CH=CH-

Aldehyde (27) is obtained by allowing compound (22) to react with n-butyllithium and subsequently with N,N-dimethylformamide (DMF). Compound (1D) is prepared by allowing phosphorus ylide generated by allowing phosphonium salt (28) to react with potassium t-butoxide to react with aldehyde (27). A cis isomer may be generated depending on reaction conditions, and therefore the cis isomer is isomerized into a trans isomer according to a publicly known method when necessary.

### (V) Formation of -CH₂CH₂-

Compound (1E) is prepared by hydrogenating compound (1D) in the presence of a palladium on carbon catalyst.

### (VI) Formation of -C=C-

Compound (29) is obtained by allowing compound (23) to react with 2-methyl-3-butyn-2-ol in the presence of a catalyst of dichloropalladium and copper iodide and then performing deprotection of the resulting compound under basic conditions. Compound (1F) is prepared by allowing compound (29) to react with compound (22) in the presence of a catalyst of dichlorobis(triphenylphosphine)palladium and copper halide.

### (VII) Formation of -CH₂O- and -OCH₂-

Compound (30) is obtained by reducing compound (27) with sodium borohydride. Compound (31) is obtained by brominating the obtained compound with hydrobromic acid. Compound (1G) is prepared by allowing compound (25) to react with compound (31) in the presence of potassium carbonate. A compound having -OCH₂- is also prepared according to the method.

### 1-4-2. Formation of ring A¹ and ring A²

A starting material is commercially available or a synthetic method is well known with regard to a ring such as 1,4-cyclohexylene, 1,4-phenylene, 2-fluoro-1,4-phenylene, 2,6-difluoro-1,4-phenylene, tetrahydropyran-2,5-diyl, 1,3-dioxane-2,5-diyl, pyrimidine-2,5-diyl and pyridine-2,5-diyl.

### 1-4-3. Synthesis Example 1 (case where X¹ and X² are identical)

When X¹ and X² are identical, an example of a method for preparing compound (1) is as described below. Compound (41) is prepared with reference to a method described in "EuropeanJournal of Medical Chemistry, 44, 2009, 239-250" or the like. Compound (43) is obtained from compound (41) by constructing a dioxane ring according to a synthesis method known in general. A dithiane ring can also be obtained according to a similar synthesis method. Compound (1) is prepared from compound (43) according to formation methods (I) to (VII) of a bonding group.

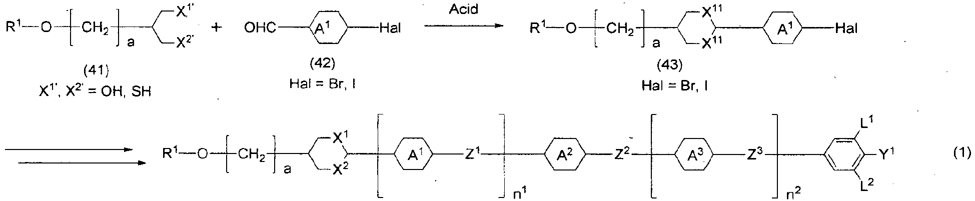

Compound (1) can also be prepared by constructing the dioxane ring or the dithiane ring in a last step. Compound (1) is prepared by allowing compound (41) to react with compound (44) prepared according to the formation methods (I) to (VII) of a bonding group.

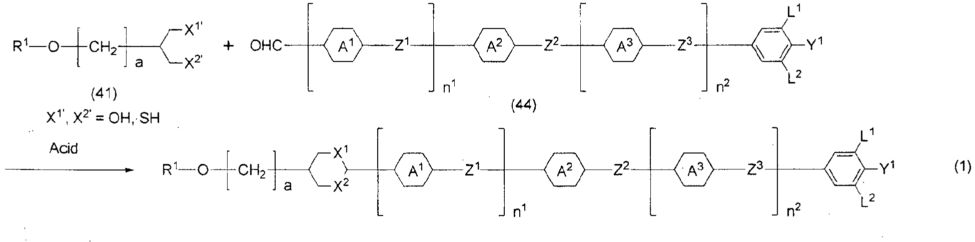

In the compounds, definitions of R¹, ring A¹, ring A², ring A³, X¹, X², Y¹, Z¹, Z², Z³, L¹, L², a, n¹ and n² are identical to definitions described above.

### 1-4-4. Synthesis Example 1 (case where X¹ and X² are different)

When X¹ and X² are different, compound (1) is prepared with reference to a method described in EP 1482019 A, WO 2004/106460 A, CN 103555344 A, "Eur. J. Org. Chem. 2006, 3326-3331" or the like.

### 2. Composition (1)

### 2-1. Compounds (2) to (16)

Liquid crystal composition (1) of the invention will be described. Composition (1) contains at least one compound (1) as component A. Composition (1) may contain two or more compounds (1). A component in a liquid crystal compound may be compound (1) only. In order to develop excellent physical properties, the composition (1) preferably contains at least one compound (1) in the range of 1 to 99% by weight. In a composition having positive dielectric anisotropy, a preferred content of compound (1) is in the range of 5 to 60% by weight. In a composition having negative dielectric anisotropy, a preferred content of compound (1) is 30% by weight or less. Composition (1) may contain compound (1) and various liquid crystal compounds that are not described herein.

A preferred composition contains a compound selected from components B, C, D and E shown below. When composition (1) is prepared, components can also be selected, for example, by taking into account dielectric anisotropy of compound (1). When a composition having positive dielectric anisotropy is prepared for the TFT mode, the IPS mode, the FFS mode or the like, main components include components A, B and E. When a composition having positive dielectric anisotropy is prepared for the STN mode, the TN mode or the like, main components include components A, C and E. When a composition having negative dielectric anisotropy is prepared for the VA mode, the PSA mode or the like, main components include components D and E, and component A is added for the purpose of adjusting a voltage-transmittance curve of a device. A composition in which the components are suitably selected has the high maximum temperature, low minimum temperature, the small viscosity, suitable optical anisotropy, the large dielectric anisotropy and a suitable elastic constant.

Component B includes compounds (2) to (5). Component C includes compound (6). Component D includes compounds (7) to (13). Component E includes compounds (14) to (16). The components will be described in the order.

Component B is a compound having hydrogen, a halogen-containing group or a fluorine-containing group at a right terminal. Specific examples of preferred component B include compounds (2-1) to (2-16), compounds (3-1) to (3-113), compounds (4-1) to (4-49), compounds (4A-1) to (4A-12), compounds (4B-1) to (4B-8), compounds (4C-1) to (4C-4), compounds (4D-1) to (4D-6) and compounds (5-1) to (5-56). In the compounds, definitions of R¹¹ and X¹¹ are identical to definitions described above.

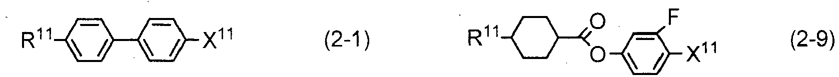

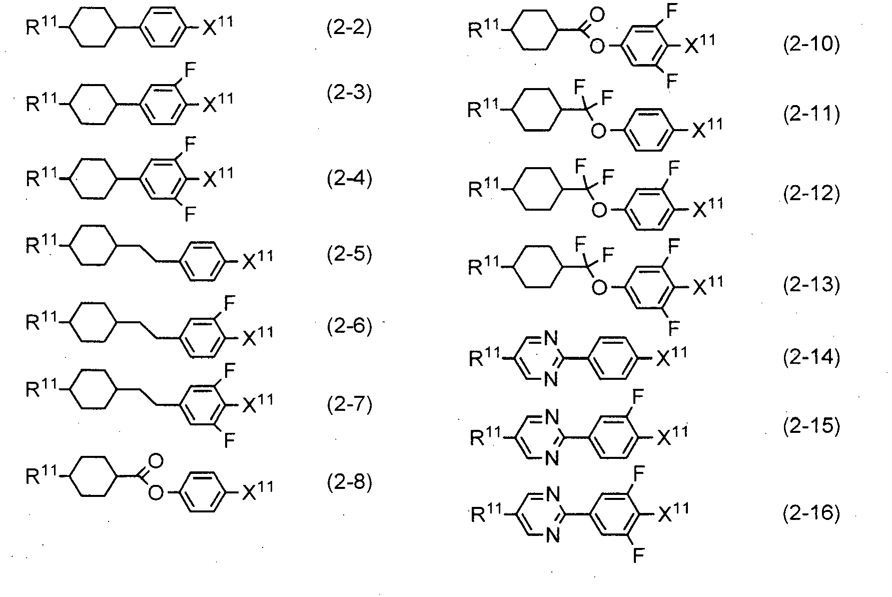

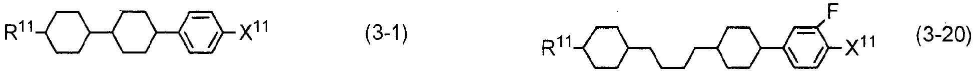

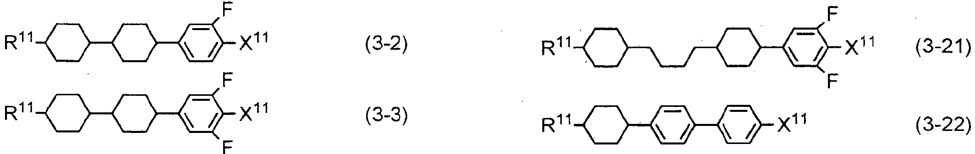

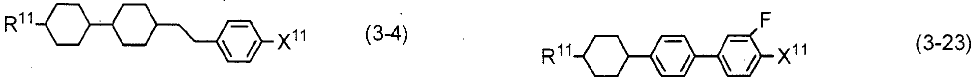

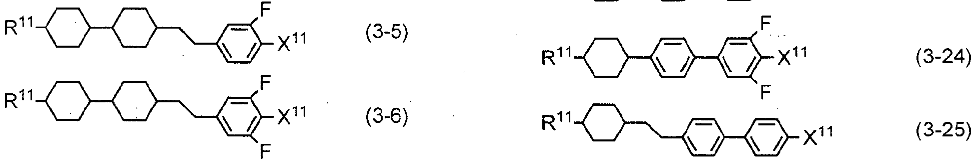

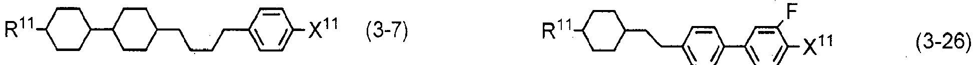

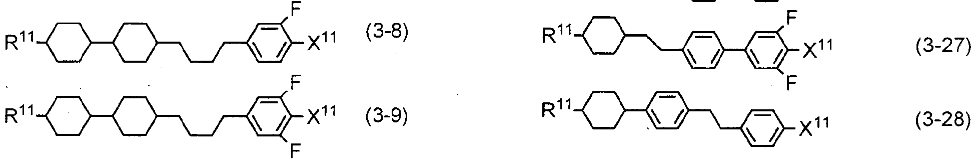

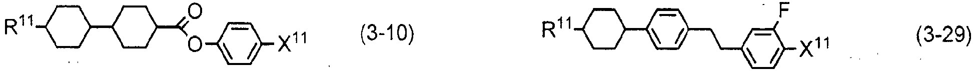

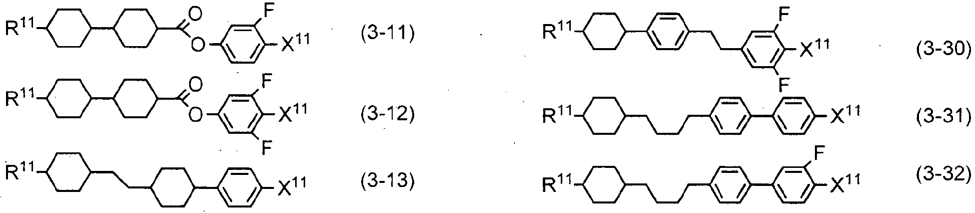

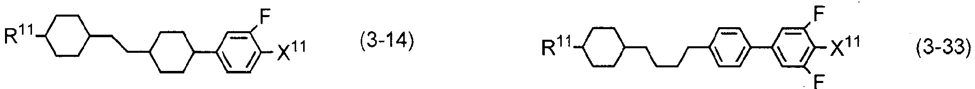

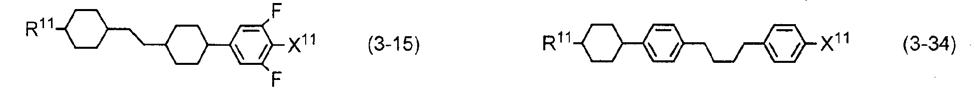

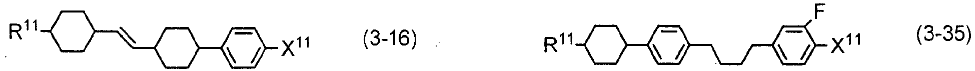

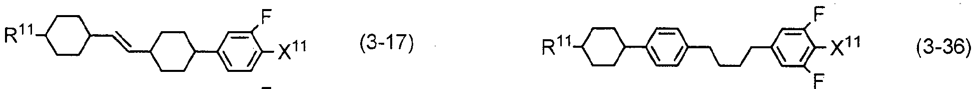

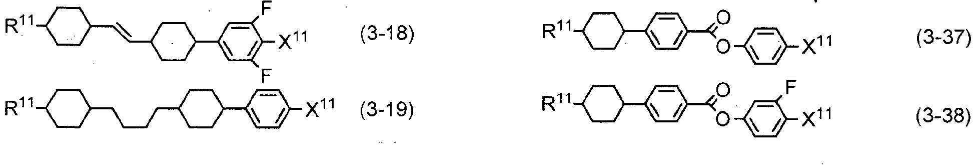

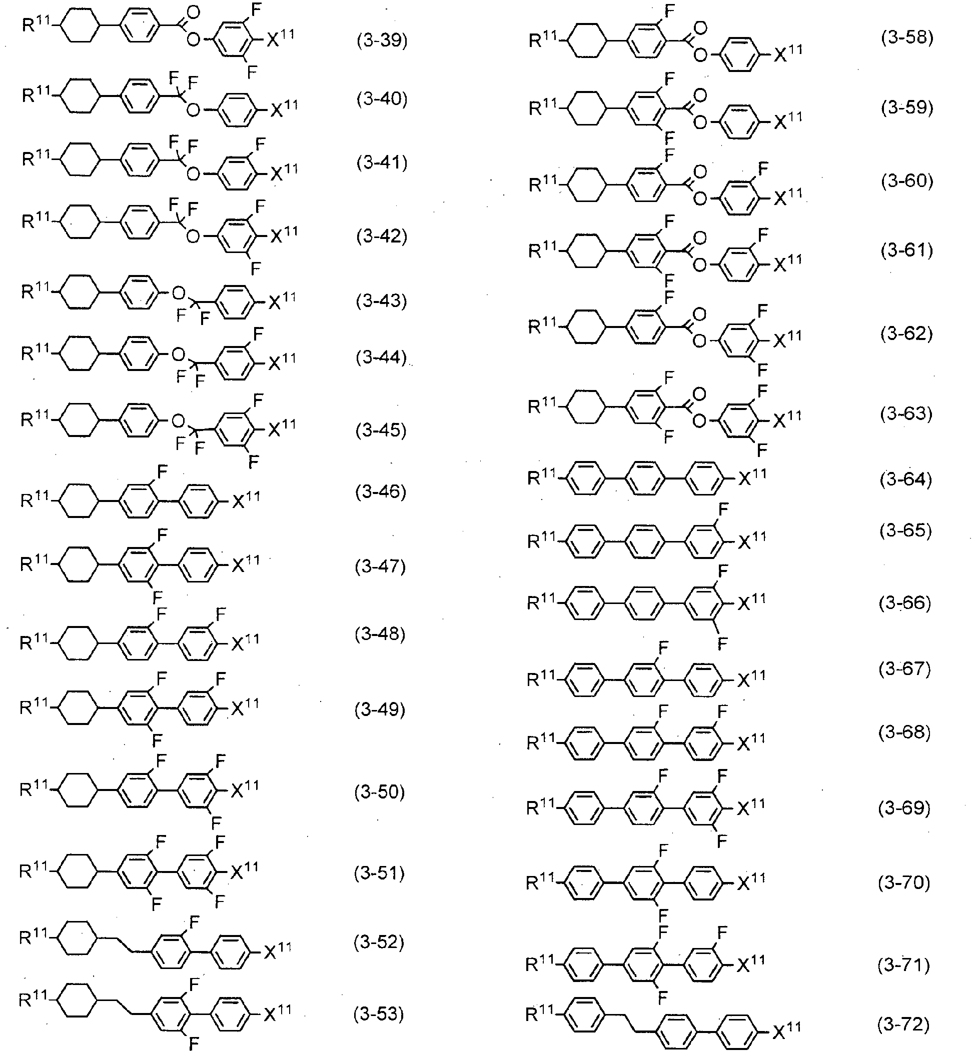

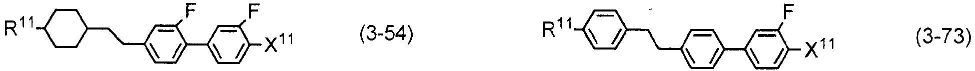

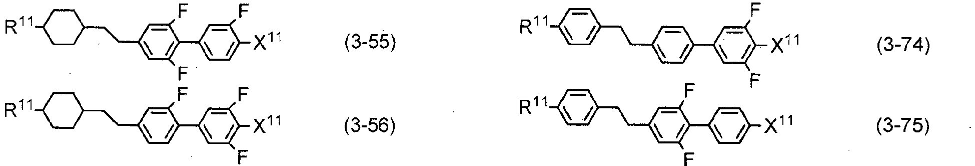

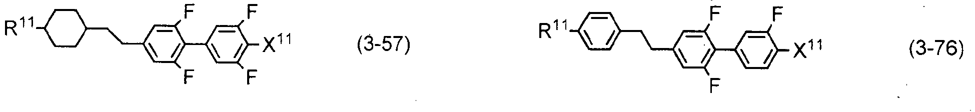

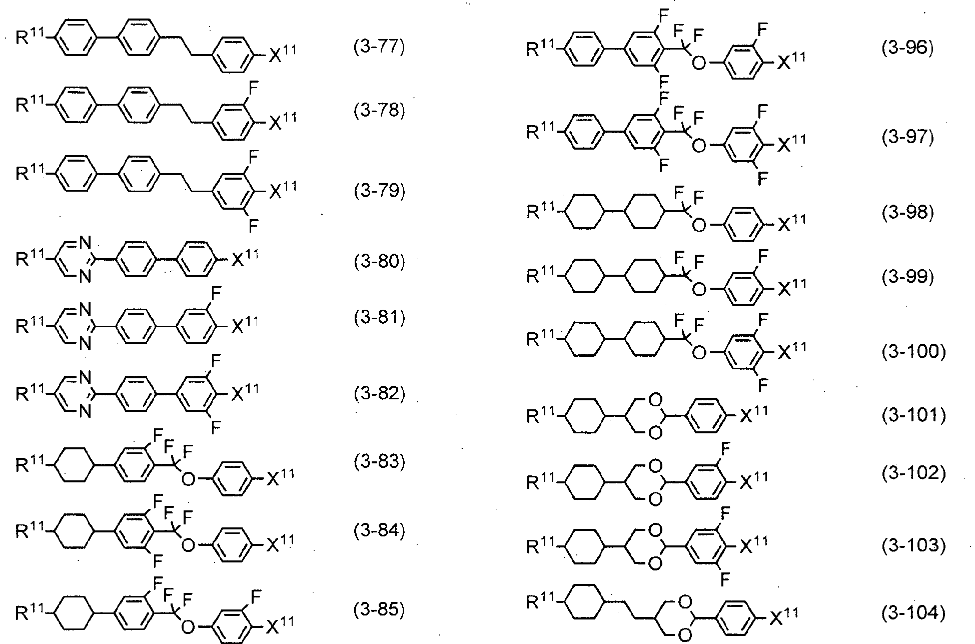

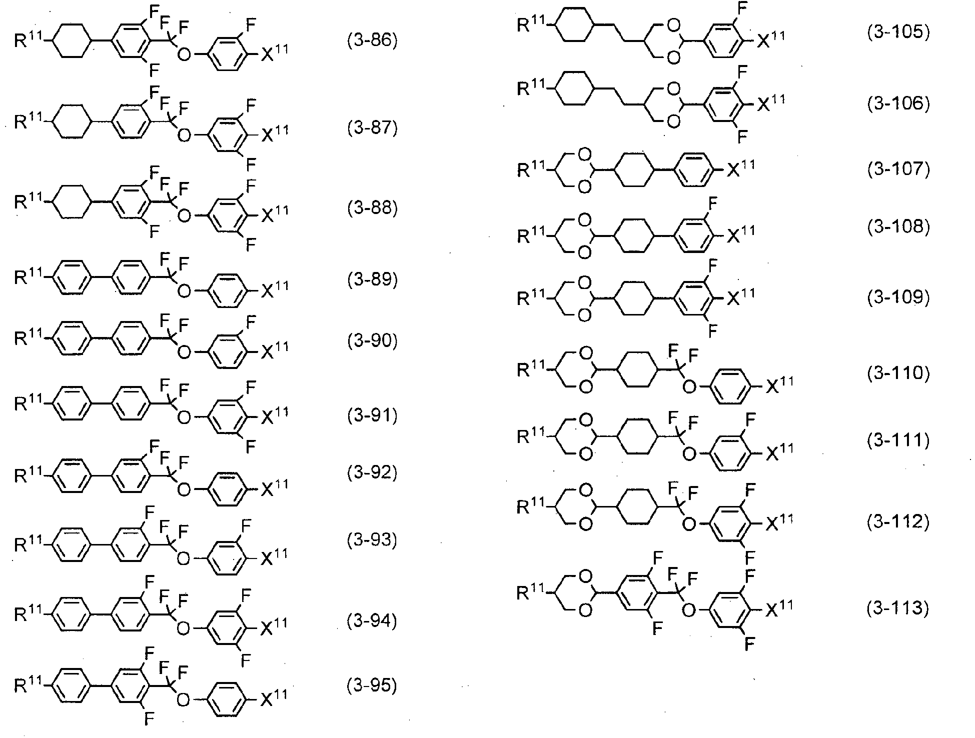

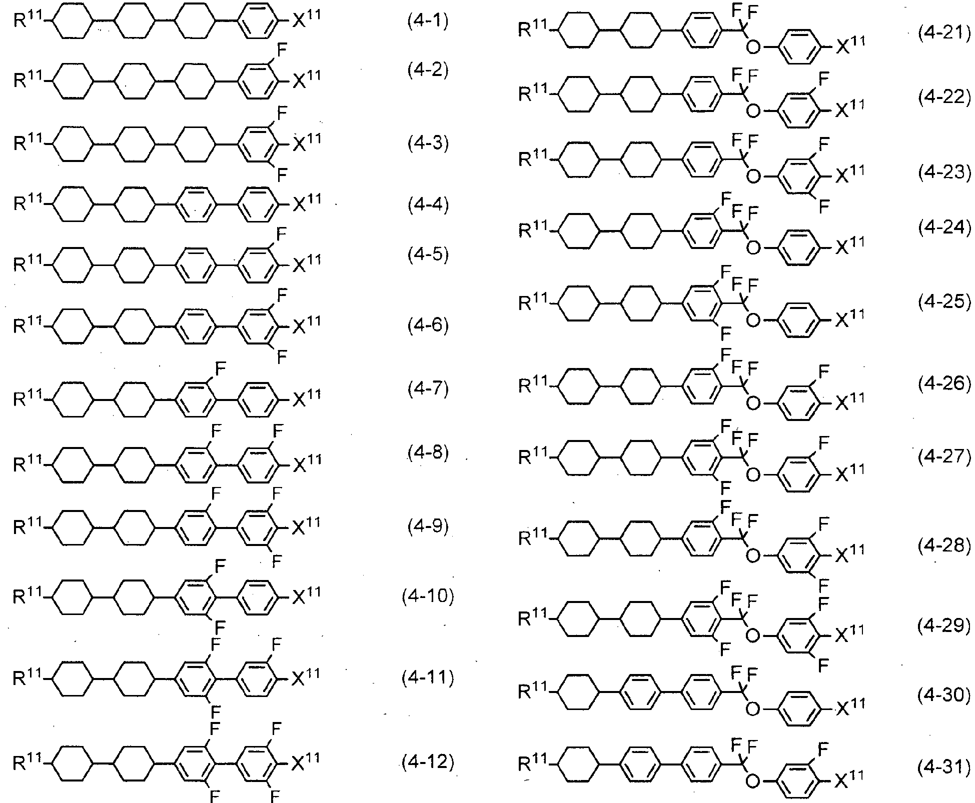

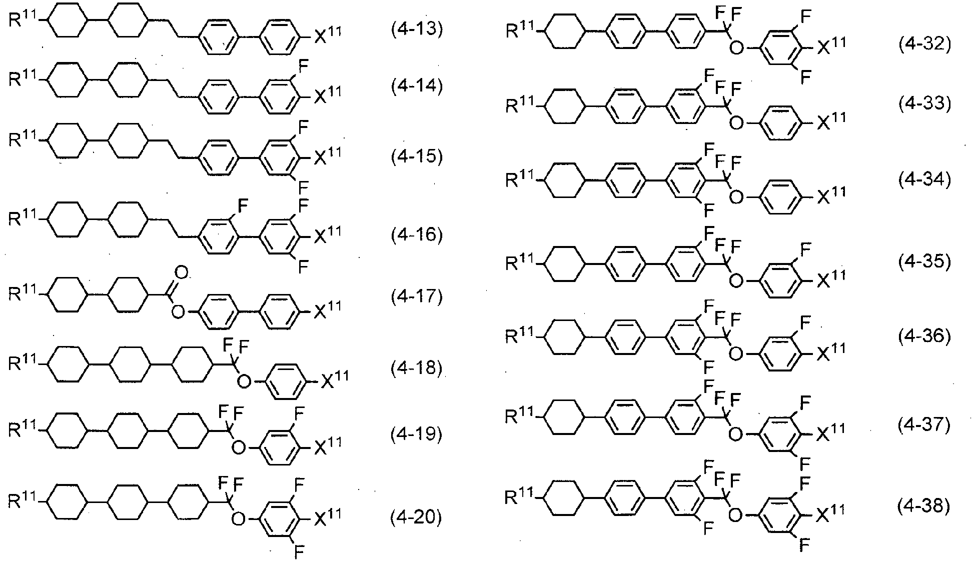

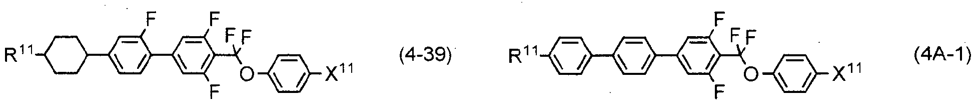

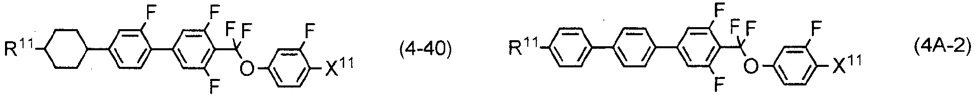

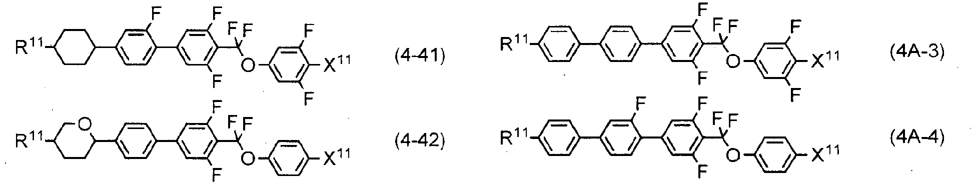

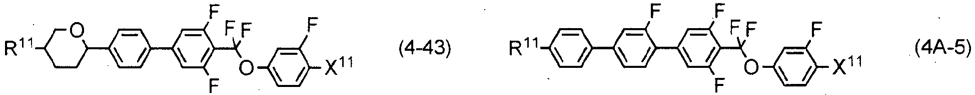

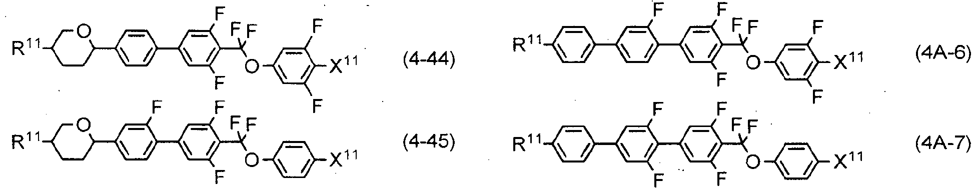

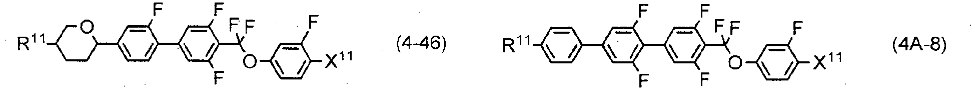

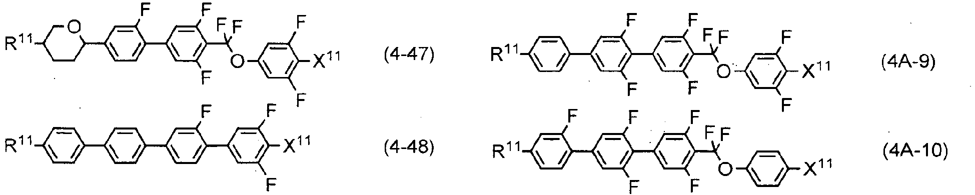

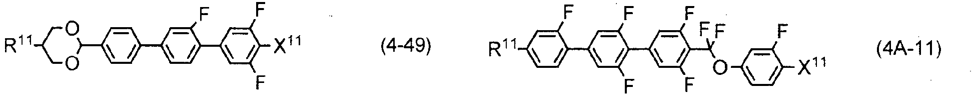

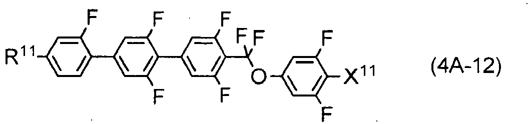

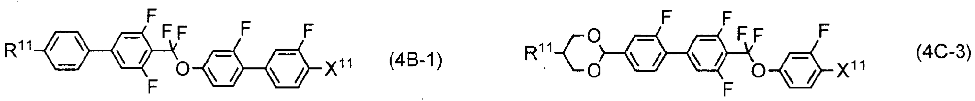

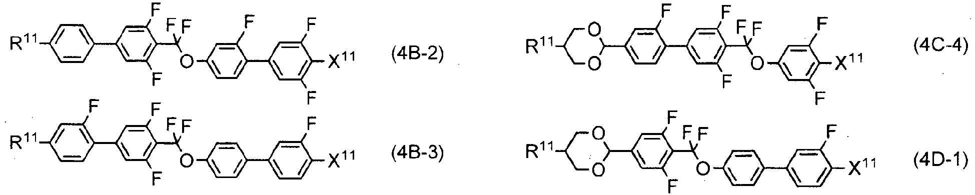

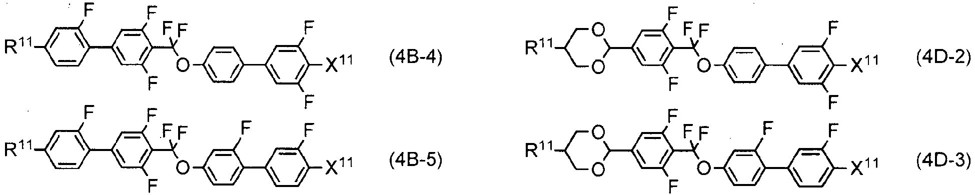

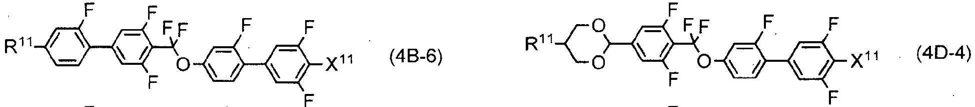

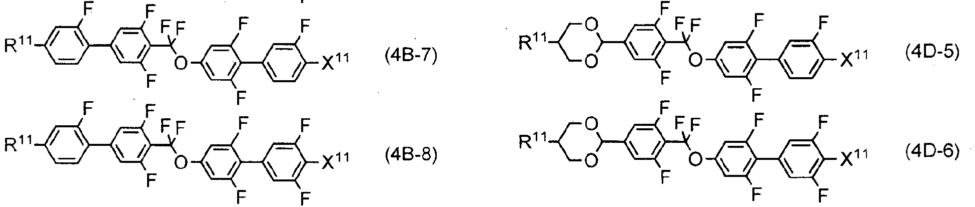

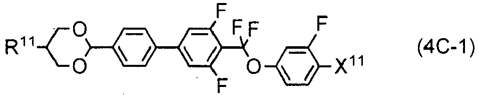

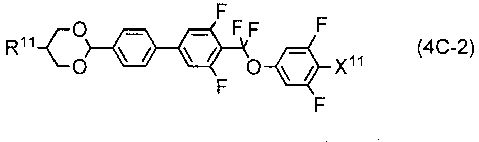

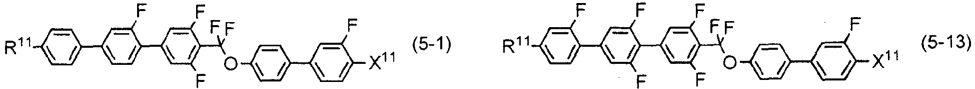

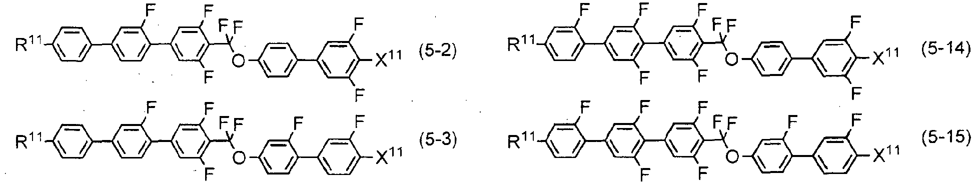

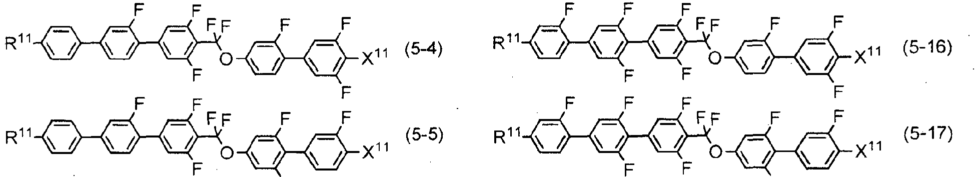

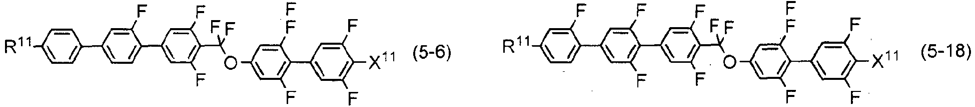

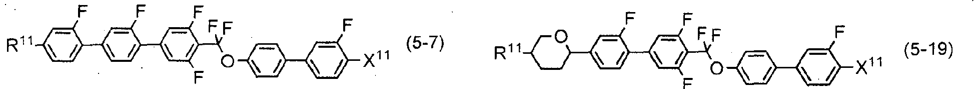

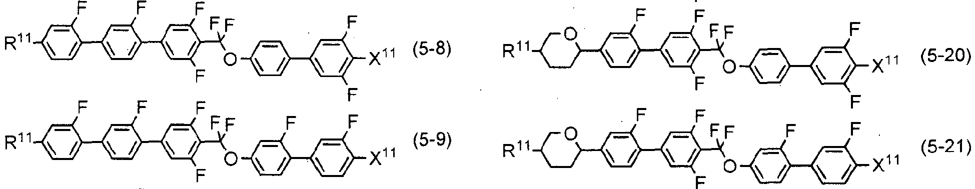

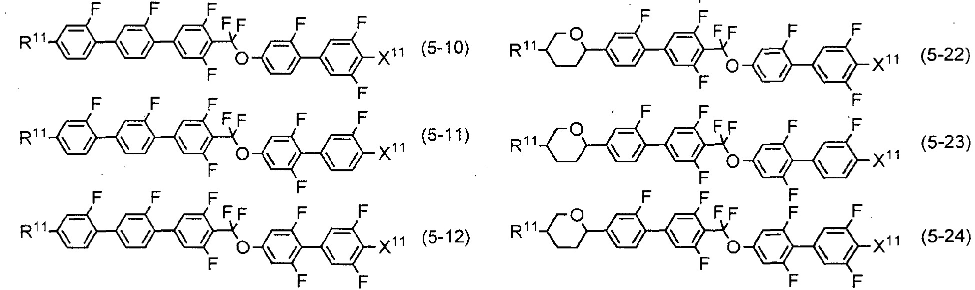

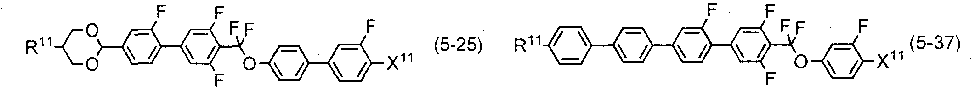

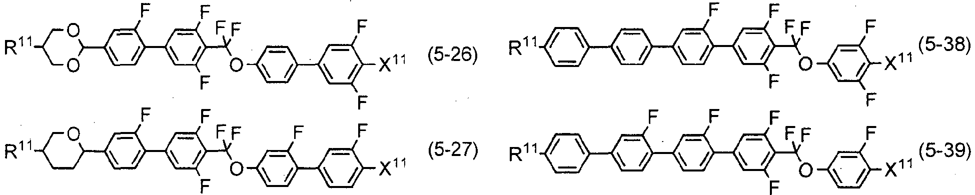

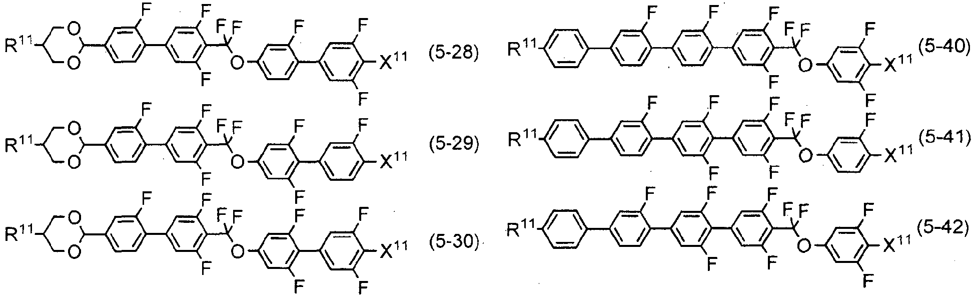

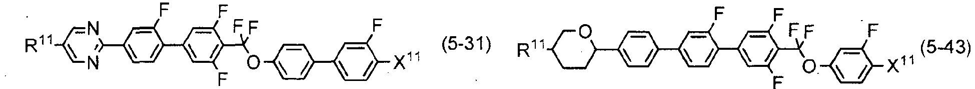

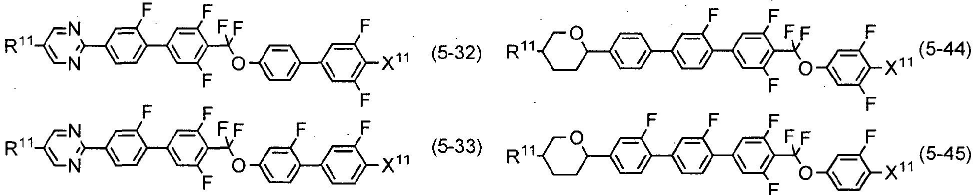

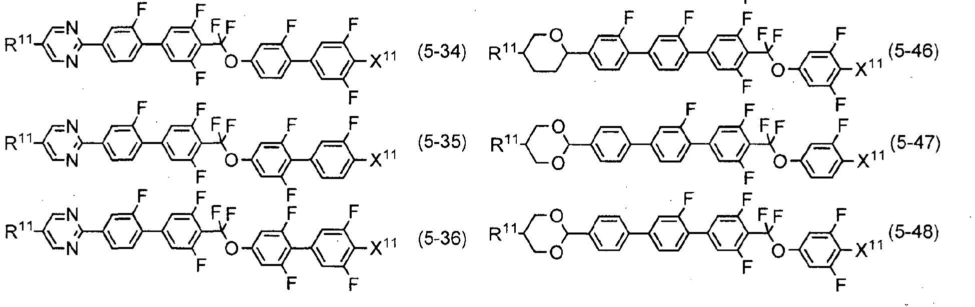

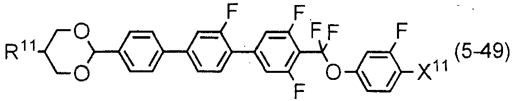

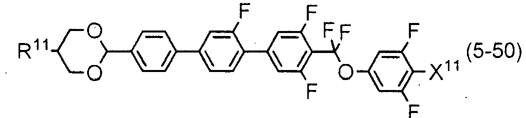

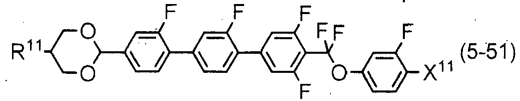

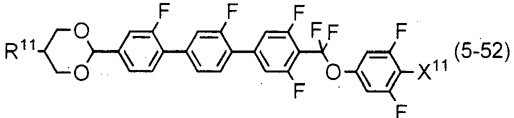

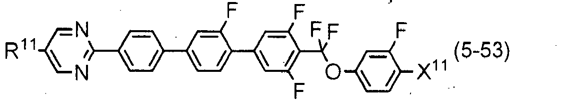

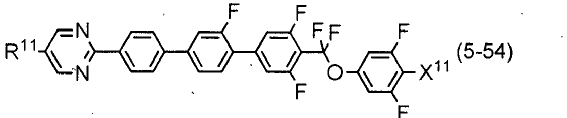

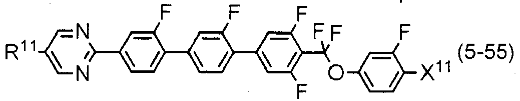

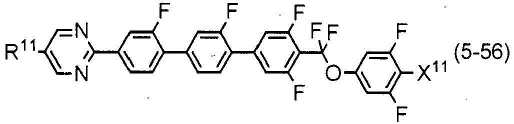

Component B has the positive dielectric anisotropy, and superb stability to heat, light and so forth, and therefore is used when a composition for the TFT mode, the IPS mode, the BP mode, the FFS mode or the like is prepared. A content of component B is suitably in the range of 1 to 99% by weight, preferably in the range of 10 to 97% by weight, and further preferably in the range of 40 to 95% by weight, based on the weight of the composition. Viscosity of the composition can be adjusted by further adding compounds (14) to (16) (component E) .

Component C is compound (6) in which a right-terminal group is -C≡N or -C=C-C=N. Specific examples of preferred component C include compounds (6-1) to (6-64) . In the compounds (component C), definitions of R¹² and X¹² are identical to definitions described above.

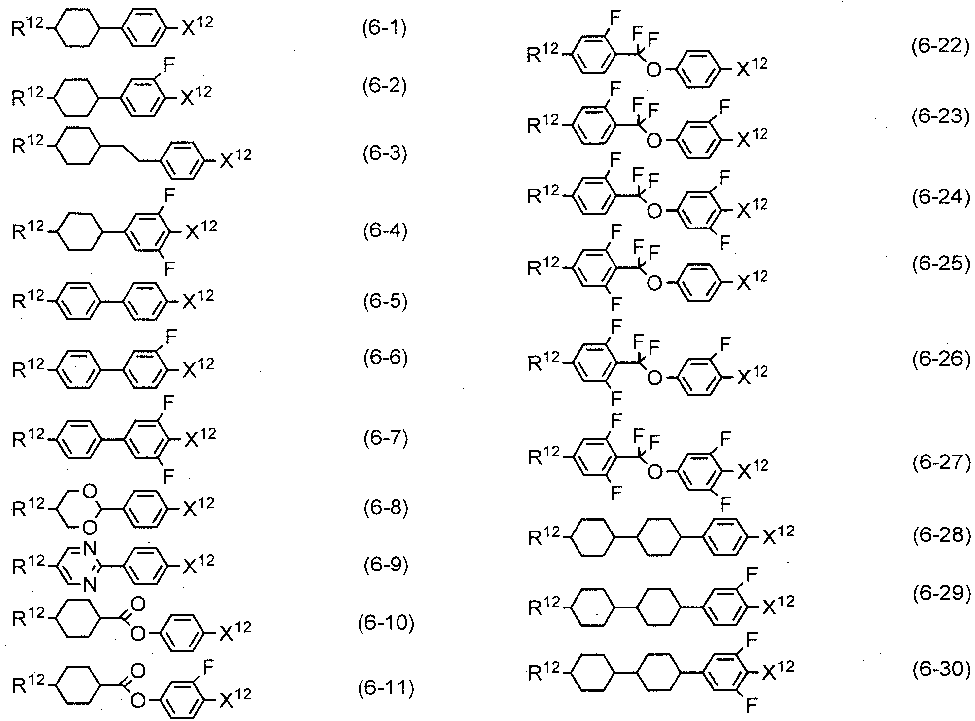

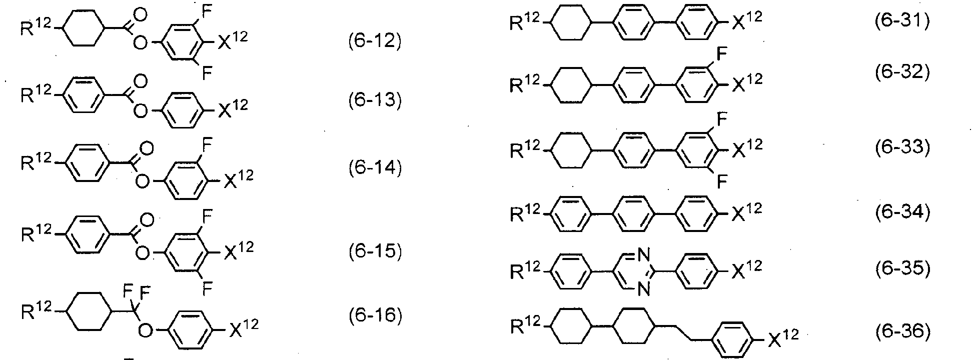

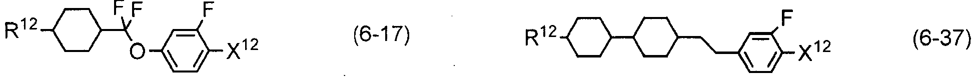

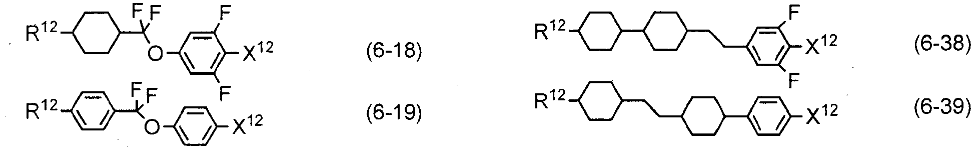

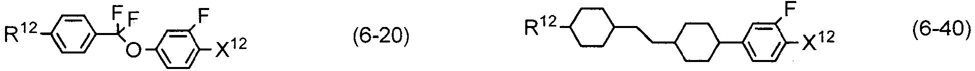

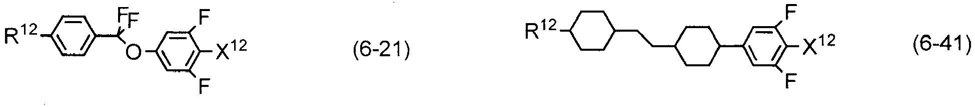

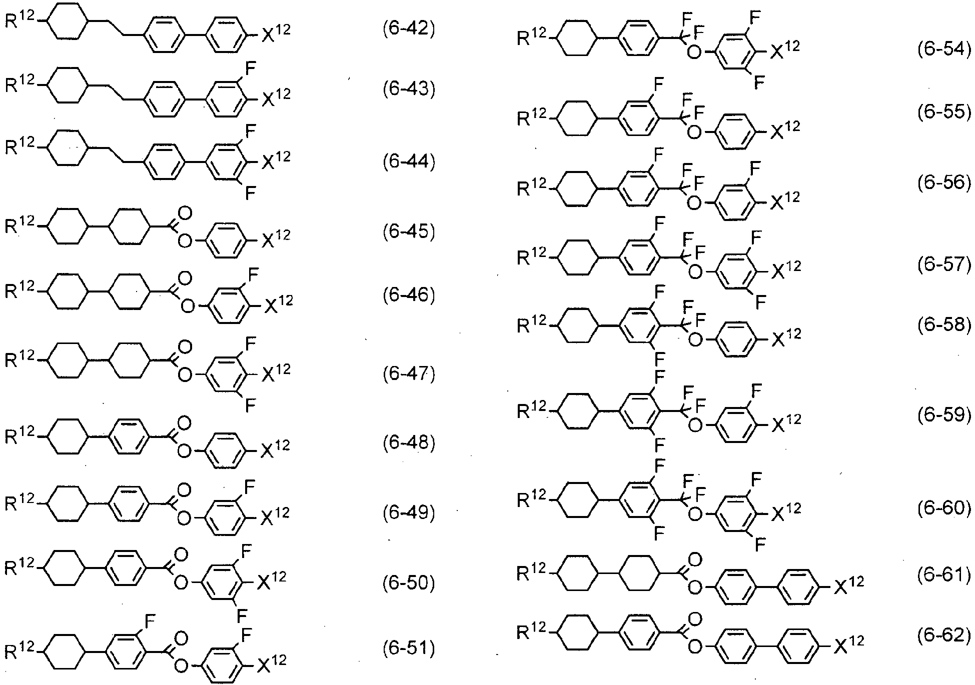

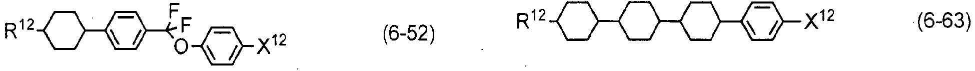

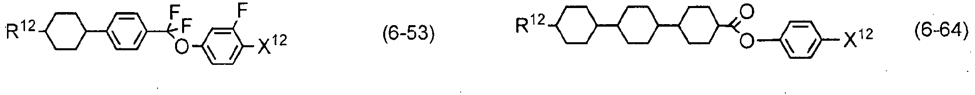

Component C has the positive dielectric anisotropy, a value of which is large, and therefore is mainly used when a composition for the STN mode, the TN mode, the PSA mode or the BP mode is prepared. Dielectric anisotropy of the composition can be increased by adding component C. Component C is effective in extending the temperature range of the liquid crystal phase, adjusting the viscosity or adjusting the optical anisotropy. Component C is also useful for adjustment of the voltage-transmittance curve of the device.

When a composition for the STN mode or the TN mode is prepared, a content of component C is suitably in the range of 1 to 99% by weight, preferably in the range of 10 to 97% by weight, and further preferably in the range of 40 to 95% by weight, based on the weight of the composition. In the composition, the temperature range of the liquid crystal phase, the viscosity, the optical anisotropy, the dielectric anisotropy or the like can be adjusted by adding component E.

Component D includes compounds (7) to (13). The compounds have a benzene ring in which hydrogen in lateral positions are replaced by two pieces of halogen, such as 2,3-difluoro-1,4-phenylene. Specific examples of preferred component D include compounds (7-1) to (7-8), compounds (8-1) to (8-17), compound (9-1), compounds (10-1) to (10-3), compounds (11-1) to (11-11), compounds (12-1) to (12-3) and compounds (13-1) to (13-3) . In the compounds (component D), definitions of R¹³, R¹⁴ and R¹⁵ are identical to definitions described above.

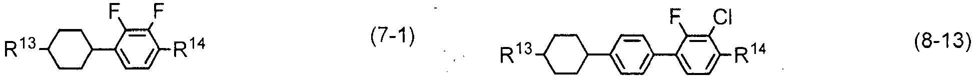

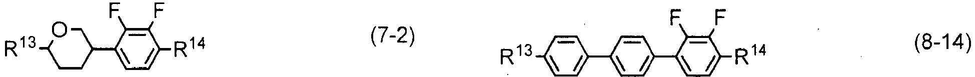

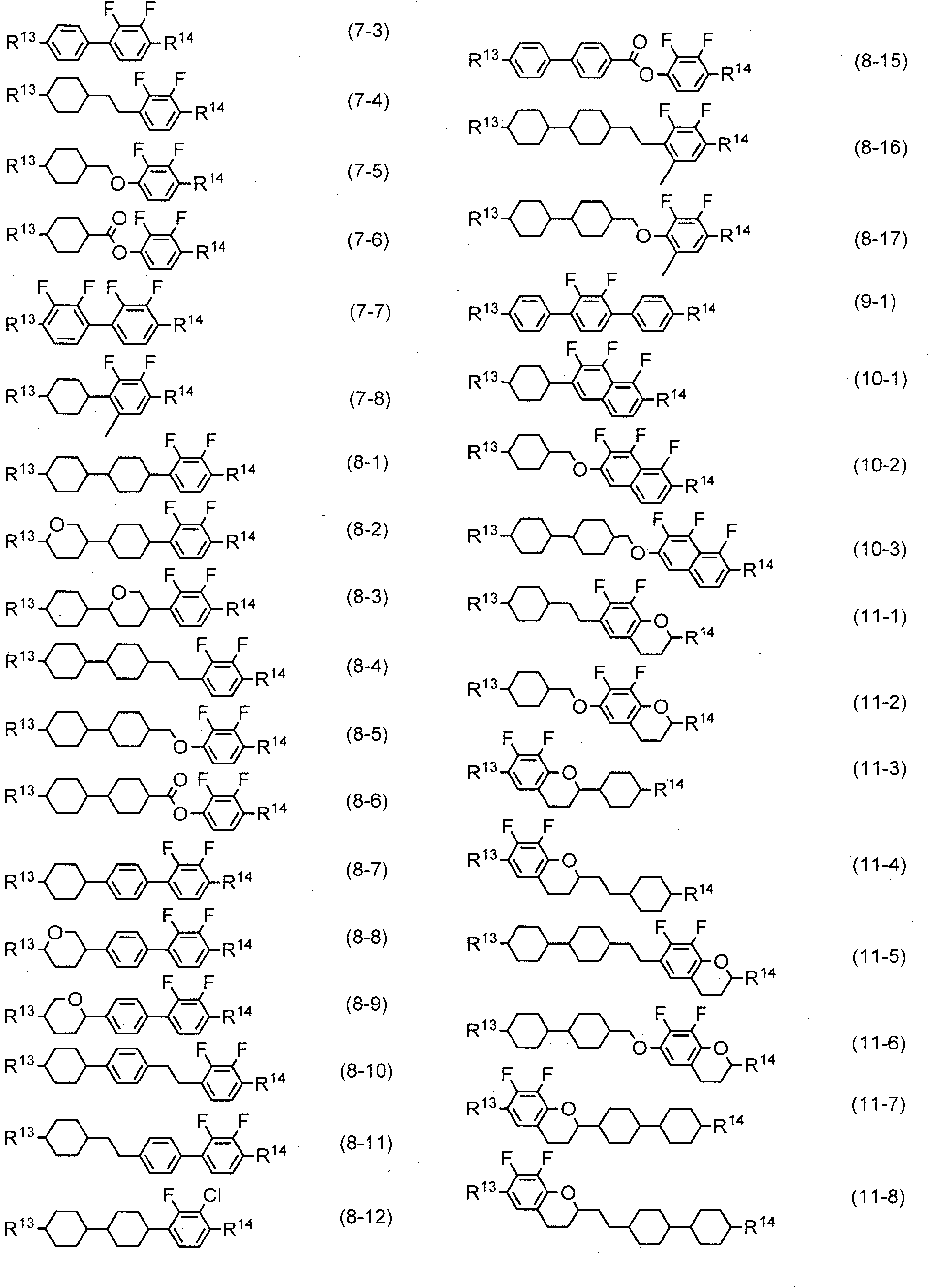

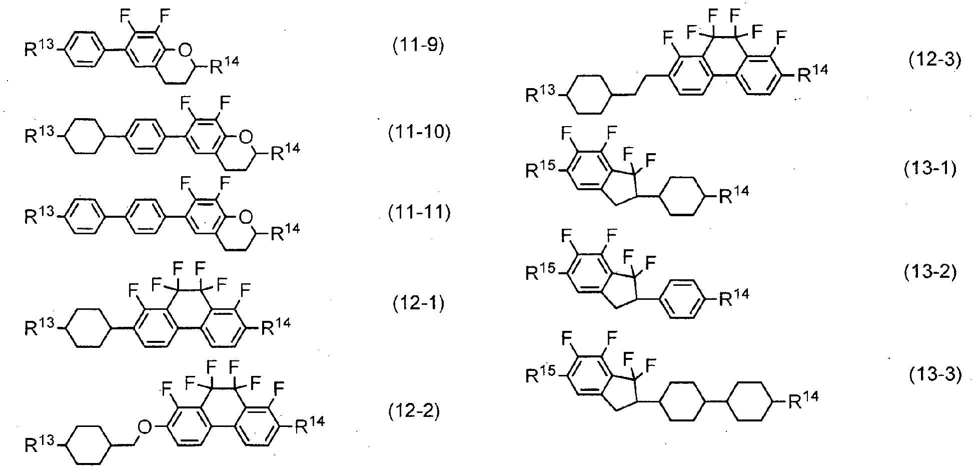

Component D is a compound having negative dielectric anisotropy. Component D is mainly used when a composition for the VA mode or the PSA mode is prepared. Among types of component D, compound (7) is a bicyclic compound, and therefore is mainly effective in adjusting the viscosity, the optical anisotropy or the dielectric anisotropy. Compounds (8) and (9) are a tricyclic compound, and therefore are effective in increasing the maximum temperature, the optical anisotropy or the dielectric anisotropy. Compounds (10) to (13) are effective in increasing the dielectric anisotropy.

When a composition for the VA mode or the PSA mode is prepared, - a content of component D is preferably 40% by weight or more, and further preferably in the range of 50 to 95% by weight, based on the weight of the composition. When component D is added to a composition having positive dielectric anisotropy, the content of component D is preferably 3 0% by weight or less based on the weight of the composition. The voltage-transmittance curve of the device in the composition can be adjusted by adding component D.

Component E includes a compound in which two terminal groups are alkyl or the like. Specific examples of preferred component E include compounds (13-1) to (13-11), compounds (14-1) to (14-19) and compounds (15-1) to (15-7). In the compounds (component E), definitions of R¹⁶ and R¹⁷ are identical to definitions described above.

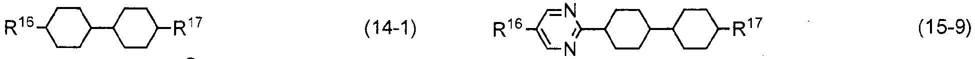

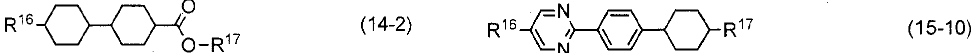

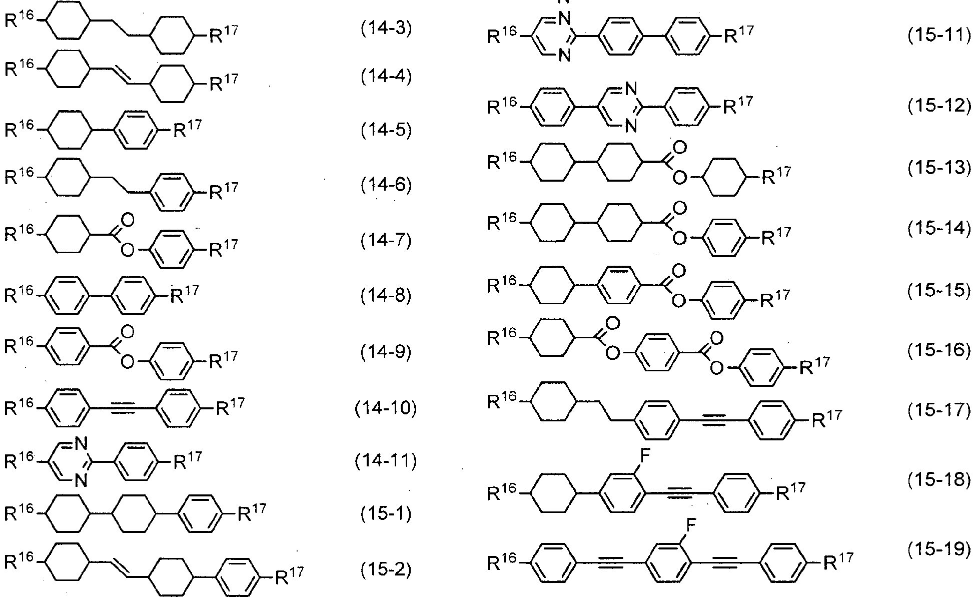

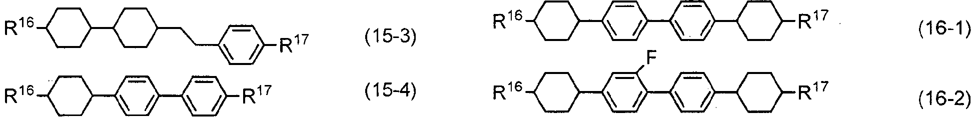

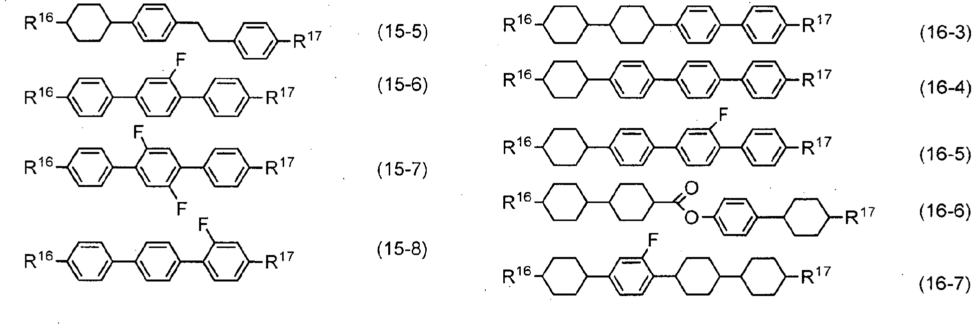

Component E has a small absolute value of dielectric anisotropy, and therefore is a compound close to neutrality. Compound (14) is mainly effective in adjusting the viscosity or adjusting the optical anisotropy. Compounds (15) and (16) are effective in extending the temperature range of the nematic phase by increasing the maximum temperature or effective in adjusting the optical anisotropy.

If a content of component E is increased, the dielectric anisotropy of the composition is decreased, but the viscosity is decreased. Thus, as long as a desired value of a threshold voltage of the device is met, the content is preferably as large as possible. Accordingly, when the composition is prepared, the content of component E is preferably 30% by weight or more, and further preferably 40% by weight or more, based on the weight of the composition.

Preparation of a composition (1) is performed by a method for dissolving required components at high temperature, or the like. According to an application, an additive maybe added to the composition. Specific examples of the additives include the optically active compound, the polymerizable compound, the polymerization initiator, the antioxidant, the ultraviolet light absorber, the light stabilizer, the heat stabilizer, the antifoaming agent and the dye. Such additives are well known to those skilled in the art, and described in literature.

Composition (1) may further contain at least one optically active compound. The optically active compound is effective in inducing a helical structure in liquid crystal molecules to give a required twist angle, and thereby preventing a reverse twist. Specific examples of a preferred optically active compound include compounds (K-1) to (K-16) described below.

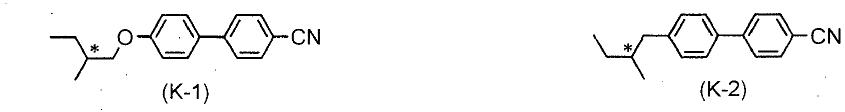

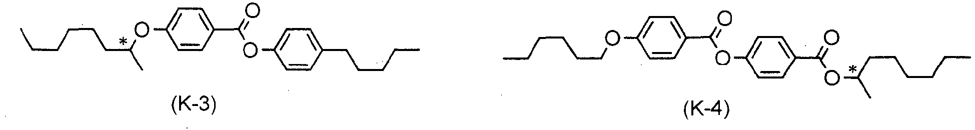

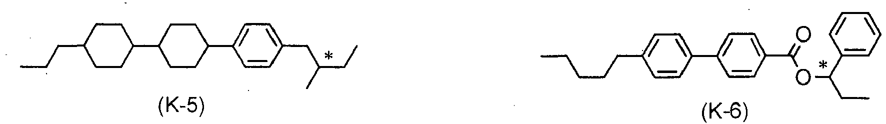

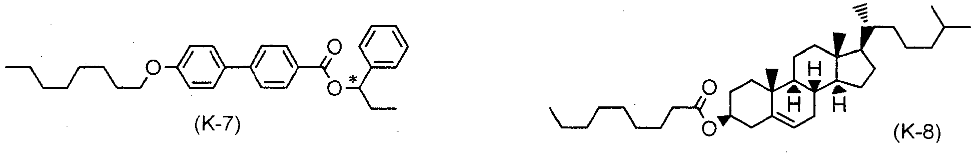

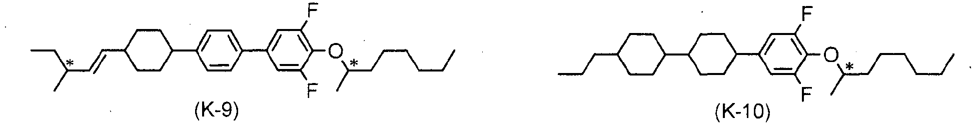

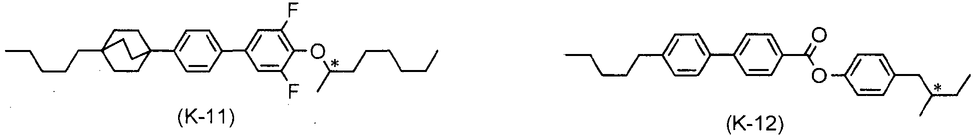

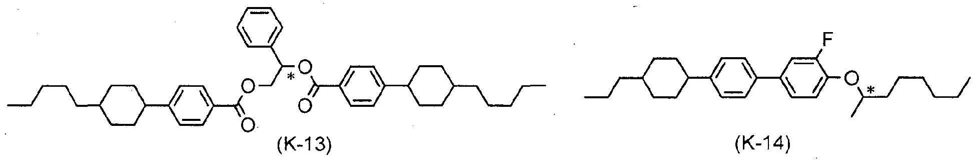

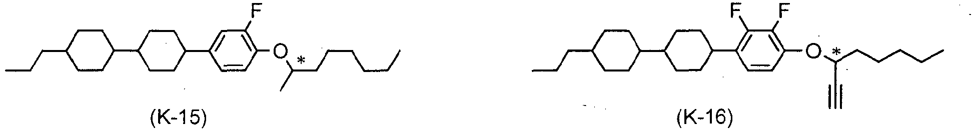

In composition (1), a helical pitch is adjusted by adding such an optically active compound. The helical pitch is preferably adjusted in the range of 40 to 200 micrometers in a composition for the TFT mode and the TN mode. In a composition for the STN mode, the helical pitch is preferably adjusted in the range of 6 to 20 micrometers. In a case of a composition for the BTN mode, the helical pitch is preferably adjusted in the range of 1.5 to 4 micrometers. Two or more optically active compounds may be added for the purpose of adjusting temperature dependence of the helical pitch.

Composition (1) can also be used for the PSA mode by adding a polymerizable compound. Specific examples of the polymerizable compounds include acrylate, methacrylate, a vinyl compound, a vinyloxy compound, propenyl ether, an epoxy compound (oxirane, oxetane) and vinyl ketone. The polymerizable compound is polymerized by irradiation with ultraviolet light or the like. An initiator such as a photopolymerization initiator may be added. Suitable conditions for polymerization, suitable types of the initiator and suitable amounts thereof are known to those skilled in the art and are described in literature. Specific examples of a preferred polymerizable compound include compounds (M2-7-1) to (M2-7-3), (M2-15-1) to (M2-15-8), (M2-28-1) to (M2-28-3), (M2-29-1) to (M2-29-2), (M4-1-1), (M4-2-1), (M4-4-1) and (M4-6-1).

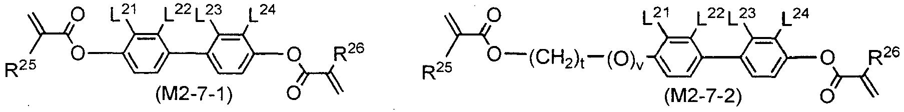

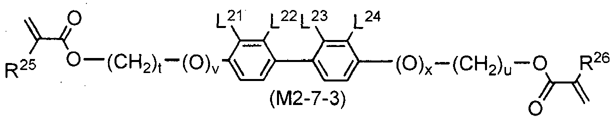

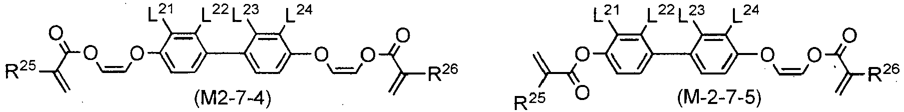

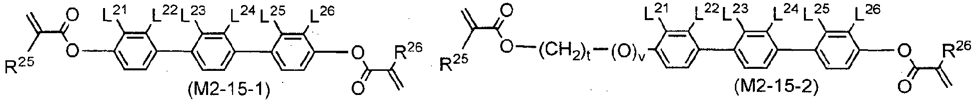

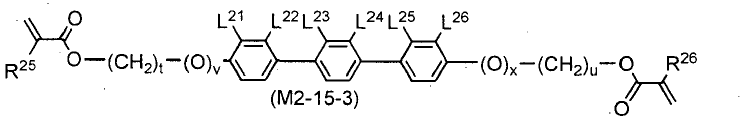

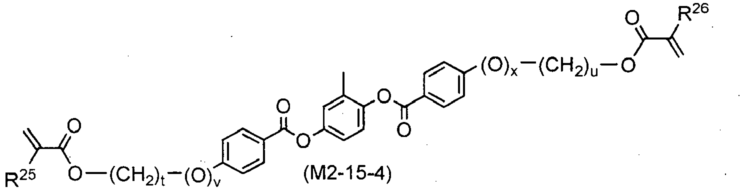

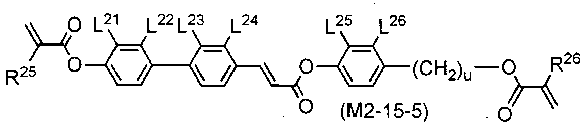

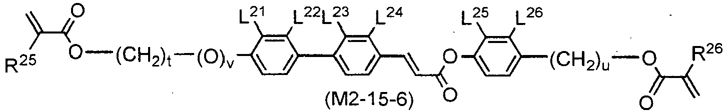

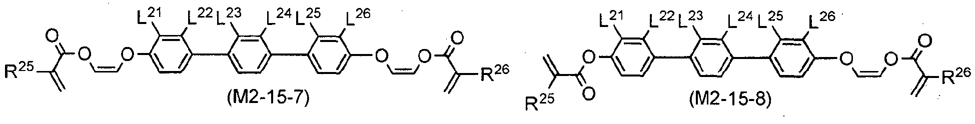

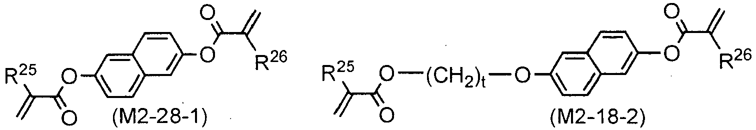

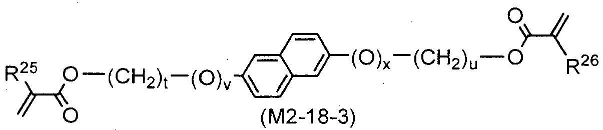

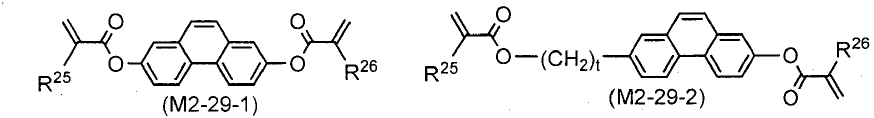

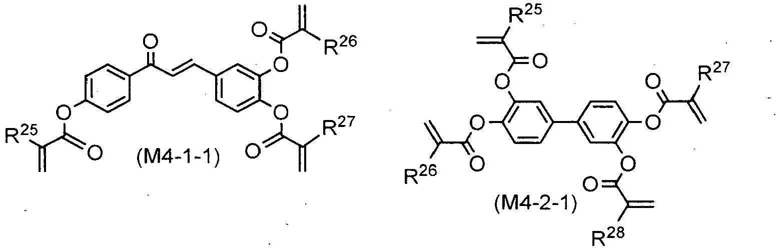

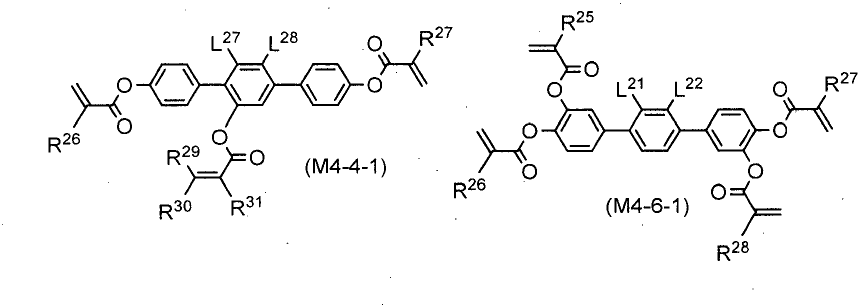

In compound compounds (M2-7-1) to (M2-7-3), (M2-15-1) to (M2-15-8), (M2-28-1) to (M2-28-3), (M2-29-1) to (M2-29-2), (M4-1-1), (M4-2-1), (M4-4-1) and (M4-6-1), R²⁵, R²⁶, R²⁷, R²⁸, R²⁹, R³⁰ and R³¹are independently hydrogen or methyl; v and x are independently 0 or 1; and t and u are independently an integer from 1 to 16. L²¹, L²², L²³, L²⁴, L²⁵ and L²⁶ are independently hydrogen or fluorine; and L²⁷ and L²⁸ are independently hydrogen, fluorine or methyl.

The antioxidant is effective for maintaining a large voltage holding ratio. Specific examples of a preferred antioxidant include compounds (AO-1) and (AO-2) described below, IRGANOX 415, IRGANOX 565, IRGANOX 1010, IRGANOX 1035, IRGANOX 3114 and IRGANOX 1098 (trade name: BASF SE). The ultraviolet light absorber is effective for preventing a decrease of the maximum temperature. Preferred examples of the ultraviolet light absorbers include a benzophenone derivative, a benzoate derivative and a triazole derivative. Specific examples include compounds (AO-3) and (AO-4) described below, TINUVIN 329, TINUVIN P, TINUVIN 326, TINUVIN 234, TINUVIN 213, TINUVIN 400, TINUVIN 328 and TINUVIN 99-2 (trade names: BASF SE), and 1,4-diazabicyclo[2.2.2]octane (DABCO). The light stabilizer such as an amine having steric hindrance is preferred for maintaining the large voltage holding ratio. Specific examples of a preferred light stabilizer include compounds (AO-5) and (AO-6) described below, TINUVIN 144, TINUVIN 765 and TINUVIN 770DF (trade name: BASF SE). The heat stabilizer is also effective for maintaining the large voltage holding ratio, and preferred examples include IRGAFOS 168 (trade name: BASF SE). The antifoaming agent is effective for preventing foam formation. Specific examples of a preferred antifoaming agent include dimethyl silicone oil and methylphenyl silicone oil.

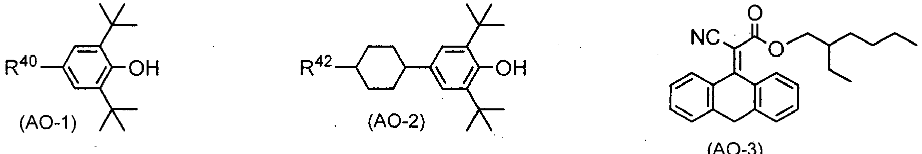

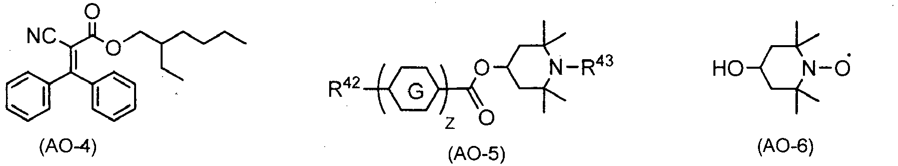

In compound (AO-1), R⁴⁰ is alkyl having 1 to 20 carbons, alkoxy having 1 to 20 carbons, -COOR⁴¹ or -CH₂CH₂COOR⁴¹, in which R⁴¹ is alkyl having 1 to 20 carbons. In compound (AO-2), R⁴² is alkyl having 1 to 20 carbons. In compound (AO-5), R⁴² is alkyl having 1 to 20 carbons; R⁴³ is hydrogen, methyl or O˙ (oxygen radical) ; ring G is 1,4-cyclohexylene or 1,4-phenylene; and z is 1, 2 or 3.

Composition (1) can also be used for a guest host (GH) mode by adding a dichroic dye such as a merocyanine type, a styryl type, an azo type, an azomethine type, an azoxy type, a quinophthalone type, an anthraquinone type and a tetrazine type.

### 2-2. Composition (1') having an optically isotropic liquid crystal phase

### 2-2-1. Formulation of a composition having an optically isotropic liquid crystal phase

Composition (1') having an optically isotropic liquid crystal phase of the invention will be described. Composition (1') is a composition containing achiral component T and the chiral agent, and a liquid crystal composition that can be used in an optical device driven in the optically isotropic liquid crystal phase. Achiral component T contains component A including a compound represented by formula (1) and by formula (4) as an addition component. A preferred component in formula (4) is the group represented by formulas (4A) to (4D). In addition to component A when necessary, achiral component T contains a compound selected from the group represented by formula (3), the group represented by formula (5), and the group represented by formula (6) . The liquid crystal composition is a composition that develops the optically isotropic liquid crystal phase.

A compound represented by formula (1) has the large dielectric anisotropy, and therefore a content thereof is about 0.5 to about 50% by weight, preferably about 1 to about 30% by weight, and further preferably about 5 to about 20% by weight, based on the total weight of component T.

Compounds represented by formulas (4A) to (4D) have a comparatively high clearing point, and the large dielectric anisotropy and large refractive index anisotropy, and therefore a content thereof is about 0.5 to about 90% by weight, preferably about 5 to about 70% by weight, and further preferably about 10 to about 50% by weight, based on the total weight of component T.

A compound represented by formula (3) has the small viscosity, good compatibility, the large dielectric anisotropy and the large refractive index anisotropy, and therefore a content thereof is about 0.5 to about 90% by weight, preferably about 5 to about 70% by weight, and further preferably about 10 to about 50% by weight, based on the total weight of component T.

A compound represented by formula (5) has the high clearing point, the large dielectric anisotropy and the large refractive index anisotropy, and therefore a content thereof is about 0.5 to about 90% by weight, preferably about 1 to about 50% by weight, and further preferably about 3 to about 30% by weight, based on the total weight of component T.

A compound represented by formula (6) has particularly large dielectric anisotropy and particularly large refractive index anisotropy, and therefore a content thereof is about 0.5 to about 90% by weight, preferably about 1 to about 50% by weight, and further preferably about 3 to about 30% by weight, based on the total weight of component T.

The liquid crystal composition contains preferably about 1 to about 40% by weight of the chiral agent, further preferably about 3 to about 25% by weight of the chiral agent, and most preferably about 5 to about 15% by weight of the chiral agent, based on the total weight of the liquid crystal composition. The liquid crystal composition containing the chiral agent in the range described above easily has the optically isotropic liquid crystal phase, and therefore is preferred.

The chiral agent contained in the liquid crystal composition may be one kind or two or more kinds of chiral agents.

### 2-2-2. Chiral Agent

A chiral agent contained in an optically isotropic liquid crystal composition is an optically active compound, and as the chiral agent, a compound having large twisting power (Helical Twisting Power) is preferred. In the compound having large twisting power, an adding amount required for obtaining a desired pitch can be decreased, and therefore an increase in driving voltage can be suppressed, and the compound having large twisting power is practically advantageous. Specifically, compounds represented by formulas (K21) to (K27) described below are preferred.

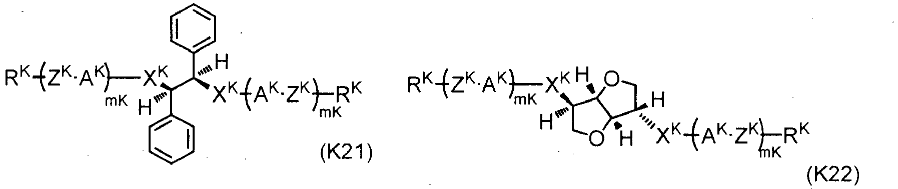

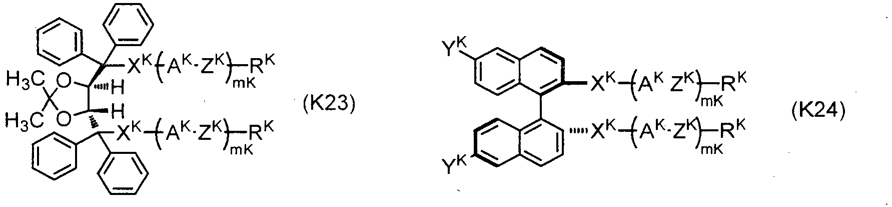

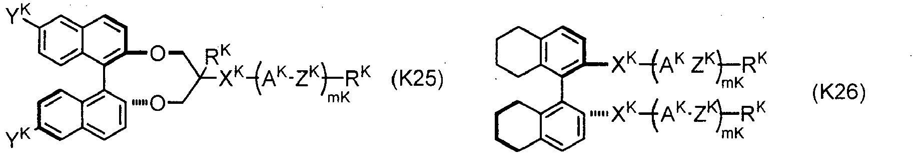

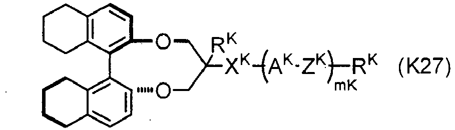

In formulas (K21) to (K27),
R^{K} is each independently hydrogen, halogen, -C=N, -N=C=O, -N=C=S or alkyl having 1 to 12 carbons, at least one piece of -CH₂- in R^{K} is may be replaced by -O-, -S-, -COO- or -OCO-, at least one piece of -CH₂-CH₂- in R^{K} may be replaced by -CH=CH-, -CF=CF- or -C≡C-, and at least one piece of hydrogen in R^{K} may be replaced by fluorine or chlorine ;
A^{K} is each independently an aromatic 6-membered ring to an aromatic 8-membered ring, a non-aromatic 3-membered ring to a non-aromatic 8-membered ring, or a condensed ring having 9 or more carbons, at least one piece of hydrogen in the rings may be replaced by halogen, alkyl having 1 to 3 carbons or haloalkyl, and -CH₂- in the ring may be replaced by -O-, -S- or -NH-, and -CH= may be replaced by -N=;
Y^{K} is each independently hydrogen, halogen, alkyl having 1 to 3 carbons, haloalkyl having 1 to 3 carbons, an aromatic 6-membered ring to an aromatic 8-membered ring, a non-aromatic 3-membered ring to a non-aromatic 8-membered ring, or a condensed ring having 9 or more carbons, at least one piece of hydrogen in the rings may be replaced by halogen, alkyl having 1 to 3 carbons or haloalkyl, -CH₂- in the alkyl may be replaced by -O-, -S- or -NH-, and -CH= may be replaced by -N=;
Z^{K} is each independently a single bond and alkylene having 1 to 8 carbons, at least one piece of -CH₂- in Z^{K} may be replaced by -O-, -S-, -COO-, -OCO-, -CSO-, -OCS-, -N=N-, -CH=N- or -N=CH-, at least one piece of -CH₂-CH₂- in Z^{K} may be replaced by -CH=CH-, -CF=CF- or -C≡C-, and at least one piece of hydrogen in Z^{K} may be replaced by halogen;
X^{K} is each independently a single bond, -COO-, -OCO-, -CH₂O-, -OCH₂-, -CF₂O-, -OCF₂- or -CH₂CH₂-; and
mK is each independently an integer from 1 to 3).

As the chiral agent to be added to the liquid crystal composition, thereamong, formula (K22-1) to formula (K22-8) included in formula (K22), formula (K24-1) to formula (K24-6) included in formula (K24), formula (K25-1) to formula (K25-3) included in formula (K25), formula (K26-1) to formula (K26-6) included in formula (K26), and formula (K27-1) to formula (K27-3) included in formula (K27) are further preferred, and formula (K24-1) to formula (K24-6), formula (K25-1) to formula (K25-3). and formula (K26-1) to formula (K26-6) are still further preferred.

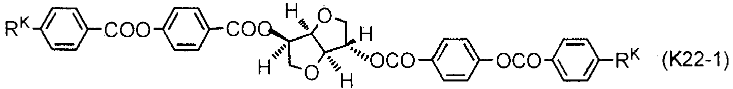

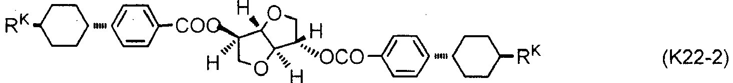

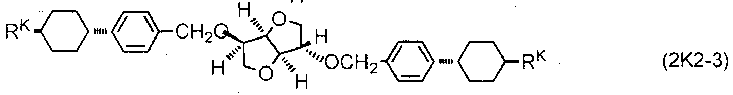

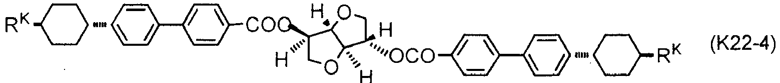

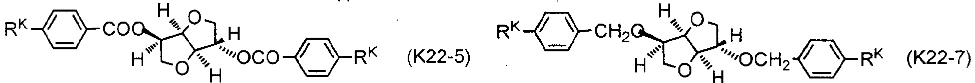

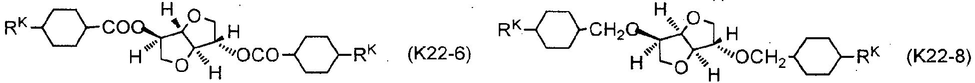

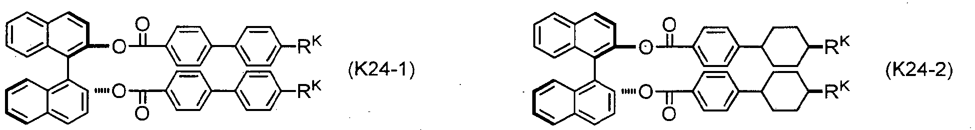

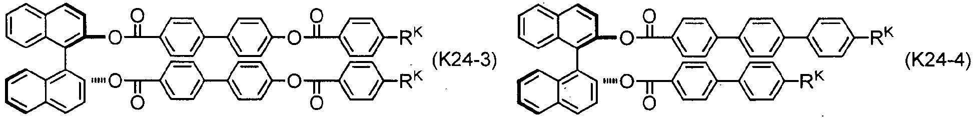

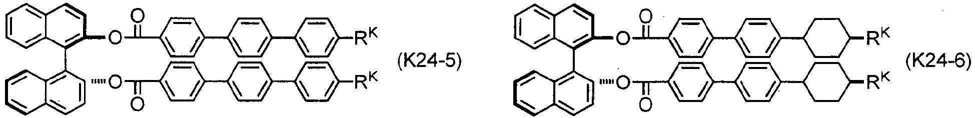

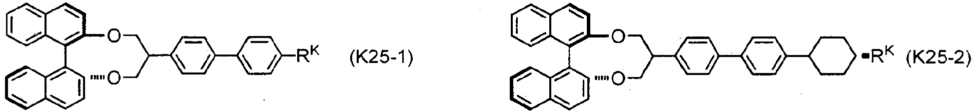

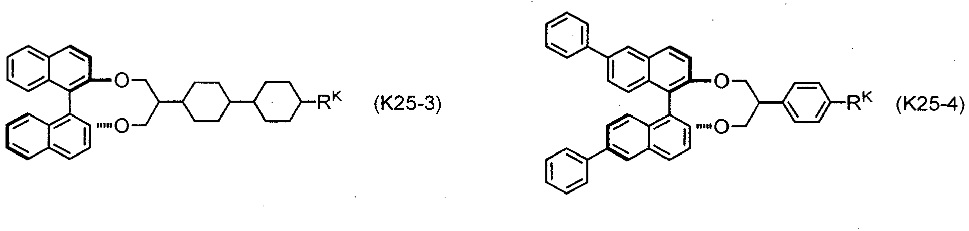

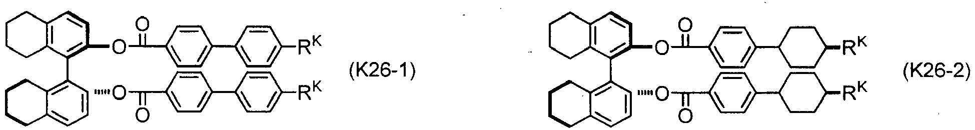

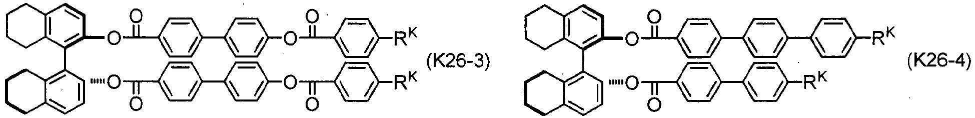

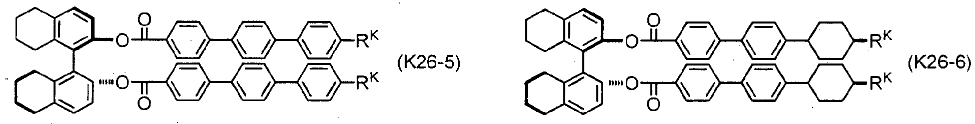

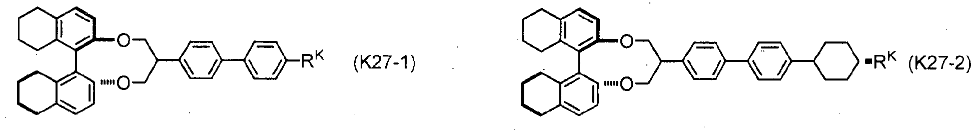

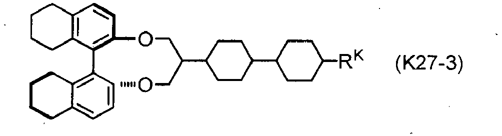

In the formulas, R^{K} is each independently alkyl having 3 to 10 carbons, -CH₂- adjacent to a ring in the alkyl may be replaced by -O-, and arbitrary -CH₂- in the alkyl or in a group in which -CH₂- adjacent to the ring in the alkyl is replaced by -O- may be replaced by -CH=CH-.

### 2-2-3. Optically isotropic liquid crystal phase

When the liquid crystal composition is optically isotropic, a liquid crystal molecule arrangement is macroscopically isotropic, and therefore optically shows isotropy, but a liquid-crystalline order microscopically exists. A "Pitch based on the liquid-crystalline order that the liquid crystal composition microscopically has (hereinafter, referred to as "pitch" sometimes)" is preferably 700 nanometers or less, further preferably 500 nanometers or less, and most preferably 350 nanometers or less.

Herein, "non-liquid crystal isotropic phase" is an isotropic phase that is generally defined, more specifically, a disorder phase, and an isotropic phase in which, even in generation of a region in which no local order parameter is zero, the cause is based on fluctuation. For example, herein, an isotropic phase that is developed in the nematic phase on a high temperature side corresponds to the non-liquid crystal isotropic phase. A chiral liquid crystal herein is also defined in a similar manner. Then, herein, "optically isotropic liquid crystal phase" represents a phase subjected to development of an optically isotropic liquid crystal phase without fluctuation, and for example, a phase subjected to development of platelet texture (blue phase in a narrow sense) is included as one example thereof.

In the optically isotropic liquid crystal composition of the invention, regardless of the optically isotropic liquid crystal phase, the platelet texture that is typical in the blue phase may not be occasionally observed under observation with a polarizing microscope. Then, herein, a phase subjected to development of platelet texture is referred to as a blue phase, and an optically isotropic liquid crystal phase including a blue phase is referred to as an optically isotropic liquid crystal phase. More specifically, the blue phase is included by the optically isotropic liquid crystal phase.

In general, the blue phase is classified into three kinds: blue phase I, blue phase II and blue phase III, and all of three kinds of the blue phases are optical activity, and isotropic. In a blue phase of blue phase I and blue phase II, two or more kinds of diffracted light resulting from Bragg reflection from a different lattice plane is observed. A blue phase is generally observed between the non-liquid crystal isotropic phase and the chiral nematic phase.

A state in which the optically isotropic liquid crystal phase represents no diffracted light having two colors or more means that the platelet texture to be observed in blue phase I and blue phase II is not observed, and an almost whole surface represents a single color. An optically isotropic liquid crystal phase representing no diffracted light having two colors or more needs no uniform tone of a color in plane.

The optically isotropic liquid crystal phase representing no diffracted light having two colors or more has an advantage that reflected light intensity by the Bragg reflection is suppressed, or an advantage of shifting to a low wavelength side.

Moreover, a liquid crystal material to reflect light of visible light may occasionally have a problem of color in utilization as a display device, but in liquid crystals representing no diffracted light having two colors or more, a reflection wavelength is subjected to a low wavelength shift, and therefore reflection of visible light can be vanished at a longer pitch in comparison with a blue phase in a narrow sense (phase subjected to development of platelet texture).

The optically isotropic liquid crystal composition of the invention can be obtained by adding the chiral agent to a composition having the nematic phase. On the occasion, the chiral agent is added at a concentration such that a pitch preferably becomes 700 nanometers or less. In addition, the composition having the nematic phase contains a compound represented by formula (1) and other components when necessary. Moreover, the optically isotropic liquid crystal composition of the invention can also be obtained by adding the chiral agent to a composition having the chiral nematic phase and having no optically isotropic liquid crystal phase. In addition, a composition having the chiral nematic phase and having no optically isotropic liquid crystallinity includes the compound represented by formula (1), the optically active compound, and other components when necessary. On the occasion, for preventing development of the optically isotropic liquid crystal phase, the optically active compound is added at a concentration such that the pitch preferably becomes 700 nanometers or more. Herein, in the optically active compound to be added, formulas (K21) to (K27) that are the compounds having large twisting power can be used, and compounds represented by formulas (K22-1) to (K22-8), formulas (K24-1) to (K24-6), formulas (K25-1) to (K25-3), formulas (K26-1) to (K26-6) or formulas (K27-1) to (K27-3) can be further preferably used. Moreover, the optically active compound to be added may be a compound in which twisting power is not so large. Specific examples of such an optically active compound include a compound to be added to a liquid crystal composition for the device (the TN mode, the STN mode or the like) driven in the nematic phase.

Specific examples of the optically active compounds in which twisting power is not so large include optically active compounds (K-1) to (K-16) in the formulas described above.

In addition, the temperature range of the optically isotropic liquid crystal composition of the invention can be extended by adding the chiral agent to a liquid crystal composition having a wide temperature range in which the nematic phase or the chiral nematic phase and the isotropic phase coexist, and by developing the optically isotropic liquid crystal phase. For example, a liquid crystal compound having the high clearing point and a liquid crystal compound having a low clearing point are mixed, a liquid crystal composition having a wide temperature range in which the nematic phase and the isotropic phase coexist at a wide temperature range is prepared, and the chiral agent is added thereto, and thus a composition to develop the optically isotropic liquid crystal phase in a wide temperature range can be prepared.

As the liquid crystal composition having a wide temperature range in which the nematic phase or the chiral nematic phase and the isotropic phase coexist, a liquid crystal composition in which a difference between a maximum temperature and a minimum temperature in which the chiral nematic phase and the non-liquid crystal isotropic phase coexist is 3 to 150°C is preferred, and a liquid crystal composition in which the difference is 5 to 150°C is further preferred. Moreover, the liquid crystal composition in which a difference between a maximum temperature and a minimum temperature in which the chiral nematic phase and the non-liquid crystal isotropic phase coexist is 3 to 150°C is preferred.

If an electric field is applied to the liquid crystal medium of the invention in the optically isotropic liquid crystal phase, electric birefringence is generated, but is not necessary to be the Kerr effect.

The electric birefringence in the optically isotropic liquid crystal phase is increased as the pitch becomes longer, and therefore as long as a requirement of other optical properties (transmittance, diffraction wavelength or the like) is satisfied, the electric birefringence can be increased by adjusting a type and a content of the chiral agent to set the pitch to be longer.

### 2-2-4. Other components that can be contained by optically isotropic liquid crystal composition

Other compounds such as a polymer substance may be further added to the optically isotropic liquid crystal composition of the invention without influence on characteristics of the composition. The liquid crystal composition of the invention may contain dichroic dye and a photochromic compound in addition to the polymer substance, for example. Specific examples of the dichroic dye include merocyanine, styryl, azo, azomethine, azoxy, quinophthalone, anthraquinone and tetrazine.

### 2-2-5. Optically isotropic polymer-liquid crystal composite material

An optically isotropic polymer-liquid crystal composite material of the invention will be described. The optically isotropic polymer-liquid crystal composite material is a liquid crystal composition containing a compound represented by formula (1) and a chiral agent, and is a composite material of a polymer, and optically shows isotropy. The material can be used in an optical device driven in an optically isotropic liquid crystal phase. Such a polymer-liquid crystal composite material is composed of the liquid crystal composition (liquid crystal composition CLC) according to items 9 to 16 and a polymer, for example.

"Polymer-liquid crystal composite material" of the invention is not particularly limited in the case of a composite material containing both a liquid crystal material and a polymer compound, but may have a state in which some or all of the polymer are not dissolved in the liquid crystal material and a state in which the polymer is subjected to phase separation to the liquid crystal material. In addition, unless otherwise noted herein, a nematic phase means a nematic phase in a narrow sense without containing a chiral nematic phase.

An optically isotropic polymer-liquid crystal composite material as related to a preferred aspect of the invention can develop an optically isotropic liquid crystal phase in a wide temperature range. Moreover, in the polymer-liquid crystal composite material as related to a preferred aspect of the invention, response speed is extremely large. Moreover, the polymer-liquid crystal composite material as related to a preferred aspect of the invention can be suitably used in an optical device such as a display device, or the like based on the effects.

### 2-2-6. Polymer

A composite material of the invention can also be produced by mixing an optically isotropic liquid crystal composition and a polymer obtained by previous polymerization, but is preferably produced by mixing a monomer, a macromonomer, an oligomer or the like (hereinafter, referred to as "monomer or the like" as a whole) having low molecular weight and used as a polymer material and liquid crystal composition CLC, and then by subjecting the mixture to a polymerization reaction. The mixture containing the monomer or the like and the liquid crystal composition is herein referred to as a "polymerizable monomer-liquid crystal mixture. " The "polymerizable monomer-liquid crystal mixture" may contain a polymerization initiator, a curing agent, a catalyst, a stabilizer, dichroic dye, a photochromic compound or the like described later when necessary in a range in which advantageous effects of the invention are not lost. For example, the polymerizable monomer-liquid crystal mixture of the invention may contain 0.1 to 20 parts by weight of the polymerization initiator based on 100 parts by weight of a polymerizable monomer when necessary.

Polymerization temperature is preferably temperature at which a polymer-liquid crystal composite material exhibits high transparency and isotropy. The Polymerization temperature is further preferably temperature at which a mixture of a monomer and a liquid crystal material develops an isotropic phase or a blue phase, and polymerization is completed in the isotropic phase or an optically isotropic liquid crystal phase. More specifically, temperature at which the polymer-liquid crystal composite material does not substantially scatter light having a longer wavelength in comparison with visible light after polymerization to develop an optically isotropic state is preferred.

As a raw material of a polymer composing the composite material of the invention, for example, the monomer, the macromonomer and the oligomer having low molecular weight can be used, and a raw material monomer of a polymer is herein used as a meaning that includes the monomer, the macromonomer, the oligomer or the like having low molecular weight. Moreover, a polymer obtained preferably has a three-dimensional crosslink structure, and therefore a polyfunctional monomer having two or more polymerizable functional groups is preferably used as the raw material monomer of a polymer. The polymerizable functional groups are not particularly limited, but specific examples include an acrylic group, a methacrylic group, a glycidyl group, an epoxy group, an oxetanyl group and a vinyl group, but the acrylic group and the methacrylic group are preferred from a viewpoint of a polymerization rate. In the raw material monomer of a polymer, if the monomer is allowed to contain 10% by weight or more of a monomer having two or more polymerizable functional groups, high transparency and isotropy becomes easily developed in the composite material of the invention, and therefore such a case is preferred.

Moreover, a polymer having a mesogen moiety is preferred in order to obtain a suitable composite material, and as the raw material monomer of a polymer, in some or all thereof, a raw material monomer having the mesogen moiety can be used.

### 2-2-7. Monofunctional-bifunctional monomer having mesogen moiety

A monofunctional or bifunctional monomer having a mesogen moiety is not particularly limited in structure, but specific examples include a compound represented by formula (M1) or formula (M2) described below.

R^{MA}-Y^{M}- (A^{M}-Z^{M})ₘ₁-A^{M}-Y^{M}-R^{MB} (M1)

R^{MA}-Y^{M}-(A^{M}-Z^{M})ₘ₁-A^{M}-Y^{M}-R^{MB} (M2)

In compound (M1), R^{MA} is hydrogen, halogen, -C≡N, -N=C=O, -N=C=S or alkyl having 1 to 20 carbons, and in the alkyl, at least one piece of -CH₂- may be replaced by -O-, -S-, -CO-, -COO- or -OCO-, and at least one piece of -CH₂-CH₂- in the alkyl may be replaced by -CH=CH-, -CF=CF- or -C≡C-, and in the alkyl groups, in a group in which at least one piece of -CH₂- in the alkyl is replaced by -O-, -S-, -COO- or -OCO-, or in a group in which at least one piece of -CH₂-CH₂- in the alkyl is replaced by -CH=CH- or -C≡C-, at least one piece of hydrogen may be replaced by halogen or -C≡N. R^{MB} is each independently a polymerizable group in formulas (M3-1) to (M3-7):

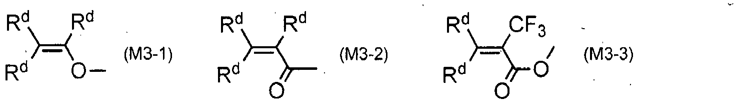

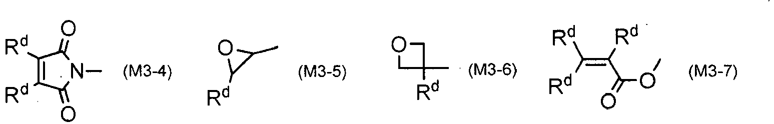

Preferred R^{MA} is hydrogen, halogen, -C≡N, -CF₃, -CF₂H, -CFH₂, -OCF₃, -OCF₂H, alkyl having 1 to 20 carbons, alkoxy having 1 to 19 carbons, alkenyl having 2 to 21 carbons or alkynyl having 2 to 21 carbons. Particularly preferred R^{a} is -C≡N, alkyl having 1 to 20 carbons or alkoxy having 1 to 19 carbons.

In compounds (M1) and (M2), R^{MB} is each independently any one of polymerizable groups represented by formulas (M3-1) to (M3-7).

Here, R^{d} in formulas (M3-1) to (M3-7) is each independently hydrogen, halogen or alkyl having 1 to 5 carbons, and in the alkyl, at least one piece of hydrogen may be replaced by halogen. Preferred R^{d} is hydrogen, halogen or methyl. Particularly preferred R^{d} is hydrogen, fluorine or methyl.

Moreover, polymerization by radical polymerization is suitable in formulas (M3-2) to (M3-4) and (M3-7). Polymerization by cationic polymerization is suitable in formulas (M3-1), (M3-5) and (M3-6). Polymerization is started if a small amount of radicals or cation active species are generated in a reaction system. The polymerization initiator can be used for the purpose of accelerating generation of the active species. Light or heat can be used for generation of the active species, for example.

In compounds (M1) and (M2), A^{M} is each independently an aromatic or non-aromatic 5-membered ring, an aromatic or non-aromatic 6-membered ring or an aromatic or non-aromatic condensed ring having 9 or more carbons, but -CH₂- in a ring may be replaced by -O-, -S-, -NH- or -NCH₃-, -CH= in a ring may be replaced by -N=, and a hydrogen atom on a ring may be replaced by halogen, alkyl having 1 to 5 carbons or alkyl halide. Specific examples of preferred A^{M} include 1,4-cyclohexylene, 1,4-cyclohexenylene, 1,4-phenylene, naphthalene-2,6-diyl, tetrahydronaphthalene-2,6-diyl, fluorene-2,7-diyl or bicyclo[2.2.2]octane-1,4-diyl, at least one piece of -CH₂- in the rings may be replaced by -O-, at least one piece of -CH= may be replaced by -N=, and at least one piece of hydrogen in the rings may be replaced by halogen, alkyl having 1 to 5 carbons or alkyl halide having 1 to 5 carbons.

In consideration of stability of a compound, -CH₂-O-CH₂-O- in which oxygen is not adjacent to oxygen is preferred to -CH₂-O-O-CH₂- in which oxygen is adjacent to oxygen. A same rule applies also to sulfur.

Particularly preferred A^{M} thereamong include 1,4-cyclohexylene, 1,4-cyclohexenylene, 1,4-phenylene, 2-fluoro-1,4-phenylene, 2,3-difluoro-1,4-phenylene, 2,5-difluoro-1,4-phenylene, 2,6-difluoro-1,4-phenylene, 2-methyl-1,4-phenylene, 2-trifluoromethyl-1,4-phenylene, 2,3-bis(trifluoromethyl)-1,4-phenylene, naphthalene-2,6-diyl, tetrahydronaphthalene-2,6-diyl, fluorene-2,7-diyl, 9-methylfluorene-2,7-diyl, 1,3-dioxane-2,5-diyl, pyridine-2,5-diyl or pyrimidine-2,5-diyl. In addition, in a configuration of 1,4-cyclohexylene and 1,3-dioxane-2,5-diyl as described above, a trans configuration is preferred to a cis configuration.

Then, 2-fluoro-1,4-phenylene is structurally identical to 3-fluoro-1,4-phenylene, and therefore the latter is not exemplified. A same rule applies also to a relationship between 2,5-difluoro-1,4-phenylene and 3,6-difluoro-1,4-phenylene, or the like.

In compounds (M1) and (M2), Y^{M} is each independently a single bond or alkylene having 1 to 20 carbons, and in the alkylene, at least one piece of -CH₂- may be replaced by -O- or -S-, and at least one piece of -CH₂-CH₂- in the alkyl may be replaced by -CH=CH-, -C≡C-, -COO- or -OCO- . Preferred Y^{M} is a single bond, -(CH₂)ₘ₂-, -O(CH₂)ₘ₂- or -(CH₂)ₘ₂O-(in the formulas described above, m2 is an integer from 1 to 20). Particularly preferred Y^{M} is a single bond, -(CH₂)ₘ₂-, -O(CH₂)ₘ₂- or -(CH₂)ₘ₂O- (in the formulas described above, m2 is an integer from 1 to 10) . In consideration of stability of a compound, -Y^{M}-R^{MA} and -Y^{M}-R^{MB} do not preferably have -O-O-, -O-S-, -S-O- or -S-S- in the groups.

In compounds (M1) and (M2), Z^{M} is each independently a single bond, -(CH₂)ₘ₃-, -O(CH₂)ₘ₃-, -(CH₂)ₘ₃O-, -O(CH₂)ₘ₃O-, -CH=CH-, -C≡C-, -COO-, -OCO-, -(CF₂)₂-, -(CH₂)₂-COO-, -OCO-(CH₂)₂-, -CH=CH-COO-, -OCO-CH=CH-, -C≡C-COO-, -OCO-C≡C-, -CH=CH-(CH₂)₂-, -(CH₂)₂-CH=CH-, -CF=CF-, -C≡C-CH=CH-, -CH=CH-C≡C-, -OCF₂-(CH₂)₂-, -(CH₂)₂-CF₂O-, -OCF₂- or -CF₂O-(in the formulas described above, m3 is an integer from 1 to 20) .

Specific examples of preferred Z^{M} include -(CH₂)ₘ₃-, -O(CH₂)ₘ₃-, -(CH₂)ₘ₃O-, -CH=CH-, -C≡C-, -COO-, -OCO-, -(CH₂)₂-COO-, -OCO-(CH₂)₂-, -CH=CH-COO-, -OCO-CH=CH-, -OCF₂- or -CF₂O-.

In compounds (M1) and (M2), m1 is an integer from 1 to 6. Preferred m1 is an integer from 1 to 3. When m1 is 1, the compound includes a bicyclic compound having two rings such as a 6-membered ring. When m1 is 2 or 3, the compound each include a tricyclic compound or a tetracyclic compound. For example, when m1 is 1, two of A^{M} may be identical or different. Moreover, for example, when m1 is 2, three of A^{M} (or two of Z^{M}) may be identical or different. When m1 is 3 to 6, a same rule applies also thereto. A same rule applies also to R^{MA}, R^{MB}, R^{d}, Z^{M}, A^{M} and Y^{M}.

Compounds (M1) and (M2) have similar characteristics even if the compounds contain more isotopes such as ²H (deuterium) and ¹³C than an amount of natural abundance, and therefore can be preferably used.

Further preferred examples of compound (M1) and compound (M2) include compounds (M1-1) to (M1-41) and (M2-1) to (M2-29), respectively. In the compounds, definitions of R^{MA}, R^{MB}, R^{d}, Z^{M}, A^{M}, Y^{M} and p are identical to definitions of compound (M1) and formula (M2) as described in an aspect of the invention.

A partial structure described below in compounds (M1-1) to (M1-41) and (M2-1) to (M2-29) will be described. Partial structure (a1) represents 1,4-phenylene in which at least one piece of hydrogen is replaced by fluorine. Partial structure (a2) represents 1,4-phenylene in which at least one piece of hydrogen may be replaced by fluorine. Partial structure (a3) represents 1,4-phenylene in which at least one piece of hydrogen may be replaced by any one of fluorine and methyl. Partial structure (a4) represents fluorene in which hydrogen in a 9 position may be replaced by methyl.

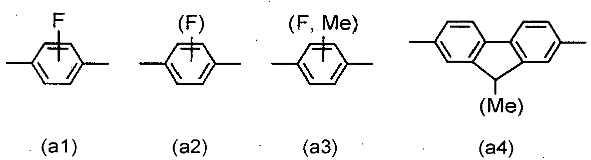

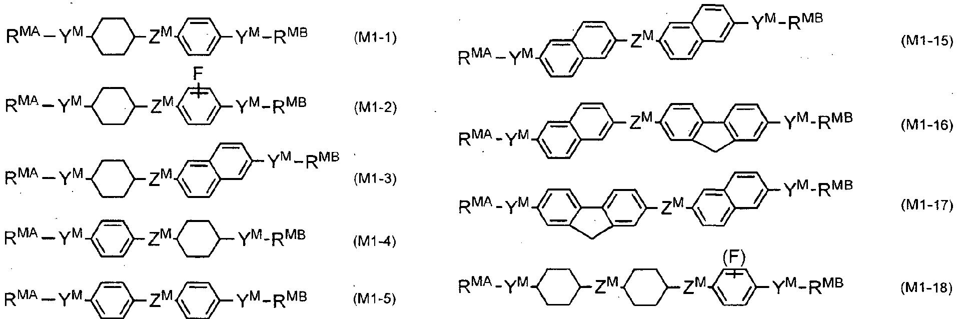

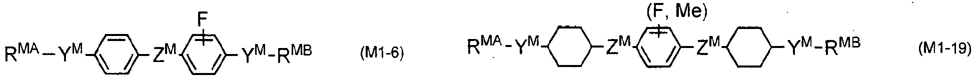

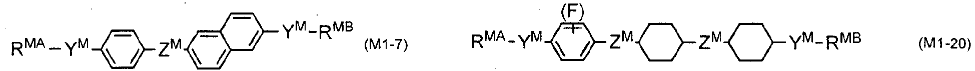

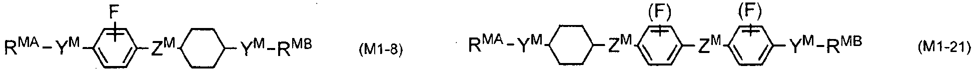

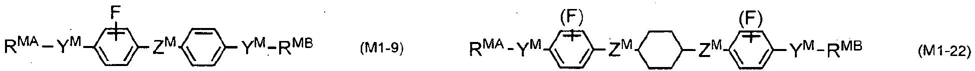

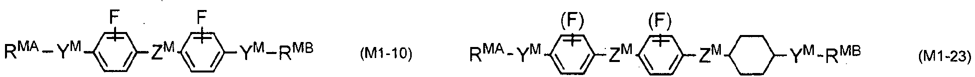

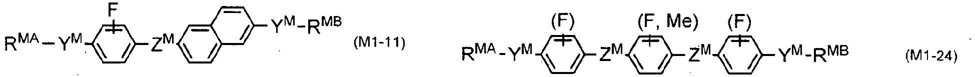

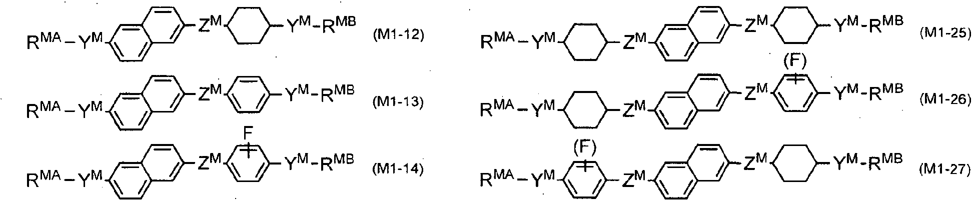

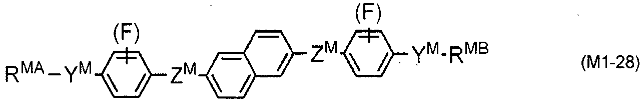

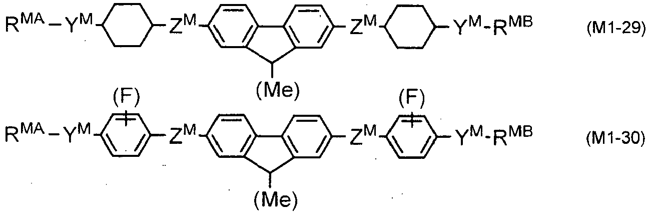

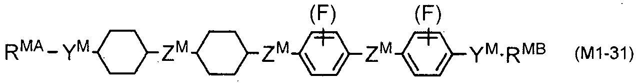

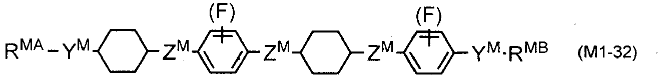

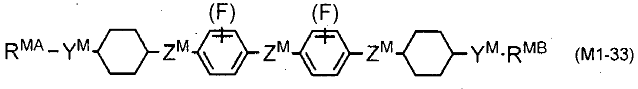

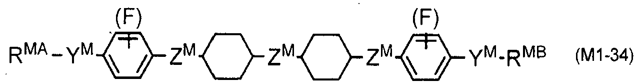

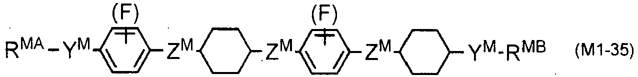

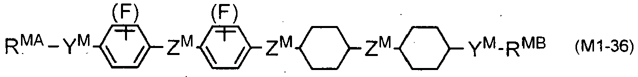

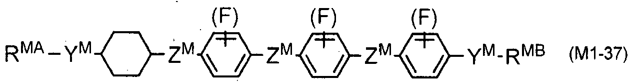

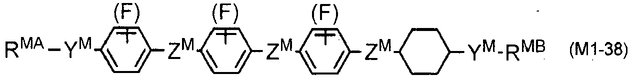

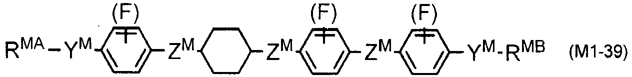

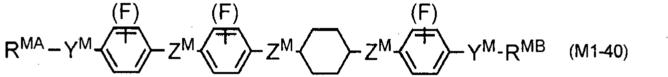

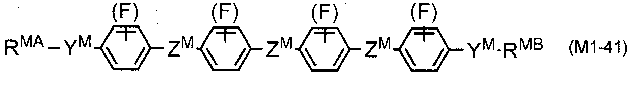

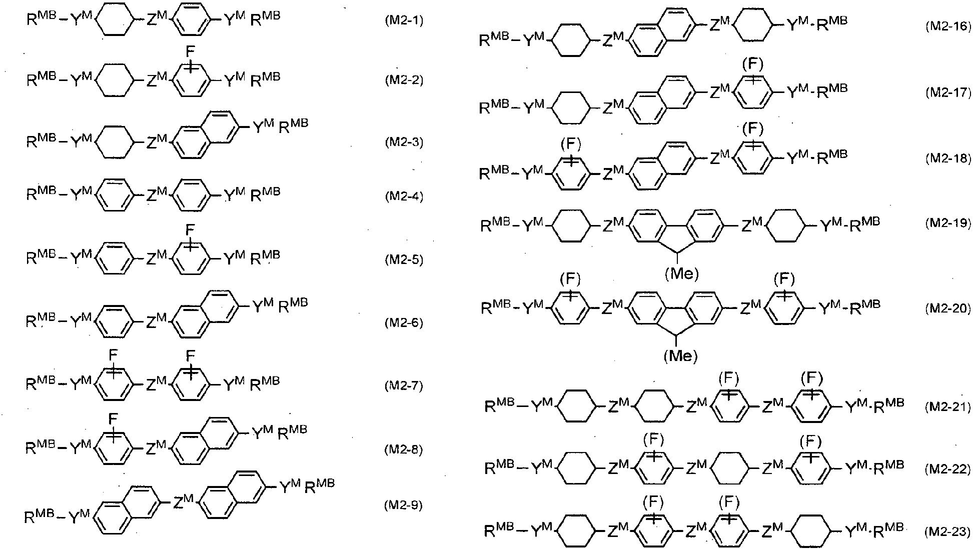

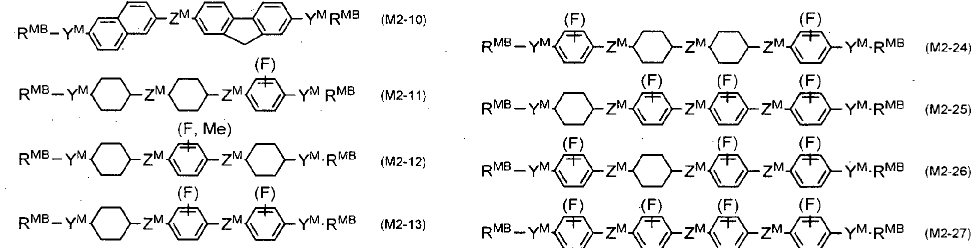

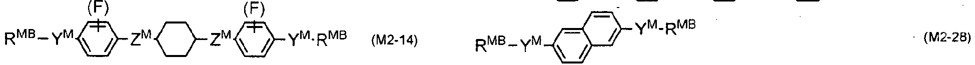

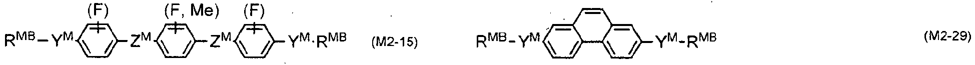

As the raw material monomer of a polymer, a polymerizable compound other than the monomer having no mesogen moiety, and monomers (M1) and (M2) each having the mesogen moiety as described above can be used when necessary.

A monomer having a mesogen moiety and three or more polymerizable functional groups can also be used for the purpose of optically optimizing isotropy of the polymer-liquid crystal composite material of the invention. A publicly-known compound can be suitably used as a monomer having a mesogen moiety and three or more polymerizable functional groups, but for example, compounds (M4-1) to (M4-6) are included, and more specific examples include compounds described in JP 2000-327632 A, JP 2004-182949 A and JP 2004-59772 A. However, in (M4-1) to (M4-6), R^{MB}, Z^{M}, Y^{M} and (F) indicate definitions identical to definitions described above.

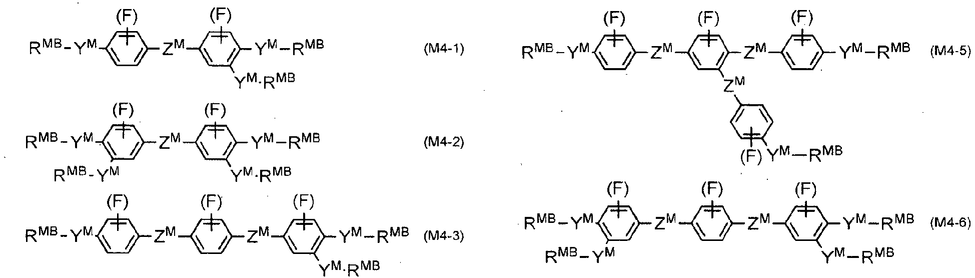

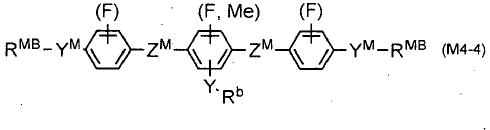

### 2-2-8. Monomer having a polymerizable functional group having no mesogen moiety

Specific examples of a monomer having a polymerizable functional group having no mesogen moiety include straight-chain or branched acrylate having 1 to 30 carbons, and straight-chain or branched diacrylate having 1 to 30 carbons, and specific examples of a monomer having three or more polymerizable functional groups include glycerol propoxylate (IPO/OH) triacrylate, pentaerythritol propoxylate triacrylate, pentaerythritol triacrylate, trimethylolpropane ethoxylate triacrylate, trimethylolpropane propoxylate triacrylate, trimethylolpropane triacrylate, di(trimethylolpropane) tetraacrylate, pentaerythritol tetraacrylate, di(pentaerythritol) pentaacrylate, di (pentaerythritol) hexa acrylate and trimethylolpropane triacrylate, but the monomers are not limited thereto.

### 2-2-9. Polymerization initiator

A polymerization reaction in production of a polymer composing a composite material of the invention is not particularly limited, and for example, photoradical polymerization, thermal radical polymerization, photocationic polymerization or the like is performed.

Specific examples of a photoradical polymerization initiator that can be used in the photoradical polymerization include DAROCUR (registered trade name) 1173 and 4265 (all are a trade name, BASF Japan Ltd.), and IRGACURE (registered trade name) 184, 369, 500, 651, 784, 819, 907, 1300, 1700, 1800, 1850 and 2959 (all are a trade name, BASF Japan Ltd.).

Specific examples of a preferred initiator of radical polymerization with heat that can be used in the thermal radical polymerization include benzoyl peroxide, diisopropyl peroxydicarbonate, t-butylperoxy-2-ethylhexanoate, t-butyl peroxypivalate, t-butyl peroxydiisobutyrate, lauroyl peroxide, dimethyl 2,2'-azobisisobutyrate (MAIB), di-t-butyl peroxide (DTBPO), azobisisobutyronitrile (AIBN) and azobis cyclohexanecarbonitrile (ACN) .

Specific examples of a photocationic polymerization initiator that can be used in the photocationic polymerization include diaryliodonium salt (hereinafter, referred to as "DAS") and triarylsulfonium salt (hereinafter, referred to as "TAS").

Specific examples of DAS include diphenyliodonium tetrafluoroborate, diphenyliodonium hexafluorophosphonate, diphenyliodonium hexafluoroarsenate, diphenyliodonium trifluoromethanesulfonate, diphenyliodonium trifluoroacetate, diphenyliodonium-p-toluenesulfonate, diphenyliodonium tetra(pentafluorophenyl)borate, 4-methoxyphenyl phenyliodonium tetrafluoroborate, 4-methoxyphenyl phenyliodonium hexafluorophosphonate, 4-methoxyphenyl phenyliodonium hexafluoroarsenate, 4-methoxyphenyl phenyliodonium trifluoromethanesulfonate, 4-methoxyphenyl phenyliodonium trifluoroacetate and 4-methoxyphenyl phenyliodonium-p-toluenesulfonate.

DAS can also be allowed to have high sensitivity by adding a photosensitizer such as thioxanthone, phenothiazine, chlorothioxanthone, xanthone, anthracene, diphenylanthracene and rubrene.

Specific examples of TAS include triphenylsulfonium tetrafluoroborate, triphenylsulfonium hexafluorophosphonate, triphenylsulfonium hexafluoroarsenate, triphenylsulfonium trifluoromethanesulfonate, triphenylsulfonium trifluoroacetate, triphenylsulfonium-p-toluenesulfonate, triphenylsulfonium tetra (pentafluorophenyl) borate, 4-methoxyphenyl diphenylsulfonium tetrafluoroborate, 4-methoxyphenyl diphenylsulfonium hexafluorophosphonate, 4-methoxyphenyl diphenylsulfonium hexafluoroarsenate, 4-methoxyphenyl diphenylsulfonium trifluoromethanesulfonate, 4-methoxyphenyl diphenylsulfonium trifluoroacetate and 4-methoxyphenyl diphenylsulfonium-p-toluenesulfonate.

Specific examples of a trade name of the photocationic polymerization initiator include Cyracure (registered trade name) UVI-6990, Cyracure UVI-6974 and Cyracure UVI-6992 (trade names respectively, Union Carbide Corporation), Adekaoptomer SP-150, Adekaoptomer SP-152, Adekaoptomer SP-170 and Adekaoptomer SP-172 (trade names respectively, ADEKA Corporation), Rhodorsil Photoinitiator 2074 (trade name, Rhodia Japan, LTD.), IRGACURE (registered trade name) 250 (trade name, BASF Japan Ltd.) and UV-9380C (trade name, GE Toshiba Silicones Co., Ltd.).

### 2-2-10. Curing agent or the like

In production of a polymer composing a composite material of the invention, one or more kinds of other suitable components in addition to the monomer or the like and the polymerization initiators, for example, a curing agent, a catalyst, a stabilizer or the like may be further added.

As the curing agent, a conventional publicly-known latent curing agent that is ordinarily used as a curing agent of epoxy resin can be used. Specific examples of the latent curing agent for epoxy resin include an amine curing agent, a novolak resin curing agent, an imidazole curing agent and an acid anhydride curing agent. Specific examples of the amine curing agents include: aliphatic polyamine such as diethylenetriamine, triethylenetetramine, tetraethylenepentamine, m-xylenediamine, trimethylhexamethylenediamine, 2 -methylpentamethylenediamine and diethylaminopropylamine; alicyclic polyamine such as isophorone diamine, 1,3-bisaminomethylcyclohexane, bis(4-aminocyclohexyl)methane, norbornenediamine, 1,2-diaminocyclohexane and laromine; and aromatic polyamine such as diaminodiphenylmethane, diaminodiphenylethane and metaphenylene diamine.

Specific examples of the novolak resin curing agent include phenol novolak resin and bisphenol novolak resin. Specific examples of the imidazole curing agent include 2-methylimidazole, 2-ethylhexylimidazole, 2-phenylimidazole and 1-cyanoethyl-2-phenylimidazolium trimellitate.

Specific examples of the acid anhydride curing agent include tetrahydrophthalic anhydride, hexahydrophthalic anhydride, methyltetrahydrophthalic anhydride, methylhexahydrophthalic anhydride, methylcyclohexene tetracarboxylic dianhydride, phthalic anhydride, trimellitic anhydride, pyromellitic anhydride and benzophenonetetracarboxylic dianhydride.

Moreover, a curing accelerator for accelerating a curing reaction of a polymerizable compound having a glycidyl group, an epoxy group and an oxetanyl group with a curing agent may be further used. Specific examples of the curing accelerator include: tertiary amines such as benzyldimethylamine, tris(dimethylaminomethyl)phenol and dimethylcyclohexylamine; imidazoles such as 1-cyanoethyl-2-ethyl-4-methylimidazole and 2-ethyl-4-methylimidazole; an organophosphorus compound such as triphenyl phosphine; quaternary phosphonium salts such as tetraphenyl phosphonium bromide; diazabicyclo alkene such as 1,8-diazabicyclo[5.4.0]undecene-7 and organic acid salt thereof; quaternary ammonium salts such as tetraethylammonium bromide and tetrabutylammonium bromide; and a boron compound such as boron trifluoride and triphenyl borate. The curing accelerators can be used alone or by mixing two or more kinds thereof.

Moreover, for example, for preventing undesired polymerization in storage, the stabilizer is preferably added. As the stabilizer, all the compounds known by those skilled in the art can be used. Specific examples of a representative stabilizer include 4-ethoxyphenol, hydroquinone and butylated hydroxytoluene (BHT).

### 2-2-9. Content of liquid crystal composition or the like

A content of a liquid crystal composition in a polymer-liquid crystal composite material of the invention is preferably a higher content as far as possible if the composite material is in a range in which an optically isotropic liquid crystal phase can be developed. The reason is that, as the content of the liquid crystal composition is higher, an electric birefringence value of the composite material of the invention becomes larger.

In the polymer-liquid crystal composite material of the invention, a content of the liquid crystal composition is preferably 60 to 99% by weight, further preferably 60 to 95% by weight, and particularly preferably 65 to 95% by weight, based on the composite material. A content of a polymer is preferably 1 to 40% by weight, further preferably 5 to 40% by weight, and particularly preferably 5 to 35% by weight, based on the composite material.

### 2-2-11. Other components that can be contained in a polymer-liquid crystal composite material

A polymer-liquid crystal composite material of the invention may contain dichroic dye and a photochromic compound in a range in which advantageous effects of the invention are not lost, for example.

Hereinafter, the invention will be described in greater detail by way of Examples, but the invention is not limited by the Examples. In addition, unless otherwise noted, "%" means "% by weight".

### 3. Liquid crystal display device

Composition (1) can be used in a liquid crystal display device having an operating mode such as the PC mode, the TN mode, the STN mode, the OCB mode and the PSA mode, and driven by an active matrix (AM mode) . Composition (1) can also be used in the liquid crystal display device having the operating mode such as the PC mode, the TN mode, the STN mode, the OCB mode, the VA mode and the IPS mode, and driven by a passive matrix (PM) mode. The AM mode device and the PM mode device can also be applied to any of a reflective type, a transmissive type and a transflective type.

Composition (1) can also be used in a nematic curvilinear aligned phase (NCAP) device prepared by microencapsulating a nematic liquid crystal, and a polymer dispersed liquid crystal display device (PDLCD) and a polymer network liquid crystal display device (PNLCD) in which a three-dimensional network-polymer is formed in the liquid crystal.

Specific examples of the polymer network liquid crystal display device (PNLCD) include an optical device to be driven in an optically isotropic liquid crystal phase including a liquid crystal composition or a polymer-liquid crystal composite material (hereinafter, referred to as a liquid crystal medium as a generic term for the liquid crystal composition and the polymer-liquid crystal composite material of the invention).

The liquid crystal medium is optically isotropic during no application of an electric field, but if the electric field is applied, the liquid crystal medium has optical anisotropy generated, and light modulation by the electric field becomes possible.

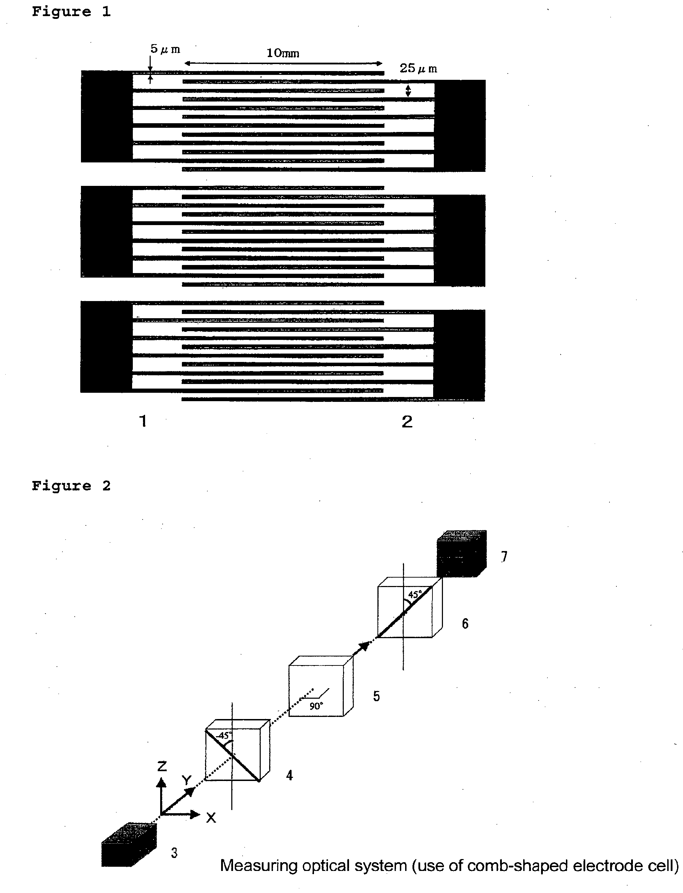

As shown in Figure 1, specific examples of a structure of a liquid crystal display device include a structure in which, in electrodes of a comb-shaped electrode substrate, electrode 1 extended from a left side and electrode 2 extended from a right side are alternately arranged. When a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

### Examples

The invention will be described in greater detail by way of Examples . The invention is not limited by the Examples.

### 4-1. Example of Compound (1)

Compound (1) was prepared according to procedures described below. The thus prepared compound was identified by methods such as an NMR analysis . Physical properties of the compound were measured by methods described below.

### NMR analysis

For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In explaining nuclear magnetic resonance spectra obtained, s, d, t, q, quin, sex and m stand for a singlet, a doublet, a triplet, a quartet, a quintet, a sextet and a multiplet, and br being broad, respectively.

### Sample for measurement

Upon measuring phase structure and transition temperature, a liquid crystal compound itself was used as a sample. Upon measuring physical properties such as a maximum temperature of a nematic phase, viscosity, optical anisotropy and dielectric anisotropy, a composition prepared by mixing the compound with a base liquid crystal was used as a sample.

When a sample in which the compound was mixed with the base liquid crystal was used, measurement was carried out as described below. The sample was prepared by mixing 15% by weight of the compound and 85% by weight of the base liquid crystal. From a measured value of the sample, an extrapolated value was calculated according to an extrapolation method represented by the following formula, and the calculated value was described: [extrapolated value] = (100 × [measured value of a sample] - [% by weight of a base liquid crystal] × [measured value of the base liquid crystal]) / [% by weight of a compound].

When crystals (or a smectic phase) precipitated at 25°C even at the ratio of the compound to the base liquid crystal, a ratio of the compound to the base liquid crystal was changed in the order of (10% by weight : 90% by weight), (5% by weight : 95% by weight) and (1% by weight : 99% by weight), and physical properties of the sample were measured at a ratio at which no crystal (or no smectic phase) precipitated at 25°C. In addition, unless otherwise noted, the ratio of the compound to the base liquid crystal was (15% by weight : 85% by weight).

As the base liquid crystal, base liquid crystal (i) described below was used. Proportions of components in base liquid crystal (i) were expressed in terms of % by weight.

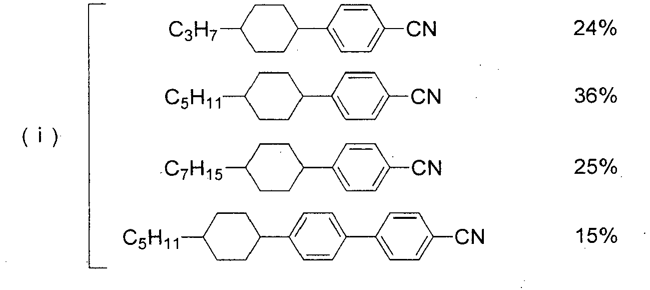

### Measuring method

Physical properties were measured according to methods described below. Most of the measuring methods are applied as described in the Standard of Japan Electronics and Information Technology Industries Association (hereinafter abbreviated as JEITA) (JEITA ED-2521B) discussed and established by JEITA, or modified thereon. No TFT was attached to a TN device used for measurement.

### (1) Phase structure

A sample was placed on a hot plate in a melting point apparatus (FP-52 Hot Stage made by Mettler-Toledo International Inc.) equipped with a polarizing microscope, a state of phase and a change thereof were observed with the polarizing microscope while the sample was heated at a rate of 3°C per minute, and a kind of the phase was specified.

### (2) Transition temperature (°C)

For measurement, a differential scanning calorimeter, Diamond DSC System, made by PerkinElmer, Inc. , or a high sensitivity differential scanning calorimeter, X-DSC7000, made by SII NanoTechnology Inc. was used. A sample was heated and then cooled at a rate of 3°C per minute, and a starting point of an endothermic peak or an exothermic peak caused by a phase change of the sample was determined by extrapolation, and thus a transition temperature was determined. Temperature at which a compound undergoes transition from a solid to a liquid crystal phase such as the smectic phase and the nematic phase may be occasionally abbreviated as "minimum temperature of the liquid crystal phase." Temperature at which the compound undergoes transition from the liquid crystal phase to an isotropic liquid may be occasionally abbreviated as "clearing point."

Hereinafter, crystals was expressed as C, and further, when the crystals were distinguishable, each of the crystals was expressed as C₁ or C₂. Moreover, the smectic phase or the nematic phase was expressed as Sm or N. A liquid (isotropic) was expressed as I. When smectic B phase or smectic A phase was distinguishable among the smectic phases, the phases were expressed as SmB or SmA, respectively. A chiral nematic phase was expressed as N*. BP represents a blue phase or an optically isotropic liquid crystal phase. A coexisting state of two phases may be occasionally described in the form of (N*+I) and (N*+BP). Specifically, (N*+I) represents a phase in which a non-liquid crystal isotropic phase and the chiral nematic phase coexist, and (N*+BP) represents a phase in which the BP phase or the optically isotropic liquid crystal phase and the chiral nematic phase coexist, respectively. Un represents a unconfirmed phase having no optical isotropy. As an expression of phase transition temperature, for example, an expression "C 50.0 N 100.0 I" indicates that phase transition temperature (CN) from the crystals to the nematic phase is 50.0°C, and phase transition temperature (NI) from the nematic phase to the liquid is 100.0°C. A same rule applies also to any other expression.

### (3) Compatibility at low temperature

Samples in which the base liquid crystal and the compound were mixed for proportions of the compounds to be 20% by weight, 15% by weight, 10% by weight, 5% by weight, 3% by weight and 1% by weight were prepared, and put in glass vials. After the glass vials were kept in freezers at -10°C or -20°C for a predetermined period of time, whether or not crystals or a smectic phase precipitated was observed.

### (4) Maximum temperature of nematic phase (T_{NI} or NI; °C)

A sample was placed on a hot plate in a melting point apparatus equipped with a polarizing microscope, and heated at a rate of 1°C per minute. Temperature when part of the sample began to change from a nematic phase to an isotropic liquid was measured. A maximum temperature of the nematic phase may be occasionally abbreviated as "maximum temperature." When the sample was a mixture of a compound and the base liquid crystal, the maximum temperature was expressed in terms of a symbol T_{NI} . When the sample was a mixture of a compound and component B or the like, the maximum temperature was expressed in terms of a symbol NI.

### (5) Minimum temperature of nematic phase (T_{c}; °C)

Samples each having a nematic phase were kept in freezers at temperatures of 0°C, -10°C, -20°C, -30°C and -40°C for 10 days, and then liquid crystal phases were observed. For example, when the sample was maintained in the nematic phase at -20°C and changed to crystals or a smectic phase at -30°C, Tc was expressed as T_{c} ≤ -20°C. A minimum temperature of the nematic phase may be occasionally abbreviated as "minimum temperature."

### (6) Viscosity (bulk viscosity; η; measured at 20°C; mPa·s)

For measurement, a cone-plate (E type) rotational viscometer made by Tokyo Keiki Inc. was used.

### (7) Viscosity (rotational viscosity; γ1; measured at 25°C; mPa·s)

Measurement was carried out according to a method described in M. Imai et al. , Molecular Crystals and Liquid Crystals, Vol. 259, 37 (1995) . A sample was put in a TN device in which a twist angle was 0 degrees and a distance (cell gap) between two glass substrates was 5 micrometers . Voltage was applied stepwise to the device in the range of 16 V to 19.5 V at an increment of 0.5 V. After a period of 0.2 second with no voltage application, voltage was repeatedly applied under conditions of only one rectangular wave (rectangular pulse; 0.2 second) and no voltage application (2 seconds) . A peak current and a peak time of transient current generated by the applied voltage were measured. A value of rotational viscosity was obtained from the measured values and calculation equation (8) described on page 40 of the paper presented by M. Imai et al. A value of dielectric anisotropy required for the calculation was determined using the device by which the rotational viscosity was measured and by a method described below.

### (8) Optical anisotropy (refractive index anisotropy; measured at 25°C; Δn)

Measurement was carried out by an Abbe refractometer with a polarizing plate mounted on an ocular, using light at a wavelength of 589 nanometers. A surface of a main prism was rubbed in one direction, and then a sample was added dropwise onto the main prism. A refractive index (n||) was measured when a direction of polarized light was parallel to a direction of rubbing. A refractive index (n⊥) was measured when the direction of polarized light was perpendicular to the direction of rubbing. A value of optical anisotropy (Δη) was calculated according to an equation: Δn = n|| - n⊥.

### (9) Dielectric anisotropy (Δε; measured at 25°C)

A sample was put in a TN device in which a distance (cell gap) between two glass substrates was 9 micrometers and a twist angle was 80 degrees. Sine waves (10 V, 1 kHz) were applied to the device, and after 2 seconds, a dielectric constant (ε||) of liquid crystal molecules in a major axis direction was measured. Sine waves (0.5 V, 1 kHz) were applied to the device, and after 2 seconds, a dielectric constant (ε⊥) of liquid crystal molecules in a minor axis direction was measured. A value of dielectric anisotropy was calculated according to an equation: Δε = εll - ε⊥.

### (10) Elastic constant (K; measured at 25°C; pN)

For measurement, HP4284A LCR Meter made by Yokogawa-Hewlett-Packard Co. was used. A sample was put in a horizontal alignment device in which a distance (cell gap) between two glass substrates was 20 micrometers. An electric charge of 0 V to 20 V was applied to the device, and electrostatic capacity and applied voltage were measured. The measured values of electrostatic capacity (C) and applied voltage (V) were fitted to equation (2.98) and equation (2.101) on page 75 of "Liquid Crystal Device Handbook" (Handbook of Liquid Crystals (Ekisho Debaisu Handobukku in Japanese) ; Nikkan Kogyo Shimbun, Ltd.) and values of K₁₁ and K₃₃ were obtained from equation (2.99) . Next, K₂₂ was calculated using the previously determined values of K₁₁ and K₃₃ in equation (3.18) on page 171. Elastic constant K was expressed in terms of a mean value of the thus determined K₁₁, K₂₂ and K₃₃.

### (11) Threshold voltage (Vth; measured at 25°C; V)

For measurement, an LCD5100 luminance meter made by Otsuka Electronics Co., Ltd. was used. A light source was a halogen lamp. A sample was put in a normally white mode TN device in which a distance (cell gap) between two glass substrates was about 0.45/Δn (µm) and a twist angle was 80 degrees. A voltage (32 Hz, rectangular waves) to be applied to the device was stepwise increased from 0 V to 10 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of a voltage at 90% transmittance.

### (12) Voltage holding ratio (VHR-1; measured at 25°C; %)

A TN device used for measurement had a polyimide alignment film, and a distance (cell gap) between two glass substrates was 5 micrometers . A sample was put in the device, and then the device was sealed with an ultraviolet-curable adhesive. The device was charged by applying a pulse voltage (60 microseconds at 5 V) at 25°C. A decaying voltage was measured for 16.7 milliseconds with a high-speed voltmeter, and area A between a voltage curve and a horizontal axis in a unit cycle was determined. Area B is an area without decay. The voltage holding ratio is expressed in terms of a percentage of area A to area B.

### (13) Voltage holding ratio (VHR-2; measured at 80°C; %)

A voltage holding ratio (VHR-2) was determined according to a method similar to the method in VHR-1 except that measurement was carried out at 80°C.

### (14) Pitch (P; measured at 25°C; nm)

Pitch length was measured using selective reflection (Handbook of Liquid Crystals (Ekisho Binran in Japanese), page 196, issued in 2000, Maruzen Co. , Ltd.) . In selective reflection wavelength λ, a relational expression: <n> p / λ = 1 hold. Here, <n> represents an average refractive index, and is given by the following equation: <n> = {(n||² + n⊥²) / 2}^{1/2}. The selective reflection wavelength was measured by a microspectrophotometer (trade name "MSV-350," JEOL Ltd.). A pitch was determined by dividing the obtained reflection wavelength by the average refractive index. A pitch of a cholesteric liquid crystal having the reflection wavelength in a longer wavelength region in comparison with visible light was proportional to a reciprocal of a concentration of a chiral agent in a region in which the chiral agent concentration is low, and therefore the pitch was determined by a linear extrapolation method by measuring several pitch lengths of the liquid crystal having the selective reflection wavelength in a visible light region. "Optically active compound" corresponds to the chiral agent in the invention.

### Raw material

Solmix A-11 (trademark) is a mixture of ethanol (85.5%), methanol (13.4%) and isopropanol (1.1%), and was purchased from Japan Alcohol Trading Co., Ltd.

### Example 1

### Synthesis of

### 2-(4-(difluoro-((2,3',4',5'-tetrafluoro-[1,1'-biphenyl]-4-yl)oxy)m ethyl)-3,5-difluorophenyl)-5-(ethoxymethyl)-1,3-dioxane (No. 148)

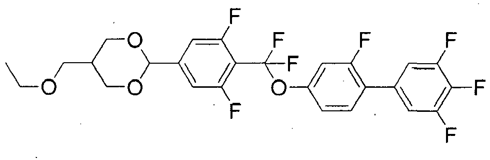

A synthesis scheme is shown in a diagram below.

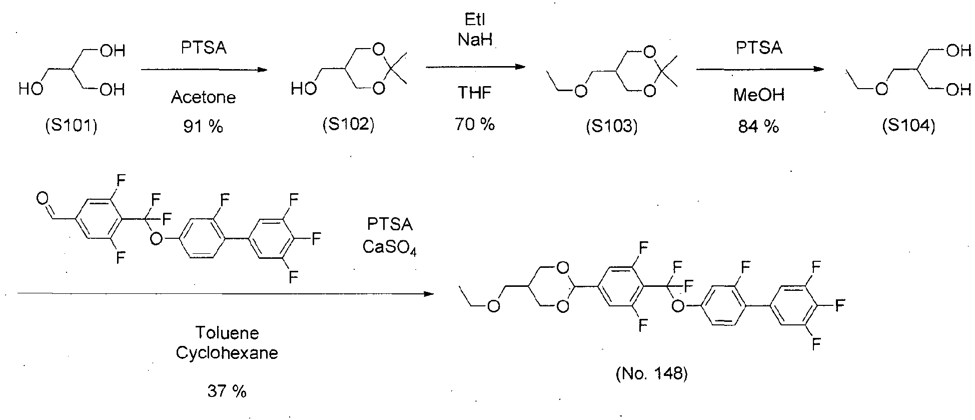

### First step

Under a nitrogen atmosphere, triethyl methanetricarboxylate (0.90 g, 8.5 mmol), p-toluenesulfonic acid monohydrate (0.05 g, 0.25 mmol), and acetone (27 mL) were put in a reaction vessel, and the resulting mixture was stirred at room temperature for 12 hours. Triethylamine was added to the resulting reaction mixture, and the solvent was distilled off by an evaporator. The residue was purified by silica gel chromatography to obtain compound (S102) (1.1 g, 7.8 mmol; 91%).

### Second step

Under a nitrogen atmosphere, compound (S102) (1.1 g, 7.8 mmol) obtained in the first step, sodium hydride (60%; 0.47 g, 11.7 mmol) and THF (11 mL) were put in a reaction vessel, and the resulting mixture was stirred at room temperature for 30 minutes. Thereto, 1-iodoethane (3.6 g, 23.4 mmol) was added, and the resulting mixture was stirred at room temperature for 5 hours. The resulting reaction mixture was poured into pure water, and an aqueous layer was subjected to extraction with ethyl acetate. Combined organic layers were washed with pure water and saturated brine, and then dried over magnesium sulfate, and the solvent was distilled off by an evaporator. The residue was purified by silica gel chromatography to obtain compound (S103) (0.95 g, 5.4 mmol; 70%).

### Third step

Under a nitrogen atmosphere, compound (S103) (0.95 g, 5.4 mmol) obtained in the second step, p-toluenesulfonic acid monohydrate (0.1 g, 0.54 mmol) and methanol (4.7 mL) were put in a reaction vessel, and the resulting mixture was stirred at room temperature for 12 hours. Triethylamine was added to the resulting reaction mixture, and the solvent was distilled off by an evaporator. The residue was purified by silica gel chromatography to obtain compound (S104) (0.62 g, 4.6 mmol; 84%).

### Fourth step

Under a nitrogen atmosphere, compound (S104) (0.62 g, 4.6 mmol) obtained in the third step, 4-(difluoro((2,3',4',5'-tetrafluoro-[1,1'-biphenyl]-4-yl)oxy)methy l)-3,5-difluorobenzaldehyde (2.0 g, 4.6 mmol), p-toluenesulfonic acid monohydrate (0.06 g, 0.32 mmol), calcium sulfate (0.6 g, 4.5 mmol), toluene (6 mL) and cyclopropane (6 mL) were put in a reaction vessel, and the resulting mixture was refluxed under heating for 3 hours. The resulting reaction mixture was returned to room temperature, and then poured into pure water, and an aqueous layer was subjected to extraction with toluene. Combined organic layers were washed with pure water and saturated brine, and then dried over magnesium sulfate, and the solvent was distilled off by an evaporator. The residue was purified by silica gel chromatography and recrystallization to obtain compound (No. 148) (0.95 g, 1.7 mmol; 37%).

¹H-NMR (δ ppm; CDCl₃) : 7.34 (dd, 1H, J = 8.4 Hz, 8.4 Hz), 7.19 - 7.10 (m, 6H), 5.39 (s, 1H), 4.28 (dd, 2H, J = 4.6 Hz, 11.8 Hz) 3.74 (dd, 2H, J = 11.8 Hz, 11.8 Hz), 3.45 (q, 2H, J = 7.0 Hz), 3 .28 (d, 2H, J = 5.8 Hz) 2.43 (m, 1H), 1.19 (t, 3H, J = 7.0 Hz).
¹⁹F-NMR (δ ppm; CFCl₃) : -61.43 (t, 2F, J = 28.5 Hz), -111.47 (dt, 2F, J = 11.3 Hz, 28.5 Hz), -114.86 (dd, 1F, J = 8.5 Hz, 10.6 Hz), -134.71 (dd, 2F, J = 9.4 Hz, 21.4 Hz), -161.72 (tt, 1F, J = 6.7 Hz, 21.4 Hz) .

Physical properties of compound (No. 148) were as described below.

Attached data were determined according to the methods described above. Upon measuring transition temperature, a compound itself was used as a sample. Upon measuring maximum temperature (T_{NI}), viscosity (η), optical anisotropy (Δn) and dielectric anisotropy (Δε), a mixture of a compound (15% by weight) and base liquid crystal (A) (85% by weight) was used as a sample. From a measured value thereof, an extrapolated value was calculated according to the extrapolation method described above, and the calculated value was described:
Transition temperature: C 64.7 N 64.7 I. T_{NI} = 43; η = 70 mPa·s; Δn = 0.117; Δε = 54.57.

### Example 2

### Synthesis of

### 5-(butoxymethyl)-2-(4-(difluoro-((2,3',4'-trifluoro-[1,1'-biphenyl]-4-yl)oxy)methyl)-3,5-difluorophenyl)-1,3-dioxane (No. 142) .

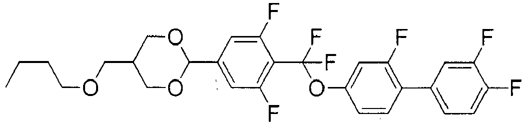

Compound (No. 142) was prepared in a manner similar to Example 1.

¹H-NMR (δ ppm; CDCl₃): 7.38 - 7.32 (m, 2H), 7.25 - 7.10 (m, 2H), 7.17 - 7.09 (m, 4H), 5.39 (s, 1H), 4.28 (dd, 2H, J = 4.6 Hz, 11.8 Hz) 3.74 (dd, 2H, J = 11.8 Hz, 11.8 Hz), 3.38 (t, 2H, J = 6.6 Hz), 3.27 (d, 2H, J = 5.8 Hz), 2.43 (m, 1H), 1.57 - 1.50 (m, 2H), 1.36 (dq, 2H, J = 7.5 Hz, 7.5 Hz), 0.93 (t, 3H, J = 7.5 Hz).
¹⁹F-NMR (δ ppm; CFCl₃) : -61.40 (t, 2F, J = 28.4 Hz), -110.48 (dt, 2F, J = 10.6 Hz, 28.4 Hz), -115.14 (dd, 1F, J = 8.5 Hz, 10.6 Hz), -137.97--138.07 (m, 1F), -139.25--139.35 (m, 1F).

Physical properties of compound (No. 142) were as described below.

A mixture of a compound (15% by weight) and base liquid crystal (A) (85% by weight) was used as a sample. From a measured value thereof, an extrapolated value was calculated according to the extrapolation method described above, and the calculated value was described:
Transition temperature: C 46.6 I. T_{NI} = 38.4; η = 72.6 mPa·s; Δn = 0.1037; Δε = 35.8.

### Example 3

### Synthesis of

### 5-(butoxymethyl)-2-(4'-((3,4-difluorophenoxy)difluoromethyl)-2,3', 5'-trifluoro-[1,1'-biphenyl]-4-yl)-1,3-dioxane (No. 124)

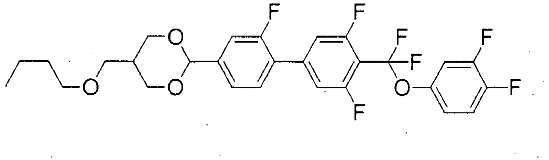

Compound (No. 124) was prepared in a manner similar to Example 1.

¹H-NMR (δ ppm; CDCl₃) : 7.42 (dd, 1H, J = 8.1, 8.1 Hz), 7.39 - 7.34 (m, 2H), 7.22 - 7.13 (m, 4H), 7.18 - 7.03 (m, 1H), 5.45 (s, 1H), 4.30 (dd, 2H, J = 4.6 Hz, 11. 7 Hz) 3.77 (dd, 2H, J = 11.7 Hz, 11.7 Hz), 3.39 (t, 2H, J = 6.5 Hz), 3.28 (d, 2H, J = 5.9 Hz), 2.47 (m, 1H), 1.58 - 1.51 (m, 2H), 1.37 (dq, 2H, J = 7.5 Hz, 7.5 Hz), 0.93 (t, 3H, J = 7.4 Hz) .
¹⁹F-NMR (δ ppm; CFCl₃) : -61.94 (t, 2F, J = 28.2 Hz), -111.10 (dt, 2F, J = 11.8 Hz, 28.2 Hz), -117.30 (dd, 1F, J = 8.3 Hz, 10.6 Hz), -134.77--134.88 (m, 1F), -140.88--140.11 (m, 1F).

Physical properties of compound (No. 124) were as described below.

A mixture of a compound (15% by weight) and base liquid crystal (A) (85% by weight) was used as a sample. From a measured value thereof, an extrapolated value was calculated according to the extrapolation method described above, and the calculated value was described:
Transition temperature: C 43.1 N 61.8 I. T_{NI} = 61. 0; η = 82.6 mPa·s; Δn = 0.117; Δε = 36.97.

### Example 4

### Synthesis of

### 5-(ethoxymethyl)-2-(2,3',4',5'-tetrafluoro-[1,1'-biphenyl]-4-yl)-1 ,3-dioxane (No. 12)

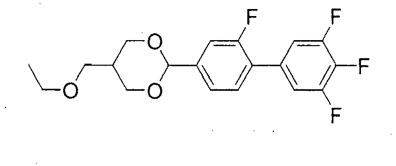

Compound (No. 12) was prepared in a manner similar to Example 1.

¹H-NMR (δ ppm; CDCl₃) : 7.39 - 7.31 (m, 3H), 7.20 - 7.13 (m, 2H), 5.45 (s, 1H), 4.30 (dd, 2H, J = 4.6 Hz, 11.7 Hz) 3.77 (dd, 2H, J = 11. 7 Hz, 11.7 Hz), 3.45 (q, 2H, J = 6.9 Hz), 3.28 (d, 2H, J = 5.9 Hz), 2.47 (m, 1H), 1.20 (t, 3H, J = 6.9 Hz) .
¹⁹F-NMR (δ ppm; CFCl₃) : -111.09 (dd, 1F, J = 7.1 Hz, 8.7 Hz), -134.97 (dd, 1F, J = 8.7 Hz, 21.3 Hz), -161.98 (dt, 1F, J = 8.7 Hz, 21.3 Hz) .

Physical properties of compound (No. 12) were as described below.

A mixture of a compound (15% by weight) and base liquid crystal (A) (85% by weight) was used as a sample. From a measured value thereof, an extrapolated value was calculated according to the extrapolation method described above, and the calculated value was described:
Transition temperature: C 49.8 SB 53.2 I. T_{NI} = 4.4; η = 65.7 mPa·s; Δn = 0.084; Δε = 37.43.

### Example 5

### Synthesis of 2-(4-(difluoro-(3,4,5-trifluorophenoxy)methyl)-3,5-difluorophenyl) -5-(ethoxymethyl)-1,3-dioxane (No.44)

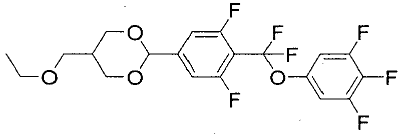

Compound (No. 44) was prepared in a manner similar to Example 1.

¹H-NMR (δ ppm; CDCl₃): 7.14 (d, 2H, J = 10.0 Hz), 6.95 (dd, 2H, J = 5.9 Hz, 7.6 Hz), 5.38 (s, 1H), 4.28 (dd, 2H, J = 4.6 Hz, 11.7 Hz) 3.74 (dd, 2H, J = 11.7 Hz, 11.7 Hz), 3.45 (q, 2H, J = 6.9 Hz), 3.28 (d, 2H, J = 5.9 Hz), 2.43 (m, 1H), 1.19 (t, 3H, J = 6.9 Hz).
¹⁹F-NMR (δ ppm; CFCl₃) : -62.29 (t, 2F, J = 28.5 Hz), -111.66 (dt, 2F, J = 11.0 Hz, 28.5 Hz), -132.99 (dd, 2F, J = 9.4 Hz, 21.4 Hz), -163.70 (tt, 1F, J = 6.7 Hz, 21.4 Hz).

Physical properties of compound (No. 44) were as described below.

A mixture of a compound (15% by weight) and base liquid crystal (A) (85% by weight) was used as a sample . From a measured value thereof, an extrapolated value was calculated according to the extrapolation method described above, and the calculated value was described:
Transition temperature: C 63.4 I. T_{NI} = -32.3; η = 40.0 mPa·s; Δn = 0.050; Δε = 42.57.

### Example 6

### Synthesis of

### 5-(ethoxymethyl)-2-(2,3',3",4",5"-pentafluoro-[1,1':4',1"-terpheny 1]-4-yl)-1,3-dioxane (No. 75)

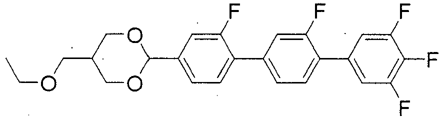

Compound (No. 75) was prepared in a manner similar to Example 1.

¹H-NMR (δ ppm; CDCl₃): 7.49 - 7.32 (m, 6H), 7.25 - 7.19 (m, 2H), 5.46 (s, 1H), 4.31 (dd, 2H, J = 4.6 Hz, 11.7 Hz) 3.77 (dd, 2H, J = 11. 7 Hz, 11.7 Hz), 3.46 (q, 2H, J = 7.0 Hz), 3.29 (d, 2H, J = 5.9 Hz), 2.48 (m, 1H), 1.20 (t, 3H, J = 7.0 Hz) .
¹⁹F-NMR (δ ppm; CFCl₃) : -117.85 (dd, 1F, J = 8.5 Hz, 11.5 Hz), -118. 08 (dd, 1F, J = 8.0 Hz, 12.5 Hz), -134.78 (dd, 1F, J = 8.6 Hz, 21.3 Hz), -161.78 (tt, 1F, J = 6.6 Hz, 21.3 Hz).

Physical properties of compound (No. 75) were as described below.

A mixture of a compound (5% by weight) and base liquid crystal (A) (95% by weight) was used as a sample. From a measured value thereof, an extrapolated value was calculated according to the extrapolation method described above, and the calculated value was described:
Transition temperature: C 113.6 SB 150.7 N 166.4 I. T_{NI} = 113.7; η = 81.1 mPa·s; Δn = 0.177; Δε = 41.8.

### Example 7

### Synthesis of

### 2-(4'-(difluoro-(3,4,5-trifluorophenoxy)methyl)-3',5'-difluoro-[1, 1'-biphenyl]-4-yl)-5 -(ethoxymethyl)-1,3-dioxane (No. 122)

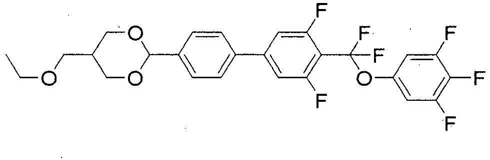

Compound (No. 122) was prepared in a manner similar to Example 1.

¹H-NMR (δ ppm; CDCl₃) : 7.61 (d, 2H, J = 8.5 Hz), 7.56 (d, 2H, J = 8.5 Hz), 7.20 (d, 2H, J = 11.3 Hz), 6.99 (dd, 2H, J = 6.0, 7.6 Hz), 5.48 (s, 1H), 4.31 (dd, 2H, J = 4.6 Hz, 11.7 Hz) 3.77 (dd, 2H, J = 11.7 Hz, 11.7 Hz), 3.45 (q, 2H, J = 7.0 Hz), 3.29 (d, 2H, J = 6.0 Hz), 2.48 (m, 1H), 1.20 (t, 3H, J = 7.0 Hz).
¹⁹F-NMR (δ ppm; CFCl₃) : -62.18 (t, 2F, J = 28.2 Hz), -111.87 (dt, 2F, J = 11.3 Hz, 28.2 Hz), -132.97 (dd, 1F, J = 7.6 Hz, 21.3 Hz), -163.67 (dd, 1F, J = 6.0 Hz, 21.3 Hz).

Physical properties of compound (No. 122) were as described below.

A mixture of a compound (5% by weight) and base liquid crystal (A) (95% by weight) was used as a sample. From a measured value thereof, an extrapolated value was calculated according to the extrapolation method described above, and the calculated value was described:
Transition temperature: C 110.1 SE 121.1 SB 129.3 I. T_{NI} = 95.7; η = 69.5 mPa·s; Δn = 0.157; Δε = 43.9.

### Example 8

### Synthesis of

### 2-(4'-(difluoro-(3,4,5-trifluorophenoxy)methyl)-2,3',5'-trifluoro-[1,1'-biphenyl]-4-yl)-5-(ethoxymethyl)-1,3-dioxane (No. 128)

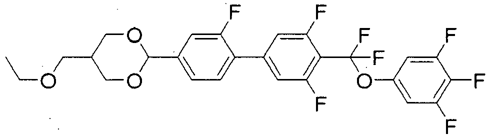

Compound (No. 128) was prepared in a manner similar to Example 1.

¹H-NMR (δ ppm; CDCl₃) : 7.46 - 7.33 (m, 3H), 7.20 (d, 2H, J = 10.6 Hz), 7.04 - 6.95 (m, 2H), 5.46 (s, 1H), 4.30 (dd, 2H, J = 4.6 Hz, 11.7 Hz) 3.77 (dd, 2H, J = 11.7 Hz, 11.7 Hz), 3.46 (q, 2H, J = 6.9 Hz), 3.29 (d, 2H, J = 5.9 Hz), 2.47 (m, 1H), 1.20 (t, 3H, J = 6.9 Hz).
¹⁹F-NMR (δ ppm; CFCl₃) : -62.26 (t, 2F, J = 28.0 Hz), -111.10 (dt, 2F, J = 10.7 Hz, 28.0 Hz), -117.29 (dd, 1F, J = 8.3 Hz, 11.3 Hz), -132.92 (dd, 1F, J = 8.3 Hz, 21.7 Hz), -163.60 (tt, 1F, J = 5.8 Hz, 21.7 Hz) .

Physical properties of compound (No. 128) were as described below.

A mixture of a compound (15% by weight) and base liquid crystal (A) (85% by weight) was used as a sample. From a measured value thereof, an extrapolated value was calculated according to the extrapolation method described above, and the calculated value was described:
Transition temperature: C 72.7 N 95.3 N 99.4 I. T_{NI} = 71; η = 79.8 mPa·s; Δn = 0.130; Δε = 57.9.

### Example 9

### Synthesis of

### 2-(4'-((3,5-difluoro-4-(trifluoromethyl)phenoxy)difluoromethyl-2,3 ',5'-trifluoro-[1,1'-biphenyl]-4-yl)-5-(ethoxymethyl)-1,3-dioxane (No. 129)

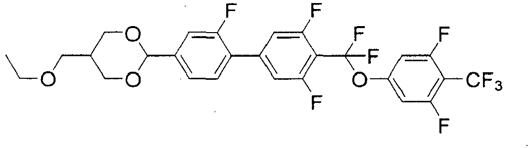

Compound (No. 129) was prepared in a manner similar to Example 1.

¹H-NMR (δ ppm; CDCl₃) : 7.46 - 7.33 (m, 3H), 7.20 (d, 2H, J = 10.7 Hz)6.98 (d, 2H, J = 9.9 Hz), 5.46 (s, 1H), 4.30 (dd, 2H, J = 4.6 Hz, 11.8 Hz) 3. 7 7 (dd, 2H, J = 11.8 Hz, 11.8 Hz), 3.46 (q, 2H, J = 6.9 Hz), 3.29 (d, 2H, J = 5.9 Hz), 2.47 (m, 1H), 1.20 (t, 3H, J = 6.9 Hz).
¹⁹F-NMR (δ ppm; CFCl₃) : 56.75 (t, 2F, J = 23.0 Hz), -2.27 (t, 2F, J = 27.8 Hz), -108.74 (dq, 2F, J = 10.4 Hz, 23.0 Hz), -111.00 (dt, 1F, J = 8.3 Hz, 27.8 Hz), -117.27 (dd, 1F, J = 8.3 Hz, 11.8 Hz).

Physical properties of compound (No. 129) were as described below.

A mixture of a compound (10% by weight) and base liquid crystal (A) (90% by weight) was used as a sample. From a measured value thereof , an extrapolated value was calculated according to the extrapolation method described above, and the calculated value was described:

Transition temperature: C 102.4 SB 122.7 I. T_{NI} = 67.7; η = 85.6 mPa·s; Δn = 0.137; Δε = 67.8.

### Example 10

### Synthesis of

### 2-(4-(difluoro((2,3',4'-trifluoro-4-[1,1'-biphenyl]-4-yl)oxy)methy l)-3-fluorophenyl)-5-(ethoxymethyl)-1,3-dioxane (No. 140)

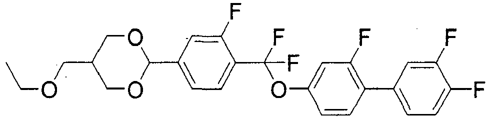

Compound (No. 140) was prepared in a manner similar to Example 1.

¹H-NMR (δ ppm; CDCl₃) : 7.70 (d, 1H, J = 8.7 Hz) 7.39 - 7.32 (m, 4H), 7.27 - 7.20 (m, 2H), 7.17 - 7.09 (m, 2H), 5.44 (s, 1H), 4.29 (dd, 2H, J = 4.6 Hz, 11.8 Hz) 3.75 (dd, 2H, J = 11.8 Hz, 11.8 Hz), 3.45 (q, 2H, J = 6.9 Hz), 3.28 (d, 2H, J = 5.9 Hz), 2.45 (m, 1H), 1.19 (t, 3H, J 6. 9 Hz) .
¹⁹F-NMR (δ ppm; CFCl₃) : -65.66 (d, 2F, J = 13.2 Hz), -114.22--114.36 (m, 1F), -115.26 (dd, 1F, J = 8.5 Hz, 10.6 Hz), -137.97--138.09 (m, 1F), -139.28--139.40 (m, 1F).

Physical properties of compound (No. 140) were as described below.

A mixture of a compound (15% by weight) and base liquid crystal (A) (85% by weight) was used as a sample . From a measured value thereof , an extrapolated value was calculated according to the extrapolation method described above, and the calculated value was described:
Transition temperature : C 67.7 N 119.3 I. T_{NI} = 85 ; η = 74.2 mPa·s ; Δn = 0.144; Δε = 36.6.

### Example 11

### Synthesis of 2-(4-(difluoro((2,3',4',6-tetrafluoro-[1,1'-biphenyl]-4-yl)oxy)met hyl)-3,5-difluorophenyl)-5-(ethoxymethyl)-1,3-dioxane (No. 151)

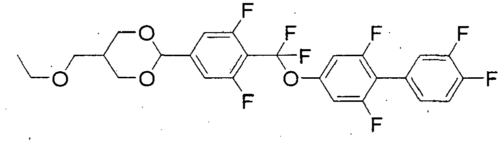

Compound (No. 151) was prepared in a manner similar to Example 1.

¹H-NMR (δ ppm; CDCl₃): 7.32 - 7.12 (m, 5H), 6.95 (dd, 2H, J = 8.2 Hz), 5.39 (s, 1H), 4.29 (dd, 2H, J = 4.6 Hz, 11.7 Hz)3.75 (dd, 2H, J = 11.7 Hz, 11.7 Hz), 3.45 (q, 2H, J = 6.9 Hz), 3.28 (d, 2H, J = 5.9 Hz), 2.43 (m, 1H), 1.19 (t, 3H, J = 6.9 Hz) .
¹⁹F-NMR (δ ppm; CFCl₃) : -61.78 (t, 2F, J = 28.0 Hz), -111.48 (dt, 2F, J = 11.4 Hz, 28.0 Hz), -112. 86 (d, 2F, J = 8.2 Hz), -137.93--138. 15 (m, 2F).

Physical properties of compound (No. 151) were as described below.

A mixture of a compound (5% by weight) and base liquid crystal (A) (95% by weight) was used as a sample. From a measured value thereof, an extrapolated value was calculated according to the extrapolation method described above, and the calculated value was described:
Transition temperature: C 109.4 I. T_{NI} = 47.7; η = 63 mPa·s; Δn = 0.137; Δε = 46.1.

### Example 12

### Synthesis of

### 2-(4-(((2,3'-difluoro-4'-trifluoromethoxy-[1,1-'-biphenyl]-4-yl)oxy )difluoromethyl)-3,5-difluorophenyl)-5-(ethoxymethyl)-1,3-dioxane (No. 153)

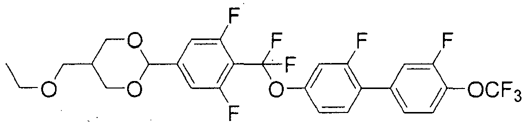

Compound (No. 153) was prepared in a manner similar to Example 1.

¹H-NMR (δ ppm; CDCl₃): 7.42 - 7.33 (m, 3H), 7.33 - 7.27 (m, 1H), 7.19 - 7.08 (m, 4H)5.39 (s, 1H), 4.28 (dd, 2H, J = 4.6 Hz, 11.8 Hz) 3.74 (dd, 2H, J = 11.8 Hz, 11.8 Hz), 3.45 (q, 2H, J = 7.0 Hz), 3.28 (d, 2H, J = 5.8 Hz) 2.43 (m, 1H), 1.19 (t, 3H, J = 7.0 Hz).
¹⁹F-NMR (δ ppm; CFCl₃) : -59.21 (d, 3F, J = 4.9 Hz), -61.48 (t, 2F, J = 28.5 Hz), -111.52 (dt, 2F, J = 11.0 Hz, 28.5 Hz), -114.93 (dd, 1F, J = 8.5 Hz, 10.6 Hz), -128.90--129.05 (m, 1F) .

Physical properties of compound (No. 153) were as described below.

A mixture of a compound (15% by weight) and base liquid crystal (A) (85% by weight) was used as a sample . From a measured value thereof, an extrapolated value was calculated according to the extrapolation method described above, and the calculated value was described:
Transition temperature: C 69.4 N 82.3 I. T_{NI} = 59; η = 67.1 mPa·s; Δn = 0.124; Δε = 44.6.

### Example 13

### Synthesis of a 2,3',4',5'-tetrafluoro-[1,1'-biphenyl]-4-yl) 4-(5-(ethoxymethyl)-1,3-dioxane-2-yl)2,6-difluorobenzoate (No. 157)

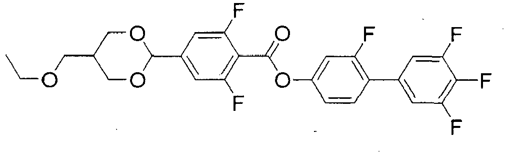

Compound (No. 157) was prepared in a manner similar to Example 1.

¹H-NMR (δ ppm; CDCl₃) : 7.43 (dd, 1H, J = 8.3 Hz, 8.3 Hz), 7.23 - 7.13 (m, 6H), 5.40 (s, 1H), 4.26 (dd, 2H, J = 4.6 Hz, 11.8 Hz) 3.54 (dd, 2H, J = 11.8 Hz, 11.8 Hz), 3.45 (q, 2H, J = 7.0 Hz), 3.28 (d, 2H, J = 5.8 Hz)2.11 (m, 1H), 1.19 (t, 3H, J = 7.0 Hz).
¹⁹F-NMR (δ ppm; CFCl₃) : -108.92 (d, 2F, J = 10.0 Hz), -114.81 (dd, 1F, J = 8.3 Hz, 10.6 Hz), -134.62 (dd, 2F, J = 8.4 Hz, 21.4 Hz), -161.60 (tt, 1F, J = 6.7 Hz, 21.4 Hz).

Physical properties of compound (No. 157) were as described below.

A mixture of a compound (10% by weight) and base liquid crystal (A) (90% by weight) was used as a sample. From a measured value thereof, an extrapolated value was calculated according to the extrapolation method described above, and the calculated value was described:
Transition temperature: C 92.4 N 135.6 N 146.3 I. T_{NI} = 84.7; η = 81.1 mPa·s; Δn = 0.137; Δε = 61.8.

### Example 14

### Synthesis of

### 2-(4'-(difluoro-((2,3',4',5'-tetrafluoro)methyl)-[1,1'-biphenyl]-4 -yl)oxy)methyl)2,3',5'-trifluoro-[1,1'-biphenyl]-4-yl)-5-(ethoxyme thyl)-1,3-dioxane (No. 220)

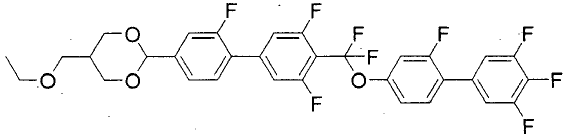

Compound (No. 220) was prepared in a manner similar to Example 1.

¹H-NMR (δ ppm; CDCl₃): 7.46 - 7.33 (m, 4H), 7.25 - 7.12 (m, 6H), 5.45 (s, 1H), 4.30 (dd, 2H, J = 4.6 Hz, 11.7 Hz) 3.76 (dd, 2H, J = 11.7 Hz, 11.7 Hz), 3.46 (q, 2H, J = 6.9 Hz), 3.28 (d, 2H, J = 5.8 Hz), 2.47 (m, 1H), 1.20 (t, 3H, J = 6.9 Hz).
¹⁹F-NMR (δ ppm; CFCl₃): -61.57 (t, 2F, J = 28.1 Hz), -111.00 (dt, 2F, J = 11.0 Hz, 28. 1 Hz), -114.84 (dd, 1F, J = 8.3 Hz, 12.1 Hz), -117.30 (dd, 1F, J = 8.3 Hz, 12.1 Hz), -134.68 (dd, 2F, J = 8.9 Hz, 21.8 Hz), -161.69 (tt, 1F, J = 6.9 Hz, 21.8 Hz).

Physical properties of compound (No. 220) were as described below.

A mixture of a compound (10% by weight) and base liquid crystal (A) (90% by weight) was used as a sample. From a measured value thereof, an extrapolated value was calculated according to the extrapolation method described above, and the calculated value was described:
Transition temperature: C 82 C 90.4 N 148.1 191.1 I. T_{NI} = 120.7; η = 95.7 mPa·s; Δn = 0.177; Δε = 61.6.

### Example 15

### Synthesis of

### 2-(4-{difluoro-((2,3',4',5'-tetrafluoro-[1,1'-biphenyl]-4-yl)oxy)m ethyl)-3,5-difluorophenyl)-5-(methoxyethyl)-1,3-dioxane (No. 149)

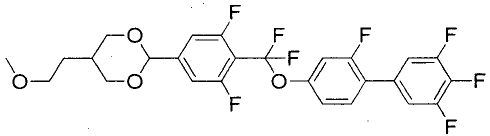

A synthesis scheme is shown in a diagram below.

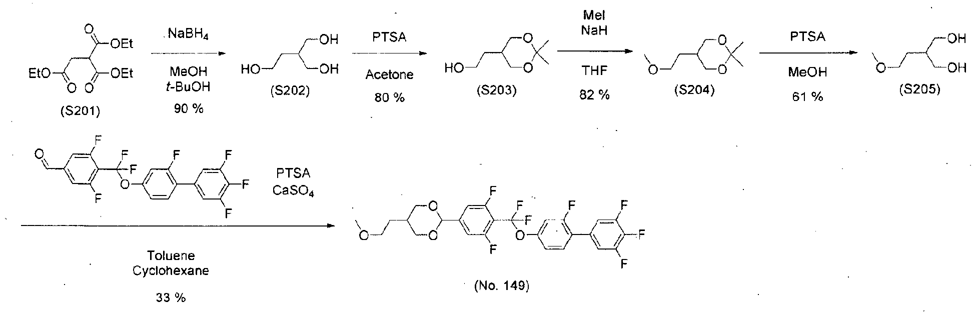

### First step

Under a nitrogen atmosphere, triethyl ethane-1,1,2-tricarboxylate (10.0 g, 40.6 mmol) , sodium borohydride (4.1 g, 109.6 mmol), methanol (5 mL) and t-butanol (80 mL) were put in a reaction vessel, and the resulting mixture was refluxed under heating for 3 hours. The resulting reaction mixture was returned to room temperature, and then neutralized by adding 2N hydrochloric acid. The resulting precipitate was removed by Celite filtration, and the solvent of filtrate was distilled off by an evaporator. The residue was purified by silica gel chromatography to obtain compound (S202) (4.4 g, 36.6 mmol; 90%).

### Second step

Under a nitrogen atmosphere, compound (S202) (4.1 g, 34.1 mmol) obtained in the first step, p-toluenesulfonic acid monohydrate (0.19 g, 1.02 mmol) and acetone (123 mL) were put in a reaction vessel, and the resulting mixture was stirred at room temperature for 12 hours. Triethylamine was added to the resulting reaction mixture, and the solvent was distilled off by an evaporator. The residue was purified by silica gel chromatography to obtain compound (S203) (4.4 g, 27.5 mmol; 80%).

### Third step

Under a nitrogen atmosphere, compound (S203) (4.2 g, 26.2 mmol) obtained in the second step, sodium hydride (60%; 1.6 g, 39.3 mmol) and THF (21 mL) were put in a reaction vessel, and the resulting mixture was stirred at room temperature for 30 minutes. Thereto, 1-iodomethane (11.1 g, 78.6 mmol) was added, and the resulting mixture was stirred at room temperature for 5 hours. The resulting reaction mixture was poured into pure water, and an aqueous layer was subjected to extraction with ethyl acetate. Combined organic layers were washed with pure water and saturated brine, and then dried over magnesium sulfate, and the solvent was distilled off by an evaporator. The residue was purified by silica gel chromatography to obtain compound (S204) (3.8 g, 21.7 mmol; 82%).

### Fourth step

Under a nitrogen atmosphere, compound (S204) (4.2 g, 24.1 mmol) obtained in the third step, p-toluenesulfonic acid monohydrate (0.46 g, 2.4 mmol) and methanol (21 mL) were put in a reaction vessel, and the resulting mixture was stirred at room temperature for 12 hours. Triethylamine was added to the resulting reaction mixture, and the solvent was distilled off by an evaporator. The residue was purified by silica gel chromatography to obtain compound (S205) (2.0 g, 14.9 mmol; 61%).

### Fourth step

Under a nitrogen atmosphere, compound (S205) (2.0 g, 14.9 mmol) obtained in the fourth step, 4-(difluoro((2,3',4',5'-tetrafluoro-[1,1'-biphenyl]-4-yl)oxy)methy l)-3,5-difluorobenzaldehyde (6.4 g, 14.9 mmol), p-toluenesulfonic acid monohydrate (0.19 g, 1.0 mmol), calcium sulfate (1.9g, 14.2 mmol), toluene (16 mL) and cyclopropane (16 mL) were put in a reaction vessel, and the resulting mixture was refluxed under heating for 3 hours. The resulting reaction mixture was returned to room temperature, and then poured into pure water, and an aqueous layer was subjected to extraction with toluene. Combined organic layers were washed with pure water and saturated brine, and then dried over magnesium sulfate, and the solvent was distilled off by an evaporator. The residue was purified by silica gel chromatography and recrystallization to obtain compound (No. 149) (2.7 g, 4.9 mmol; 33%) .

¹H-NMR (δ ppm; CDCl₃): 7.34 (dd, 1H, J = 8.4 Hz, 8.4 Hz), 7.19 - 7.10 (m, 6H), 5.38 (s, 1H), 4.27 (dd, 2H, J = 4.6 Hz, 11.7 Hz) 3.57 (dd, 2H, J = 11.7 Hz, 11.7 Hz), 3.40 (t, 2H, J = 6.3 Hz), 3.34 (s, 3H) 2.25 (m, 1H), 1.38 (dt, 3H, J = 6.3 Hz).
¹⁹F-NMR (δ ppm; CFCl₃) : -61.43 (t, 2F, J = 28.5 Hz), -111.50 (dt, 2F, J = 11.3 Hz, 28.5 Hz), -114.86 (dd, 1F, J = 8.5 Hz, 10.6 Hz), -134.71 (dd, 2F, J = 9.4 Hz, 21.4 Hz), -161.72 (tt, 1F, J = 6.7 Hz, 21.4 Hz) .

Physical properties of compound (No. 149) were as described below.

A mixture of a compound (5% by weight) and base liquid crystal (A) (95% by weight) was used as a sample. From a measured value thereof, an extrapolated value was calculated according to the extrapolation method described above, and the calculated value was described:

Transition temperature: C 86.5 I. T_{NI} = 39.7; η = 62.9 mPa·s; Δn = 0.137; Δε = 47.8.

### Example 16

### Synthesis of

### 2-(4-(difluoro-((2,3',4',5'-tetrafluoro-[1,1'-biphenyl]-4-yl)oxy)m ethyl)-3,5-difluorophenyl) -5- (ethoxymethyl)tetrahydro-2H-pyran (No. 107)

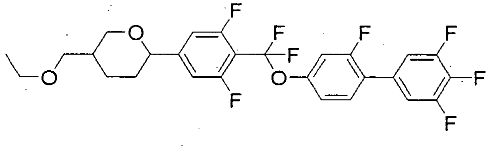

Compound (No. 107) was prepared according to a synthesis method of a compound having a pyran ring described in EP 1482019 A.

¹H-NMR (δ ppm; CDCl₃) : 7.35 (dd, 1H, J = 8.7 Hz, 8.7 Hz), 7.20 - 7.10 (m, 4H), 6.99 (d, 2H, J = 10.4H), 4.27 (dd, 1H, J = 1.8 Hz, 11.0 Hz), 4.23 (ddd, 1H, J = 2.0 Hz, 4.3 Hz, 11.4 Hz), 3.53 - 3.41 (m, 2H), 3.33 (dd, 1H, J = 11.4 Hz, 11.4 Hz), 3.32 - 3.22 (m, 2H), 2.07 - 1.87 (m, 3H), 1.57 - 1.35 (m, 2H), 1.20 (t, 3H, J = 7.0 Hz).
¹⁹F-NMR (δ ppm; CFCl₃) : -61.41 (t, 2F, J = 28.4 Hz), -111.04 (dt, 2F, J = 10.4 Hz, 28.4 Hz), -114.93 (dd, 1F, J = 8.7 Hz, 12.1 Hz), - 134.70 (dd, 2F, J = 8.7 Hz, 22.3 Hz), -161.72 (tt, 1F, J = 6.9 Hz, 22.3 Hz) .

Physical properties of compound (No. 107) were as described below.

A mixture of a compound (10% by weight) and base liquid crystal (A) (90% by weight) was used as a sample. From a measured value thereof, an extrapolated value was calculated according to the extrapolation method described above, and the calculated value was described:
Transition temperature: C 59.6 N 59.6 I. T_{NI} = 35.0; Δn = 0.110; Δε = 40.1.

Compounds (Nos. 1 to 260) described below can be prepared according to the synthesis method of compound (1) as described above and synthesis procedures described in Examples 1 to 16.

| No. | | No. | |
|---|---|---|---|
| 1 | 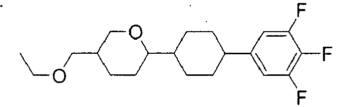 | 13 | 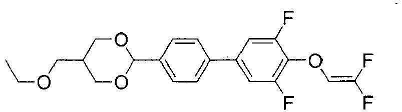 |
| 2 | 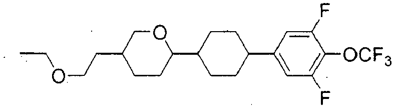 | 14 | 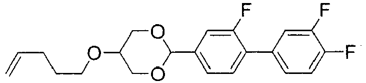 |
| 3 | 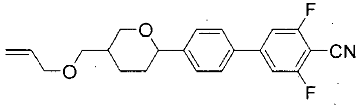 | 15 | 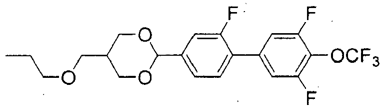 |
| 4 | 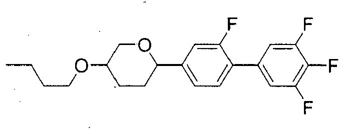 | 16 | 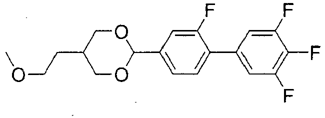 |
| 5 | 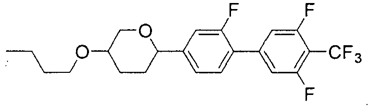 | 17 | 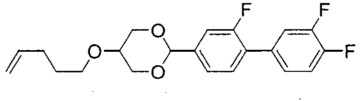 |
| 6 | 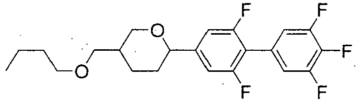 | 18 | 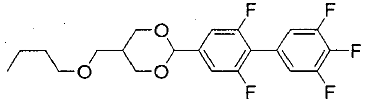 |
| 7 | 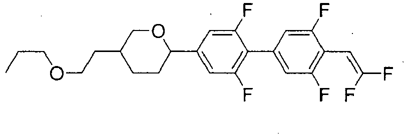 | 19 | 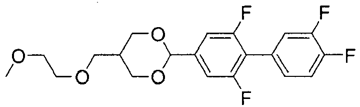 |
| 8 | 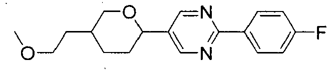 | 20 | 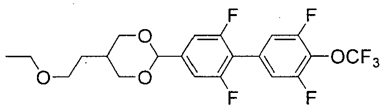 |
| 9 | 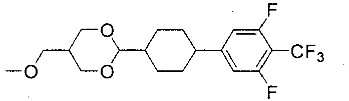 | 21 | 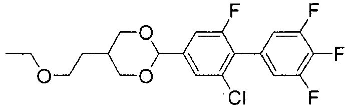 |
| 10 | 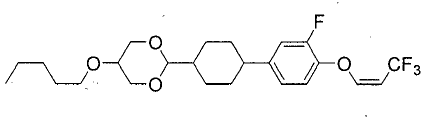 | 22 | 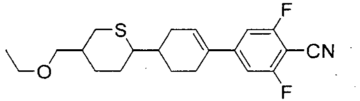 |
| 11 | 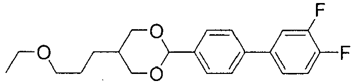 | 23 | 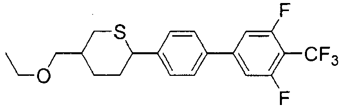 |
| 12 | 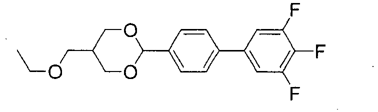 | 24 | 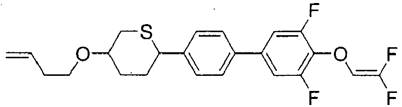 |
| | C 49.8 SB 53.2 I T_{NI}=4.4°C, *η* =65.7mPa·S, Δ n=0.084, Δ *ε* =37.43 | | |

| No. | | No. | |
|---|---|---|---|
| 25 | 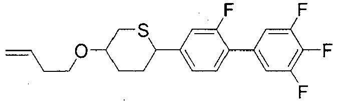 | 37 | 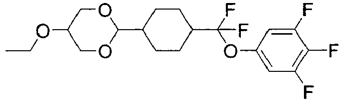 |
| 26 | 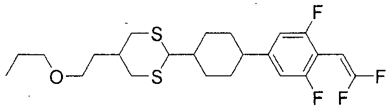 | 38 | 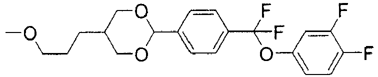 |
| 27 | 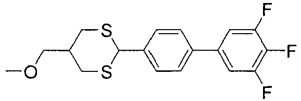 | 39 | 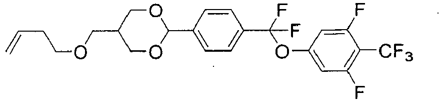 |
| 28 | 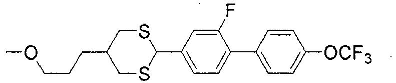 | 40 | 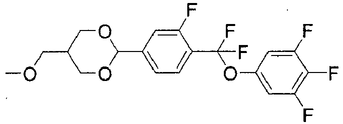 |
| 29 | 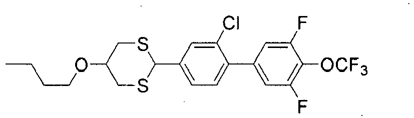 | 41 | 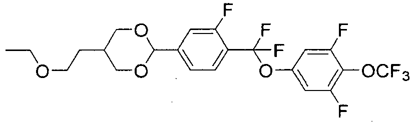 |
| 30 | 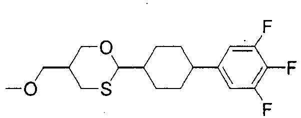 | 42 | 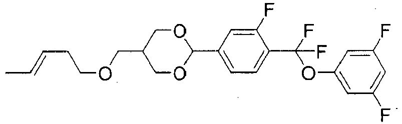 |
| 31 | 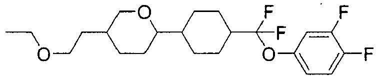 | 43 | 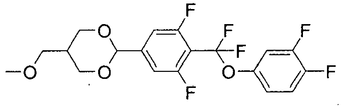 |
| 32 | 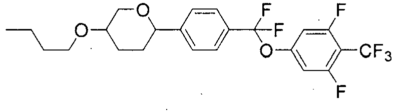 | 44 | 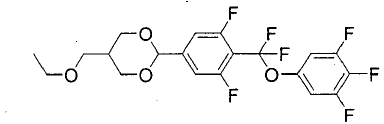 |
| | | | C 63.4 I T_{NI}=-32.3°C, *η*=40.0mPa·S, Δ n=0.050, Δ *ε* =42.57 |
| 33 | 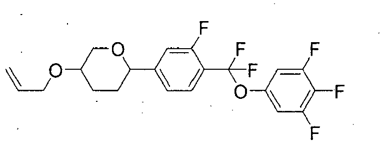 | 45 | 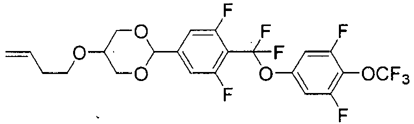 |
| 34 | 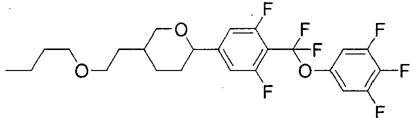 | 46 | 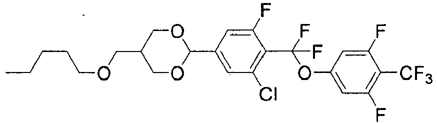 |
| 35 | 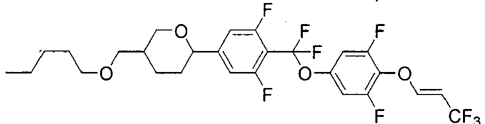 | 47 | 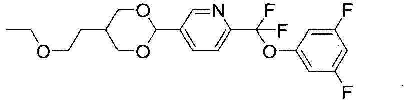 |
| 36 | 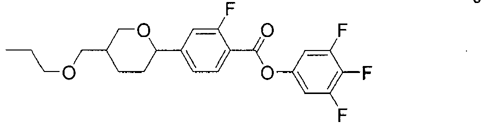 | 48 | 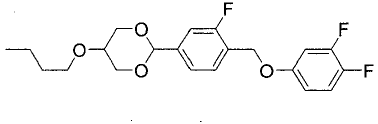 |

| No. | | No. | |
|---|---|---|---|
| 49 | 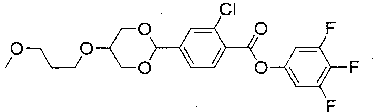 | 61 | 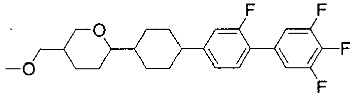 |
| 50 | 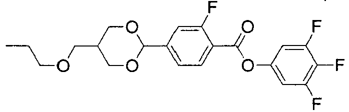 | 62 | 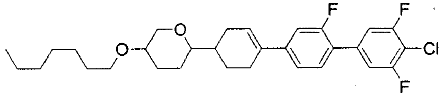 |
| 51 | 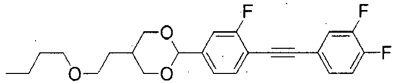 | 63 | 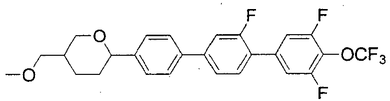 |
| 52 | 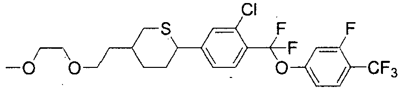 | 64 | 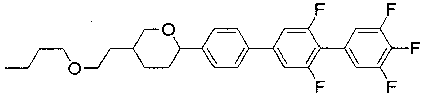 |
| 53 | 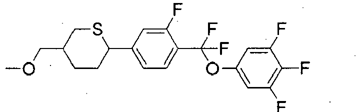 | 65 | 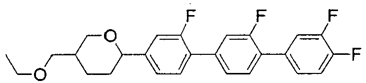 |
| 54 | 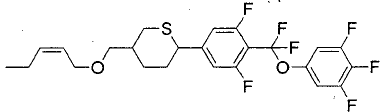 | 66 | 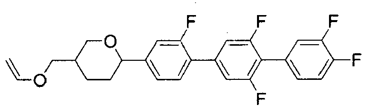 |
| 55 | 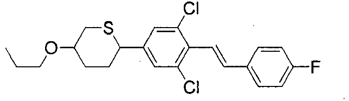 | 67 | 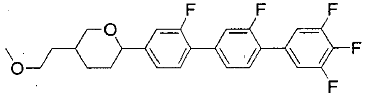 |
| 56 | 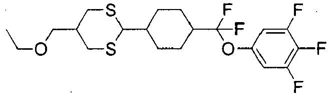 | 68 | 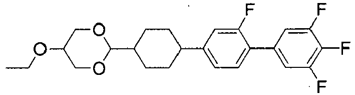 |
| 57 | 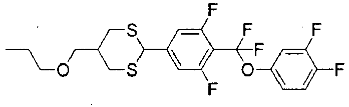 | 69 | 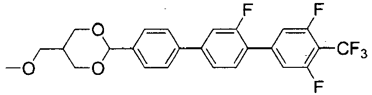 |
| 58 | 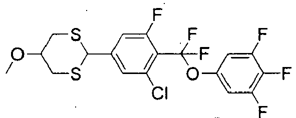 | 70 | 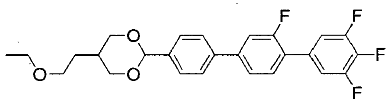 |
| 59 | 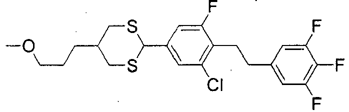 | 71 | 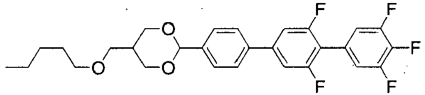 |
| 60 | 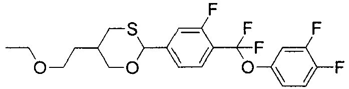 | 72 | 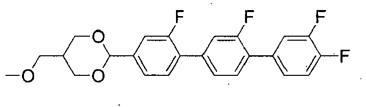 |

| No. | | No. | |
|---|---|---|---|
| 73 | 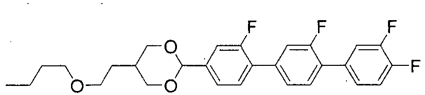 | 85 | 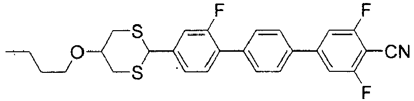 |
| 74 | 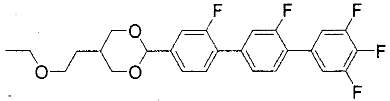 | 86 | 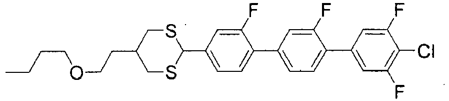 |
| 75 | 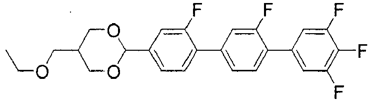 | 87 | 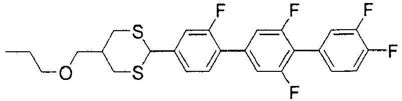 |
| | C 113.6 SB 150.7 N 166.4 I T_{NI}=1137°C, *η*=81.1mPa·S, Δ n=0.177, Δ *ε* =41.8 | | |
| 76 | 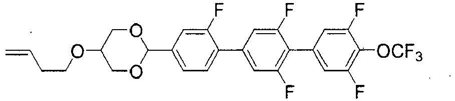 | 88 | 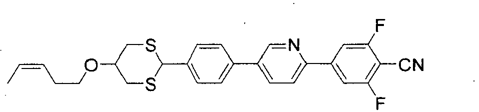 |
| 77 | 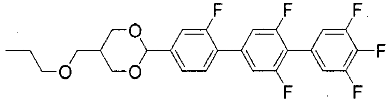 | 89 | 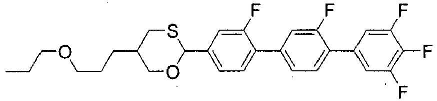 |
| 78 | 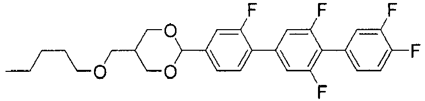 | 90 | 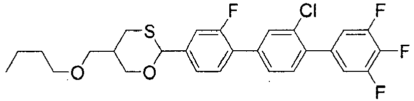 |
| 79 | 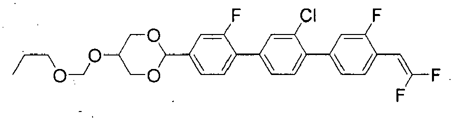 | 91 | 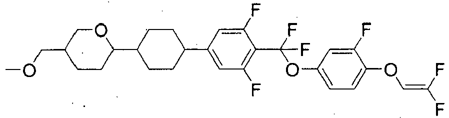 |
| 80 | 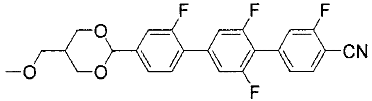 | 92 | 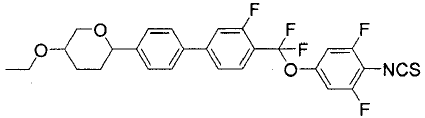 |
| 81 | 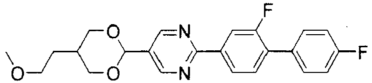 | 93 | 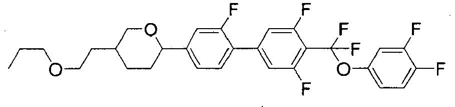 |
| 82 | 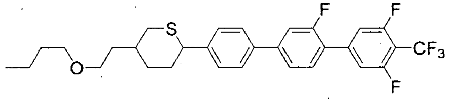 | 94 | 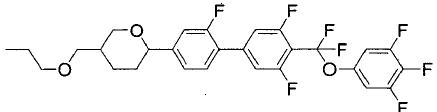 |
| 83 | 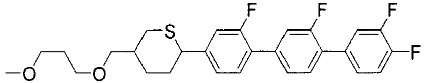 | 95 | 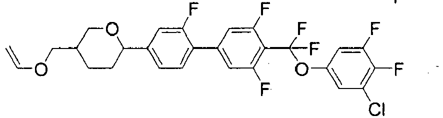 |
| 84 | 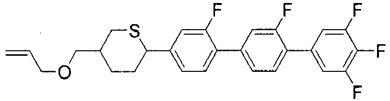 | 96 | 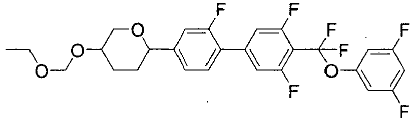 |

| No. | | No. | |
|---|---|---|---|
| 97 | 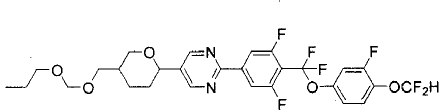 | 109 | 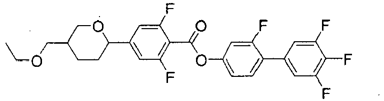 |
| 98 | 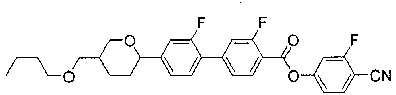 | 110 | 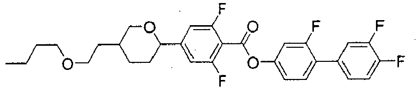 |
| 99 | 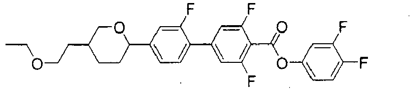 | 111 | 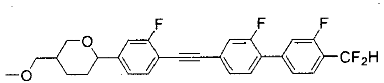 |
| 100 | 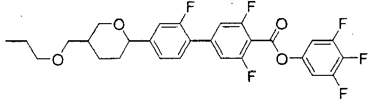 | 112 | 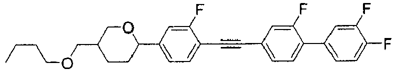 |
| 101 | 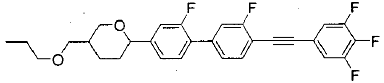 | 113 | 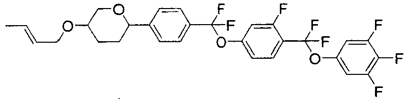 |
| 102 | 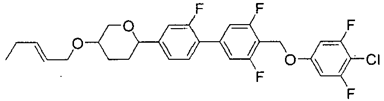 | 114 | 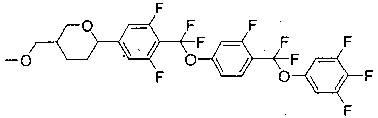 |
| 103 | 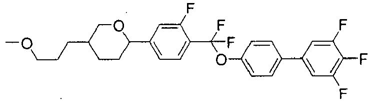 | 115 | 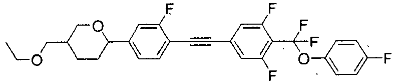 |
| 104 | 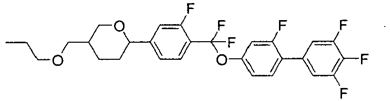 | 116 | 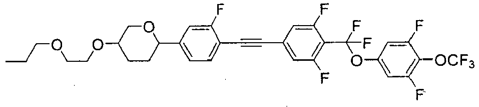 |
| 105 | 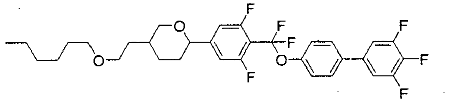 | 117 | 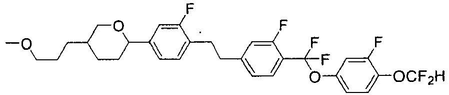 |
| 106 | 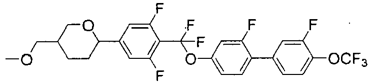 | 118 | 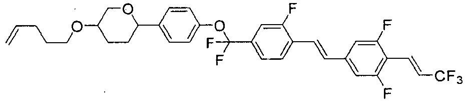 |
| 107 | 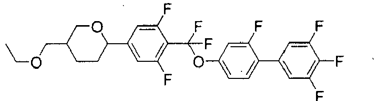 | 119 | 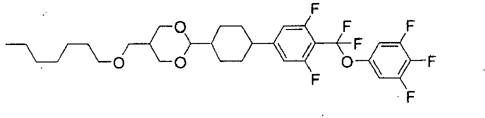 |
| | C 59.6 N 59.6 I T_{NI}=35.0°C, Δ n=0.110, Δ *ε* =40.1 | | |
| 108 | 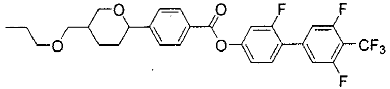 | 120 | 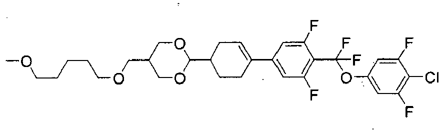 |

| No. | | No. | |
|---|---|---|---|
| 121 | 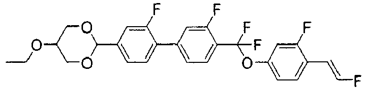 | 133 | 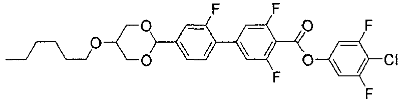 |
| 122 | 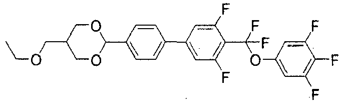 | 134 | 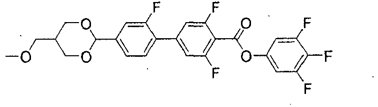 |
| | C 110.1 SE 121.1 SB 129.3 I T_{NI}=95.7°C, *η*=69 5mPa·S, Δn=0.157, Δ *ε* =43.9 | | |
| 123 | 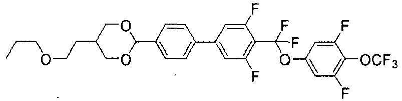 | 135 | 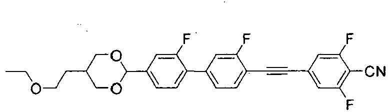 |
| 124 | 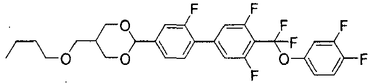 | 136 | 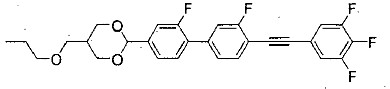 |
| | C 43.1 N 61.8 I T_{NI}=61.0°C, *η*=82.6mPa·S, Δn=0.117, Δ *ε* =36.97 | | |
| 125 | 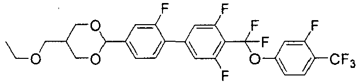 | 137 | 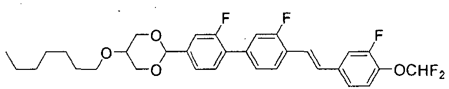 |
| 126 | 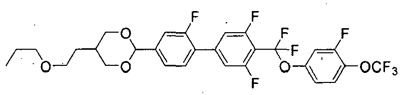 | 138 | 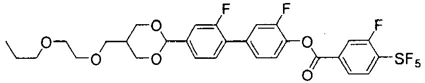 |
| 127 | 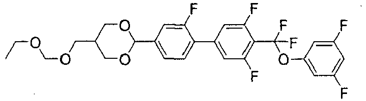 | 139 | 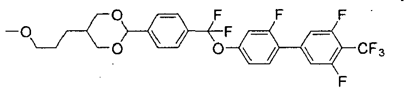 |
| 128 | 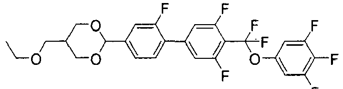 | 140 | 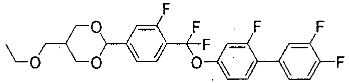 |
| | C 72.7 N 95.3 N 99.4 I T_{NI}=71°C, *η* =79.8mPa·S, Δ n=0.130, Δ *ε* =57.9 | | C 67.7 N 119.3 I T_{NI}=85°C, *η* =74.2mPa·S, Δ n=0.144, Δ *ε* =36.6 |
| 129 | 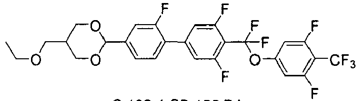 | 141 | 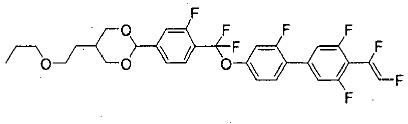 |
| | C 102.4 SB 122.7 I T_{NI}=677°C, *η* =85.6mPa·S, Δ n=0.137, Δ *ε* =67.8 | | |
| 130 | 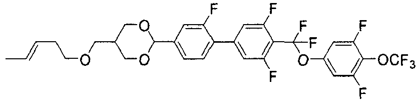 | 142 | 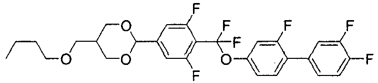 |
| | | | C 46.6 I T_{NI}=38.4°C, *η* =72.6mPa·S, Δ n=0.137, Δ *ε* =35.8 |
| 131 | 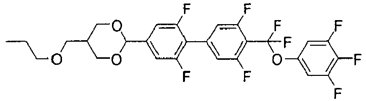 | 143 | 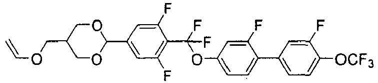 |
| 132 | 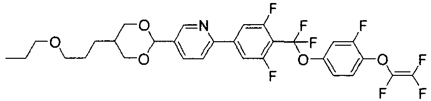 | 144 | 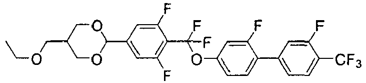 |

| No. | | No. | |
|---|---|---|---|
| 145 | 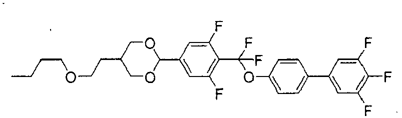 | 157 | 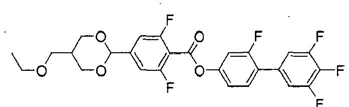 |
| | | | C 92.4 N 135.6 N 146.3 I T_{NI}=84.7°C, *η* =81.1mPa·S, Δ n=0.137, Δ *ε* =61.8 |
| 146 | 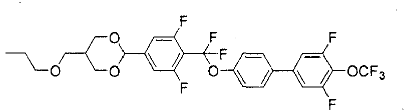 | 158 | 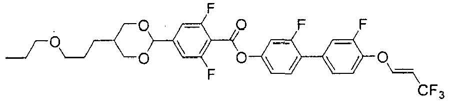 |
| 147 | 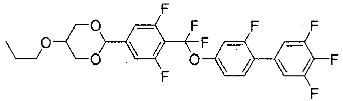 | 159 | 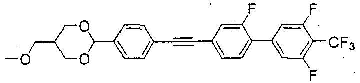 |
| 148 | 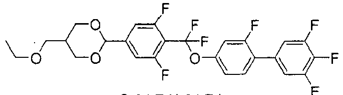 | 160 | 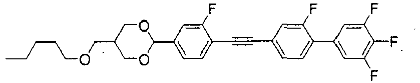 |
| | C 64.7 N 64.1 I T_{NI}=43°C, *η* =70mPa·S, Δ n=0.117, Δ *ε* =54.57 | | |
| 149 | 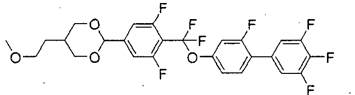 | 161 | 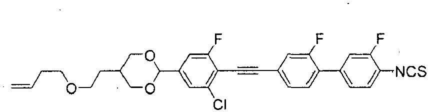 |
| | C 86.5 I T_{NI}=397°C, *η* =62.9mPa·S, Δ n=0.137, Δ *ε* =47.8 | | |
| 150 | 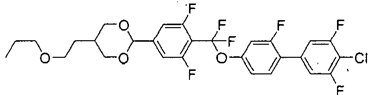 | 162 | 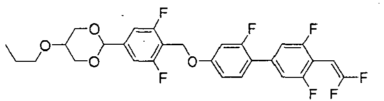 |
| 151 | 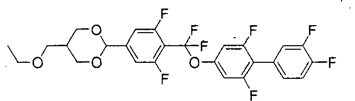 | 163 | 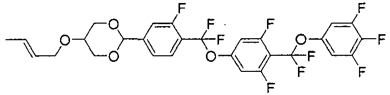 |
| | C 109.4 I T_{NI}=477°C, *η* =63mPa·S, Δ n=0.137, Δ *ε* =46.1 | | |
| 152 | 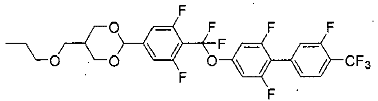 | 164 | 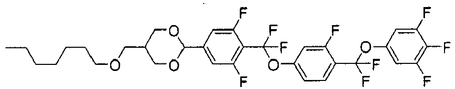 |
| 153 | 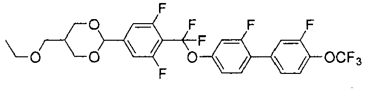 | 165 | 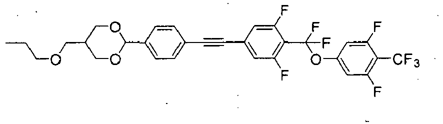 |
| | C 69.4 N 82.3 I T_{NI}=59°C, *η*=67.1mPa·S, Δ n=0.124, Δ *ε* =44.6 | | |
| 154 | 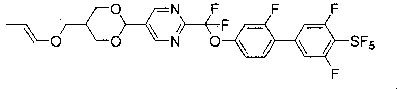 | 166 | 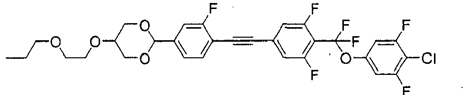 |
| 155 | 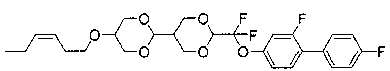 | 167 | 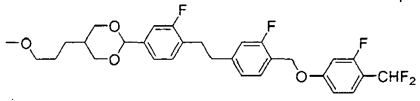 |
| 156 | 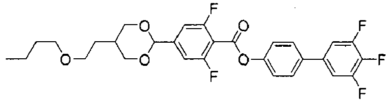 | 168 | 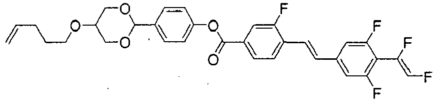 |

| No. | | No. | |
|---|---|---|---|
| 169 | 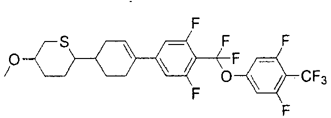 | 181 | 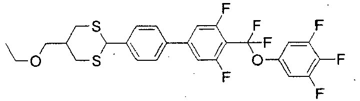 |
| 170 | 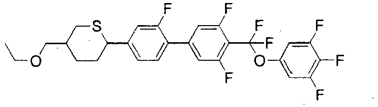 | 182 | 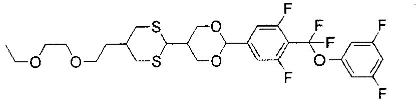 |
| 171 | 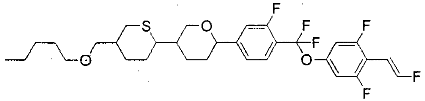 | 183 | 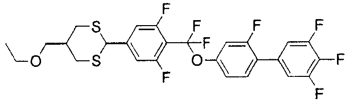 |
| 172 | 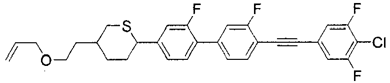 | 184 | 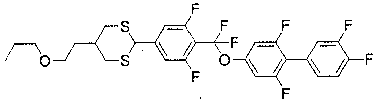 |
| 173 | 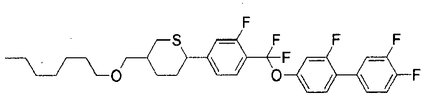 | 185 | 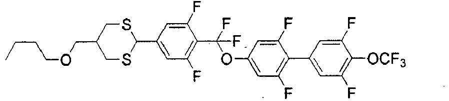 |
| 174 | 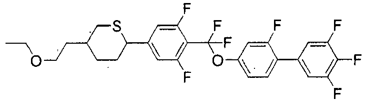 | 186 | 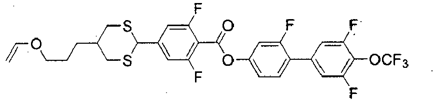 |
| 175 | 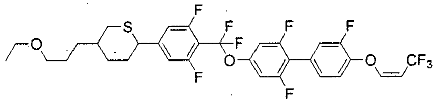 | 187 | 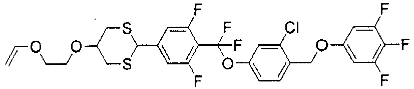 |
| 176 | 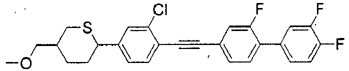 | 188 | 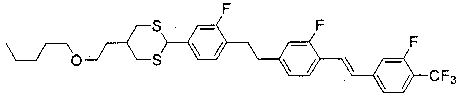 |
| 177 | 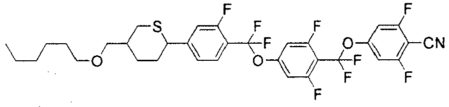 | 189 | 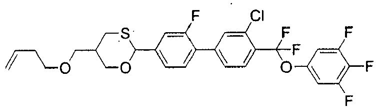 |
| 178 | 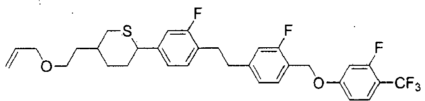 | 190 | 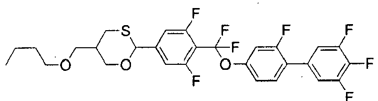 |
| 179 | 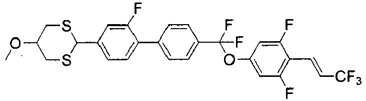 | 191 | 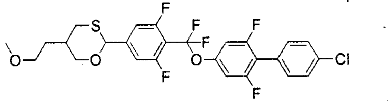 |
| 180 | 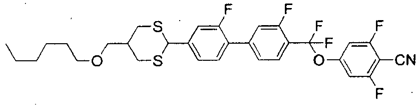 | 192 | 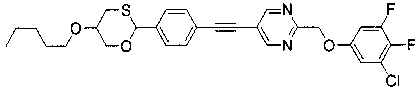 |

| No. | | No. | |
|---|---|---|---|
| 193 | 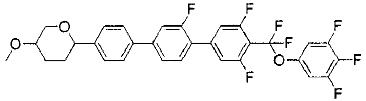 | 205 | 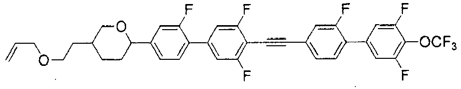 |
| 194 | 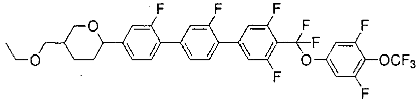 | 206 | 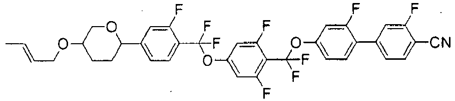 |
| 195 | 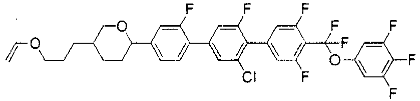 | 207 | 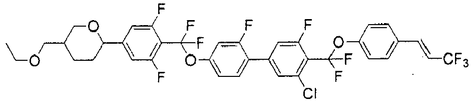 |
| 196 | 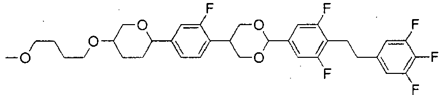 | 208 | 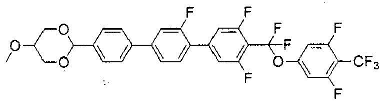 |
| 197 | 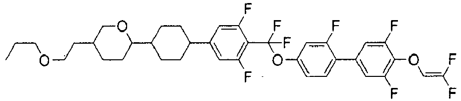 | 209 | 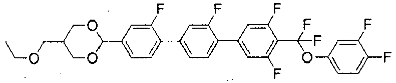 |
| 198 | 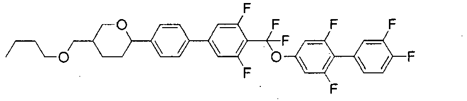 | 210 | 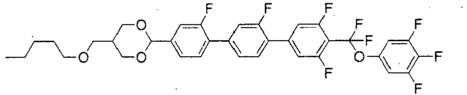 |
| 199 | 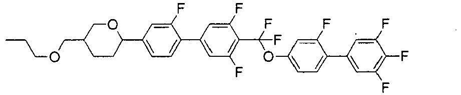 | 211 | 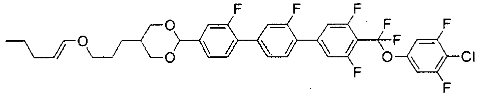 |
| 200 | 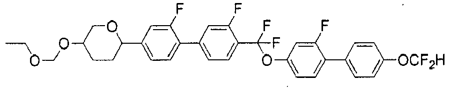 | 212 | 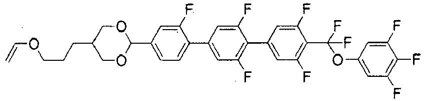 |
| 201 | 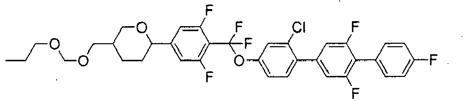 | 213 | 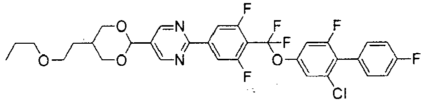 |
| 202 | 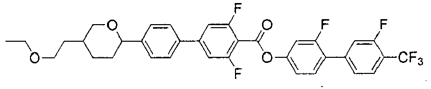 | 214 | 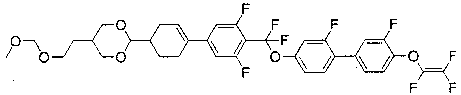 |
| 203 | 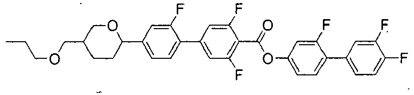 | 215 | 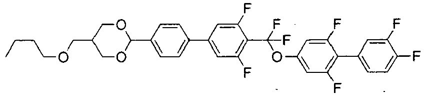 |
| 204 | 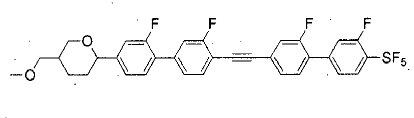 | 216 | 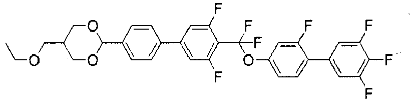 |

| No. | | No. | |
|---|---|---|---|
| 217 | 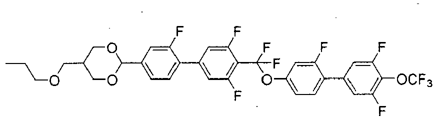 | 229 | 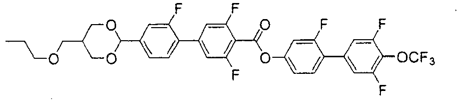 |
| 218 | 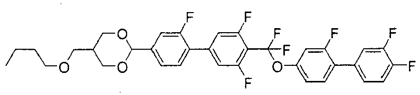 | 230 | 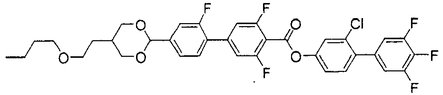 |
| 219 | 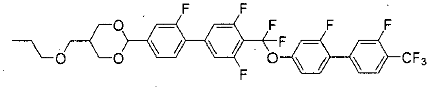 | 231 | 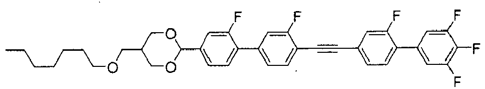 |
| 220 | 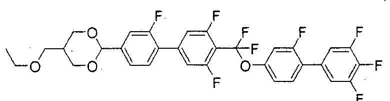 | 232 | 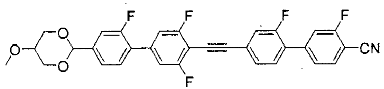 |
| 221 | 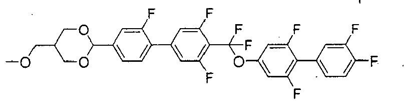 | 233. | 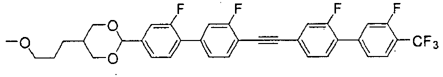 |
| 222 | 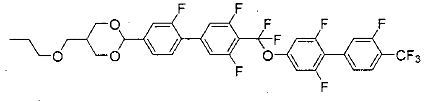 | 234 | 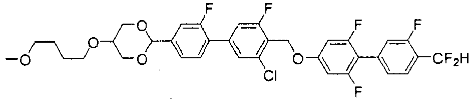 |
| 223 | 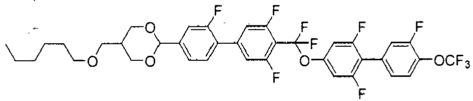 | 235 | 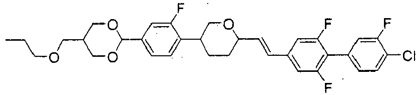 |
| 224 | 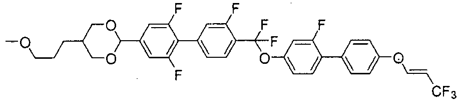 | 236 | 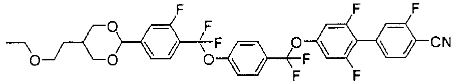 |
| 225 | 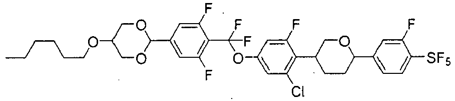 | 237 | 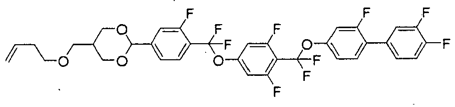 |
| 226 | 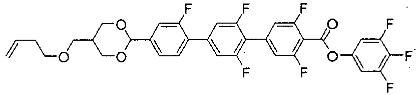 | 238 | 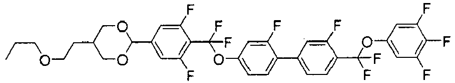 |
| 227 | 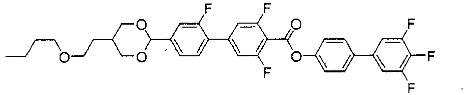 | 239 | 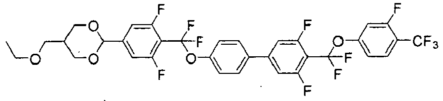 |
| 228 | 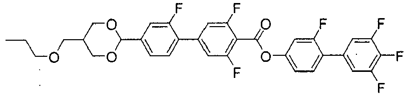 | 240 | 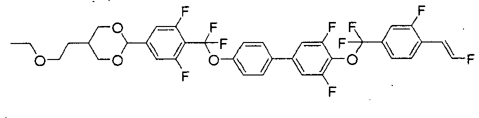 |

| No. | | No. | |
|---|---|---|---|
| 241 | 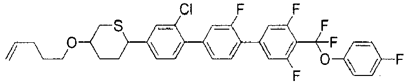 | 253 | 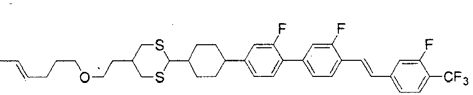 |
| 242 | 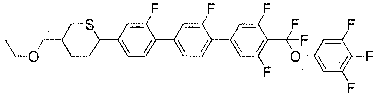 | 254 | 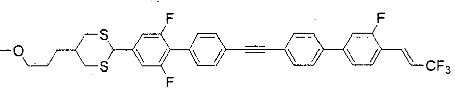 |
| 243 | 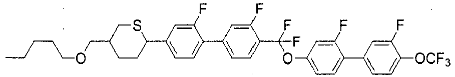 | 255 | 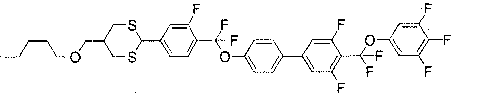 |
| 244 | 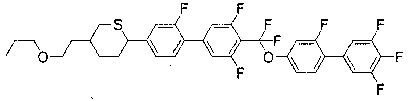 | 256 | 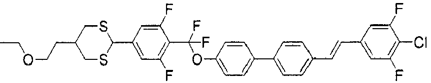 |
| 245 | 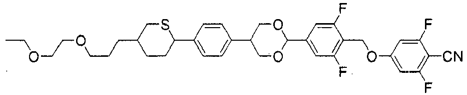 | 257 | 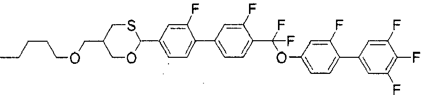 |
| 246 | 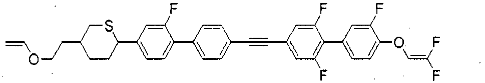 | 258 | 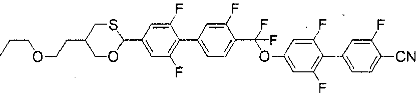 |
| 247 | 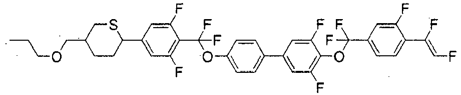 | 259 | 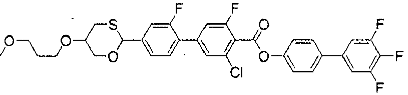 |
| 248 | 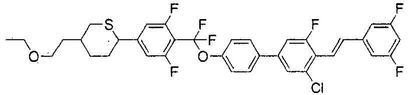 | 260 | 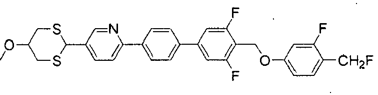 |
| 249 | 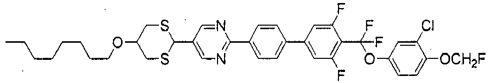 | | |
| 250 | 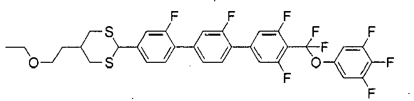 | | |
| 251 | 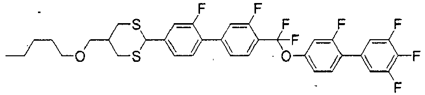 | | |
| 252 | 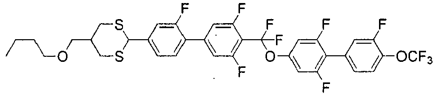 | | |

### Comparative Example

Physical properties of comparative compound (R) were as described below.

A mixture of a compound (15% by weight) and base liquid crystal (A) (85% by weight) was used as a sample. From a measured value thereof, an extrapolated value was calculated according to the extrapolation method described above, and the calculated value was described:

Dielectric anisotropy (Δε) = 39.1.

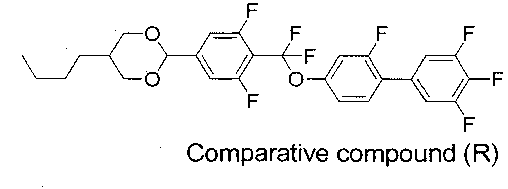

Dielectric anisotropy of compound (No. 148) obtained in Example 1, compound (No. 149) obtained in Example 4 and comparative compound (R) are summarized in Table 1. From the results, compound (No. 148) and compound (No. 149) were found to be superb in having larger dielectric anisotropy (Δ*ε*).

**Table 1 Physical properties of compounds (No. 148) and (No. 149) and comparative compound (R)**

| Structure | Δε |
|---|---|
| 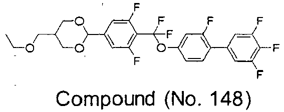 | 54.6 |
| 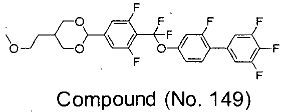 | 47.8 |
| 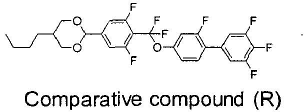 | 39.1 |

### 4-2. Example of composition (1)

Liquid crystal composition (1) of the invention will be described in detail by way of Examples. However, the invention is not limited by the Examples. The invention includes a mixture of a composition in Example 1 and a composition in Example 2. The invention also includes a mixture in which at least two compositions in Examples were mixed. The compounds in Examples were represented using symbols according to definitions in Table 2 described below. In Table 2, a configuration of 1,4-cyclohexylene is trans. A parenthesized number next to a symbolized compounds in Examples corresponds to the number of the compound. A symbol (-) means any other liquid crystal compound. A proportion (percentage) of the liquid crystal compound is expressed in terms of weight percent (% by weight) based on the weight of the liquid crystal composition. Values of the physical properties of the composition are summarized in a last part. The physical properties were measured according to the methods described above, and measured values are directly described (without extrapolation) . Other physical properties were measured according to the methods described above. Most of the measuring methods are applied as described in the Standard of Japan Electronics and Information Technology Industries Association (hereinafter abbreviated as JEITA) (JEITA ED-2521B) discussed and established by JEITA, or modified thereon. No TFT was attached to a TN device used for measurement.

### (15) Transition temperature of an optically isotropic liquid crystal phase

A sample was placed on a hot plate of a melting point measuring apparatus equipped with a polarizing microscope. A sample was first heated, in a crossed nicol state, to temperature at which the sample becomes a non-liquid crystal isotropic phase, and then the temperature was decreased at a rate of 1°C/min to develop a liquid crystal phase that is completely a chiral nematic phase or an optically isotropic liquid crystal phase. Temperature at which phase transition was caused in a temperature-decreasing process was measured, subsequently the temperature was increased at a rate of 1°C/min, and temperature at which the phase transition was caused in a temperature-increasing process was measured. In the invention, unless otherwise noted, the temperature at which the phase transition was caused in the temperature-increasing process was taken as a phase transition temperature. When discrimination of the phase transition temperature was difficult in a dark field under crossed nicols in an optically isotropic liquid crystal phase, the phase transition temperature was measured by shifting the polarizing plate by 1 to 10° from the crossed nicol state.

### (16) Helical pitch (measured at 20°C; µm)

A Cano wedge cell method was applied to measurement of a helical pitch. A sample was injected into a Cano wedge cell, and a distance (a; unit: µm) between disclination lines as observed from a cell was measured. Helical pitch (P) was calculated according to an equation: P = 2•a•tan θ. Then, θ is an angle between two glass plates in the wedge cell.

Alternatively, a pitch length was measured using selective reflection (Ekisho Binran in Japanese, page 196, issued in 2000, Maruzen Co., Ltd.). In selective reflection wavelength λ, a relational expression: <n> p / λ = 1 holds. Here, <n> represents an average refractive index, and is given by the following equation: <n> = {(n∥² + n⊥²) / 2}^{1/2}. The selective reflection wavelength was measured by using a microspectrophotometer (trade name "MSV-350," JEOL Ltd.). A pitch was determined by dividing the obtained reflection wavelength by the average refractive index.

A pitch of a cholesteric liquid crystal having the reflection wavelength in a longer wavelength region in comparison with visible light was proportional to a reciprocal of a concentration of a chiral agent in a region in which the chiral agent concentration is low, and therefore the pitch was determined by a linear extrapolation method by measuring several pitch lengths of the liquid crystal having the selective reflection wavelength in a visible light region.

**Table Method for Description of Compounds using Symbols R-(A1)-Z₁-····-Zₙ-(Aₙ)-R'**

| 1) Left-terminal Group R- | Symbol | 4) Ring Structure -Aₙ- | Symbol |
|---|---|---|---|
| CₙH₂ₙ₊₁- | n- | 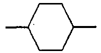 | H |
| CₙH₂ₙ₊₁O- | nO- | | |
| CₘH₂ₘ₊₁OCₙH₂ₙ- | mOn- | 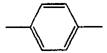 | B |
| CH₂=CH- | V- | | |
| CₙH₂ₙ₊₁-CH=CH- | nV- | | |
| CH₂=CH-CₙH₂ₙ- | Vn- | 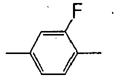 | B(F) |
| CₘH₂ₘ₊₁-CH=CH-CₙH₂ₙ- | mVn- | | |
| CF₂=CH- | VFF- | 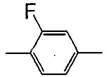 | B(2F) |
| CF₂=CH-CₙH₂ₙ- | VFFn- | | |
| 2) Right-terminal Group -R' | Symbol | | |
| -CₙH₂ₙ₊₁ | -n | 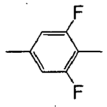 | B(F,F) |
| -OCₙH₂ₙ₊₁ | -On | | |
| -COOCH₃ | -EMe | | |
| -CH=CH₂ | -V | | |
| -CH=CH-OₙH₂ₙ₊₁ | -Vn | 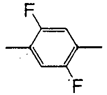 | B(2F,5F) |
| -CₙH₂ₙ-CH=CH₂ | -nV | | |
| -CₘH₂ₘCH=CH-CₙH₂ₙ₊₁ | -mVn | | |
| -CH=CF₂ | -VFF | 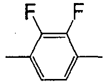 | B(2F,3F) |
| -F | -F | | |
| -Cl | -CL | | |
| -OCF₃ | -OCF3 | 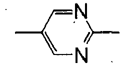 | Py |
| -OCF₂H | -OCF2H | | |
| -CF₃ | -CF3 | | |
| -OCH=CH-CF₃ | -OVCF3 | 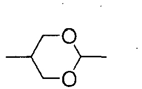 | G |
| -C≡N | -C | | |
| 3) Bonding Group -Zₙ- | Symbol | | |
| -CₙH₂ₙ- | n | 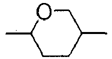 | Dh |
| -COO- | E | | |
| -CH=CH- | V | 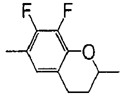 | Cro |
| -CH₂O- | 1O | | |
| -OCH₂- | O1 | | |
| -CF₂O- | X | 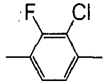 | B(2F,3CL) |
| -C≡C- | T | | |

| 5) Examples of Description | | | |
|---|---|---|---|
| Example 1 2O1-GB(F,F)XB(F)B(F,F)-F | | Example 2 3-HBB(F,F)-F | |
| 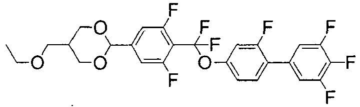 | | 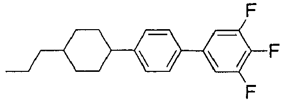 | |

| Example 3 3-HH-4 | | Example 4 3-HBB(2F,3F)-O2 | |
|---|---|---|---|
| 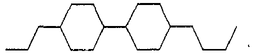 | | 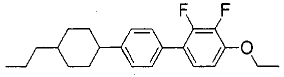 | |

### Example 17

| | |
|---|---|
| 2O1-GB(F,F)XB(F)B(F,F)-F | 10% |
| 3-HB-O2 | 12% |
| 5-HB-CL | 14% |
| 3-HBB(F,F)-F | 7% |
| 3-PyB(F)-F | 10% |
| 5-PyB(F)-F | 10% |
| 3-PyBB-F | 8% |
| 4-PyBB-F | 8% |
| 5-PyBB-F | 7% |
| 5-HBB(F)B-2 | 7% |
| 5-HBB(F)B-3 | 7% |

NI = 80.0°C; η = 39.6 mPa·s; Δn = 0.172; Δε = 12.2.

### Example 18

| | |
|---|---|
| 4O1-GB(F,F)XB(F)B(F)-F | 8% |
| 2-HB-C | 5% |
| 3-HB-C | 12% |
| 3-HB-O2 | 15% |
| 2-BTB-1 | 3% |
| 3-HHB-F | 4% |
| 3-HHB-1 | 8% |
| 3-HHB-O1 | 5% |
| 3-HHB-3 | 14% |
| 5-HHEB-F | 4% |
| 2-HHB(F)-F | 7% |
| 3-HHB(F)-F | 5% |
| 5-HHB(F)-F | 5% |
| 3-HHB(F,F)-F | 5% |

NI = 89.6°C; η = 20.1 mPa·s; Δn = 0.099; Δ*ε* = 6.9.

### Example 19

| | |
|---|---|
| 1O2-GB(F,F)XB(F)B(F,F)-F | 5% |
| 7-HB(F,F)-F | 3% |
| 3-HB-O2 | 7% |
| 2-HHB(F)-F | 9% |
| 3-HHB(F)-F | 9% |
| 5-HHB(F)-F | 9% |
| 2-HBB(F)-F | 8% |
| 3-HBB(F)-F | 9% |
| 5-HBB(F)-F | 16% |
| 2-HBB-F | 4% |
| 3-HBB-F | 4% |
| 5-HBB-F | 3% |
| 3-HBB(F,F)-F | 5% |
| 5-HBB(F,F)-F | 9% |

NI = 96.5°C; η = 38.8 mPa·s; Δn = 0.186; Δε = 7.2.

### Example 20

| | |
|---|---|
| 2O1-GB(F)B(F,F)XB(F) -F | 4% |
| 5-HB-CL | 16% |
| 3-HB-O2 | 16% |
| 3-HHB-F | 4% |
| 3-HHB-CL | 3% |
| 4-HHB-CL | 4% |
| 3-HHB(F)-F | 8% |
| 4-HHB(F)-F | 7% |
| 5-HHB(F)-F | 9% |
| 7-HHB(F)-F | 8% |
| 5-HBB(F)-F | 4% |
| 1O1-HBBH-5 | 3% |
| 3-HHBB(F,F)-F | 2% |
| 4-HHBB(F,F)-F | 3% |
| 5-HHBB(F,F)-F | 3% |
| 3-HH2BB(F,F)-F | 3% |
| 4-HH2BB(F,F)-F | 3% |

### Example 21

| | |
|---|---|
| 2O1-GB(F)B(F,F)XB(F,F)-F | 3% |
| 3-HHB(F,F)-F | 9% |
| 3-H2HB(F,F)-F | 8% |
| 4-H2HB(F,F)-F | 8% |
| 5-H2HB(F,F)-F | 8% |
| 3-HBB(F,F)-F | 18% |
| 5-HBB(F,F)-F | 20% |
| 3-H2BB(F,F)-F | 10% |
| 5-HHBB(F,F)-F | 3% |
| 5-HHEBB-F | 2% |
| 3-HH2BB(F,F)-F | 3% |
| 1O1-HBBH-4 | 4% |
| 1O1-HBBH-5 | 4% |

### Example 22

| | |
|---|---|
| 2O1-GB(F,F)XB(F)B(F,F)-F | 9% |
| 5-HB-F | 12% |
| 6-HB-F | 9% |
| 7-HB-F | 7% |
| 2-HHB-OCF3 | 8% |
| 3-HHB-OCF3 | 7% |
| 4-HHB-OCF3 | 7% |
| 5-HHB-OCF3 | 5% |
| 3-HH2B-OCF3 | 4% |
| 5-HH2B-OCF3 | 4% |
| 3-HHB(F,F)-OCF3 | 3% |
| 3-HH2B(F)-F | 3% |
| 3-HBB(F)-F | 9% |
| 5-HBB(F)-F | 7% |
| 5-HBBH-3 | 3% |
| 3-HB(F)BH-3 | 3% |

NI = 81.6°C; η = 17.4 mPa·s; Δn = 0.093; Δε = 8.4.

### Example 23

| | |
|---|---|
| 401-GB(F,F)XB(F)B(F)-F | 10% |
| 5-HB-CL | 11% |
| 3-HB-02 | 8% |
| 3-HHB-1 | 5% |
| 3-HHB(F,F)-F | 7% |
| 3-HBB(F,F)-F | 20% |
| 5-HBB(F,F)-F | 12% |
| 3-HHEB(F,F)-F | 8% |
| 4-HHEB(F,F)-F | 3% |
| 5-HHEB(F,F) -F | 3% |
| 2-HBEB(F,F)-F | 3% |
| 3-HBEB(F,F)-F | 3% |
| 5-HBEB(F,F)-F | 3% |
| 3-HHBB(F,F)-F | 4% |

NI = 70.7°C; η = 24.9 mPa·s; Δn = 0.106; Δε = 11.0.

### Example 24

| | |
|---|---|
| 1O2-GB(F,F)XB(F)B(F,F)-F | 4% |
| 3-HB-CL | 4% |
| 5-HB-CL | 3% |
| 3-HHB-OCF3 | 5% |
| 3-H2HB-OCF3 | 5% |
| 5-H4HB-OCF3 | 15% |
| V-HHB(F)-F | 5% |
| 3-HHB(F)-F | 5% |
| 5-HHB(F)-F | 6% |
| 3-H4HB(F,F)-CF3 | 8% |
| 5-H4HB(F,F)-CF3 | 10% |
| 5-H2HB(F,F)-F | 5% |
| 5-H4HB(F,F)-F | 7% |
| 2-H2BB(F)-F | 5% |
| 3-H2BB(F)-F | 8% |
| 3-HBEB(F,F)-F | 5% |

NI = 69.6°C; η = 25.3 mPa·s; Δn = 0.093; Δε = 8.0.

### Example 25

| | |
|---|---|
| 2O1-GB(F)B(F,F)XB(F)-F | 3% |
| 5-HB-CL | 17% |
| 7-HB(F,F)-F | 3% |
| 3-HB-02 | 15% |
| 5-HB-02 | 12% |
| 3-HHB-1 | 9% |
| 3-HHB-O1 | 5% |
| 2-HHB(F)-F | 7% |
| 3-HHB(F)-F | 6% |
| 5-HHB(F)-F | 6% |
| 3-HHB(F,F)-F | 7% |
| 3-H2HB(F,F)-F | 5% |
| 4-H2HB(F,F)-F | 5% |

### Example 26

| | |
|---|---|
| 2O1-GB(F)B(F,F)XB(F,F)-F | 5% |
| 5-HB-CL | 3% |
| 7-HB(F)-F | 7% |
| 3-HB-02 | 16% |
| 5-HB-02 | 15% |
| 3-HHEB-F | 8% |
| 5-HHEB-F | 8% |
| 3-HHEB(F,F)-F | 10% |
| 4-HHEB(F,F)-F | 5% |
| 4-HGB(F,F)-F | 5% |
| 5-HGB(F,F)-F | 4% |
| 2-H2GB(F,F)-F | 4% |
| 3-H2GB(F,F) -F | 5% |
| 5-GHB(F,F)-F | 5% |

### Example 27

| | |
|---|---|
| 2O1-GB(F,F)XB(F)B(F,F)-F | 8% |
| 3-HB-O1 | 13% |
| 3-HH-4 | 5% |
| 3-HB(2F,3F)-O2 | 12% |
| 5-HB(2F,3F)-O2 | 12% |
| 2-HHB(2F,3F)-1 | 10% |
| 3-HHB(2F,3F)-1 | 13% |
| 3-HHB(2F,3F)-O2 | 11% |
| 5-HHB(2F,3F)-O2 | 10% |
| 3-HHB-1 | 6% |

NI = 81.1°C; η = 38.4 mPa·s; Δn = 0.092; Δε = -3.1.

### Example 28

| | |
|---|---|
| 4O1-GB(F,F)XB(F)B(F)-F | 9% |
| 2-HH-5 | 3% |
| 3-HH-4 | 15% |
| 3-HH-5 | 4% |
| 3-HB-O2 | 10% |
| 3-H2B(2F,3F)-O2 | 13% |
| 5-H2B(2F,3F)-O2 | 14% |
| 3-HHB(2F,3CL)-O2 | 5% |
| 2-HBB(2F,3F)-O2 | 3% |
| 3-HBB(2F,3F)-O2 | 7% |
| 5-HBB(2F,3F)-O2 | 7% |
| 3-HHB-1 | 3% |
| 3-HHB-3 | 4% |
| 3-HHB-O1 | 3% |

NI = 72.0°C; η = 23.0 mPa·s; Δn = 0.091; Δε = -3.6.

### Example 29

| | |
|---|---|
| 1O2-GB(F,F)XB(F)B(F,F)-F | 5% |
| 2-HH-3 | 21% |
| 3-HH-4 | 9% |
| 1-BB-3 | 9% |
| 3-HB-O2 | 2% |
| 3-BB(2F,3F)-O2 | 8% |
| 5-BB(2F,3F)-O2 | 6% |
| 2-HH1OB(2F,3F)-O2 | 11% |
| 3-HH1OB(2F,3F)-O2 | 20% |
| 3-HHB-1 | 4% |
| 3-HHB-O1 | 3% |
| 5-B(F)BB-2 | 2% |

NI = 70.9°C; η = 16.4 mPa·s; Δn = 0.098; Δε = -2.9.

### Example 30

| | |
|---|---|
| 2O1-GB(F)B(F,F)XB(F)-F | 5% |
| 2-HH-3 | 16% |
| 7-HB-1 | 10% |
| 5-HB-O2 | 8% |
| 3-HB(2F,3F)-O2 | 15% |
| 5-HB(2F,3F)-O2 | 16% |
| 3-HHB(2F,3CL)-O2 | 3% |
| 4-HHB(2F,3CL)-O2 | 3% |
| 3-HH1OCro(7F,8F)-5 | 5% |
| 5-HBB(F)B-2 | 10% |
| 5-HBB(F)B-3 | 9% |

### Example 31

| | |
|---|---|
| 2O1-GB(F)B(F,F)XB(F,F)-F | 4% |
| 1-BB-3 | 10% |
| 3-HH-V | 26% |
| 3-BB(2F,3F)-O2 | 13% |
| 2-HH1OB(2F,3F)-O2 | 20% |
| 3-HH1OB(2F, 3F)-O2 | 13% |
| 3-HHB-1 | 8% |
| 5-B(F)BB-2 | 6% |

### Example 32

| | |
|---|---|
| 2O1-GB(F,F)XB(F)B(F,F)-F | 10% |
| 2-HH-3 | 6% |
| 3-HH-V1 | 9% |
| 1V2-HH-1 | 8% |
| 1V2-HH-3 | 7% |
| 3-BB(2F,3F)-O2 | 8% |
| 5-BB(2F,3F)-O2 | 4% |
| 3-H1OB(2F,3F)-O2 | 5% |
| 2-HH1OB(2F,3F)-O2 | 5% |
| 3-HH1OB(2F,3F)-O2 | 18% |
| 3-HDhB(2F,3F)-O2 | 6% |
| 3-HHB-1 | 3% |
| 3-HHB-3 | 2% |
| 2-BB(2F,3F)B-3 | 9% |

NI = 80.3°C; η = 24.3 mPa·s; Δn = 0.108; Δε = -3.8.

### Example 33

| | |
|---|---|
| 4O1-GB(F,F)XB(F)B(F)-F | 10% |
| 1V2-BEB(F,F)-C | 6% |
| 3-HB-C | 10% |
| 2-BTB-1 | 10% |
| 3-HB-02 | 15% |
| 5-HB-02 | 15% |
| 3-HHB-1 | 4% |
| VFF-HHB-1 | 8% |
| VFF2-HHB-1 | 10% |
| 3-H2BTB-2 | 4% |
| 3-H2BTB-3 | 4% |
| 3-H2BTB-4 | 4% |

NI = 72.7°C; η = 18.6 mPa·s; Δn = 0.135; Δε = 9.8.

### 4-3. Example of optically isotropic liquid crystal composition (1')

### Example 34

Liquid crystal composition A corresponding to achiral component T was prepared by mixing a liquid crystal compound shown in a diagram below in a proportion described below.

Correspondence to a general formula was described on a right side of a structure formula.

### Liquid crystal composition A

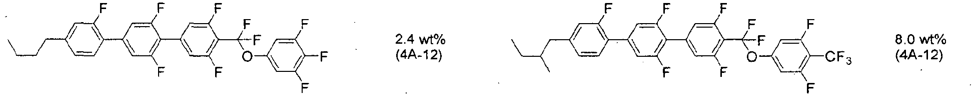

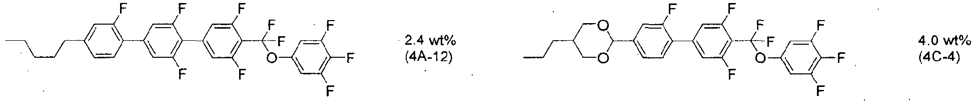

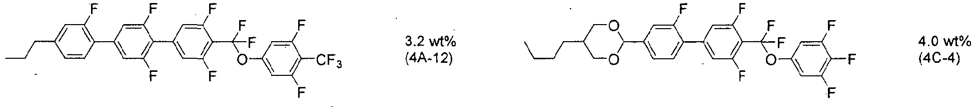

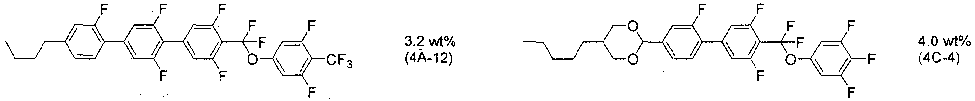

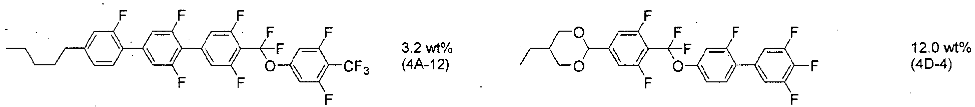

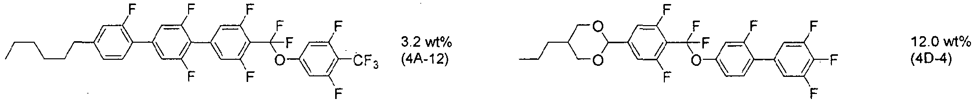

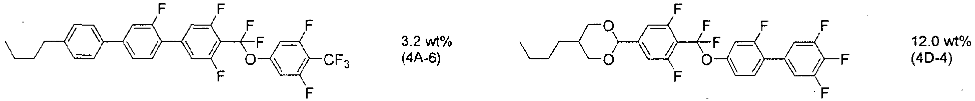

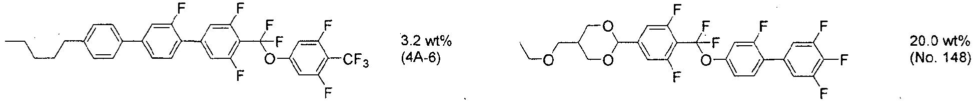

Next, liquid crystal composition A1 composed of liquid crystal composition A (94.8 wt%) and chiral agents BN-H4 (2.65 wt%) and BN-H5 (2.65 wt%) represented by formulas described below was obtained.

In addition, BN-H4 or BN-H5 was obtained by performing esterification from (R)-(+)-1,1-bi(2-naphthol) and carboxylic acid corresponding thereto by using dicyclohexylcarbodiimide (DCC).

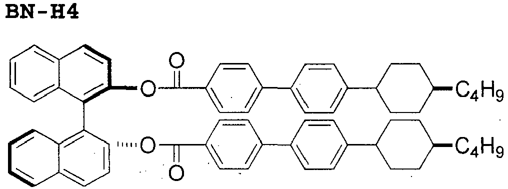

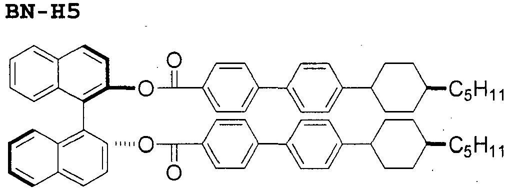

### Adjustment of a mixture of a liquid crystal composition and a monomer

As a mixture of a liquid crystal composition and a monomer, liquid crystal composition A1-1M was prepared by mixing 88.8% by weight of liquid crystal composition A1, 6.0% by weight of n-hexadecyl acrylate, 4.8% by weight of 1,4-di(4-(12-(acryloyloxy)dodecyloxy)-benzoyloxy)-2-methylbenzene (LCA-12) and 0.4% by weight of 2,2' -dimethoxyphenylacetophenone as a photopolymerization initiator.

A phase transition temperature (°C) of the liquid crystal composition A-1M was
A-1M: N* 39.2 N*+BP 39.6 BP - I.

A latter row indicates a phase transition temperature observed in a cooling process, and BP was developed also in the cooling process.

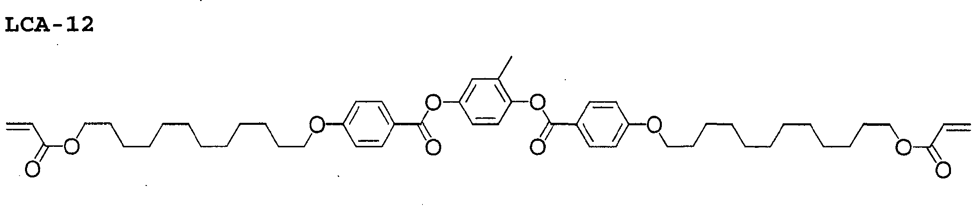

### Preparation of a polymer-liquid crystal composite material

Liquid crystal composition A-1M was interposed between a comb-shaped electrode substrate subjected to no alignment treatment and a facing glass substrate (not provided with an electrode) (cell thickness 8 µm), and an obtained cell was heated to a blue phase of 39.6°C. In a state described above, a polymerization reaction was performed by irradiation with ultraviolet light (ultraviolet light intensity 23 mWcm⁻² (365 nm)) for 1 minute.

Thus obtained polymer-liquid crystal composite material A-1P was maintained in an optically isotropic liquid crystal phase even if cooled to room temperature.

In addition, as shown in Figure 1, in electrodes of the comb-shaped electrode substrate, electrode 1 extended rightward from an electrode part for connection on a left side and electrode 2 extended leftward from an electrode part for connection on a right side were alternately arranged. Accordingly, when a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

### Example 35

A cell in which polymer-liquid crystal composite material A-1P obtained in Example 34 was interposed therebetween was set in an optical system shown in Figure 2, and electrooptical characteristics were measured. A white light source of a polarizing microscope (made by Nikon Corporation, ECLIPSE LV100POL) was used as a light source, and the cell was set in the optical system in such a manner that an angle incidence to the cell was adjusted to be perpendicular to a cell plane, and a line direction of the comb-shaped electrode became 45 degrees relative to Polarizer and Analyzer, respectively. A relationship between applied voltage and a transmittance was investigated at room temperature. In A-1P, if a rectangular wave of 21.6 V was applied thereto, a transmittance was 74.4%, and transmitted light intensity was saturated.

### Comparative Example

Liquid crystal composition W was prepared by mixing a liquid crystal compound shown in a figure below in a proportion described below. Liquid crystal composition W is a composition containing no compound (1). Correspondence to a general formula was described on a right side of a structure formula.

### Liquid crystal composition W

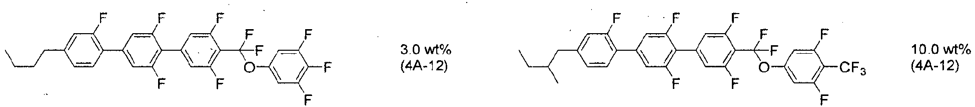

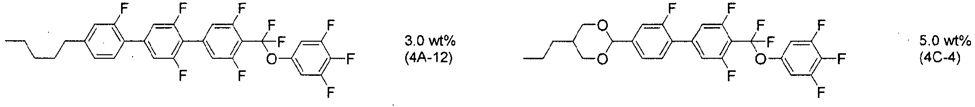

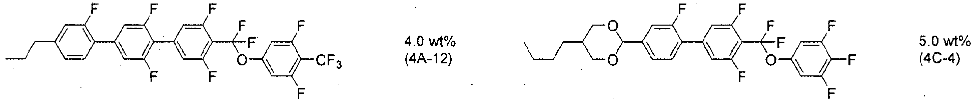

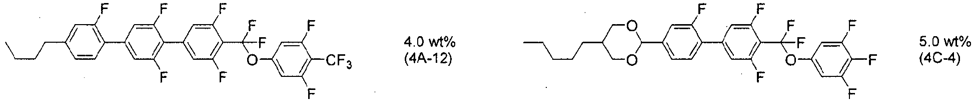

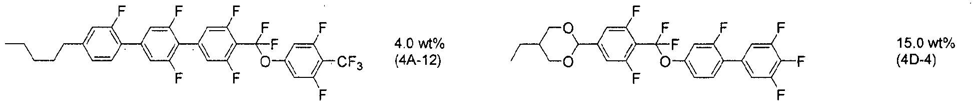

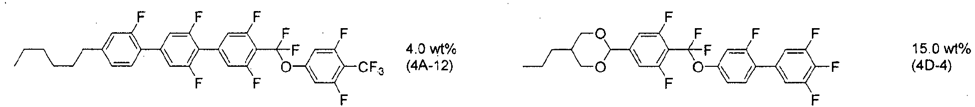

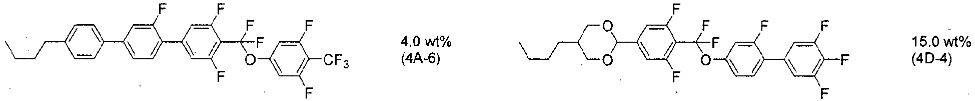

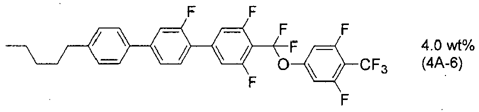

Next, liquid crystal composition W1 composed of liquid crystal composition W (94.8 wt%) and chiral agents BN-H4 (2.65 wt%) and BN-H5 (2.65 wt%) was obtained.

### Preparation of a mixture of a monomer and a liquid crystal composition

As a mixture of a liquid crystal composition and a monomer, liquid crystal composition W-1M was prepared by mixing 88.8% by weight of liquid crystal composition W1, 6.0% by weight of n-dodecylacrylate, 4.8% by weight of 1,4-di(4-(6-(acryloyloxy)hexyloxy)benzoyloxy)-2-methylbenzene (LCA-6) and 0.4% by weight of 2,2'-dimethoxyphenylacetophenone as a photopolymerization initiator.

### Preparation of a polymer-liquid crystal composite material

Liquid crystal composition W-1M was interposed between a comb-shaped electrode substrate subjected to no alignment treatment and a facing glass substrate (not provided with an electrode) (cell thickness 10 µm), and an obtained cell was heated to a blue phase of 40.1°C. In a state described above, a polymerization reaction was performed by irradiation with ultraviolet light (ultraviolet light intensity 23 mWcm⁻² (365 nm)) for 1 minute.

Thus obtained polymer-liquid crystal composite material W-1P was maintained in an optically isotropic liquid crystal phase even if cooled to room temperature.

In addition, as shown in Figure 1, in electrodes of the comb-shaped electrode substrate, electrode 1 extended rightward from an electrode part for connection on a left side and electrode 2 extended leftward from an electrode part for connection on a right side were alternately arranged. Accordingly, when a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

A cell in which polymer-liquid crystal composite material W-1P was interposed therebetween as obtained was set in an optical system shown in Figure 2, and electrooptical characteristics were measured. A white light source of a polarizing microscope (ECLIPSE LV100POL, made by Nikon Corporation) was used as a light source, and the cell was set in the optical system in such a manner that an angle incidence to the cell was adjusted to be perpendicular to a cell plane, and a line direction of the comb-shaped electrode became 45 degrees relative to Polarizer and Analyzer, respectively. A relationship between applied voltage and transmittance was investigated at room temperature. If a rectangular wave of 43 V was applied thereto, the transmittance reached 83%, and transmitted light intensity was saturated.

Driving voltage of polymer-liquid crystal composite material A-1P obtained in Example 35 and W-1P in Comparative Example each was summarized in Table 3. From the results, A-1P had 49.8% are found to be improved in driving voltage, in comparison with W-1P, and a device using compound (1) to be superb in having a larger effect of reduced voltage.

**Table 3 Driving voltage of polymer-liquid crystal composite materials A-1P and W-1P**

| Polymer-liquid crystal composite material | Driving voltage [V] |
|---|---|
| A-1P | 21.6 |
| W-1P (Comparative Example) | 43 |

### Example 36

Liquid crystal composition B corresponding to achiral component T was prepared by mixing a liquid crystal compound shown in a diagram below in a proportion described below.

Correspondence to a general formula was described on a right side of a structure formula.

### Liquid crystal composition B

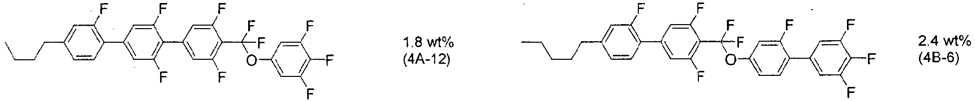

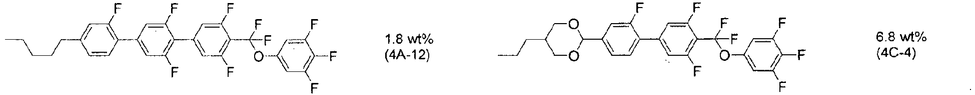

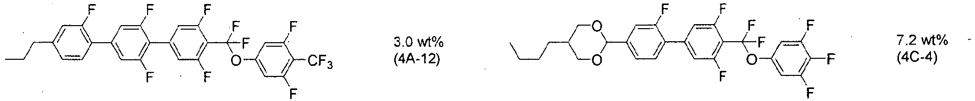

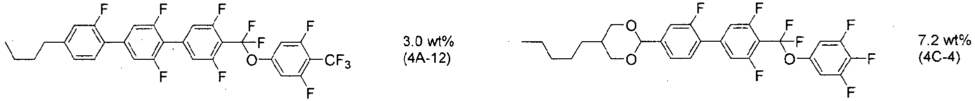

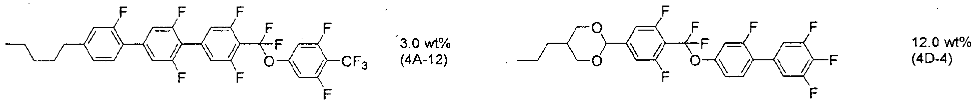

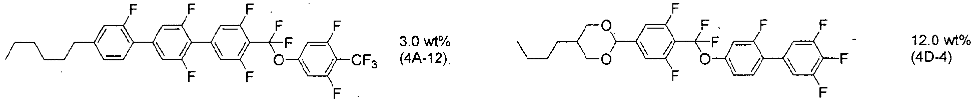

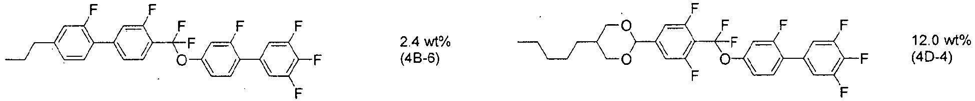

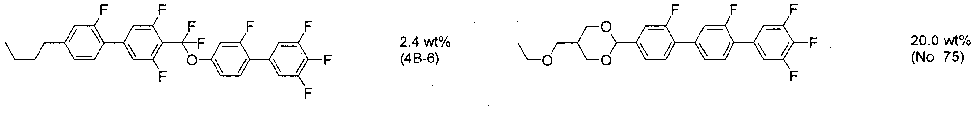

Next, liquid crystal composition B1 composed of liquid crystal composition B (95.2 wt%) and chiral agent (8H) BN-H5 (4.8 wt%) represented by a formula described below was obtained.

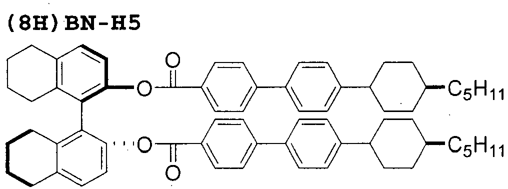

### Adjustment of a mixture of a liquid crystal composition and a monomer

As a mixture of a liquid crystal composition and a monomer, liquid crystal composition B-1M was prepared by mixing 88.8% by weight of liquid crystal composition B1, 6.0% by weight of n-hexadecyl acrylate, 4.8% by weight of 1,4-di-(4-(12-(acryloyloxy)dodecyloxy)-benzoyloxy)-2-methylbenzene (LCA-12) and 0.4% by weight of 2,2'-dimethoxyphenylacetophenone as a photopolymerization initiator.

### Preparation of a polymer-liquid crystal composite material

Liquid crystal composition B-1M was interposed between a comb-shaped electrode substrate subjected to no alignment treatment and a facing glass substrate (not provided with an electrode) (cell thickness 8 µm), and an obtained cell was heated to B-1M: a blue phase of 56.6°C. In a state described above, a polymerization reaction was performed by irradiation with ultraviolet light (ultraviolet light intensity 2 mWcm⁻² (365 nm)) for 7 minutes.

Thus obtained polymer-liquid crystal composite material B-1P was maintained in an optically isotropic liquid crystal phase even if cooled to room temperature.

In addition, as shown in Figure 1, in electrodes of the comb-shaped electrode substrate, electrode 1 extended rightward from an electrode part for connection on a left side and electrode 2 extended leftward from an electrode part for connection on a right side were alternately arranged. Accordingly, when a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

### Example 37

A cell in which polymer-liquid crystal composite material B-1P obtained in Example 36 was interposed therebetween was set in an optical system shown in Figure 2, and electrooptical characteristics were measured. A white light source of a polarizing microscope (ECLIPSE LV100POL, made by Nikon Corporation) was used as a light source, and the cell was set in the optical system in such a manner that an angle of incidence to the cell was adjusted to be perpendicular to a cell plane, and a line direction of the comb-shaped electrode became 45 degrees relative to Polarizer and Analyzer, respectively. A relationship between applied voltage and transmittance was investigated at room temperature. In B-1P, if a rectangular wave of 45.3 V was applied thereto, the transmittance reached 87.5%, and transmitted light intensity was saturated.

### Example 38

Liquid crystal composition C corresponding to achiral component T was prepared by mixing a liquid crystal compound shown in a diagram below in a proportion described below.

Correspondence to a general formula was described on a right side of a structure formula.

### Liquid crystal composition C

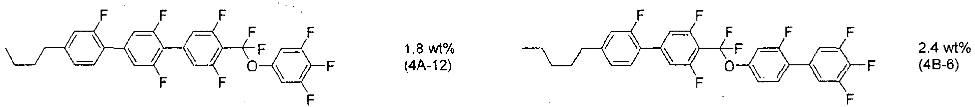

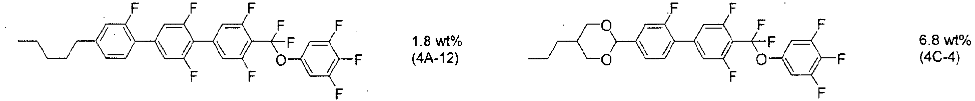

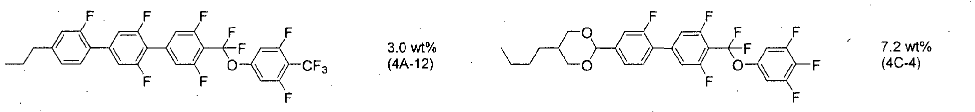

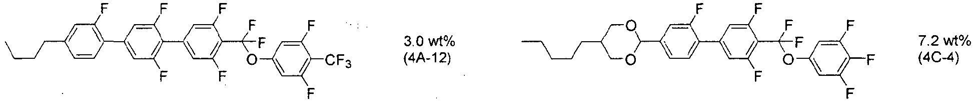

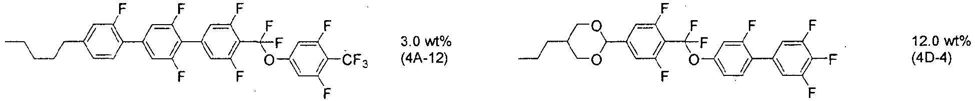

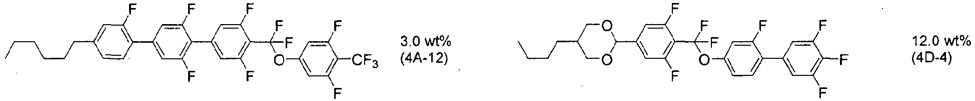

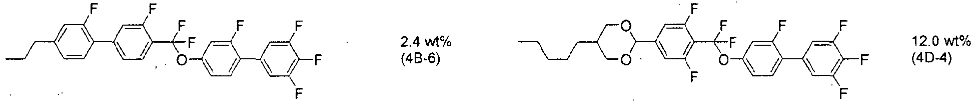

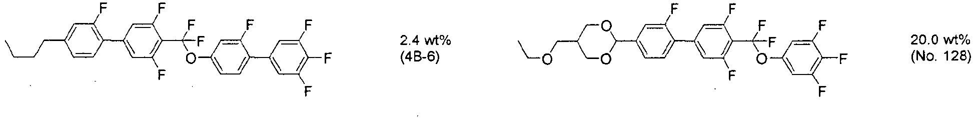

Next, liquid crystal composition C1 composed of liquid crystal composition C (95.2 wt%) and chiral agent (8H) BN-H5 (4.8 wt%) was obtained.

### Adjustment of a mixture of a liquid crystal composition and a monomer

As a mixture of a liquid crystal composition and a monomer, liquid crystal composition C-1M was prepared by mixing 88.8% by weight of liquid crystal composition C1, 6.0% by weight of n-hexadecyl acrylate, 4.8% by weight of 1,4-di-(4-(12-(acryloyloxy)dodecyloxy)-benzoyloxy)-2-methylbenzene (LCA-12) and 0.4% by weight of 2,2'-dimethoxyphenylacetophenone as a photopolymerization initiator.

### Preparation of a polymer-liquid crystal composite material

Liquid crystal composition C-1M was interposed between a comb-shaped electrode substrate subjected to no alignment treatment and a facing glass substrate (not provided with an electrode) (cell thickness 8 µm), and an obtained cell was heated to C-1M: a blue phase of 51.3°C. In a state described above, a polymerization reaction was performed by irradiation with ultraviolet light (ultraviolet light intensity 2 mWcm⁻² (365 nm)) for 7 minutes.

Thus obtained polymer-liquid crystal composite material C-1P was maintained in an optically isotropic liquid crystal phase even if cooled to room temperature.

In addition, as shown in Figure 1, in electrodes of the comb-shaped electrode substrate, electrode 1 extended rightward from an electrode part for connection on a left side and electrode 2 extended leftward from an electrode part for connection on a right side were alternately arranged. Accordingly, when a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

### Example 39

A cell in which polymer-liquid crystal composite material C-1P obtained in Example 38 was interposed therebetween was set in an optical system shown in Figure 2, and electrooptical characteristics were measured. A white light source of a polarizing microscope (ECLIPSE LV100POL, made by Nikon Corporation) was used as a light source, and the cell was set in the optical system in such a manner that an angle of incidence to the cell was adjusted to be perpendicular to a cell plane, and a line direction of the comb-shaped electrode became 45 degrees relative to Polarizer and Analyzer, respectively. A relationship between applied voltage and transmittance was investigated at room temperature. In C-1P, if a rectangular wave of 45.4 V was applied thereto, the transmittance reached 84.0%, and transmitted light intensity was saturated.

### Example 40

Liquid crystal composition D corresponding to achiral component T was prepared by mixing a liquid crystal compound shown in a diagram below in a proportion described below.

Correspondence to a general formula was described on a right side of a structure formula.

### Liquid crystal composition D

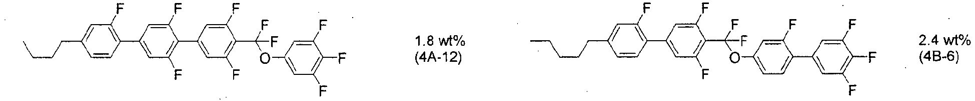

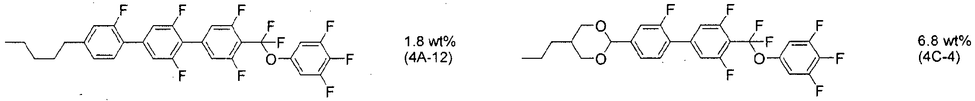

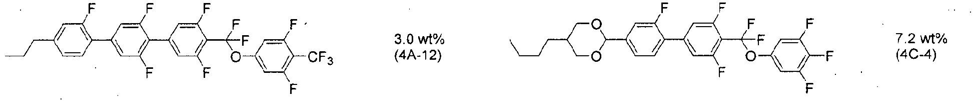

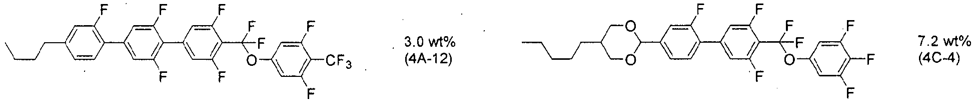

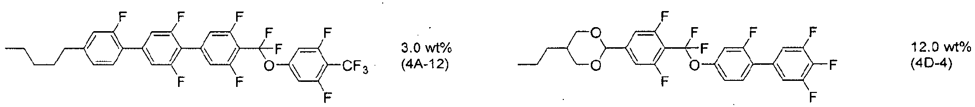

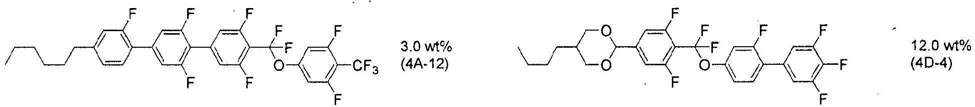

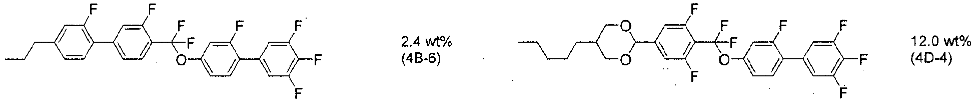

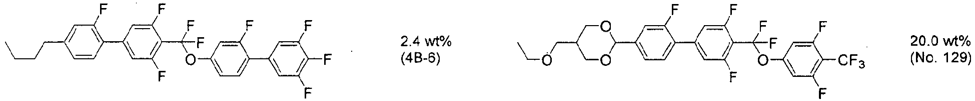

Next, liquid crystal composition D1 composed of liquid crystal composition D (95.2 wt%) and chiral agent (8H) BN-H5 (4.8 wt%) was obtained.

### Adjustment of a mixture of a liquid crystal composition and a monomer

As a mixture of a liquid crystal composition and a monomer, liquid crystal composition D-1M was prepared by mixing 88.8% by weight of liquid crystal composition D1, 6.0% by weight of n-hexadecyl acrylate, 4.8% by weight of 1,4-di-(4-(12-(acryloyloxy)dodecyloxy)-benzoyloxy)-2-methylbenzene (LCA-12) and 0.4% by weight of 2,2'-dimethoxyphenylacetophenone as a photopolymerization initiator.

### Preparation of a polymer-liquid crystal composite material

Liquid crystal composition D-1M was interposed between a comb-shaped electrode substrate subjected to no alignment treatment and a facing glass substrate (not provided with an electrode) (cell thickness 8 µm), and an obtained cell was heated to D-1M: a blue phase of 50.1°C. In a state described above, a polymerization reaction was performed by irradiation with ultraviolet light (ultraviolet light intensity 2 mWcm⁻² (365 nm)) for 7 minutes.

Thus obtained polymer-liquid crystal composite material D-1P was maintained in an optically isotropic liquid crystal phase even if cooled to room temperature.

In addition, as shown in Figure 1, in electrodes of the comb-shaped electrode substrate, electrode 1 extended rightward from an electrode part for connection on a left side and electrode 2 extended leftward from an electrode part for connection on a right side were alternately arranged. Accordingly, when a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

### Example 41

A cell in which polymer-liquid crystal composite material D-1P obtained in Example 40 was interposed therebetween was set in an optical system shown in Figure 2, and electrooptical characteristics were measured. A white light source of a polarization microscope (ECLIPSE LV100POL, made by Nikon Corporation) was used as a light source, and the cell was set in the optical system in such a manner that an angle of incidence to the cell was adjusted to be perpendicular to a cell plane, and a line direction of the comb-shaped electrode became 45 degrees relative to Polarizer and Analyzer, respectively. A relationship between applied voltage and transmittance was investigated at room temperature. In D-1P, if a rectangular wave of 40.4 V was applied thereto, the transmittance reached 87.1%, and transmitted light intensity was saturated.

### Example 42

Liquid crystal composition E corresponding to achiral component T was prepared by mixing a liquid crystal compound shown in a diagram below in a proportion described below.

Correspondence to a general formula was described on a right side of a structure formula.

### Liquid crystal composition E

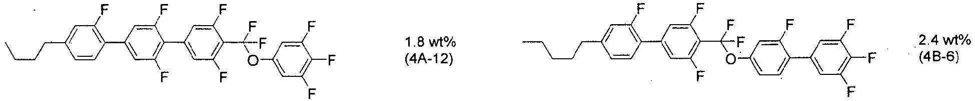

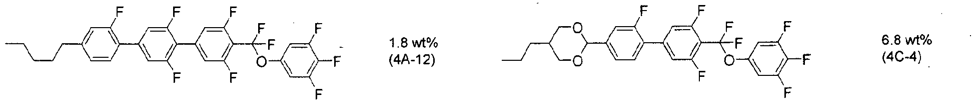

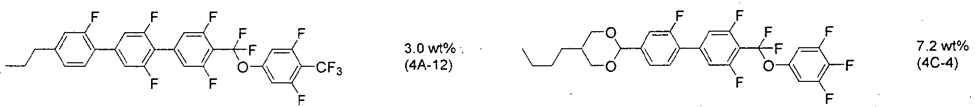

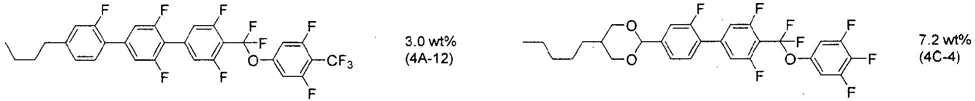

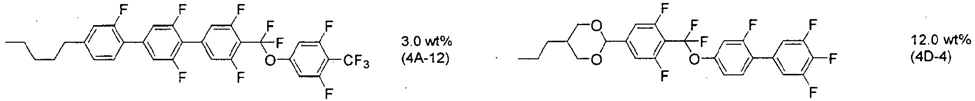

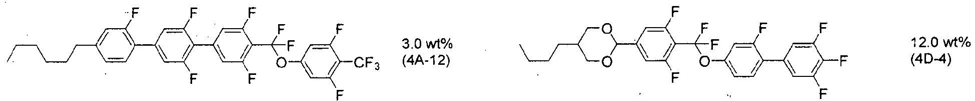

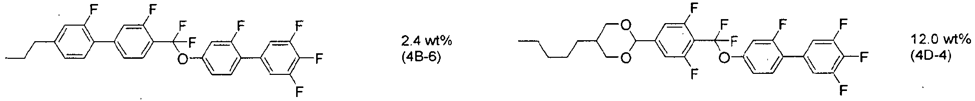

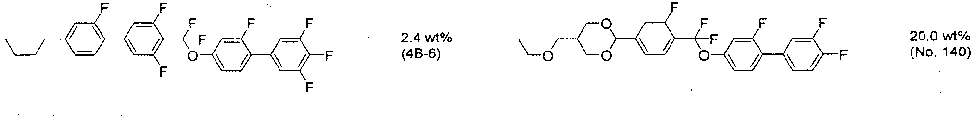

Next, liquid crystal composition E1 composed of liquid crystal composition E (95.2 wt%) and chiral agent (8H) BN-H5 (4.8 wt%) was obtained.

### Adjustment of a mixture of a liquid crystal composition and a monomer

As a mixture of a liquid crystal composition and a monomer, liquid crystal composition E-1M was prepared by mixing 88.8% by weight of liquid crystal composition E1, 6.0% by weight of n-hexadecyl acrylate, 4.8% by weight of 1,4-di-(4-(12-(acryloyloxy)dodecyloxy)-benzoyloxy)-2-methylbenzene (LCA-12) and 0.4% by weight of 2,2'-dimethoxyphenylacetophenone as a photopolymerization initiator.

### Preparation of a polymer-liquid crystal composite material

Liquid crystal composition E-1M was interposed between a comb-shaped electrode substrate subjected to no alignment treatment and a facing glass substrate (not provided with an electrode) (cell thickness 8 µm), and an obtained cell was heated to E-1M: a blue phase of 54.1°C. In a state described above, a polymerization reaction was performed by irradiation with ultraviolet light (ultraviolet light intensity 2 mWcm⁻² (365 nm)) for 7 minutes.

Thus obtained polymer-liquid crystal composite material E-1P was maintained in an optically isotropic liquid crystal phase even if cooled to room temperature.

In addition, as shown in Figure 1, in electrodes of the comb-shaped electrode substrate, electrode 1 extended rightward from an electrode part for connection on a left side and electrode 2 extended leftward from an electrode part for connection on a right side were alternately arranged. Accordingly, when a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

### Example 43

A cell in which polymer-liquid crystal composite material E-1P obtained in Example 42 was interposed therebetween was set in an optical system shown in Figure 2, and electrooptical characteristics were measured. A white light source of a polarization microscope (ECLIPSE LV100POL, made by Nikon Corporation) was used as a light source, and the cell was set in the optical system in such a manner that an angle of incidence to the cell was adjusted to be perpendicular to a cell plane, and a line direction of the comb-shaped electrode became 45 degrees relative to Polarizer and Analyzer, respectively. A relationship between applied voltage and transmittance was investigated at room temperature. In E-1P, if a rectangular wave of 57.8 V was applied thereto, the transmittance reached 85.1%, and transmitted light intensity was saturated.

### Comparative Example

Liquid crystal composition W2 composed of liquid crystal composition W (95.2 wt%) and chiral agent (8H) BN-H5 (4.8 wt%) was obtained.

### Adjustment of a mixture of a liquid crystal composition and a monomer

As a mixture of a liquid crystal composition and a monomer, liquid crystal composition W-2M was prepared by mixing 88.8% by weight of liquid crystal composition W2, 6.0% by weight of n-hexadecyl acrylate, 4.8% by weight of 1,4-di-(4-(12-(acryloyloxy)dodecyloxy)-benzoyloxy)-2-methylbenzene (LCA-12) and 0.4% by weight of 2,2'-dimethoxyphenylacetophenone as a photopolymerization initiator.

### Preparation of a polymer-liquid crystal composite material

Liquid crystal composition W-2M was interposed between a comb-shaped electrode substrate subjected to no alignment treatment and a facing glass substrate (not provided with an electrode) (cell thickness 8 µm), and an obtained cell was heated to W-2M: a blue phase of 52.8°C. In a state described above, a polymerization reaction was performed by irradiation with ultraviolet light (ultraviolet light intensity 2 mWcm⁻² (365 nm)) for 7 minutes.

Thus obtained polymer-liquid crystal composite material W-2P was maintained in an optically isotropic liquid crystal phase even if cooled to room temperature.

In addition, as shown in Figure 1, in electrodes of the comb-shaped electrode substrate, electrode 1 extended rightward from an electrode part for connection on a left side and electrode 2 extended leftward from an electrode part for connection on a right side were alternately arranged. Accordingly, when a potential difference exists between electrode 1 and electrode 2, on the comb-shaped electrode substrate as shown in Figure 1, a state in which an electric field with two directions of an upward direction and a downward direction on a drawing exists can be provided if attention is paid to one electrode.

A cell in which the polymer/liquid crystal composite material was interposed therebetween as obtained was set in an optical system shown in Figure 2, and electrooptical characteristics were measured. A white light source of a polarization microscope (ECLIPSE LV100POL, made by Nikon Corporation) was used as a light source, and the cell was set in the optical system in such a manner that an angle of incidence to the cell was adjusted to be perpendicular to a cell plane, and a line direction of the comb-shaped electrode became 45 degrees relative to Polarizer and Analyzer, respectively. A relationship between applied voltage and transmittance was investigated at room temperature. If a rectangular wave of 60.5 V was applied thereto, the transmittance reached 82.7%, and transmitted light intensity was saturated.

Driving voltage of polymer-liquid crystal composite materials B-1P to E-1P and W-2P being Comparative Example each was summarized in Table 4. From the results, B-1P to E-1P are found to be improved in the driving voltage, in comparison with W-1P' , and a device using compound (1) to be superb in having a larger effect of reduced voltage.

**Table 4 Driving voltage of polymer-liquid crystal composite materials B-1P to E-1P and W-2P**

| Polymer/liquid crystal composite material | Driving voltage [V] |
|---|---|
| B-1P | 45.3 |
| C-1P | 45.4 |
| D-1P | 40.4 |
| E-1P | 57.8 |
| W-2P (Comparative Example) | 60.5 |

### Industrial Applicability

A liquid crystal compound of the invention satisfies at least one of physical properties such as high stability to heat, light and so forth, a high clearing point, low minimum temperature of a liquid crystal phase, small viscosity, suitable optical anisotropy, large dielectric anisotropy, a suitable elastic constant and excellent compatibility with other liquid crystal compounds. A liquid crystal composition contains the compound and satisfies at least one of physical properties such as high maximum temperature, low minimum temperature, small viscosity, suitable optical anisotropy, large dielectric anisotropy and a suitable elastic constant. The composition has a suitable balance regarding at least two of the physical properties. A liquid crystal display device includes the composition and has a wide temperature range in which the device can be used, a short response time, a large voltage holding ratio, low threshold voltage, a large contrast ratio and a long service life. A liquid crystal display optical device using an optically isotropic liquid crystal composition of the invention can be driven at low voltage, and is superior to a conventional technology in development of BP in a cooling process. In addition, the development of BP in the cooling process means that a polymer-liquid crystal composite material can be easily adjusted in a production process of the optical device, and therefore exhibits usefulness of the optical device of the invention. Accordingly, the composition can be widely applied to a liquid crystal display device used in a personal computer, a television and so forth.

### Reference Signs List

- 1:: Electrode 1
- 2:: Electrode 2
- 3:: Light source
- 4:: Polarizer
- 5:: Comb-shaped electrode cell
- 6:: Analyzer
- 7:: Photodetector

## Claims

1. A liquid crystal composition, containing at least one compound represented by formula (1): 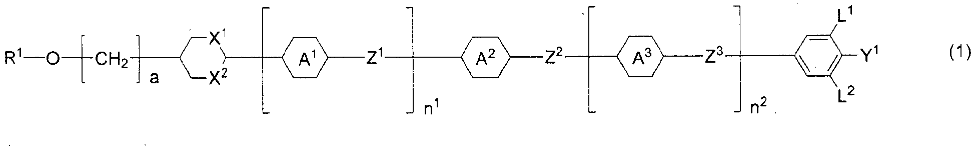 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine;
ring A¹, ring A² and ring A³ are independently represented by a formula described below; 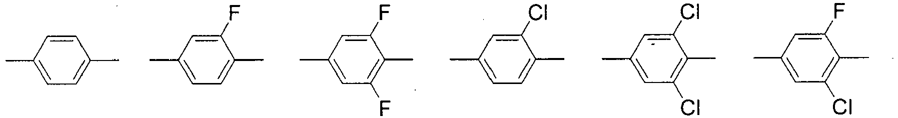 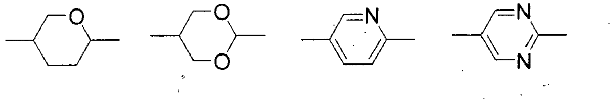 wherein, X¹ and X² are independently -O-, -S- or -CH₂-, and a case where both of X¹ and X² are -CH₂- is excluded;
Y¹ is hydrogen, fluorine, chlorine, -C=N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-;
Z¹ and Z³ are independently a single bond, -CH₂CH₂-, -CH₂O-, -CF₂O-, -OCF₂-, -COO-, -OCO-, -CH=CH- or -C≡C-;
Z² is a single bond, -CF₂O- or -COO-;
L¹ and L² are independently hydrogen or halogen;
a is 0, 1, 2 or 3; and
n¹ and n² are independently 0, 1 or 2, and an expression: n¹ + n² ≤ 2 holds; and
when n¹ + n² = 0 and Z² is -CF₂O, Y¹ is hydrogen, fluorine, chlorine, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

2. The liquid crystal composition according to claim 1, containing at least one compound represented by formula (1-1): 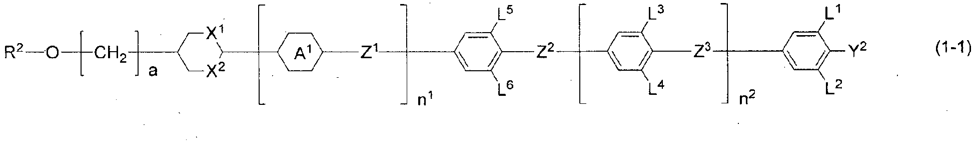 wherein, in formula (1-1),
R² is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
ring A¹ is represented by a formula described below; 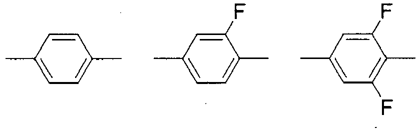 wherein, X¹ and X² are independently -O- or -CH₂-, and a case where both X¹ and X² are -CH₂- is excluded;
Y² is hydrogen, fluorine, chlorine, -C≡N, -CH₂F, -CHF₂, -CF₃, -OCH₂F, -OCHF₂, -OCF₃, -CH=CHF, -CH=CF₂, -CF=CHF, -CH=CHCF₃, -OCH=CF₂, -OCF=CF₂ or -OCH=CHCF₃;
Z¹ and Z³ are independently a single bond, -CH₂CH₂-, -CH₂O-, -CF₂O-, -OCF₂-, -COO-, -OCO-, -CH=CH- or -C≡C-;
Z² is a single bond, -CF₂O- or -COO-;
L¹, L², L³, L⁴, L⁵ and L⁶ are independently hydrogen, fluorine or chlorine;
a is 0, 1, 2 or 3; and
n¹ and n² are independently 0, 1 or 2, and an expression: n¹ + n² ≤ 2 holds.

3. The liquid crystal composition according to claim 1 or 2, containing at least one compound represented by formula (1-1-1): 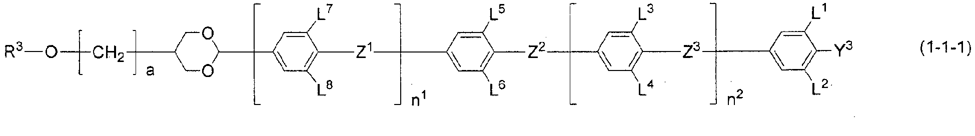 wherein, in formula (1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C≡N, -CF₃, -OCF₃, -OCH=CF₂ or -OCH=CHCF₃ ;
Z¹ and Z³ are independently a single bond, -CH₂O-, -CF₂0-, -COO-, -CH=CH- or -C≡C-;
Z² is a single bond, -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

4. The liquid crystal composition according to any one of claims 1 to 3, wherein a is 1.

5. The liquid crystal composition according to any one of claims 1 to 3, wherein a is 2.

6. The liquid crystal composition according to any one of claims 1 to 5, containing at least one compound represented by formula (1-1-1-1) : 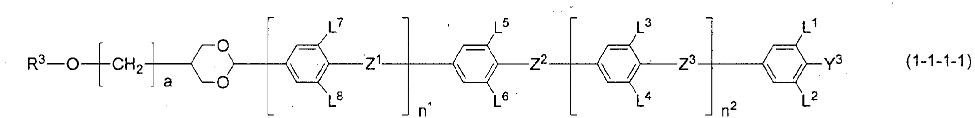 wherein, in formula (1-1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C≡N, -CF₃, -OCF₃, -OCH=CF₂ or -OCH=CHCF₃ ;
Z¹ and Z³ are independently a single bond, -CF₂0-, -COO- or -C≡C-;
Z² is -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

7. The liquid crystal composition according to any one of claims 1 to 6, containing at least one compound represented by formulas (1-1-1-1-1) to (1-1-1-1-5): 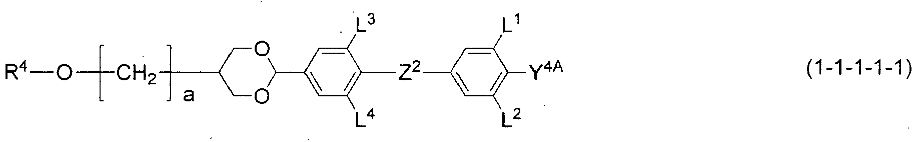 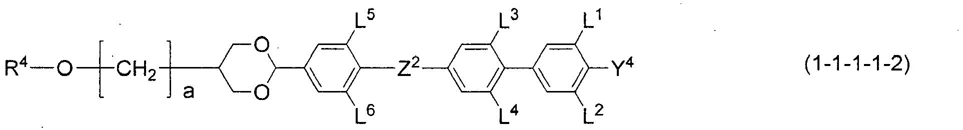 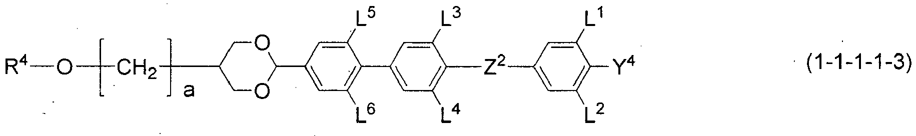 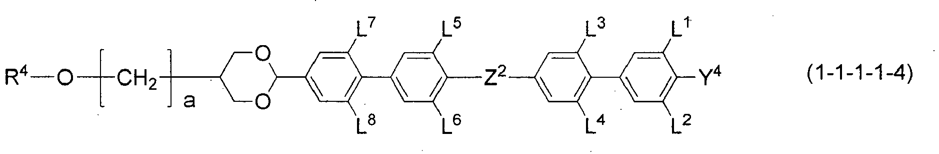 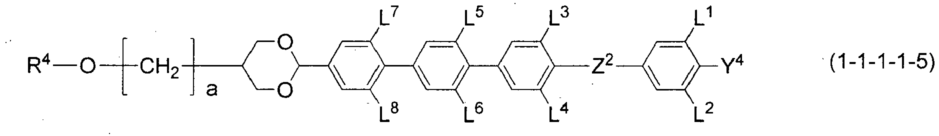 wherein, in formulas (1-1-1-1-1) to (1-1-1-1-5),
R⁴ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁴ is hydrogen, fluorine, chlorine, -C≡N, -CF₃, -OCF₃, -OCH=CF₂ or -OCH=CHCF₃ ;
Y^{4A} is hydrogen, fluorine, chlorine, -CF₃, -OCF₃, -OCH=CF₂ or -OCH=CHCF₃ ;
Z² is -CF₂O- or -COO-;
L¹, L², L³, L⁴, L⁵, L⁶, L⁷ and L⁸ are independently hydrogen or fluorine; and
a is 0, 1, 2 or 3.

8. The compound according to any one of claims 1 to 6, containing at least one compound represented by formula (1-1-1-1-11) or (1-1-1-1-12) : 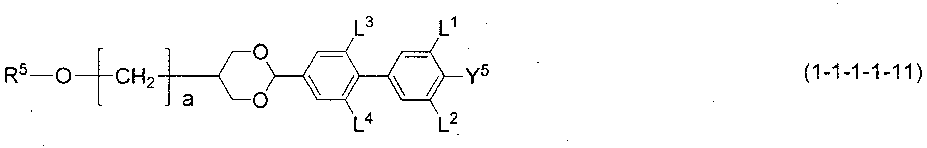 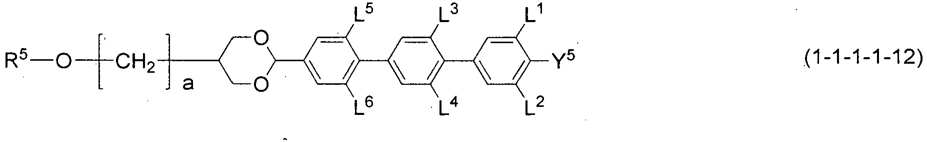 wherein, in formulas (1-1-1-1-11) and (1-1-1-1-12),
R⁵ is alkyl having 1 to 12 carbons, and in the alkyl, at least one piece of -CH₂- may be replaced by -O-, however, a case where two pieces of -O- are adjacent to each other is excluded, and at least one piece of -CH₂CH2- may be replaced by -CH=CH-;
Y⁵ is hydrogen, fluorine, chlorine, -C≡N, -CF₃, -OCF₃, -OCH=CF₂ or -OCH=CHCF₃ ;
L¹, L², L³, L⁴, L⁵ and L⁶ are independently hydrogen or fluorine; and
a is 0, 1, 2 or 3.

9. The liquid crystal composition according to any one of claims 1 to 8, further containing at least one compound selected from the group of compounds represented by formulas (2) to (5): 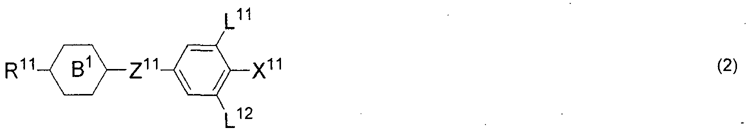 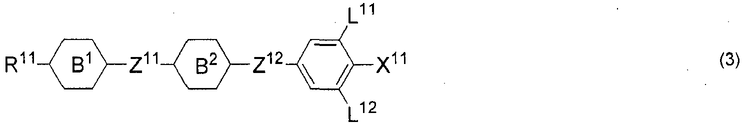 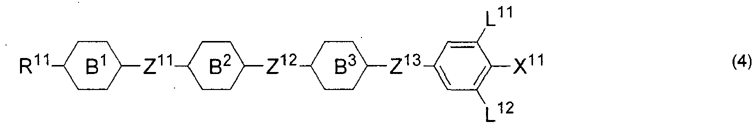 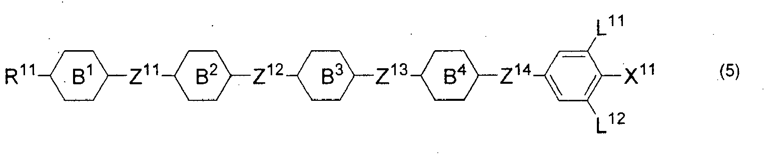 wherein, in formulas (2) to (5),
R¹¹ is alkyl having 1 to 10 carbons or alkenyl having 2 to 10 carbons, and in the alkyl and the alkenyl, at least one piece of hydrogen may be replaced by fluorine;
X¹¹ is hydrogen, fluorine, chlorine, -OCF₃, -OCHF₂, -CF₃, -CHF₂, -CH₂F, -OCF₂CHF₂ or -OCF₂CHFCF₃;
ring B¹, ring B², ring B³ and ring B⁴ are independently 1,4-cyclohexylene, 1,4-phenylene in which at least one piece of hydrogen may be replaced by fluorine, tetrahydropyran-2,5-diyl, 1,3-dioxane-2,5-diyl or pyrimidine-2,5-diyl;
Z¹¹, Z¹², Z¹³ and Z¹⁴ are independently a single bond, -CH₂CH₂- , -CH=CH- , -C=C-, -COO-, -CF₂0-, -OCF₂-, -CH₂O- or - (CH₂)₄-; and
L¹¹ and L¹² are independently hydrogen or fluorine.

10. The liquid crystal composition according to any one of claims 1 to 9, further containing at least one compound selected from the group of compounds represented by formula (6): 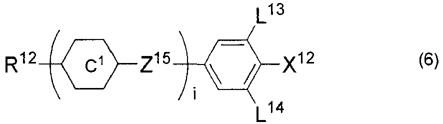 wherein, in formula (6),
R¹² is alkyl having 1 to 10 carbons or alkenyl having 2 to 10 carbons, and in the alkyl and the alkenyl, at least one piece of hydrogen may be replaced by fluorine;
X¹² is -C≡N or -C≡C-CN;
ring C¹ is 1, 4-cyclohexylene, 1,4-phenylene in which at least one piece of hydrogen may be replaced by fluorine, tetrahydropyran-2,5-diyl, 1,3-dioxane-2,5-diyl or pyrimidine-2,5-diyl;
Z¹⁵ is a single bond, -CH₂CH₂-, -C≡C-, -COO-, -CF₂0-, -OCF₂- or -CH₂O- ;
L¹³ and L¹⁴ are independently hydrogen or fluorine; and
i is 1, 2, 3 or 4.

11. The liquid crystal composition according to any one of claims 1 to 10, further containing at least one compound selected from the group of compounds represented by formulas (7) to (13): 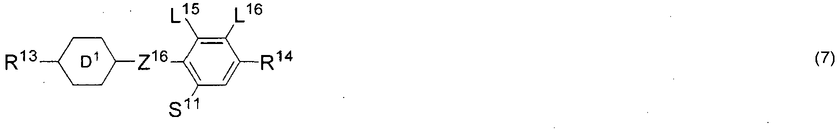 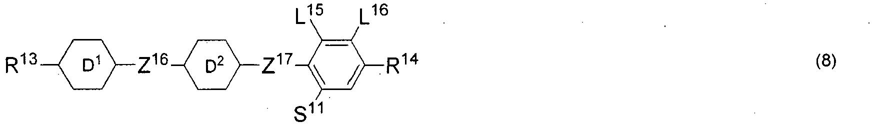 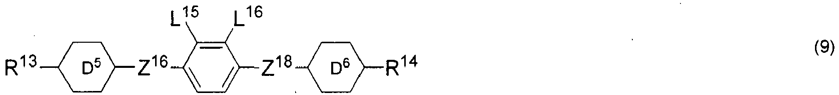 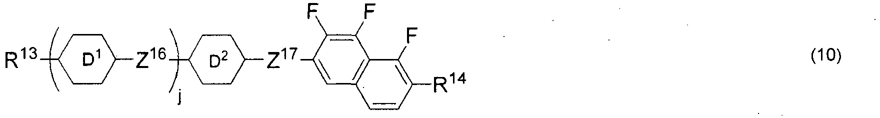 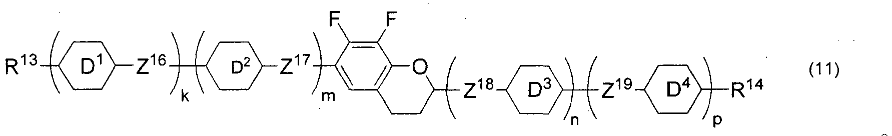 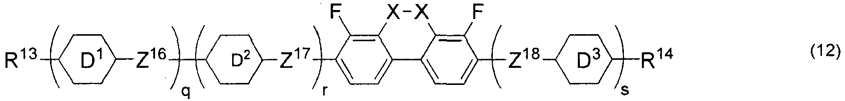 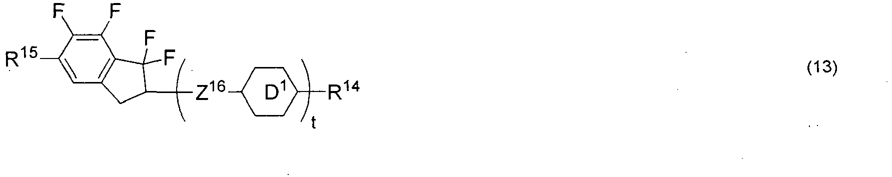 wherein, in formulas (7) to (13),
R¹³ and R¹⁴ are independently alkyl having 1 to 10 carbons or alkenyl having 2 to 10 carbons, and in the alkyl and the alkenyl, at least one piece of -CH₂- may be replaced by -O-, and at least one piece of hydrogen may be replaced by fluorine;
R¹⁵ is hydrogen, fluorine, alkyl having 1 to 10 carbons or alkenyl having 2 to 10 carbons, and in the alkyl and the alkenyl, at least one piece of -CH₂- may be replaced by -O- , and at least one piece of hydrogen may be replaced by fluorine;
S¹¹ is hydrogen or methyl;
X is -CF₂-, -O- or -CHF-;
ring D¹, ring D², ring D³ and ring D⁴ are independently 1,4-cyclohexylene, 1,4-cyclohexenylene, 1,4-phenylene in which at least one piece of hydrogen may be replaced by fluorine, tetrahydropyran-2,5-diyl or decahydronaphthalene-2,6-diyl;
ring D⁵ and ring D⁶ are independently 1,4-cyclohexylene, 1,4-cyclohexenylene, 1,4-phenylene, tetrahydropyran-2,5-diyl or decahydronaphthalene-2,6-diyl;
Z¹⁶, Z¹⁷, Z¹⁸ and Z¹⁹ are independently a single bond, -CH₂CH₂- , -COO- , -CH₂O-, -OCF₂- or -OCF₂CH₂CH₂-;
L¹⁵ and L¹⁶ are independently fluorine or chlorine; and
j, k, m, n, p, q, r and s are independently 0 or 1, a sum of k, m, n and p is 1 or 2, a sum of q, r and s is 0, 1, 2 or 3, and t is 1, 2 or 3.

12. The liquid crystal composition according to any one of claims 1 to 11, further containing at least one compound selected from the group of compounds represented by formulas (14) to (16): 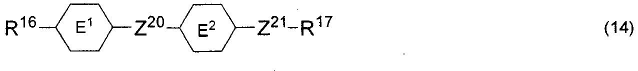 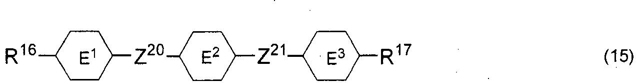 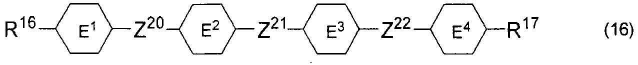 wherein, in formulas (14) to (16),
R¹⁶ and R¹⁷ are independently alkyl having 1 to 10 carbons or alkenyl having 2 to 10 carbons;
ring E¹, ring E², ring E³ and ring E⁴ are independently 1,4-cyclohexylene, 1,4-phenylene, 2-fluoro-1,4-phenylene, 2,5-difluoro-1,4-phenylene or pyrimidine-2,5-diyl; and
Z²⁰, Z²¹ and Z²² are independently a single bond, -CH₂CH₂-, -CH=CH-, -C≡C- or -COO-.

13. The liquid crystal composition according to any one of claims 1 to 12, further containing at least one of a polymerizable compound, an optically active compound, an antioxidant, an ultraviolet light absorber, a light stabilizer, a heat stabilizer and an antifoaming agent.

14. A liquid crystal composition that contains a compound represented by formula (1) and a chiral agent, and develops an optically isotropic liquid crystal phase: 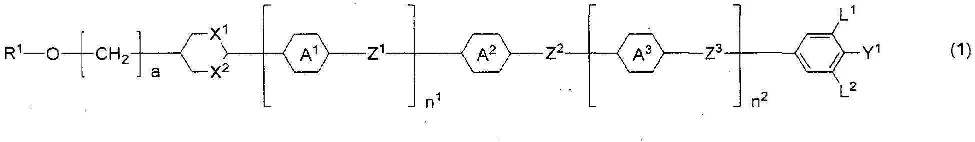 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine;
ring A¹, ring A² and ring A³ are independently represented by a formula described below; 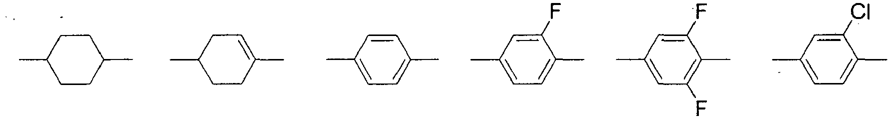 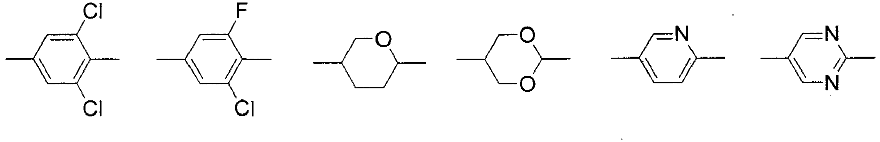 wherein, X¹ and X² are independently -O-, -S- or -CH₂-, and a case where both X¹ and X² are -CH₂- is excluded;
Y¹ is hydrogen, fluorine, chlorine, -C≡N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-;
Z¹ and Z³ are independently a single bond, -CH₂CH₂-, -CH₂O-, -CF₂0-, -OCF₂-, -COO-, -OCO-, -CH=CH- or -C≡C-;
Z² is a single bond, -CF₂O- or -COO-;
L¹ and L² are independently hydrogen or halogen;
a is 0, 1, 2 or 3; and
n¹ and n² are independently 0, 1 or 2, and an expression: n¹ + n² ≤ 2 holds; and
when n¹+ n² = 0 and Z² is -CF₂O, Y¹ is hydrogen, fluorine, chlorine, -C≡N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

15. The liquid crystal composition according to claim 14, containing at least one compound represented by formula (1-1): 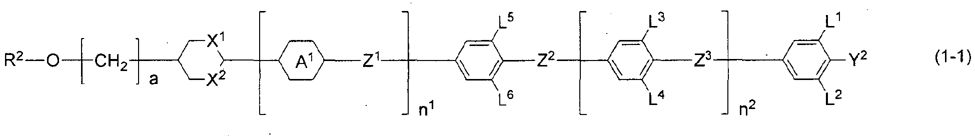 wherein, in formula (1-1),
R² is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
ring A¹ is represented by a formula described below; 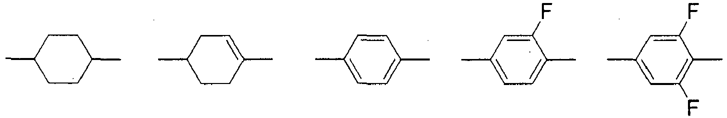 wherein, X¹ and X² are independently -0- or -CH₂-, and a case where both X¹ and X² are -CH₂- is excluded;
Y² is hydrogen, fluorine, chlorine, -C≡N, -CH₂F, -CHF₂, -CF₃, -OCH₂F, -OCHF₂, -OCF₃, -CH=CHF, -CH=CF₂, -CF=CHF, -CH=CHCF₃, -OCH=CF₂, -OCF=CF₂ or -OCH=CHCF₃;
Z¹ and Z³ are independently a single bond, -CH₂CH₂-, -CH₂O-, -CF₂0-, -OCF₂-, -COO-, -OCO-, -CH=CH- or -C≡C-;
Z² is a single bond, -CF₂O- or -COO-;
L¹, L², L³, L⁴, L⁵ and L⁶ are independently hydrogen, fluorine or chlorine;
a is 0, 1, 2 or 3; and
n¹ and n² are independently 0, 1 or 2, and an expression: n¹ + n² ≤ 2 holds.

16. The liquid crystal composition according to claim 14 or 15, containing at least one compound represented by formula (1-1-1): 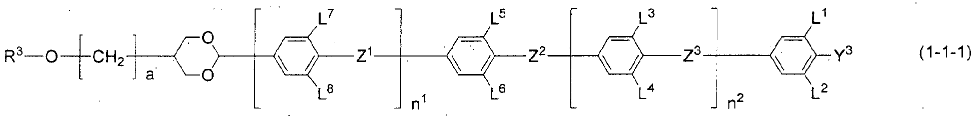 wherein, in formula (1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C≡N, -CF₃, -OCF₃, -OCH=CF₂ or -OCH=CHCF₃ ;
Z¹ and Z³ are independently a single bond, -CH₂O-, -CF₂0-, -COO-, -CH=CH- or -C≡C-;
Z² is a single bond, -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

17. The liquid crystal composition according to any one of claims 14 to 16, wherein a is 1.

18. The liquid crystal composition according to any one of claims 14 to 16, wherein a is 2.

19. The liquid crystal composition according to any one of claims 14 to 18, containing at least one compound represented by formula (1-1-1-1) : 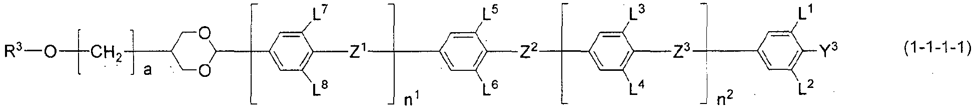 wherein, in formula (1-1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C≡N, -CF₃, -OCF₃, -OCH=CF₂ or -OCH=CHCF₃ ;
Z¹ and Z³ are independently a single bond, -CF₂0-, -COO- or -C≡C-; Z² is -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, an expression: n¹ + n² ≤ 2 holds.

20. The liquid crystal composition according to any one of claims 14 to 19, containing at least one compound represented by formulas (1-1-1-1-1) to (1-1-1-1-5): 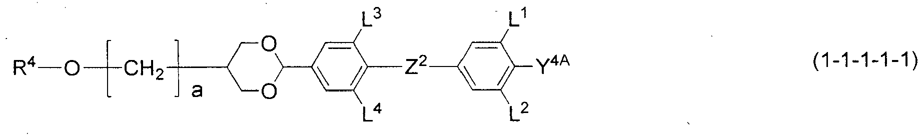 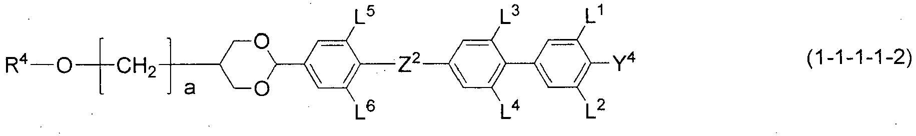 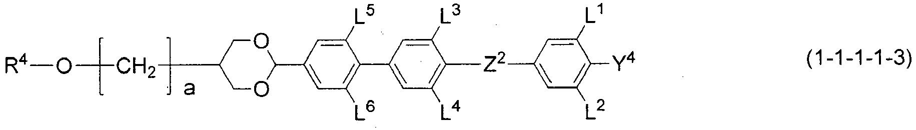 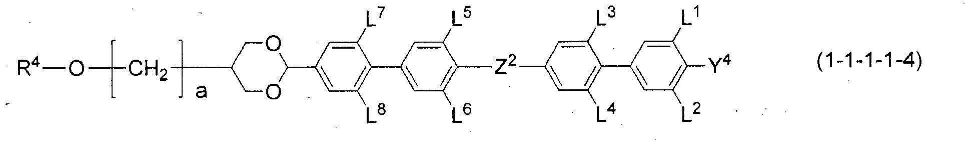 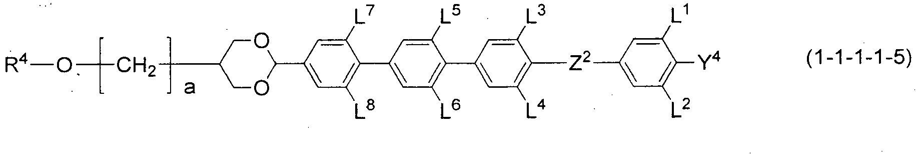 wherein, in formulas (1-1-1-1-1) to (1-1-1-1-5),
R⁴ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁴ is hydrogen, fluorine, chlorine, -C≡N, -CF₃, -OCF₃, -OCH=CF₂ or -OCH=CHCF₃ ;
Y^{4A} is hydrogen, fluorine, chlorine, -CF₃, -OCF₃, -OCH=CF₂ or -OCH=CHCF₃ ;
Z² is -CF₂O- or -COO-;
L¹, L², L³, L⁴, L⁵, L⁶, L⁷ and L⁸ are independently hydrogen or fluorine; and
a is 0, 1, 2 or 3.

21. The liquid crystal composition according to any one of claims 14 to 19, containing at least one compound represented by formula (1-1-1-1-11) or (1-1-1-1-12): 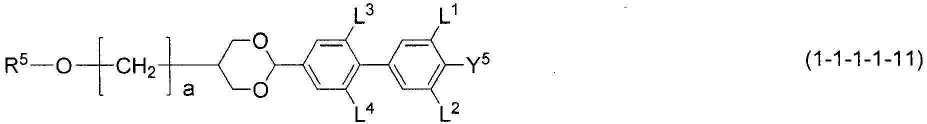 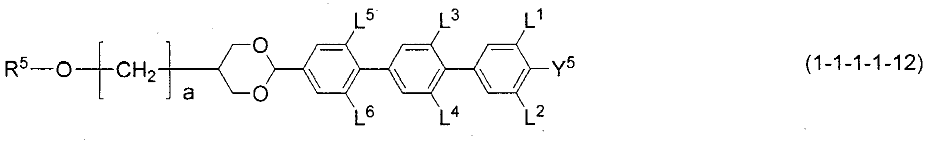 wherein, in formulas (1-1-1-1-11) and (1-1-1-1-12),
R⁵ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂₋ may be replaced by -CH=CH-;
Y⁵ is hydrogen, fluorine, chlorine, -C≡N, -CF₃, -OCF₃, -OCH=CF₂ or -OCH=CHCF₃;
L¹, L², L³, L⁴, L⁵ and L⁶ are independently hydrogen or fluorine; and
a is 0, 1, 2 or 3.

22. The liquid crystal composition according to any one of claims 14 to 21, further containing at least one compound selected from the group of compounds represented by formulas (4A) to (4D): 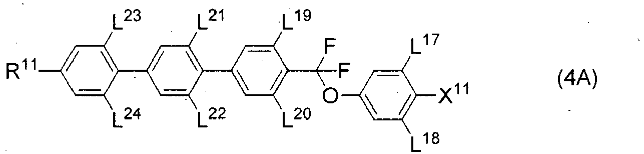 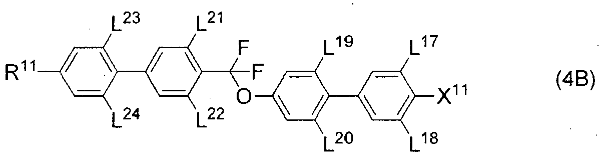 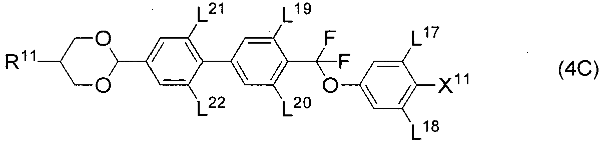 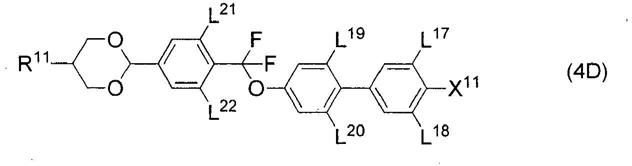 wherein, in formulas (4A) to (4D),
R¹¹ is alkyl having 1 to 10 carbons or alkenyl having 2 to 10 carbons, and in the alkyl and the alkenyl, at least one piece of hydrogen may be replaced by fluorine,
L¹⁷, L¹⁸, L¹⁹, L²⁰, L²¹, L²², L²³ and L²⁴ are independently hydrogen, fluorine or chlorine; and
X¹¹ is hydrogen, fluorine, chlorine, -OCF₃, -OCHF₂, -CF₃, -CHF₂, -CH₂F, -OCF₂CHF₂ or -OCF₂CHFCF₃.

23. The liquid crystal composition according to any one of claims 14 to 22, containing at least one compound selected from the group of compounds represented by formulas (K21) to (K27) as a chiral agent (optically active compound): 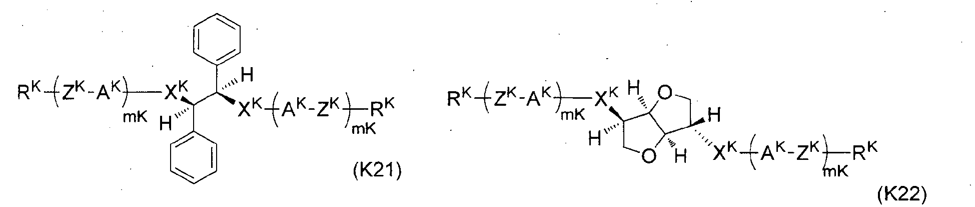 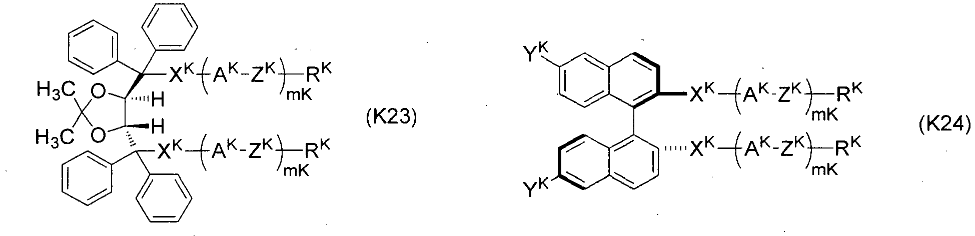 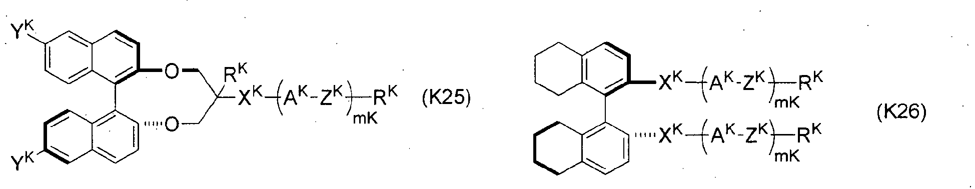 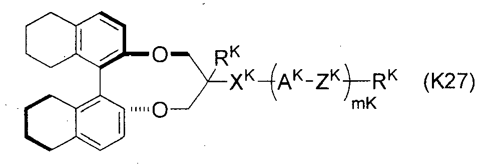 wherein, in formulas (K21) to (K27),
R^{K} is each independently hydrogen, halogen, -C=N, -N=C=O, -N=C=S or alkyl having 1 to 12 carbons, at least one piece of -CH₂- in R^{K} is may be replaced by -O-, -S-, -COO- or -OCO-, at least one piece of -CH₂-CH₂- in R^{K} may be replaced by -CH=CH-, -CF=CF- or -C≡C-, and at least one piece of hydrogen in R^{K} may be replaced by fluorine or chlorine;
A^{K} is each independently an aromatic 6-membered ring to an aromatic 8-membered ring, a non-aromatic 3-membered ring to a non-aromatic 8-membered ring, or a condensed ring having 9 or more carbons, at least one piece of hydrogen in the rings may be replaced by halogen, alkyl having 1 to 3 carbons or haloalkyl, -CH₂- in the ring may be replaced by -O-, -S- or -NH-, and -CH= may be replaced by -N=;
Y^{K} is each independently hydrogen, halogen, alkyl having 1 to 3 carbons, haloalkyl having 1 to 3 carbons, an aromatic 6-membered ring to an aromatic 8-membered ring, a non-aromatic 3-membered ring to a non-aromatic 8-membered ring, or a condensed ring having 9 or more carbons, at least one piece of hydrogen in the rings may be replaced by halogen, alkyl having 1 to 3 carbons or haloalkyl, -CH₂- in the alkyl may be replaced by -O-, -S- or -NH-, and -CH= may be replaced by -N=;
Z^{K} is each independently a single bond and alkylene having 1 to 8 carbons, at least one piece of -CH₂- in Z^{K} may be replaced by -O-, -S-, -COO-, -OCO-, -CSO-, -OCS-, -N=N-, -CH=N- or -N=CH-, at least one piece of -CH₂-CH₂- in Z^{K} may be replaced by -CH=CH-, -CF=CF- or -C≡C-, and at least one piece of hydrogen in Z^{K} may be replaced by halogen;
X^{K} is each independently a single bond, -COO-, -OCO-, -CH₂O-, -OCH₂-, -CF₂0-, -OCF₂- or -CH₂CH₂-; and
mK is each independently an integer from 1 to 3).

24. The liquid crystal composition according to any one of claims 14 to 23, further containing at least one polymerizable compound selected from the group of compounds represented by formulas (M2-15), (M4-5) and (M21): 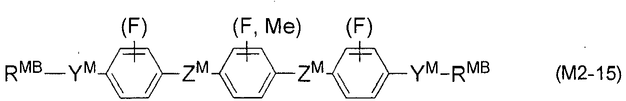 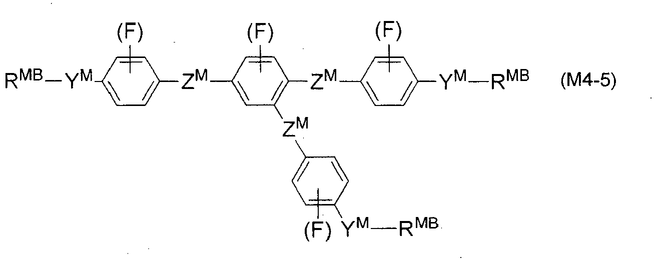
R^{MB}-R^{MC} (M21)
wherein, in formulas (M2-15), (M4-5) and (M21),
R^{MB} is each independently a polymerizable group in formulas (M3-1) to (M3-7), and R^{d} in formulas (M3-1) to (M3-7) is each independently hydrogen, halogen or alkyl having 1 to 5 carbons, and in the alkyl, at least one piece of hydrogen may be replaced by halogen; 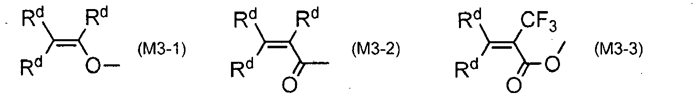 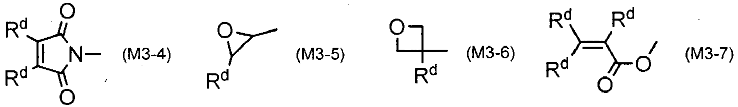
R^{MC} is each independently alkyl having 1 to 20 carbons or alkenyl having 2 to 20 carbons, and in the alkyl and the alkenyl, at least one piece of -CH₂- may be replaced by -O-, and at least one piece of hydrogen may be replaced by fluorine;
Y^{M} is each independently a single bond or alkylene having 1 to 20 carbons, and in the alkylene, at least one piece of -CH₂- may be replaced by -O- or -S-, and at least one piece of -CH₂-CH₂- in the alkyl may be replaced by -CH=CH-, -C≡C-, -COO- or -OCO-; and
Z^{M} is each independently a single bond, -(CH₂)ₘ₃-, -O(CH₂)ₘ₃-, -(CH₂)ₘ₃O-, -O(CH₂)ₘ₃O-, -CH=CH-, -C≡C-, -COO-, -OCO-, -(CF₂)₂-, -(CH₂)₂-COO-, -OCO-(CH₂)₂-, -CH=CH-COO-, -OCO-CH=CH-, -≡C-COO-, -OCO-C≡C-, -CH=CH-(CH₂)₂-, -(CH₂)₂-CH=CH-, -CF=CF-, -C≡C-CH=CH-, -CH=CH-C≡C-, -OCF₂- (CH₂)₂-, - (CH₂)₂-CF₂O-, -OCF₂- or -CF₂O- (in the formulas described above, m3 is an integer from 1 to 20); and
in partial structure of a ring, partial structure (a1) represents 1, 4-phenylene in which at least one of hydrogen is replaced by fluorine, partial structure (a2) represents 1,4-phenylene in which at least one piece of hydrogen may be replaced by fluorine, partial structure (a3) represents 1,4-phenylene in which at least one piece of hydrogen may be replaced by any one of fluorine and methyl, and partial structure (a4) represents fluorene in which hydrogen in a 9 position may be replaced by methyl.

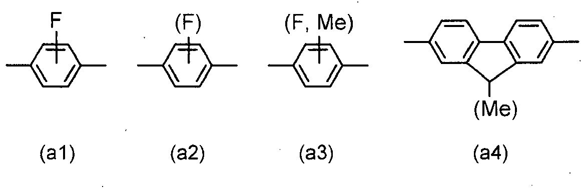

25. The liquid crystal composition according to any one of claims 14 to 24, having a chiral nematic phase in a temperature of any of -20°C to 70°C, wherein a helical pitch is 700 nanometers or less in at least part of the range of the temperature.

26. The liquid crystal composition according to any one of claims 14 to 24, used for a device driven in an optically isotropic liquid crystal phase.

27. A polymer-liquid crystal composite material, obtained by polymerizing the liquid crystal composition according to claim 24 and used for a device driven in an optically isotropic liquid crystal phase.

28. An optical device, having an electrode arranged on one or both substrates, a liquid crystal medium arranged between the substrates, and an electric field applying means for applying an electric field to the liquid crystal medium through the electrode, wherein the optical device is prepared by using the liquid crystal composition according to any one of claims 14 to 24 as the liquid crystal medium, or comprises the polymer-liquid crystal composite material according to claim 27.

29. Use of the liquid crystal composition according to any one of claims 14 to 24 in an optical device, or use of the polymer-liquid crystal composite material according to claim 27 in the optical device.

30. A compound, represented by formula (1): 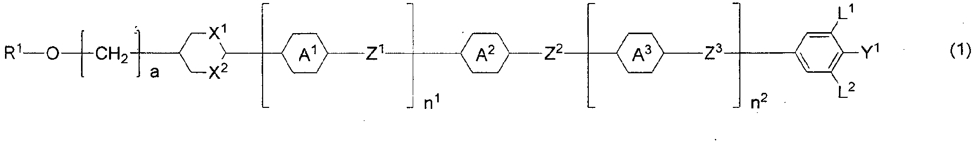 wherein, in formula (1),
R¹ is alkyl having 1 to 12 carbons or alkenyl having 2 to 12 carbons, and in the alkyl and the alkenyl, at least one piece of -CH₂- may be replaced by -O-, however, a case where two pieces of -O- are adjacent to each other is excluded, and at least one piece of hydrogen may be replaced by fluorine;
ring A¹, ring A² and ring A³ are independently represented by a formula described below; 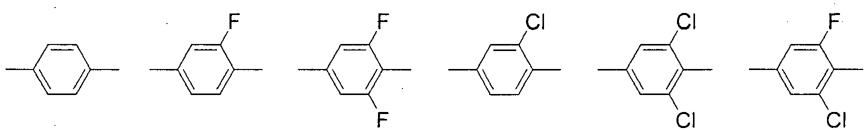 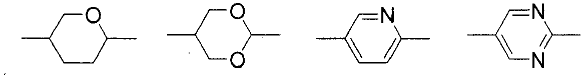 wherein, X¹ and X² are independently -O-, -S- or -CH₂-, and a case where both X¹ and X² are -CH₂- is excluded;
Y¹ is hydrogen, fluorine, chlorine, -C≡N, -N=C=S, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCH=CF₂, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-;
Z¹ and Z³ are independently a single bond, -CH₂CH₂-, -CH₂O-, -CF₂0-, -OCF₂-, -COO-, -OCO-, -CH=CH- or -C≡C-;
Z² is a single bond, -CF₂O- or -COO-;
L¹ and L² are independently hydrogen or halogen;
a is 0, 1, 2 or 3; and
n¹ and n² are independently 0, 1 or 2, an expression: n¹ + n² ≤ 2 holds; and
when n¹ + n² = 0 and Z² is -CF₂O, Y¹ is hydrogen, fluorine, chlorine, -CH₂F, -CHF₂, -CF₃, -OCH₂F, -OCHF₂, -OCF₃, -OCF₂CHF₂, -OCF₂CHFCF₃, -CH=CHF, -CH=CF₂, -CF=CHF, -CH=CHCF₃, -OCF=CF₂, -OCH=CHCF₃, alkyl having 1 to 7 carbons or alkenyl having 2 to 7 carbons, and in the alkyl and the alkenyl, at least one piece of -CH₂- may be replaced by -O-.

31. The compound according to claim 30, represented by formula (1-1) : 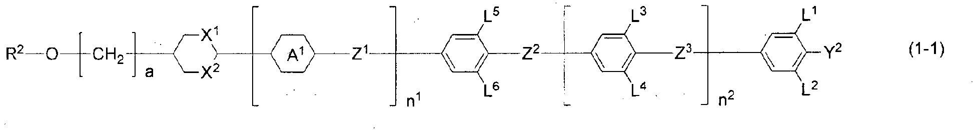 wherein, in formula (1-1),
R² is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
ring A¹ is represented by a formula described below; 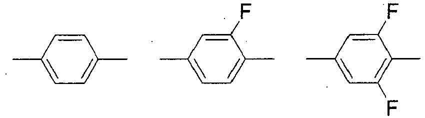 wherein, X¹ and X² are independently -O- or -CH₂-, and a case where both X¹ and X² are -CH₂- is excluded;
Y² is hydrogen, fluorine, chlorine, -C≡N, -CH₂F, -CHF₂, -CF₃, -OCH₂F, -OCHF₂, -OCF₃, -CH=CHF, -CH=CF₂, -CF=CHF, -CH=CHCF₃, -OCH=CF₂, -OCF=CF₂ or -OCH=CHCF₃;
Z¹ and Z³ are independently a single bond, -CH₂CH₂-, -CH₂O-, -CF₂0-, -OCF₂-, -COO-, -OCO-, -CH=CH- or -C≡C-;
Z² is a single bond, -CF₂O- or -COO-;
L¹, L², L³, L⁴, L⁵ and L⁶ are independently hydrogen, fluorine or chlorine;
a is 0, 1, 2 or 3; and
n¹ and n² are independently 0, 1 or 2, and an expression: n¹ + n² ≤ 2 holds.

32. The compound according to claim 30 or 31, represented by formula (1-1-1): 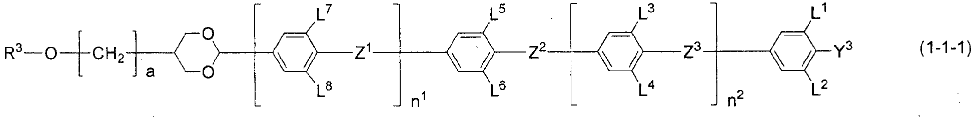 wherein, in formula (1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C≡N, -CF₃, -OCF₃, -OCH=CF₂ or -OCH=CHCF₃ ;
Z¹ and Z³ are independently a single bond, -CH₂O-, -CF₂0-, -COO-, -CH=CH- or -C≡C-;
Z² is a single bond, -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

33. The compound according to any one of claims 30 to 32, wherein a is 1.

34. The compound according to any one of claims 30 to 32, wherein a is 2.

35. The compound according to any one of claims 30 to 34, represented by formula (1-1-1-1): 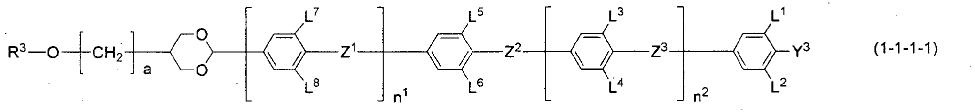 wherein, in formula (1-1-1-1),
R³ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y³ is hydrogen, fluorine, chlorine, -C≡N, -CF₃, -OCF₃, -OCH=CF₂ or -OCH=CHCF₃ ;
Z¹ and Z³ are independently a single bond, -CF₂0-, -COO- or -C=C-;
Z² is -CF₂O- or -COO-;
L¹, L², L³, L⁴, L⁵, L⁶, L⁷ and L⁸ are independently hydrogen or fluorine;
a is 0, 1, 2 or 3; and
n¹ and n² are independently 0, 1 or 2, and an expression: n¹ + n² ≤ 2 holds.

36. The compound according to any one of claims 30 to 35, represented by formulas (1-1-1-1-1) to (1-1-1-1-5): 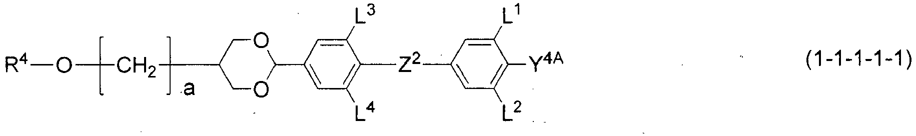 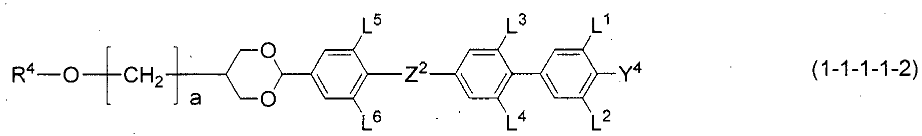 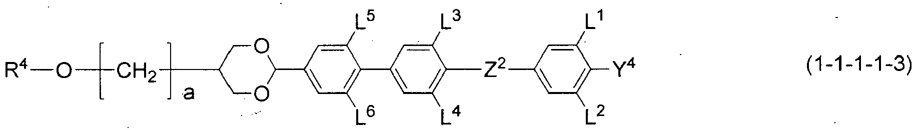 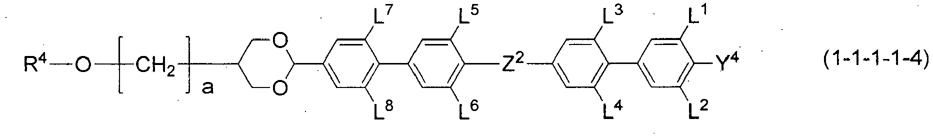 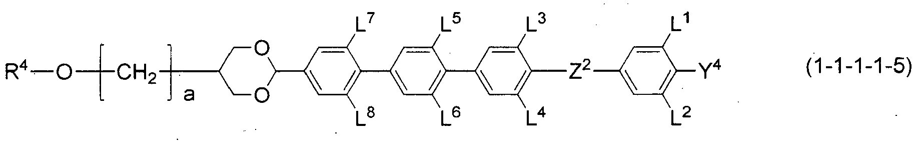 wherein, in formulas (1-1-1-1-1) to (1-1-1-1-5),
R⁴ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁴ is hydrogen, fluorine, chlorine, -C≡N, -CF₃, -OCF₃, -OCH=CF₂ or -OCH=CHCF₃ ;
Y^{4A} is hydrogen, fluorine, chlorine, -CF₃, -OCF₃, -OCH=CF₂ or -OCH=CHCF₃;
Z² is -CF₂O- or -COO-;
L¹, L², L³, L⁴, L⁵, L⁶, L⁷ and L⁸ are independently hydrogen or fluorine; and
a is 0, 1, 2 or 3.

37. The compound according to any one of claims 30 to 35, represented by formula (1-1-1-1-11) or (1-1-1-1-12): 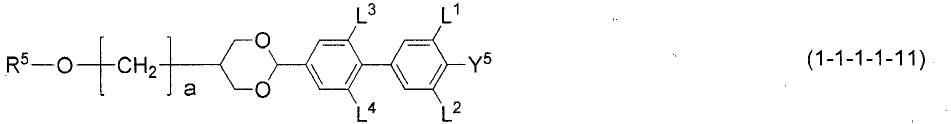 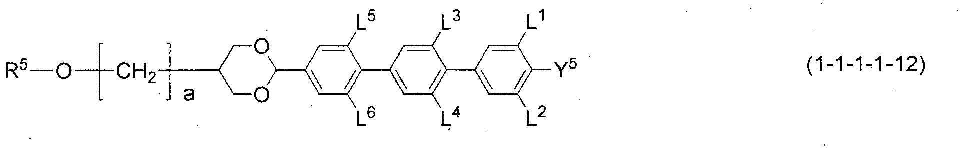 wherein, in formulas (1-1-1-1-11) and (1-1-1-1-12),
R⁵ is alkyl having 1 to 12 carbons, and in the alkyl, at least one piece of -CH₂- may be replaced by -O-, however, a case where two pieces of -O- are adjacent to each other is excluded, and at least one piece of -CH₂CH₂- may be replaced by -CH=CH-;
Y⁵ is hydrogen, fluorine, chlorine, -C≡N, -CF₃, -OCF₃, -OCH=CF₂ or -OCH=CHCF₃ ;
L¹, L², L³, L⁴, L⁵ and L⁶ are independently hydrogen or fluorine; and
a is 0, 1, 2 or 3.
